# EUROPEAN PATENT APPLICATION

(11) **EP 1 408 333 A2**
(43) Date of publication of application: **14.04.2004**
(21) Application number: 02256893.5
(22) Date of filing: 03.10.2002
(51) Int. Cl.: G01N 33/68, A61K 39/00, C07K 16/00

(54) **Diagnosis and treatment of Alzheimer's disease**

(30) Priority: 03.10.2002 US 326708 P
(71) Applicant: Pfizer Products Inc., Groton, Connecticut 06340-5196 (US); Oxford GlycoSciences (UK) Limited, Abingdon, Oxon OX14 4RY (GB)
(72) Inventor: Durham, L. Kathryn, New London, Connecticut 06320 (US); Friedman, David L., Madison, Connecticut 06433 (US); Herath, Herath Mudiyanselage Athula Chandrasiri, Abingdon OX14 4RY (GB); Kimmel, Lida H., Chester, Connecticut 06412 (US); Parekh, Rajesh Bhikhu, Abingdon OX14 4RY (GB); Potter, David M., Groton, Connecticut 06340 (US); Rohlff, Christian, Abingdon OX14 4RY (GB); Silber, B. Michael, Palo ALto California 94301 (US); Snyder, Peter Jeffrey, West Hartford, Connecticut 06107 (US); Soares, Holly Daria, Connecticut 06340 (US); Stiger, Thomas R., Pawcatuck, Connecticut 06379 (US); Sunderland, P. Trey, Chevy Chase, Maryland 20815 (US); Townsend, Robert Reid, Abingdon OX14 4RY (GB); White, W. Frost, Ledyard, Connecticut 06339 (US); Williams, Stephen A., Stonington, Connecticut 06378 (US)
(74) Representative: Lee, Nicholas John

(57) **Abstract**

The present invention provides methods and compositions for screening, diagnosis and prognosis of Alzheimer's disease, for monitoring the effectiveness of Alzheimer's disease treatment, and for drug development. Alzheimer's Disease-Associated Features (AFs), detectable by two-dimensional electrophoresis of cerebrospinal fluid, serum or plasma are described. The invention further provides Alzheimer's Disease-Associated Protein Isoforms (APIs) detectable in cerebrospinal fluid, serum or plasma, preparations comprising isolated APIs, antibodies immunospecific for APIs, pharmaceutical compositions, diagnostic and therapeutic methods, and kits comprising or based on the same.

## Description

### 1. INTRODUCTION

The present invention relates to the identification of proteins and protein isoforms that are associated with predisposition to Alzheimer's Disease and its onset and development, and of genes and nucleic acid molecules, encoding the same, and to their use for *e.g.*, clinical screening, diagnosis, treatment, as well as for drug screening and drug development.

### 2. BACKGROUND OF THE INVENTION

Alzheimer's Disease (AD) is an increasingly prevalent form of neurodegeneration that accounts for approximately 50 % - 60 % of the overall cases of dementia among people over 65 years of age. It currently affects an estimated 15 million people worldwide and owing to the relative increase of elderly people in the population its prevalence is likely to increase over the next 2 to 3 decades. AD is a progressive disorder with a mean duration of around 8.5 years between onset of clinical symptoms and death. Death of pyramidal neurons and loss of neuronal synapses in brains regions associated with higher mental functions results in the typical symptomology, characterized by gross and progressive impairment of cognitive function (Francis et al., 1999, J. Neurol. Neurosurg. Psychiatry 66:137-47). Currently, a diagnosis of AD requires a careful medical history and physical examination; a detailed neurological and psychiatric examination; laboratory blood studies to exclude underlying metabolic and medical illnesses that masquerade as AD; a mental status assessment and formal cognitive tests; and a computed tomographic scan or magnetic resonance image of the brain (Growdon, JH., 1995, Advances in the diagnosis of AD. In: Iqbal, K., Mortimer, JA., Winblad, B., Wisniewski, HM eds Research Advances in Alzheimer's Disease and Related Disorders. New York, NY: John Wiley & Sons Inc. 1995:139-153). Due to the time consuming nature of these tests, their expense, and their inconvenience to patients, it would be highly desirable to measure a substance or substances in body samples, such as samples of cerebrospinal fluid (CSF), blood or urine, that would lead to a positive diagnosis of AD or that would help to exclude AD from the differential diagnosis. Since the CSF bathes the brain, changes in its protein composition may most accurately reveal alterations in brain protein expression patterns that are causatively or diagnostically linked to the disease.

Current candidate biomarkers for AD include: (1) mutations in presenilin 1 (PS1), presenilin 2 (PS2) and amyloid precursor protein (APP) genes; (2) the detection of alleles of apolipoprotein E (ApoE); and (3) altered concentrations of amyloid β-peptides (Aβ), tau protein, and neuronal thread protein (NTP) in the CSF. See, *e.g.*, Neurobiology of Aging 19:109-116 (1998) for a review. Mutations in PS1, PS2 and APP genes are indicative of early-onset familial AD. However, early-onset familial AD is relatively rare; only 120 families worldwide are currently known to carry deterministic mutations (Neurobiology of Aging 19:109-116 (1998)). The detection of the ε4 allele of ApoE has been shown to correlate with late-onset and sporadic forms of AD. However, e4 alone cannot be used as a biomarker for AD since ε4 has been detected in many individuals not suffering from AD and the absence of ε4 does not exclude AD (Neurobiology of Aging 19:109-116 (1998)).

A decrease in the Aβ peptide Aβ42 and an increase in tau protein in the CSF of AD have been shown to correlate with the presence of AD (Neurobiology of Aging 19:109-116 (1998)). However, the specificity and sensitivity of Aβ42 and tau protein as biomarkers of AD are modest. For example, it has been difficult to determine a cut-off level of CSF tau protein that is diagnostically informative. Also, elevated levels of NTP in the CSF of postmortem subjects have been shown to correlate with the presence of AD (Neurobiology of Aging 19:109-116 (1998)). Therefore, a need exists to identify sensitive and specific biomarkers for the diagnosis of AD in living subjects.

### 3. SUMMARY OF THE INVENTION

The present invention provides methods and compositions for screening, diagnosis and treatment of AD and for screening and development of drugs for treatment of AD.

Therefore, one aspect of the invention provides methods for identification of AD that comprise analyzing a sample of CSF by two-dimensional electrophoresis to detect the presence or level of at least one Alzheimer's Disease-Associated Feature (AF), *e.g.*, one or more of the AFs disclosed herein, or any combination thereof. These methods are also suitable for clinical screening, prognosis, monitoring the results of therapy, for identifying patients most likely to respond to a particular therapeutic treatment, drug screening and development, and identification of new targets for drug treatment.

A further aspect of the invention provides methods for diagnosis of AD that comprise detecting in a sample of CSF the presence or level of at least one Alzheimer's Disease-Associated Protein Isoform (API), *e.g.*, one or more of the APIs disclosed herein or any combination thereof.

Another aspect of the invention provides antibodies, *e.g.*, monoclonal, polyclonal, humanised, chimeric or bispecific antibodies capable of immunospecific binding to an API, *e.g.*, an API disclosed herein.

A further aspect of the invention provides a preparation comprising an isolated API, *i.e.,* an API substantially free from proteins or protein isoforms having a significantly different isoelectric point or a significantly different apparent molecular weight from the API.

Another aspect of the invention provides kits that may be used in the above recited methods and that may comprise single or multiple preparations, or antibodies, together with other reagents, labels, substrates, if needed, and directions for use. The kits may be used for diagnosis of disease, or may be assays for the identification of new diagnostic and/or therapeutic agents.

An additional aspect of the invention provides methods of treating AD, comprising administering to a subject a therapeutically effective amount of an agent that modulates (*e.g.*, upregulates or downregulates) the expression or activity (*e.g*. enzymatic or binding activity), or both, of an API in subjects having AD.

A further aspect of the invention provides methods of screening for agents that modulate a characteristic of, *e.g.*, the expression or the enzymatic or binding activity, of an API, an API analog, or an API-related polypeptide.

Other objects and advantages will become apparent from a review of the ensuing detailed description taken in conjunction with the following illustrative drawings.

### 4. BRIEF DESCRIPTION OF THE FIGURE

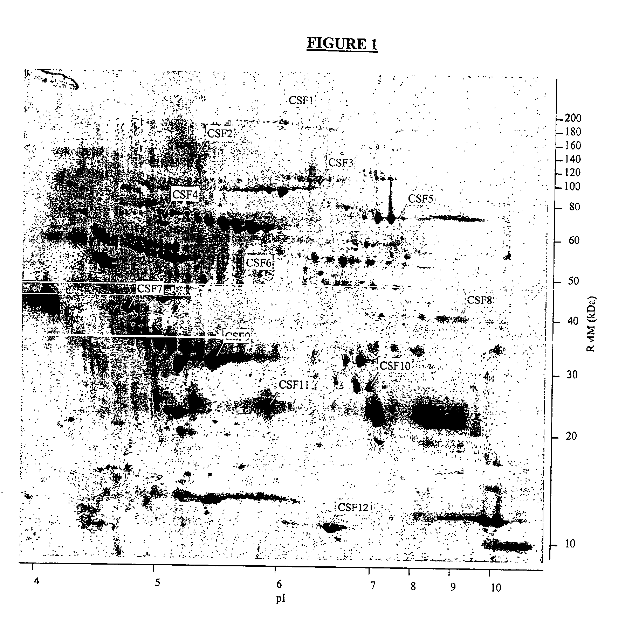

Figure 1 is an image obtained from 2-dimensional electrophoresis of normal CSF, which has been annotated to identify twelve landmark features, designated CSF1 to CSF12, and which are illustrative of an embodiment of an aspect of the present invention.

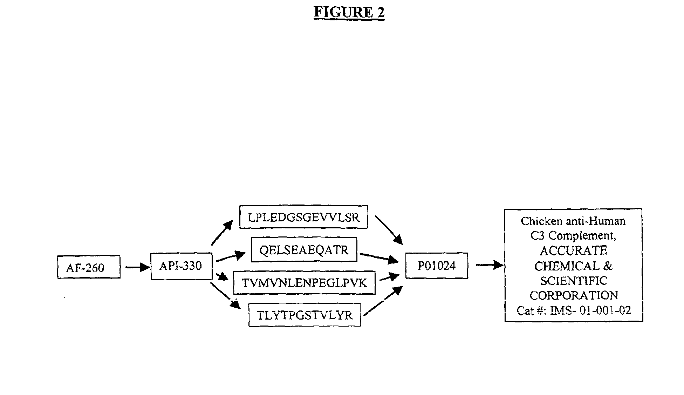

Figure 2 is a flow chart depicting the characterization of a Feature and relationship of a Feature and Protein Isoform(s). A feature may be further characterized as or by a Protein Isoform having a particular peptide sequence associated with its pI and MW. As depicted herein, a feature may comprise one or more protein isoform(s), which have indistinguishable pIs and MWs using the Preferred Technology, but which comprise distinct peptide sequences. The peptide sequence(s) of the protein isoform can be utilized to search database(s) for previously identified proteins comprising such peptide sequence(s), for which previously identified protein it can be ascertained whether a commercially available antibody exists, which may recognize the previously identified protein and/or a member of its protein family.

### 5. DETAILED DESCRIPTION OF THE INVENTION

The present invention described in detail below provides methods, compositions and kits useful, *e.g.*, for screening, diagnosis and treatment of Alzheimer's disease (AD) in a mammalian subject, and for drug screening and drug development. The invention also encompasses the administration of therapeutic compositions to a mammalian subject to treat or prevent AD a human adult, *i.e.* a human subject at least 21 (more particularly at least 35, at least 50, at least 60, at least 70, or at least 80) years old. For clarity of disclosure, and not by way of limitation, the invention will be described with respect to the analysis of CSF samples. However, as one skilled in the art will appreciate, based on the present description the assays and techniques described below can be applied to other types of samples, including a body fluid (*e.g.* blood, serum, plasma or saliva), a tissue sample from a subject at risk of having or developing AD (*e.g.* a biopsy such as a brain biopsy) or homogenate thereof. The methods and compositions of the present invention are useful, such as for example, screening, diagnosis and treatment of a living subject, but may also be used for post-mortem diagnosis of a subject, for example, to identify if family members of the subject may be at risk of developing the same disease.

The following definitions are provided to assist in the review of the instant disclosure.

### 5.1. DEFINITIONS

"Feature" refers to a spot identified in a 2D gel, and the term "Alzheimer's disease-Associated Feature" (AF) refers to a feature that is differentially present in a first sample or sample set from a subject having AD compared with a second sample or sample set from a subject free from AD. A feature or spot identified in a 2D gel is characterized by its isoelectric point (pI) and apparent molecular weight (MW) as determined by 2D gel electrophoresis, particularly utilizing the Preferred Technology described herein. As used herein, a feature is "differentially present" in a first sample or sample set with respect to a second sample or sample set when a method for detecting the said feature (*e.g.*, 2D electrophoresis) gives a different signal when applied to the first and second samples or sample sets. An AF, (or a Protein Isoform, i.e. API, as defined *infra*) is "increased" in the first sample or sample set with respect to the second sample or sample set if the method of detection indicates that the AF, or API is more abundant in the first sample or sample set than in the second sample or sample set, or if the AF, or API is detectable in the first sample or sample set and substantially undetectable in the second sample or sample set. Conversely, an AF, or API is "decreased" in the first sample or sample set with respect to the second sample or sample set if the method of detection indicates that the AF, or API is less abundant in the first sample or sample set than in the second sample or sample set or if the AF, or API is undetectable in the first sample or sample set and detectable in the second sample or sample set.

Particularly, the relative abundance of a feature in the two samples or sample sets is determined in reference to its normalized signal, in two steps. First, the signal obtained upon detecting the feature in a first sample or sample set is normalized by reference to a suitable background parameter, *e.g.*, (a) to the total protein in the sample being analyzed (*e.g.*, total protein loaded onto a gel); (b) to an Expression Reference Feature (ERF) i.e., a feature whose abundance is substantially invariant, within the limits of variability of the Preferred Technology, in the population of subjects being examined, *e.g.* the ERFs disclosed in Table III, or (c) more preferably to the total signal detected as the sum of each of all proteins in the sample.

Secondly, the normalized signal for the feature in the first sample or sample set is compared with the normalized signal for the same feature in the second sample or sample set in order to identify features that are "differentially present" in the first sample or sample set with respect to the second sample or sample set.

"Fold change" includes "fold increase" and "fold decrease" and refers to the relative increase or decrease in abundance of a MSF or the relative increase or decrease in expression or activity of a polypeptide (*e.g.* an API, as defined *infra.)* in a first sample or sample set compared to a second sample or sample set. An AF or polypeptide fold change may be measured by any technique known to those of skill in the art, albeit the observed increase or decrease will vary depending upon the technique used. Preferably, fold change is determined herein as described in the Examples *infra*.

"Alzheimer's Disease-Associated Protein Isoform" (API) refers to a polypeptide that is differentially present in a first sample or sample set from a subject having AD compared with a second sample or sample set from a subject free from AD. As used herein, an API is "differentially present" in a first sample or sample set with respect to a second sample or sample set when a method for detecting the said feature, (*e.g.*, 2D electrophoresis or immunoassay) gives a different signal when applied to the first and second samples or sample sets (as described above in relation to AFs). An API is characterised by one or more peptide sequences of which it is comprised, and further by a pI and MW, preferably determined by 2D electrophoresis, particularly utilising the Preferred Technology as described herein. Typically, APIs are identified or characterised by the amino acid sequencing of AFs (Figure 2).

An API is characterized as, or by, a particular peptide sequence associated with its pI and MW. As depicted herein, an AF may comprise one or more API(s), which have indistinguishable pIs and MWs using the Preferred Technology, but which have distinct peptide sequences. The peptide sequence(s) of the API can be utilized to search database(s) for previously identified proteins comprising such peptide sequence(s). In some instances, it can be ascertained whether a commercially available antibody exists which may recognize the previously identified protein and/or a variant thereof. Prefereably the API corresponds to the previously identified protein, or be a variant of the previously identified protein.

"Variant" as used herein refers to a polypeptide which is a member of a family of polypeptides that are encoded by a single gene or from a gene sequence within a family of related genes and which differ in their pI or MW, or both. Such variants can differ in their amino acid composition (*e.g.* as a result of alternative mRNA or premRNA processing, *e.g.* alternative splicing or limited proteolysis) and in addition, or in the alternative, may arise from differential post-translational modification (*e.g.*, glycosylation, acylation, phosphorylation).

"Modulate" in reference to expression or activity of an AF, API or API-related polypeptide refers to any change, *e.g.*, upregulation or downregulation, increase or decrease, of the expression or activity of the AF, API or API-related polypeptide. Those skilled in the art, based on the present disclosure, will understand that such modulation can be determined by assays known to those of skill in the art.

"API analog" refers to a polypeptide that possesses similar or identical function(s) as as API but need not necessarily comprise an amino acid sequence that is similar or identical to the amino acid sequence of the API, or possess a structure that is similar or identical to that of the API. As used herein, an amino acid sequence of a polypeptide is "similar" to that of an API if it satisfies at least one of the following criteria: (a) the polypeptide has an amino acid sequence that is at least 30% (more preferably, at least 35%, at least 40%, at least 45%, at least 50%, at least 55%, at least 60%, at least 65%, at least 70%, at least 75%, at least 80%, at least 85%, at least 90%, at least 95% or at least 99%) identical to the amino acid sequence of the API; (b) the polypeptide is encoded by a nucleotide sequence that hybridizes under stringent conditions to a nucleotide sequence encoding at least 5 amino acid residues (more preferably, at least 10 amino acid residues, at least 15 amino acid residues, at least 20 amino acid residues, at least 25 amino acid residues, at least 40 amino acid residues, at least 50 amino acid residues, at least 60 amino residues, at least 70 amino acid residues, at least 80 amino acid residues, at least 90 amino acid residues, at least 100 amino acid residues, at least 125 amino acid residues, or at least 150 amino acid residues) of the API; or (c) the polypeptide is encoded by a nucleotide sequence that is at least 30% (more preferably, at least 35%, at least 40%, at least 45%, at least 50%, at least 55%, at least 60%, at least 65%, at least 70%, at least 75%, at least 80%, at least 85%, at least 90%, at least 95% or at least 99%) identical to the nucleotide sequence encoding the MSPI. As used herein, a polypeptide with "similar structure" to that of a API refers to a polypeptide that has a similar secondary, tertiary or quartemary structure as that of the API. The structure of a polypeptide can determined by methods known to those skilled in the art, including but not limited to, X-ray crystallography, nuclear magnetic resonance, and crystallographic electron microscopy.

"API fusion protein" refers to a polypeptide that comprises (i) an amino acid sequence of an API, API fragment, API-related polypeptide or a fragment of an API-related polypeptide and (ii) an amino acid sequence of a heterologous polypeptide (i.e., a non-API, non-API fragment or non-API-related polypeptide).

"API homolog" refers to a polypeptide that comprises an amino acid sequence similar to that of a API but does not necessarily possess a similar or identical function as the API.

"API ortholog" refers to a non-human polypeptide that (i) comprises an amino acid sequence similar to that of an API and (ii) possesses a similar or identical function to that of the API.

"API-related polypeptide" refers to an API homolog, an API analog, a variant of an API, an API ortholog, or any combination thereof.

"Chimeric Antibody" refers to a molecule in which different portions are derived from different animal species, such as those having a human immunoglobulin constant region and a variable region derived from a murine mAb. (See, *e.g.*, Cabilly et al., U.S. Patent No. 4,816,567; and Boss et al., U.S. Patent No. 4,816397). For example, a portion of the antibody may be fused with the constant domain of immunoglobulins (IgA, IgE, IgG, IgM), or portions thereof (CH1, CH2, CH3, or any combination thereof and portions thereof) resulting in chimeric antibodies.

"Humanised Antibody" refers to a molecule from non-human species having one or more complementarily determining regions (CDRs) from the non-human species and a framework region from a human immunoglobulin molecule.

"Derivative" refers to a polypeptide that comprises an amino acid sequence of a second polypeptide that has been altered by the introduction of at least one amino acid residue substitution, deletion or addition. The derivative polypeptide possesses a similar or identical function as the second polypeptide.

"Fragment" refers to a peptide or polypeptide comprising an amino acid sequence of at least 5 amino acid residues (preferably, at least 10 amino acid residues, at least 15 amino acid residues, at least 20 amino acid residues, at least 25 amino acid residues, at least 40 amino acid residues, at least 50 amino acid residues, at least 60 amino residues, at least 70 amino acid residues, at least 80 amino acid residues, at least 90 amino acid residues, at least 100 amino acid residues, at least 125 amino acid residues, at least 150 amino acid residues, at least 175 amino acid residues, at least 200 amino acid residues, or at least 250 amino acid residues) of the amino acid sequence of a second polypeptide. Prefereably the fragment of a MSPI possesses the functional activity of the MSPI.

The "percent identity" of two amino acid sequences or of two nucleic acid sequences can be or is generally determined by aligning the sequences for optimal comparison purposes (*e.g.*, gaps can be introduced in either sequences for best alignment with the other sequence) and comparing the amino acid residues or nucleotides at corresponding positions. The "best alignment" is an alignment of two sequences that results in the highest percent identity. The percent identity is determined by the number of identical amino acid residues or nucleotides in the sequences being compared (i.e., % identity = # of identical positions/total # of positions x 100).

The determination of percent identity between two sequences can be accomplished using a mathematical algorithm known to those of skill in the art. An example of a mathematical algorithm for comparing two sequences is the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. The NBLAST and XBLAST programs of Altschul, et al. (1990) J. Mol. Biol.
215:403-410 have incorporated such an algorithm. BLAST nucleotide searches can be performed with the NBLAST program, score = 100, wordlength = 12 to obtain nucleotide sequences homologous to a nucleic acid molecule of the invention. BLAST protein searches can be performed with the XBLAST program, score = 50, wordlength = 3 to obtain amino acid sequences homologous to a protein molecule of the invention. To obtain gapped alignments for comparison purposes, Gapped BLAST can be utilized as described in Altschul et al. (1997) Nucleic Acids Res. 25:3389-3402. Alternatively, PSI-Blast can be used to perform an iterated search which detects distant relationships between molecules *(Id.).* When utilizing BLAST, Gapped BLAST, and PSI-BLAST programs, the default parameters of the respective programs (*e.g.*, XBLAST and NBLAST) can be used.

Another example of a mathematical algorithm utilized for the comparison of sequences is the algorithm of Myers and Miller, CABIOS (1989). The ALIGN program (version 2.0) which is part of the GCG sequence alignment software package has incorporated such an algorithm. Other algorithms for sequence analysis known in the art include ADVANCE and ADAM as described in Torellis and Robotti (1994) Comput. Appl. Biosci., 10 :3-5; and FASTA described in Pearson and Lipman (1988) Proc. Natl. Acad. Sci. 85:2444-8. Within FASTA, ktup is a control option that sets the sensitivity and speed of the search.

"Diagnosis" refers to diagnosis, prognosis, monitoring, characterizing, selecting patients, including participants in clinical trials, and identifying patients at risk for or having a particular disorder or those most likely to respond to a particular therapeutic treatment, or for assessing or monitoring a patient's response to a particular therapeutic treatment.

"Treatment" refers to therapy, prevention and prophylaxis and particularly refers to the administration of medicine or the performance of medical procedures with respect to a patient, for either prophylaxis (prevention) or to cure the infirmity or malady in the instance where the patient is afflicted.

"Agent" refers to all materials that may be used to prepare pharmaceutical and diagnostic compositions, or that may be compounds, agonists, antagonists, nucleic acids, polypeptides, fragments, isoforms, variants, or other materials that may be used independently for such purposes, all in accordance with the present invention.

"Highly stringent conditions" refers to hybridization to filter-bound DNA in 0.5 M NaHPO4, 7% sodium dodecyl sulfate (SDS), 1 mM EDTA at 65□C, and washing in 0.1xSSC/0.1% SDS at 68□C (Ausubel F.M. et al., eds., 1989, Current Protocols in Molecular Biology, Vol. I, Green Publishing Associates, Inc., and John Wiley & Sons, Inc., New York, at p. 2.10.3)

For some applications, less stringent conditions for duplex formation are required. As used herein "moderately stringent conditions" refers to washing in 0.2xSSC/0.1% SDS at 42□C (Ausubel et al., 1989, supra).

In the context of the present invention, the term "sample" can refer to a sample obtained from any source, such as a serum sample, a CSF sample or a tissue sample, e.g. brain tissue. In addition, as used in this specification and the appended claims, the singular forms "a", "an", and "the" include plural references unless the context clearly dictates otherwise. Thus for example, references to "Alzheimer disease-associated protein" includes one or more of such proteins, reference to "the method" includes one or more methods, and/or steps of the type described herein and/or which will become apparent to those persons skilled in the art upon reading this disclosure, references to a "sample" includes sets of more than one sample and so forth.

"Cerebrospinal fluid", (CSF), refers to the fluid that surrounds the bulk of the central nervous system, as described in Physiological Basis of Medical Practice (J.B. West, ed., Williams and Wilkins, Baltimore, MD 1985). CSF includes ventricular CSF and lumbar CSF.

"Two-dimensional electrophoresis" (2D-electrophoresis) means a technique comprising isoelectric focusing, followed by denaturing electrophoresis; this generates a two-dimensional gel (2D-gel) containing a plurality of separated proteins.

### 5.2 The "Preferred Technology"

Preferably, the step of denaturing electrophoresis uses polyacrylamide electrophoresis in the presence of sodium dodecyl sulfate (SDS-PAGE). Especially preferred are the highly accurate and automatable methods and apparatus ("the Preferred Technology") described in WO 98/23950 and in U.S. Patent Nos 6,064,654, and 6,278.794, each of which is incorporated herein by reference in its entirety with particular reference to the protocol at pages 23-35 of WO 98/23950. Briefly, the Preferred Technology provides efficient, computer-assisted methods and apparatus for identifying, selecting and characterising biomolecules (e.g. proteins, including glycoproteins) in a biological sample. A two-dimensional array is generated by separating biomolecules on a two-dimensional gel according to their electrophoretic mobility and isoelectric point. A computer-generated digital profile of the array is generated, representing the identity, apparent molecular weight, isoelectric point, and relative abundance of a plurality of biomolecules detected in the two-dimensional array, thereby permitting computer-mediated comparison of profiles from multiple biological samples, as well as computer aided excision of separated proteins of interest.

A preferred scanner for detecting fluorescently labeled proteins is described in WO 96/36882 and in the Ph.D. thesis of David A. Basiji, entitled "Development of a High-throughput Fluorescence Scanner Employing Internal Reflection Optics and Phase-sensitive Detection (Total Internal Reflection, Electrophoresis)", University of Washington (1997), Volume 58/12-B of Dissertation Abstracts International, page 6686, the contents of each of which are incorporated herein by reference. These documents describe an image scanner designed specifically for automated, integrated operation at high speeds. The scanner can image gels that have been stained with fluorescent dyes or silver stains, as well as storage phosphor screens. The Basiji thesis provides a phase-sensitive detection system for discriminating modulated fluorescence from baseline noise due to laser scatter or homogeneous fluorescence, but the scanner can also be operated in a non-phase-sensitive mode. This phase-sensitive detection capability would increase the sensitivity of the instrument by an order of magnitude or more compared to conventional fluorescence imaging systems. The increased sensitivity would reduce the sample-preparation load on the upstream instruments while the enhanced image quality simplifies image analysis downstream in the process.

A more highly preferred scanner is a modified version of the above described scanner. In the preferred scanner, the gel is transported through the scanner on a precision lead-screw drive system. This is preferable to laying the glass plate on the belt-driven system that is described in the Basiji thesis, as it provides a reproducible means of accurately transporting the gel past the imaging optics.

In the preferred scanner, the gel is secured against three alignment stops that rigidly hold the glass plate in a known position. By doing this in conjunction with the above precision transport system, the absolute position of the gel can be predicted and recorded. This ensures that co-ordinates of each feature on the gel can be determined more accurately and communicated, if desired, to a cutting robot for excision of the feature. In the preferred scanner, the carrier that holds the gel has four integral fluorescent markers for use to correct the image geometry. These markers are a quality control feature that confirms that the scanning has been performed correctly.

In comparison to the scanner described in the Basiji thesis, the optical components of the preferred scanner have been inverted. In the preferred scanner, the laser, mirror, waveguide and other optical components are above the glass plate being scanned. The scanner described in the Basiji thesis has these components underneath. In the preferred scanner, the glass plate is mounted onto the scanner gel side down, so that the optical path remains through the glass plate. By doing this, any particles of gel that may break away from the glass plate will fall onto the base of the instrument rather than into the optics. This does not affect the functionality of the system, but increases its reliability.

Still more preferred is a modified version of the preferred scanner, in which the signal output is digitised to the full 16-bit data without any peak saturation or without square root encoding of the signal. A compensation algorithm has also been applied to correct for any variation in detection sensitivity along the path of the scanning beam. This variation is due to anomalies in the optics and differences in collection efficiency across the waveguide. A calibration is performed using a perspex plate with an even fluorescence throughout. The data received from a scan of this plate are used to determine the multiplication factors needed to increase the signal from each pixel level to a target level. These factors are then used in subsequent scans of gels to remove any internal optical variations.

### 5.3 Alzheimer's Disease-Associated Features (AFs)

In one aspect of the invention, two-dimensional electrophoresis is used to analyze CSF from a subject, preferably a living subject, in order to detect or quantify the expression of one or more Alzheimer's Disease-Associated Features (AFs) for screening, treatment or diagnosis of AD.

By way of example and not of limitation, using the Preferred Technology, a number of samples from subjects having AD and samples from subjects free from AD are separated by two-dimensional electrophoresis, and the fluorescent digital images of the resulting gels are matched to a chosen representative primary master gel image. This process allows any gel feature, characterized by its pI and MW, to be identified and examined on any gel of the study. In particular, the amount of protein present in a given feature can be measured in each gel; this feature abundance can be averaged amongst gels from similar samples (e.g. gels from samples from subjects having AD). Finally, statistical analyses can be conducted on the thus created sample sets, in order to compare 2 or more sample sets to each other.

In accordance with an aspect of the present invention, the AFs disclosed herein have been identified by comparing CSF samples from subjects having AD against CSF samples from subjects free from AD. Subjects free from AD include subjects with no known disease or condition (normal subjects) and subjects with diseases (including neurological and neurodegenerative diseases) other than AD.

Two groups of AFs have been identified through the methods and apparatus of the Preferred Technology. The first group consists of AFs that are decreased in the CSF of subjects having AD as compared with the CSF of subjects free from AD. These AFs can be described by apparent molecular weight (MW) and isoelectric point (pI) as provided in Table I.

### Table I. AFs Decreased in CSF of Subiects Having AD

### (a) AFs Identified From Mastergroup Analysis

**Table I (a)**

| **AF#** | **Fold Decrease**^{**#**} | **pI** | **MW (Da)** | **p value*** |
|---|---|---|---|---|
| AF-200 | 2.217 | 6.32 | 18300 | 0.000⁽¹⁾ |
| AF-201 | 2.132 | 8.32 | 63264 | 0.000⁽¹⁾ |
| AF-202 | 2.132 | 8.32 | 63264 | 0.000⁽¹⁾ |
| AF-203 | 1.991 | 5.53 | 74605 | 0.000⁽¹⁾ |
| AF-204 | 1.955 | 7.97 | 60878 | 0.000⁽¹⁾ |
| AF-205 | 1.876 | 6.82 | 61514 | 0.000⁽¹⁾ |
| AF-206 | 1.807 | 5.55 | 28801 | 0.000⁽¹⁾ |
| AF-207 | 1.790 | 6.65 | 33533 | 0.000⁽¹⁾ |
| AF-208 | 1.747 | 5.75 | 29376 | 0.000⁽¹⁾ |
| AF-209 | 1.741 | 8.4 | 59188 | 0.000⁽¹⁾ |
| AF-210 | 1.674 | 5.96 | 29414 | 0.000⁽¹⁾ |
| AF-211 | 1.673 | 8.07 | 42384 | 0.000⁽¹⁾ |
| AF-212 | 1.670 | 6.37 | 62904 | 0.000⁽¹⁾ |
| AF-213 | 1.669 | 6.53 | 62318 | 0.000⁽¹⁾ |
| AF-214 | 1.658 | 4.28 | 18482 | 0.000⁽¹⁾ |
| AF-215 | 1.617 | 8.01 | 44616 | 0.004⁽¹⁾ |
| AF-216 | 1.603 | 6.73 | 56999 | 0.000⁽¹⁾ |
| AF-217 | 1.572 | 6.4 | 26203 | 0.000⁽¹⁾ |
| AF-218 | 1.558 | 6.74 | 33401 | 0.000⁽¹⁾ |
| AF-219 | 1.555 | 7.93 | 63355 | 0.000⁽¹⁾ |
| AF-220 | 1.555 | 6.76 | 54345 | 0.000⁽¹⁾ |
| AF-221 | 1.542 | 5.51 | 139627 | 0.000⁽¹⁾ |
| AF-222 | 1.532 | 5.62 | 132758 | 0.000⁽¹⁾ |
| AF-223 | 1.513 | 5.67 | 131516 | 0.000⁽¹⁾ |
| AF-224 | 1.513 | 6.86 | 49289 | 0.000⁽¹⁾ |
| AF-225 | 1.495 | 10.85 | 58257 | 0.000⁽¹⁾ |
| AF-226 | 1.448 | 7.23 | 19847 | 0.000⁽¹⁾ |
| AF-227 | 1.444 | 4.38 | 21160 | 0.000⁽¹⁾ |
| AF-228 | 1.444 | 4.38 | 21160 | 0.000⁽¹⁾ |
| AF-229 | 1.431 | 5.29 | 29663 | 0.000^{(1,4)} |
| AF-230 | 1.392 | 5.55 | 135815 | 0.005^{(1,4)} |
| AF-231 | 1.349 | 7.72 | 175333 | 0.000⁽¹⁾ |
| AF-232 | 1.341 | 4.41 | 42104 | 0.000⁽¹⁾ |
| AF-233 | 1.326 | 5.19 | 46876 | 0.000⁽¹⁾ |
| AF-234 | 1.320 | 5.04 | 18662 | 0.000^{(1,4)} |
| AF-235 | 1.301 | 5.38 | 62756 | 0.000⁽¹⁾ |
| AF-236 | 1.285 | 7.35 | 64620 | 0.000⁽¹⁾ |
| AF-237 | 1.283 | 7.86 | 59059 | 0.000⁽¹⁾ |
| AF-238 | 1.276 | 8.1 | 34846 | 0.000^{(1,4)} |
| AF-239 | 1.269 | 6.05 | 184171 | 0.005⁽¹⁾ |
| AF-240 | 1.264 | 5.01 | 46760 | 0.000⁽¹⁾ |
| AF-241 | 1.262 | 5.46 | 16541 | 0.000⁽¹⁾ |
| AF-242 | 1.258 | 4.48 | 34420 | 0.000⁽¹⁾ |
| AF-243 | 1.256 | 5.25 | 119795 | 0.000⁽¹⁾ |
| AF-244 | 1.251 | 7.12 | 54519 | 0.006⁽¹⁾ |
| AF-245 | 1.244 | 4.87 | 49219 | 0.000^{(1,4)} |
| AF-246 | 1.233 | 4.24 | 73972 | 0.018⁽¹⁾ |
| AF-247 | 1.232 | 6.46 | 92692 | 0.017⁽¹⁾ |
| AF-248 | 1.222 | 5.52 | 39355 | 0.002⁽¹⁾ |
| AF-249 | 1.210 | 4.25 | 20787 | 0.008⁽¹⁾ |
| AF-250 | 1.210 | 7.88 | 14371 | 0.535⁽³⁾ |
| AF-251 | 1.206 | 4.95 | 54997 | 0.001⁽¹⁾ |
| AF-252 | 1.205 | 4.53 | 12315 | 0.004⁽¹⁾ |
| AF-253 | 1.200 | 5.36 | 22610 | 0.000⁽¹⁾ |
| AF-254 | 1.199 | 7.67 | 31952 | 0.045⁽¹⁾ |
| AF-255 | 1.196 | 5.92 | 185771 | 0.048⁽¹⁾ |
| AF-256 | 1.187 | 5.04 | 185128 | 0.012⁽¹⁾ |
| AF-257 | 1.179 | 8.19 | 16738 | 0.01⁽¹⁾ |
| AF-258 | 1.178 | 5.47 | 15411 | 0.0001^{(1,2)} |
| AF-259 | 1.167 | 4.81 | 30575 | 0.024⁽¹⁾ |
| AF-260 | 1.166 | 4.93 | 154156 | 0.069⁽¹⁾ |
| AF-261 | 1.152 | 6.71 | 16524 | 0.022⁽¹⁾ |
| AF-262 | 1.151 | 5.81 | 114225 | 0.000⁽¹⁾ |
| AF-263 | 1.150 | 4.43 | 16818 | 0.001⁽¹⁾ |
| AF-264 | 1.146 | 5.12 | 184512 | 0.045⁽¹⁾ |
| AF-265 | 1.137 | 6.13 | 87723 | 0.033⁽¹⁾ |
| AF-266 | 1.131 | 6.47 | 35322 | 0.018⁽¹⁾ |
| AF-267 | 1.130 | 5.25 | 179949 | 0.074^{(1,3)} |
| AF-268 | 1.130 | 9.25 | 17981 | 0.032⁽¹⁾ |
| AF-269 | 1.125 | 6.58 | 170682 | 0.011(1) |
| AF-270 | 1.119 | 5.76 | 45729 | 0.123⁽³⁾ |
| AF-271 | 1.113 | 4.99 | 63787 | 0.044⁽¹⁾ |
| AF-272 | 1.103 | 6.38 | 37769 | 0.085⁽¹⁾ |
| AF-273 | 1.100 | 4.62 | 82833 | 0.101⁽³⁾ |
| AF-274 | 1.100 | 9.38 | 35170 | 0.232⁽³⁾ |
| AF-275 | 1.099 | 5.76 | 189295 | 0.049⁽¹⁾ |
| AF-276 | 1.099 | 6.49 | 43668 | 0.132⁽³⁾ |
| AF-277 | 1.099 | 6.05 | 29055 | 0.256⁽³⁾ |
| AF-278 | 1.097 | 5.67 | 21864 | 0.003⁽¹⁾ |
| AF-279 | 1.092 | 5.39 | 95475 | 0.098⁽¹⁾ |
| AF-280 | 1.089 | 5.3 | 102319 | 0.142⁽³⁾ |
| AF-281 | 1.087 | 6.84 | 151156 | 0.026⁽¹⁾ |
| AF-282 | 1.080 | 6.03 | 40754 | 0.146⁽³⁾ |
| AF-283 | 1.077 | 6.22 | 70359 | 0.066⁽¹⁾ |
| AF-284 | 1.076 | 5.84 | 187223 | 0.121⁽³⁾ |
| AF-285 | 1.075 | 5.33 | 183136 | 0.255⁽³⁾ |
| AF-286 | 1.072 | 6.36 | 20758 | 0.148⁽³⁾ |
| AF-287 | 1.069 | 6.79 | 14728 | 0.0001^{(1,2)} |
| AF-288 | 1.067 | 5.22 | 37843 | 0.679⁽³⁾ |
| AF-289 | 1.065 | 6.18 | 66668 | 0.066⁽¹⁾ |
| AF-290 | 1.065 | 5.78 | 19705 | 0.150⁽³⁾ |
| AF-291 | 1.063 | 4.88 | 135547 | 0.269⁽³⁾ |
| AF-292 | 1.062 | 5.18 | 184053 | 0.338⁽³⁾ |
| AF-293 | 1.061 | 5.79 | 21435 | 0.658⁽³⁾ |
| AF-294 | 1.059 | 6.38 | 21221 | 0.343⁽³⁾ |
| AF-295 | 1.052 | 6.57 | 114704 | 0.389⁽³⁾ |
| AF-296 | 1.050 | 6.2 | 35622 | 0.119⁽³⁾ |
| AF-297 | 1.046 | 5.91 | 16055 | 0.349⁽³⁾ |
| AF-298 | 1.037 | 4.98 | 91522 | 0.445⁽³⁾ |
| AF-299 | 1.032 | 4.61 | 29548 | 0.464⁽³⁾ |
| AF-300 | 1.027 | 6.19 | 174157 | 0.538⁽³⁾ |
| AF-301 | 1.025 | 6.46 | 25118 | 0.705⁽³⁾ |
| AF-302 | 1.025 | | 18011 | 0.642⁽³⁾ |
| AF-303 | 1.019 | 6.13 | 175113 | 0.0001^{(1,2)} |
| AF-304 | 1.014 | 5.97 | 60157 | 0.902⁽³⁾ |
| AF-305 | 1.013 | 4.63 | 21103 | 0.873⁽³⁾ |
| AF-306 | 1.010 | 6.58 | 27953 | 0.836⁽³⁾ |
| AF-307 | 1.006 | 6.79 | 30719 | 0.969⁽³⁾ |
| AF-308 | 1.005 | 5.57 | 179672 | 0.924⁽³⁾ |
| AF-309 | 1.000 | 10.69 | 18098 | 0.998⁽³⁾ |

| | | | | |
|---|---|---|---|---|
| * The statistical technique used to calculate a given p value is indicated by a footnote for each p value. The statistical techniques used for the group analysis were comparisons between patients with AD and control patients with no AD. Analysis included (1) a linear model, controlling for age and gender; (2) Stepwise multivariate linear discriminant analysis; (3) nearest neighbor and (4) longitudinal analaysis. | | | | |

### (b) AFs Identified By Mastergroup Analysis Which Can Be Used In Combination With The Proteins Of Table I (a) and Table II (a)

**Table I (b)**

| **AF#** | **Fold Decrease**^{**#**} | **pI** | **MW (Da)** | **p value*** |
|---|---|---|---|---|
| AF-1 | 1.41 | 4.79 | 150081 | 0.001695⁽¹⁾ |
| AF-2 | 1.34 | 4.28 | 21349 | 0.000133⁽¹⁾ |
| AF-3 | 1.47 | 8.10 | 34846 | 0.000083⁽¹⁾ |
| AF-4 | 1.56 | 4.38 | 21160 | 0.001192⁽¹⁾ |
| AF-5 | 1.51 | 7.34 | 36554 | 0.000010⁽¹⁾ |
| AF-6 | 1.46 | 4.91 | 29812 | 0.000003⁽¹⁾ |
| AF-7 | 1.24 | 4.25 | 20787 | 0.003558⁽¹⁾ |
| AF-8 | 1.44 | 4.93 | 187927 | 0.002221⁽¹⁾ |
| AF-9 | 1.34 | 5.21 | 136768 | 0.000799⁽¹⁾ |
| AF-10 | 1.30 | 5.19 | 17694 | 0.000400⁽¹⁾ |
| AF-13 | 1.37 | 6.01 | 184530 | 0.000100⁽¹⁾ |
| AF-14 | 1.52 | 4.72 | 63166 | 0.009387⁽¹⁾ |
| AF-15 | 1.38 | 4.47 | 38970 | 0.000437⁽¹⁾ |
| AF-16 | 1.22 | 5.19 | 46876 | 0.000697⁽¹⁾ |
| AF-17 | 1.38 | 5.82 | 50294 | 0.000126⁽¹⁾ |
| AF-18 | 1.30 | 4.87 | 49219 | 0.020661⁽¹⁾ |
| AT-19 | 1.24 | 4.82 | 12454 | 0.00146⁽¹⁾ |
| AF-20 | 1.30 | 4.43 | 16818 | 0.000322⁽¹⁾ |
| AF-21 | 1.30 | 5.40 | 141094 | 0.000560⁽¹⁾ |
| AF-22 | 1.36 | 4.93 | 133773 | 0.011000⁽¹⁾ |
| AF-23 | 1.21 | 4.50 | 32473 | 0.000209⁽⁴⁾ |
| AF-24 | 1.20 | 5.31 | 46663 | 0.000871⁽¹⁾ |
| AF-25 | 1.19 | 5.68 | 36700 | 0.00251⁽¹⁾ |
| AF-26 | 1.31 | 8.11 | 32305 | 0.002204⁽¹⁾ |
| AF-27 | 1.26 | 5.33 | 141371 | 0.010447⁽¹⁾ |
| AF-28 | 1.06 | 5.13 | 158568 | 0.000100⁽¹⁾ |
| AF-29 | 1.27 | 9.22 | 47059 | 0.000028⁽¹⁾ |
| AF-30 | 1.13 | 5.67 | 48057 | 0.000100⁽¹⁾ |
| AF-31 | 1.15 | 6.07 | 91258 | 0.012712⁽⁴⁾ |
| AF-32 | 1.15 | 6.17 | 48958 | 0.008321⁽⁴⁾ |
| AF-33 | 1.06 | 4.41 | 42104 | 0.000126⁽¹⁾ |
| AF-34 | 1.14 | 4.54 | 145408 | 0.017268⁽⁴⁾ |
| AF-35 | 1.22 | 5.21 | 18623 | 0.001094⁽¹⁾ |
| AF-36 | 1.17 | 5.78 | 14416 | 0.010744⁽¹⁾ |
| AF-37 | 1.29 | 6.91 | 33523 | 0.000087⁽¹⁾ |
| AF-38 | 1.18 | 6.47 | 29535 | 0.002759⁽¹⁾ |
| AF-39 | 1.30 | 7.50 | 35510 | 0.002858⁽¹⁾ |
| AF-40 | 1.22 | 7.29 | 38617 | 0.00118⁽¹⁾ |
| AF-41 | 1.11 | 5.85 | 17345 | 0.016690⁽⁴⁾ |
| AF-42 | 1.10 | 5.04 | 18662 | 0.002252⁽¹⁾ |
| AF-43 | 1.13 | 9.83 | 14065 | 0.003303⁽¹⁾ |
| AF-44 | 1.10 | 6.63 | 102328 | 0.020753⁽⁴⁾ |
| AF-45 | 1.09 | 6.04 | 46998 | 0.031910⁽⁴⁾ |
| AF-46 | 1.09 | 4.71 | 19802 | 0.008437⁽⁴⁾ |
| AF-47 | 1.09 | 5.99 | 49664 | 0.002187⁽¹⁾ |
| AF-48 | 1.32 | 5.32 | 122332 | 0.006582⁽¹⁾ |
| AF-49 | 1.07 | 6.94 | 27576 | 0.010068⁽⁴⁾ |
| AF-50 | 1.07 | 6.82 | 71337 | 0.035409⁽⁴⁾ |
| AF-51 | 1.04 | 5.70 | 34388 | 0.006156⁽⁴⁾ |
| AF-76 | 1.11 | 5.59 | 45537 | 0.001973⁽¹⁾ |
| AF-149 | 1.15 | 4.82 | 190721 | 0.003541⁽¹⁾ |
| AF-150 | 1.24 | 6.87 | 157592 | 0.000100⁽¹⁾ |
| AF-152 | 1.09 | 5.04 | 81703 | 0.002800⁽¹⁾ |
| AF-154 | 1.06 | 5.03 | 67307 | 0.000100⁽¹⁾ |
| AF-155 | 1.38 | 9.21 | 64021 | 0.000070⁽¹⁾ |
| AF-156 | 9.75 | 4.36 | 58083 | 0.001568⁽¹⁾ |
| AF-159 | 1.18 | 5.08 | 52008 | 0.000100⁽¹⁾ |
| AF-160 | 1.06 | 5.76 | 45729 | 0.004123⁽¹⁾ |
| AF-162 | 1.23 | 5.47 | 38663 | 0.001073⁽¹⁾ |
| AF-163 | 1.39 | 4.45 | 34879 | 0.001228⁽¹⁾ |
| AF-164 | 1.51 | 5.00 | 33485 | 0.01179⁽¹⁾ |
| AF-169 | 1.00 | 8.00 | 34362 | 0.000004⁽¹⁾ |
| AF-170 | 1.38 | 5.41 | 31886 | 0.005600⁽¹⁾ |
| AF-172 | 1.53 | 6.71 | 28747 | 0.01051⁽¹⁾ |
| AF-173 | 10.91 | 7.67 | 27476 | 0.003738⁽¹⁾ |
| AF-174 | 1.03 | 4.67 | 27811 | 0.002423⁽¹⁾ |
| AF-175 | 1.03 | 5.33 | 24936 | 0.044270⁽¹⁾ |
| AF-176 | 1.15 | 4.86 | 22248 | 0.012144⁽¹⁾ |
| AF-177 | 1.14 | 4.63 | 21103 | 0.006564⁽¹⁾ |
| AF-178 | 1.08 | 6.03 | 22247 | 0.05097⁽¹⁾ |
| AF-181 | 1.21 | 5.72 | 16336 | 0.004745⁽¹⁾ |
| AF-183 | 1.44 | 10.36 | 11160 | 0.000270⁽¹⁾ |
| AF-184 | 1.08 | 5.31 | 48769 | 0.004689⁽¹⁾ |
| AF-186 | 2.76 | 4.71 | 29693 | 0.002446⁽¹⁾ |
| AF-187 | 2.09 | 4.93 | 154156 | 0.000750⁽¹⁾ |
| AF-188 | 1.35 | 5.52 | 39355 | 0.007175⁽¹⁾ |
| AF-189 | 1.29 | 6.79 | 30719 | 0.000377⁽¹⁾ |
| AF-190 | 1.26 | 5.29 | 29663 | 0.000178⁽¹⁾ |
| AF-191 | 1.12 | 5.31 | 46663 | 0.000654⁽¹⁾ |

| | | | | |
|---|---|---|---|---|
| * The statistical technique used to calculate a given p value is indicated by a footnote for each p value. The statistical techniques used for these group analyzes were (1) a linear model, controlling for age and gender; (2) classification trees;(3) a logistic regression model and (4) longitudinal analysis. | | | | |
| # Fold changes reported here are those calculated before adjustment for age and gender. | | | | |

### (c) AFs Identified From Pooled Gel Analysis Which Can Be Used In Combination With The Proteins From Table I (a) and Table II (a)

**Table I (c)**

| **AF#** | **Fold Decrease*** | **pI** | **MW (Da)** |
|---|---|---|---|
| AF-78 | 2.80 | 5.59 | 158937 |
| AF-79 | 2.62 | 5.52 | 142378 |
| AF-80 | 2.78 | 5.56 | 142378 |
| AF-81 | 2.60 | 5.43 | 78299 |
| AF-82 | 7.25 | 6.69 | 74838 |
| AF-83 | 4.07 | 6.81 | 71920 |
| AF-84 | 4.44 | 6.94 | 73402 |
| AF-85 | 16.77 | 7.10 | 73878 |
| AF-86 | 4.86 | 5.24 | 67676 |
| AF-87 | 4.17 | 5.95 | 64179 |
| AF-88 | 6.48 | 5.36 | 66979 |
| AF-89 | 2.64 | 5.39 | 65155 |
| AF-90 | 4.81 | 7.61 | 62945 |
| AF-91 | 3.10 | 8.16 | 56352 |
| AF-92 | 2.63 | 6.18 | 50860 |
| AF-93 | 6.33 | 6.28 | 49268 |
| AF-94 | 11.17 | 4.38 | 45882 |
| AF-95 | 9.28 | 5.60 | 46036 |
| AF-96 | 11.07 | 4.43 | 44966 |
| AF-98 | 5.51 | 6.72 | 44664 |
| AF-99 | 10.1 | 5.92 | 44365 |
| AF-100 | 15.39 | 6.08 | 44068 |
| AF-101 | 9.82 | 4.47 | 44216 |
| AF-102 | 3.14 | 6.02 | 44216 |
| AF-103 | 3.57 | 5.93 | 42722 |
| AF-104 | 22.78 | 5.09 | 42184 |
| AF-105 | 4.11 | 5.19 | 42184 |
| AF-107 | 13.3 | 7.26 | 33226 |
| AF-108 | 19.0 | 7.54 | 33136 |
| AF-110 | 9.21 | 5.39 | 28237 |
| AF-111 | 14.85 | 5.68 | 27835 |
| AT-112 | 2.60 | 6.00 | 20681 |
| AF-114 | 8.98 | 6.80 | 18741 |
| AF-115 | 2.85 | 6.04 | 17422 |
| AF-116 | 6.85 | 6.68 | 14031 |
| AF-117 | 2.90 | 4.65 | 13983 |
| AF-118 | 15.19 | 6.94 | 11739 |
| AF-119 | 16.27 | 7.23 | 11699 |

| | | | |
|---|---|---|---|
| # These features were identified as having a differential presence that is significant on the basis of having at least a 2-fold difference in mean intensity (i.e. a fold change threshold of at least 2) between Alzheimer's CSF and normal CSF. | | | |

The second group consists of AFs that are increased in the CSF of subjects having AD as compared with the CSF of subjects free from AD. These AFs can be described by apparent molecular weight (MW) and isoelectric point (pI) as provided in Table II.

### Table II. AFs Increased in CSF of Subjects Having AD

### (a) AFs Identified From Mastergroup Analysis

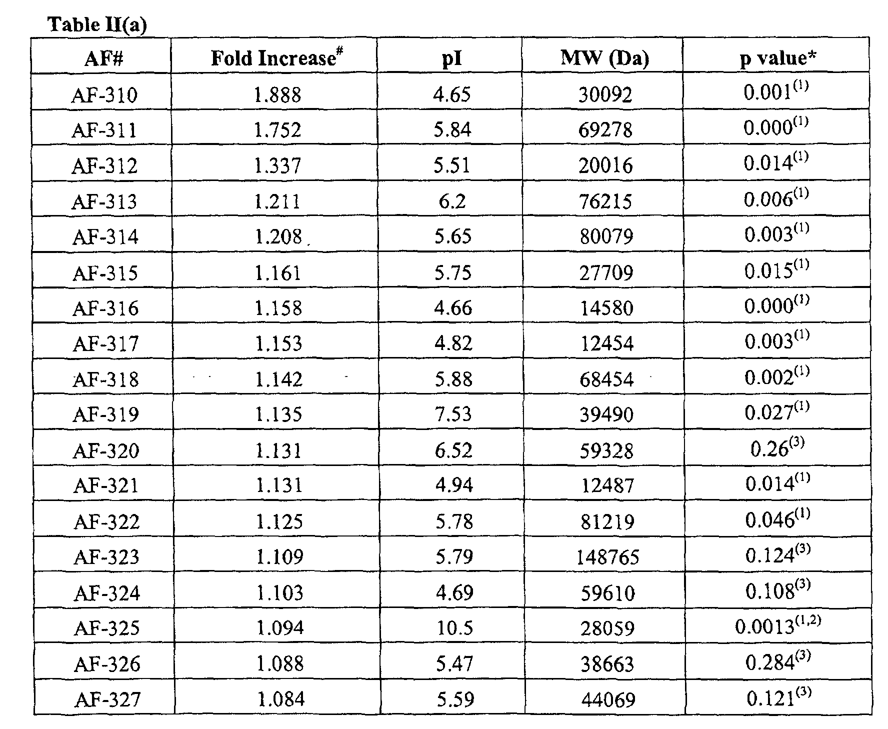

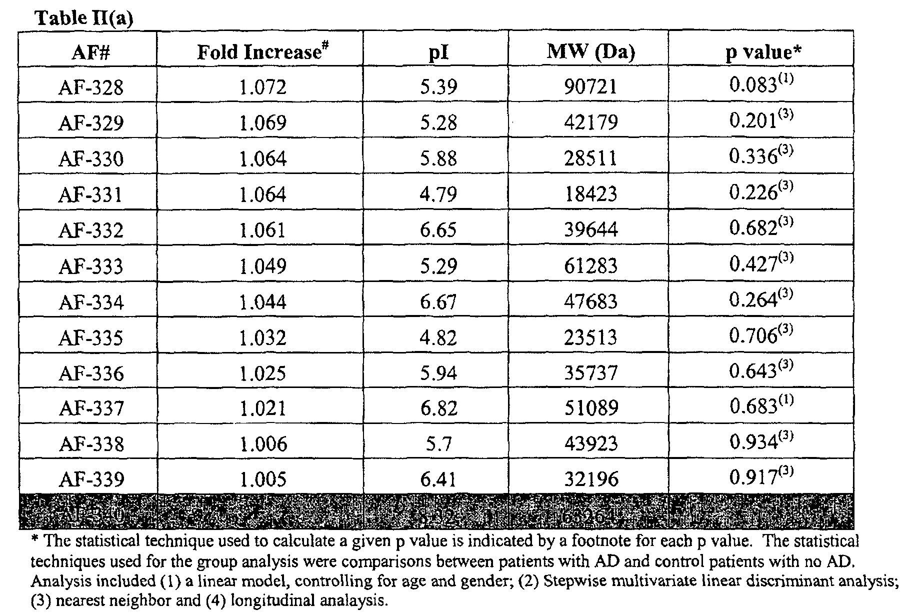

### (b) AFs Identified By Mastergroup Analysis Which Can Be Used In Combination With The Proteins Of Table I (a) and Table II (a)

**Table II (b)**

| **AF#** | **Fold Increase#** | **pI** | **MW (Da)** | **p value*** |
|---|---|---|---|---|
| AF-52 | 2.81 | 6.30 | 32573 | 0.000009⁽⁴⁾ |
| AT-53 | 1.80 | 5.84 | 45302 | 0.016106⁽⁴⁾ |
| AF-54 | 1.76 | 5.12 | 17520 | 0.003235⁽¹⁾ |
| AF-55 | 1.29 | 8.10 | 12361 | 0.000482⁽¹⁾ |
| AF-56 | 1.49 | 8.56 | 52128 | 0.005771⁽¹⁾ |
| AF-57 | 1.46 | 6.30 | 68549 | 0.000274⁽¹⁾ |
| AF-58 | 1.40 | 5.01 | 14507 | 0.01182⁽¹⁾ |
| AF-59 | 1.37 | 6.74 | 33401 | 0.001351⁽¹⁾ |
| AF-60 | 1.38 | 5.39 | 33873 | 0.009818⁽¹⁾ |
| AF-61 | 1.34 | 6.76 | 54345 | Bag Tree 4 Analysis⁽²⁾ |
| AF-62 | 1.31 | 6.60 | 31004 | 0.000027⁽¹⁾ |
| AF-63 | 1.24 | 5.97 | 14897 | 0.10696⁽¹⁾ |
| AF-64 | 1.20 | 6.67 | 68119 | 0.000731⁽¹⁾ |
| AF-65 | 1.22 | 7.19 | 58620 | 0.005833⁽⁴⁾ |
| AF-66 | 1.09 | 10.05 | 30092 | 0.000100⁽¹⁾ |
| AF-67 | 1.21 | 5.02 | 13735 | 0.006391⁽⁴⁾ |
| AF-68 | 1.21 | 9.06 | 35351 | 0.003575⁽¹⁾ |
| AF-69 | 1.19 | 5.01 | 46760 | 0.005125⁽⁴⁾ |
| AF-70 | 1.19 | 8.91 | 38789 | 0.021552⁽⁴⁾ |
| AF-71 | 1.20 | 6.44 | 68579 | 0.003848⁽¹⁾ |
| AF-72 | 1.15 | 5.00 | 43788 | 0.014917⁽⁴⁾ |
| AF-73 | 1.19 | 5.21 | 31615 | 0.000008⁽¹⁾ |
| AF-74 | 1.14 | 6.19 | 51934 | 0.054917⁽¹⁾ |
| AF-75 | 1.12 | 5.03 | 33671 | 0.002399⁽¹⁾ |
| AF-77 | 1.09 | 6.41 | 32196 | 0.035148⁽⁴⁾ |
| AF-151 | 1.13 | 5.28 | 137531 | Bag Tree Analysis⁽²⁾ |
| AF-153 | 1.23 | 9.85 | 69630 | 0.004906⁽¹⁾ |
| AF-157 | 1.70 | 4.99 | 55449 | 0.006272⁽¹⁾ |
| AT-161 | 1.00 | 5.18 | 44404 | 0.000600⁽¹⁾ |
| AF-165 | 1.88 | 7.17 | 34230 | 0.000035⁽¹⁾ |
| AF-166 | 1.20 | 8.54 | 33657 | 0.000009⁽¹⁾ |
| AF-167 | 1.31 | 5.69 | 33621 | 0.005400⁽¹⁾ |
| AF-168 | 1.00 | 7.66 | 33920 | 0.000013⁽¹⁾ |
| AF-171 | 1.10 | 4.98 | 29658 | 0.004242⁽¹⁾ |
| AF-179 | 1.64 | 5.26 | 20115 | Stepwise Analysis⁽¹⁾ |
| AF-180 | 1.62 | 6.17 | 16255 | 0.005047⁽¹⁾ |
| AF-182 | 1.37 | 4.89 | 13651 | 0.005380⁽¹⁾ |
| AF-185 | 6.00 | 5.32 | 40323 | 0.005520⁽¹⁾ |
| AF-192 | 1.04 | 5.38 | 62756 | 0.000213⁽¹⁾ |

| | | | | |
|---|---|---|---|---|
| * The statistical technique used to calculate a given p value is indicated by a footnote for each p value. The statistical techniques used for these group analyzes were (1) a linear model, controlling for age and gender; (2) classification trees;(3) a logistic regression model and (4) longitudinal analysis. | | | | |
| # Fold changes reported here are those calculated before adjustment for age and gender. | | | | |

### (c) AFs Identified From Pooled Gel Analysis Which Can Be Used In Combination With The Proteins From Table I (a) and Table II (a)

**Table II (c)**

| **AF#** | **Fold Increase#** | **pI** | **MW (Da)** |
|---|---|---|---|
| AF-121 | 11.7 | 5.42 | 105108 |
| AF-122 | 2.20 | 5.27 | 71060 |
| AF-123 | 6.35 | 7.31 | 64933 |
| AF-124 | 6.86 | 7.47 | 64736 |
| AF-125 | 2.34 | 4.77 | 61297 |
| AF-126 | 48.84 | 4.11 | 60374 |
| AF-127 | 6.79 | 4.98 | 59649 |
| AF-128 | 2.36 | 6.60 | 57865 |
| AF-129 | 2.90 | 5.29 | 54625 |
| AF-130 | 2.94 | 5.08 | 51880 |
| AF-131 | 5.19 | 6.54 | 50944 |
| AF-132 | 3.41 | 4.72 | 47414 |
| AF-133 | 3.08 | 5.12 | 44068 |
| AF-134 | 2.34 | 5.00 | 43516 |
| AF-137 | 4.41 | 4.98 | 36855 |
| AF-139 | 4.11 | 5.00 | 34295 |
| AF-140 | 110.32 | 6.80 | 32080 |
| AF-141 | 3.58 | 7.50 | 28440 |
| AF-142 | 2.66 | 6.75 | 27279 |
| AF-143 | 5.68 | 7.44 | 26066 |
| AF-144 | 2.71 | 6.56 | 20744 |
| AF-145 | 4.43 | 4.76 | 18069 |
| AF-146 | 2.18 | 4.94 | 12790 |
| AF-147 | 2.29 | 4.81 | 12790 |
| AF-148 | 5.04 | 5.32 | 11382 |

| | | | |
|---|---|---|---|
| # These features were identified as having a differential presence that is significant on the basis of having at least a 2-fold difference in mean intensity (i.e. a fold change threshold of at least 2) between Alzheimer's CSF and normal CSF. | | | |

For any given AF, the signal obtained upon analyzing CSF from subjects having AD relative to the signal obtained upon analyzing CSF from subjects free from AD will depend upon the particular analytical protocol and detection technique that is used. Accordingly, those skilled in the art will understand that any laboratory, based on the present description, can establish a suitable reference range for any AF in subjects free from AD according to the analytical protocol and detection technique in use. In particular, at least one positive control CSF sample from a subject known to have AD or at least one negative control CSF sample from a subject known to be free from AD (and more preferably both positive and negative control samples) are included in each batch of test samples analyzed. In one embodiment, the level of expression of a feature is determined relative to a background value, which is defined as the level of signal obtained from a proximal region of the image that (a) is equivalent in area to the particular feature in question; and (b) contains no substantial discernable protein feature.

In a preferred embodiment, the signal associated with an AF in the CSF of a subject (*e.g.*, a subject suspected of having or known to have AD) is normalized with reference to one or more Expression Reference Features (ERFs) detected in the same 2D gel. As will be apparent to one of ordinary skill in the art, such ERFs may readily be determined by comparing different samples using techniques and protocols such as the Preferred Technology. Suitable ERFs include (but are not limited to) that described in the following table.

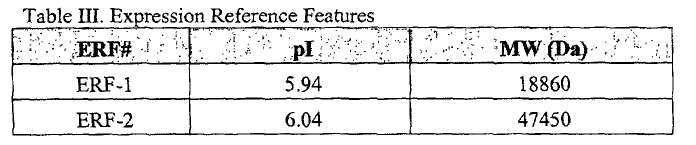

As those of skill in the art will readily appreciate, the measured MW and pI of a given feature or protein isoform will vary to some extent depending on the precise protocol used for each step of the 2D electrophoresis and for landmark matching. As used herein, the terms "MW" and "pI" are defined, respectively, to mean the apparent molecular weight in Daltons and the apparent isoelectric point of a feature or protein isoform as measured in careful accordance with the Reference Protocol identified in Section 6 below. When the Reference Protocol is followed and when samples are run in duplicate or a higher number of replicates, variation in the measured mean pI of an AF or API is typically less than 3% and variation in the measured mean MW of an AF or API is typically less than 5%. Where the skilled artisan wishes to diverge from the Reference Protocol, calibration experiments should be performed to compare the MW and pI for each AF or protein isoform as detected (a) by the Reference Protocol and (b) by the divergent.

The AFs of the invention can be used, for example, for detection, treatment, diagnosis, or the drug development or pharmaceutical products. In one embodiment of the invention, CSF from a subject (*e.g.*, a subject suspected of having AD) is analyzed by 2D electrophoresis for quantitative detection of one or more of the AFs as defined in Table I (a), optionally in combination with one or more of the Afs as defined in Tables I (b) and (c). Adecreased abundance of said one or more AFs in any suitable combination in the CSF from the subject relative to CSF from a subject or subjects free from AD (*e.g.*, a control sample or a previously determined reference range) indicates the presence of AD.

In a particular embodiment hereof, CSF from a subject (*e.g.*, a subject suspected of having AD) is analyzed by 2D electrophoresis for quantitative detection of one or more of the AFs are set forth herein in Table I (a). A decreased abundance of one or more in any suitable combination of such AFs in the CSF from the subject relative to CSF from a subject or subjects free from AD (*e.g.*, a control sample or a previously determined reference range) indicates the presence of AD.

In another embodiment of the invention, CSF from a subject is analyzed by 2D electrophoresis for quantitative detection of one or more of the Afs as defined in Table II (a), optionally in combination with one or more Afs as defined in Tables II (b) and (c). An increased abundance of said one or more AFs in any suitable combination in the CSF from the subject relative to CSF from a subject or subjects free from AD (*e.g.*, a control sample or a previously determined reference range) indicates the presence of AD. In particular embodiment hereof, CSF from a subject is analyzed by 2D electrophoresis for quantitative detection of one or more of the AFs set forth herein in Table II (a). An increased abundance of said one or more AFs in any suitable combination in the CSF from the subject relative to CSF from a subject or subjects free from AD (*e.g.*, a control sample or a previously determined reference range) indicates the presence of AD.

In yet another embodiment, CSF from a subject is analyzed by 2D electrophoresis for quantitative detection of (a) one or more AFs or any suitable combination of them, whose decreased abundance indicates the presence of AD, *i.e.*, preferably AFs as set forth in Tables I (a), optionally in combination with the AFs set out in Tables I (b), or I (c); and (b) one or more AFs or any combination of them, whose increased abundance indicates the presence of AD *i.e.*, preferably AFs set forth in Tables II (a), optionally in combination with the AFs set out in Tables II (b), or II (c).

In yet another embodiment of the invention, CSF from a subject is analyzed by 2D electrophoresis for quantitative detection of one or more of the AFs set forth in Table I (a) or II (a), optionally in combination with the AFs set forth in Tables I (b), I (c), II (b), or II (c) wherein the ratio of the one or more AFs relative to an Expression Reference Feature (ERF) indicates that AD is present. In a specific embodiment, a decrease in one or more AF/ERF ratios in a sample being tested relative to the AF/ERF ratios in a control sample or a reference range indicates the presence of AD; the AFs set forth in Table I (a), optionally combined with those set forth in Tables I (b), or I (c) are suitable are suitable AFs for this purpose. In another specific embodiment, one may measure one or more AFs in a test sample, and compare them to an ERF, as a method for detecting the presence of AD. Thus, an increase in one or more AF/ERF ratios in a test sample relative to the AF/ERF ratios in a control sample or a reference range indicates the presence of AD, the AFs set forth in Table II (a), optionally combined with the AFs set forth in Tables II (b) or II (c) are suitable AFs for this purpose.

In a further embodiment of the invention, CSF from a subject is analyzed by 2D electrophoresis for quantitative detection of (a) one or more AFs, or any suitable combination of them, whose decreased AF/ERF ratio(s) in a test sample relative to the AF/ERF ratio(s) in a control sample indicates the presence of AD, *i.e.*, preferably the AFs set forth in Table I (a), optionally combined with the AFs set forth in Tables I (b) or I (c); (b) one or more AFs, or any combination of them, whose increased AF/ERF ratio(s) in a test sample relative to the AF/ERF ratio(s) in a control sample indicates the presence of AD, *i.e.*, preferably the AFs as set forth in Table II (a), optionally combined with the AFs set forth in Tables II (b), or II (c).

In a preferred embodiment, CSF from a subject is analyzed for quantitative detection of a plurality of AFs.

### 5.4 Alzheimer's Disease-Associated Protein Isoforms (APIs)

In another aspect of the invention, CSF from a subject, is analyzed for quantitative detection of one or more Alzheimer's Disease-Associated Protein Isoforms (APIs), *e.g*. for screening, treatment or diagnosis of AD or for development of pharmaceutical products. As is well known in the art, a given protein may be expressed as one or more variants forms (isoforms) that differ in amino acid composition *(e.g,* as a result of alternative mRNA or premRNA processing, *e.g.* alternative splicing or limited proteolysis) or as a result of differential post-translational modification *(e.g.,* glycosylation, phosphorylation, acylation), or both, so that proteins of identical amino acid sequence can differ in their pI, MW, or both. "Alzheimer's Disease -Associated Protein Isoform" refers to a protein isoform that is differentially present in CSF from a subject having AD compared with CSF from a subject free from AD.

Two groups of APIs are described herein by the amino acid sequencing of AFs as depicted in Figure 2 and described above. APIs were isolated, subjected to proteolysis, and analyzed by mass spectrometry using in this instance the methods and apparatus of the Preferred Technology, it being understood that the preferred technology is set forth as representative but not restrictive of the invention. One skilled in the art can identify sequence information from proteins analyzed by mass spectrometry and/or tandem mass spectrometry using various spectral interpretation methods and database searching tools. Examples of some of these methods and tools can be found at the Swiss Institute of Bioinformatics web site at http://www.expasy.ch/, and the European Molecular Biology Laboratory web site at www.mann.embl-heidelberg.de/Services/PeptideSearch/. Identification of APIs was performed using the SEQUEST search program (Eng et al., 1994, J. Am. Soc. Mass Spectrom. 5:976-989) and the method described in PCT Application No. PCT/GB01/04034.

The first group comprises of APIs that are decreased in the CSF of subjects having AD as compared with the CSF of subjects free from AD. The amino acid sequences of peptides produced from these APIs by proteolysis using trypsin and identified by tandem mass spectrometry and database searching using the SEQUEST program are listed in Table IV, in addition to their corresponding pIs and MWs. For one API, the partial sequence information derived from tandem mass spectrometry was not found to be described in any known public database. This API is listed as 'NOVEL' in Table IV, and the partial amino acid sequence information derived from manually interpreting the MS/MS spectrum of tryptic peptides of this API as described in the Example *infra*, is given in Table IX.

### Table IV. APIs Decreased in CSF of Subjects Having AD

### (a) APIs Decreased in CSF of Subjects Having AD

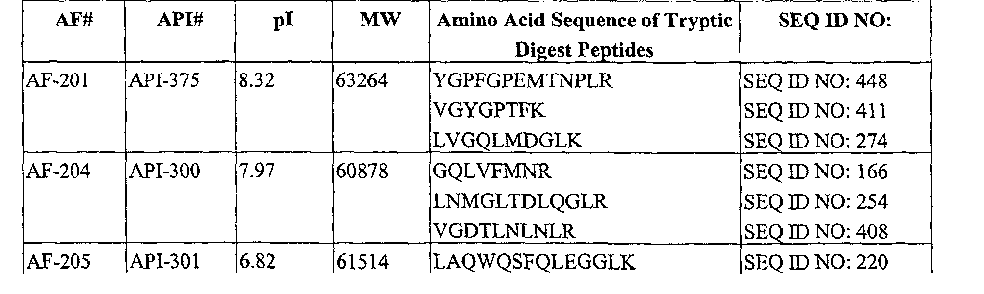

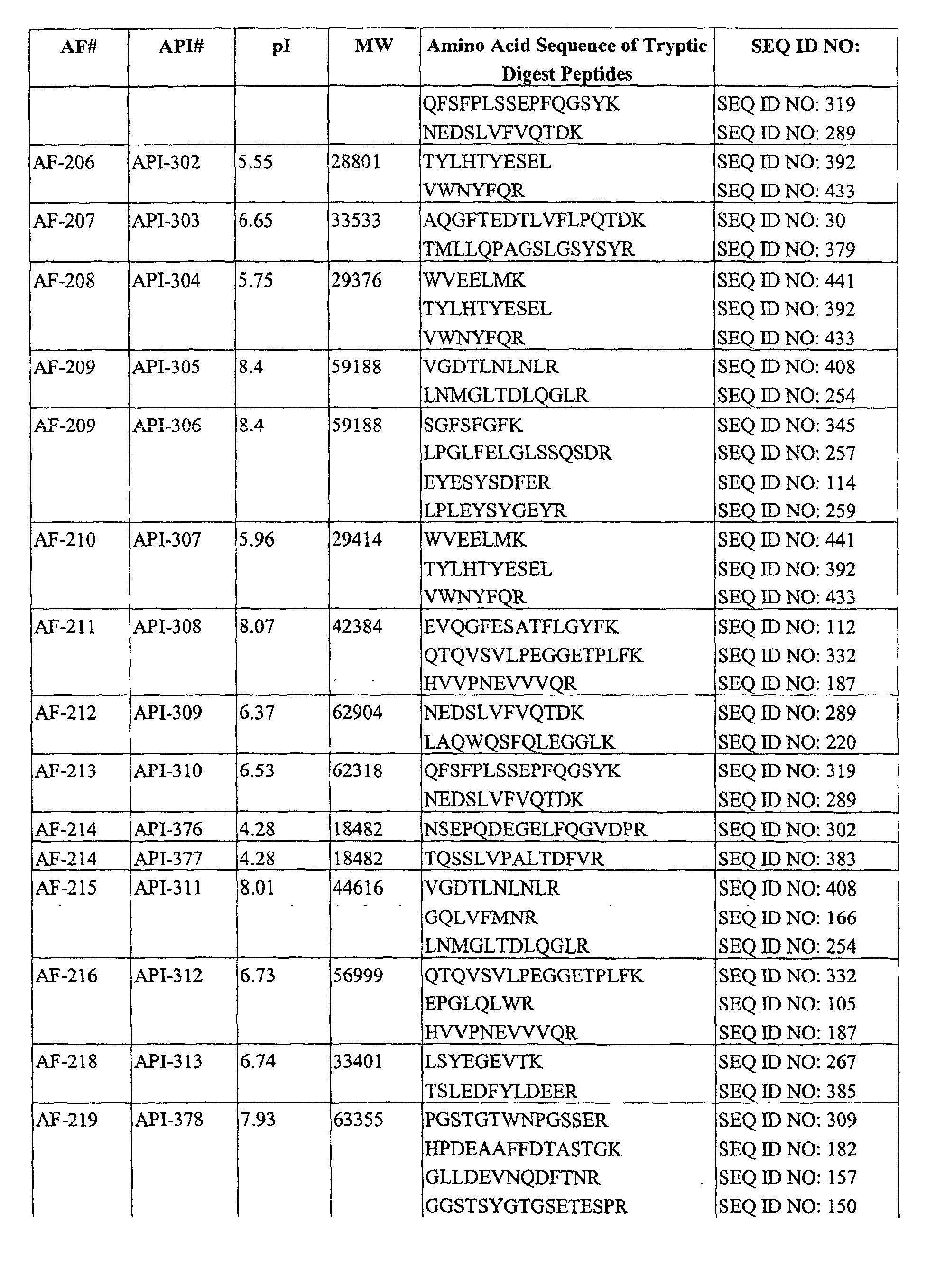

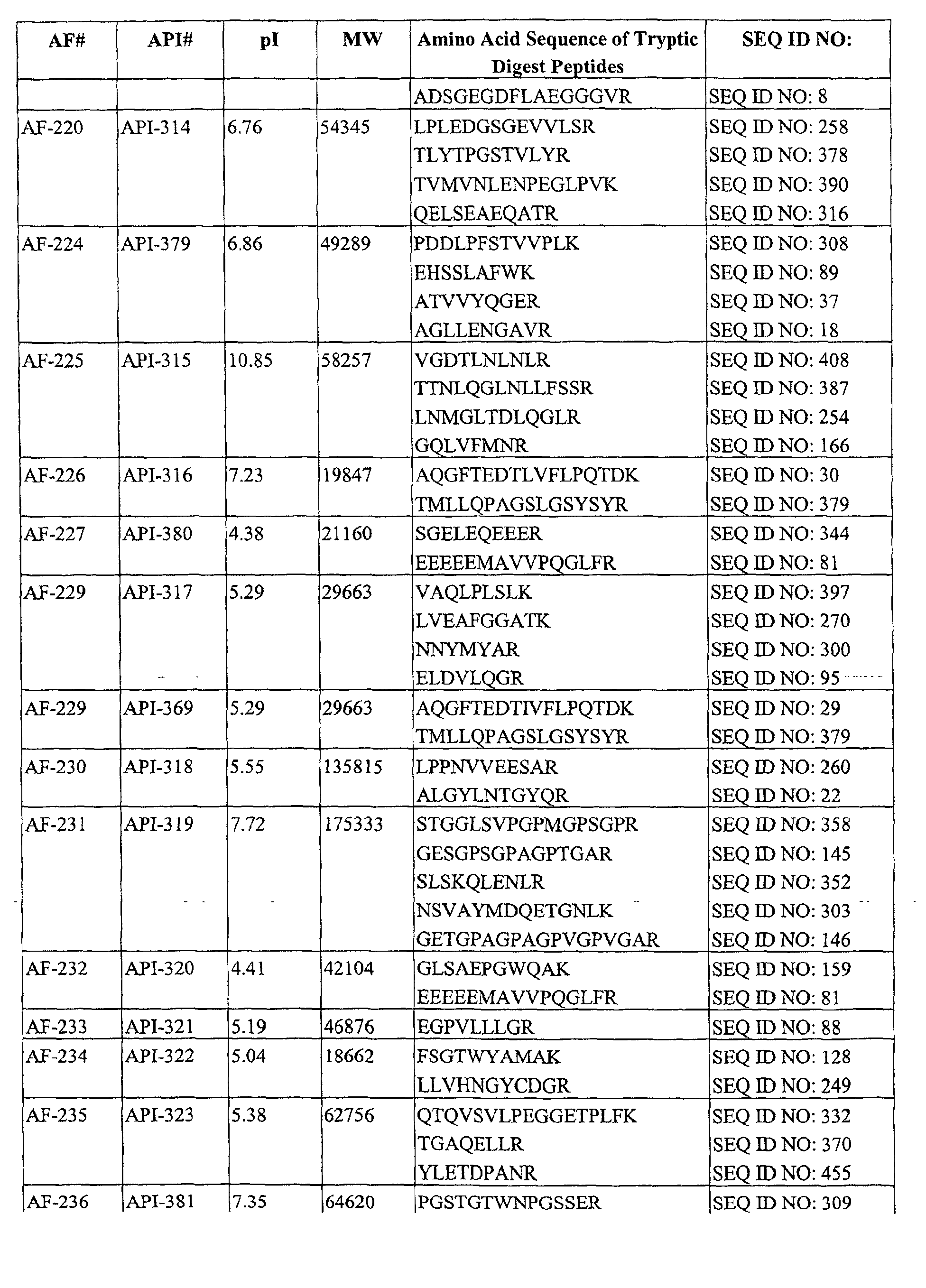

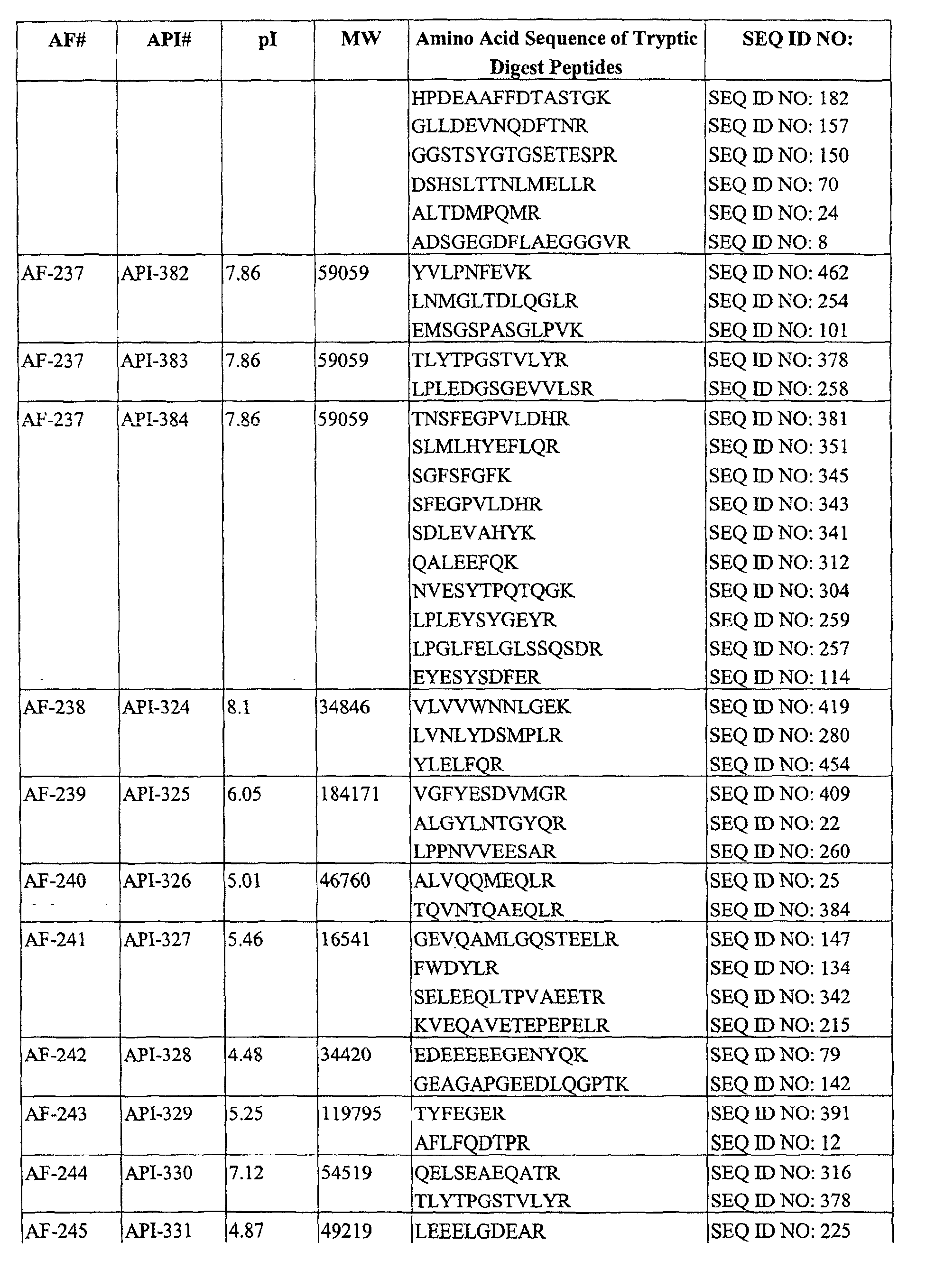

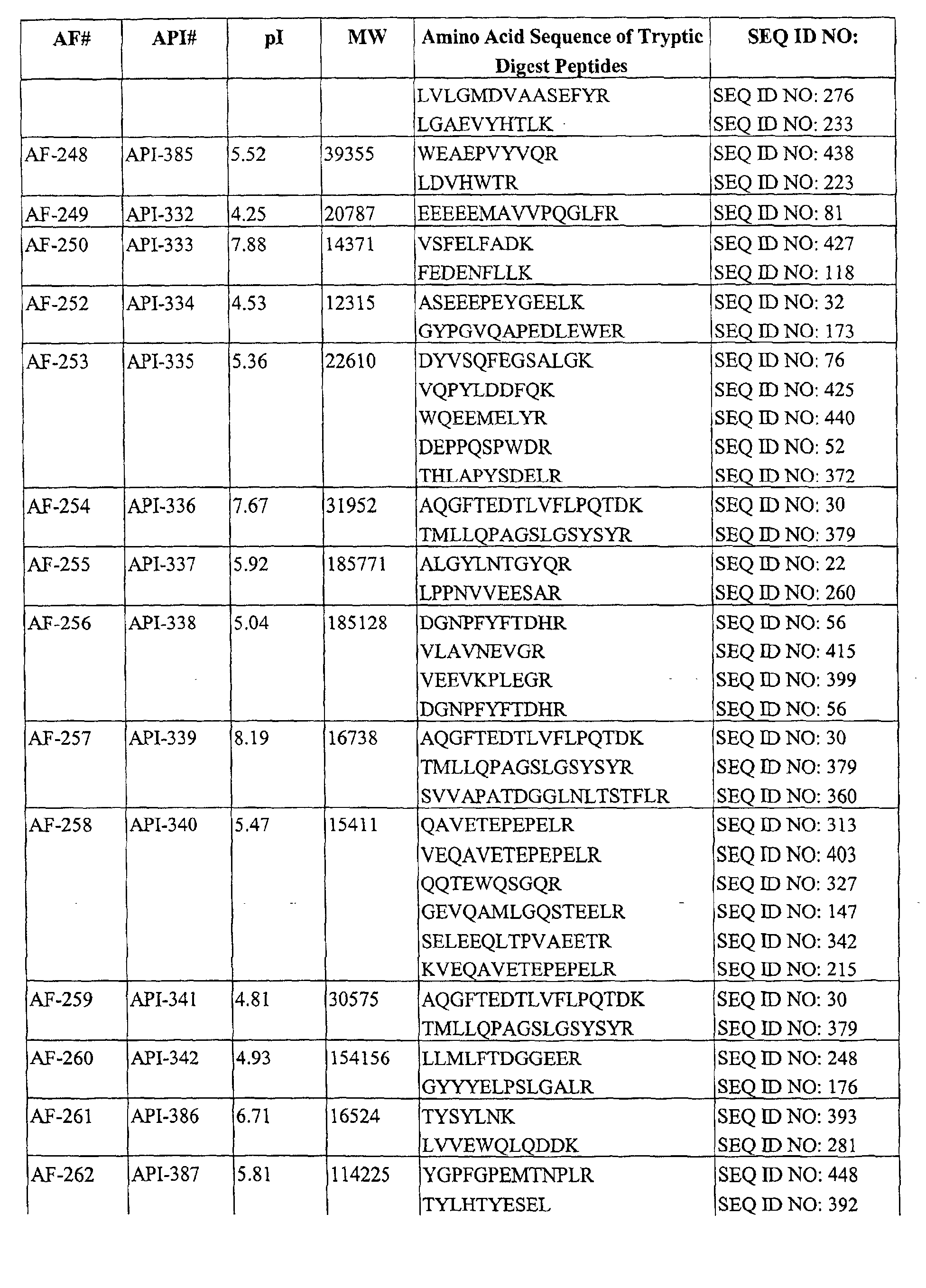

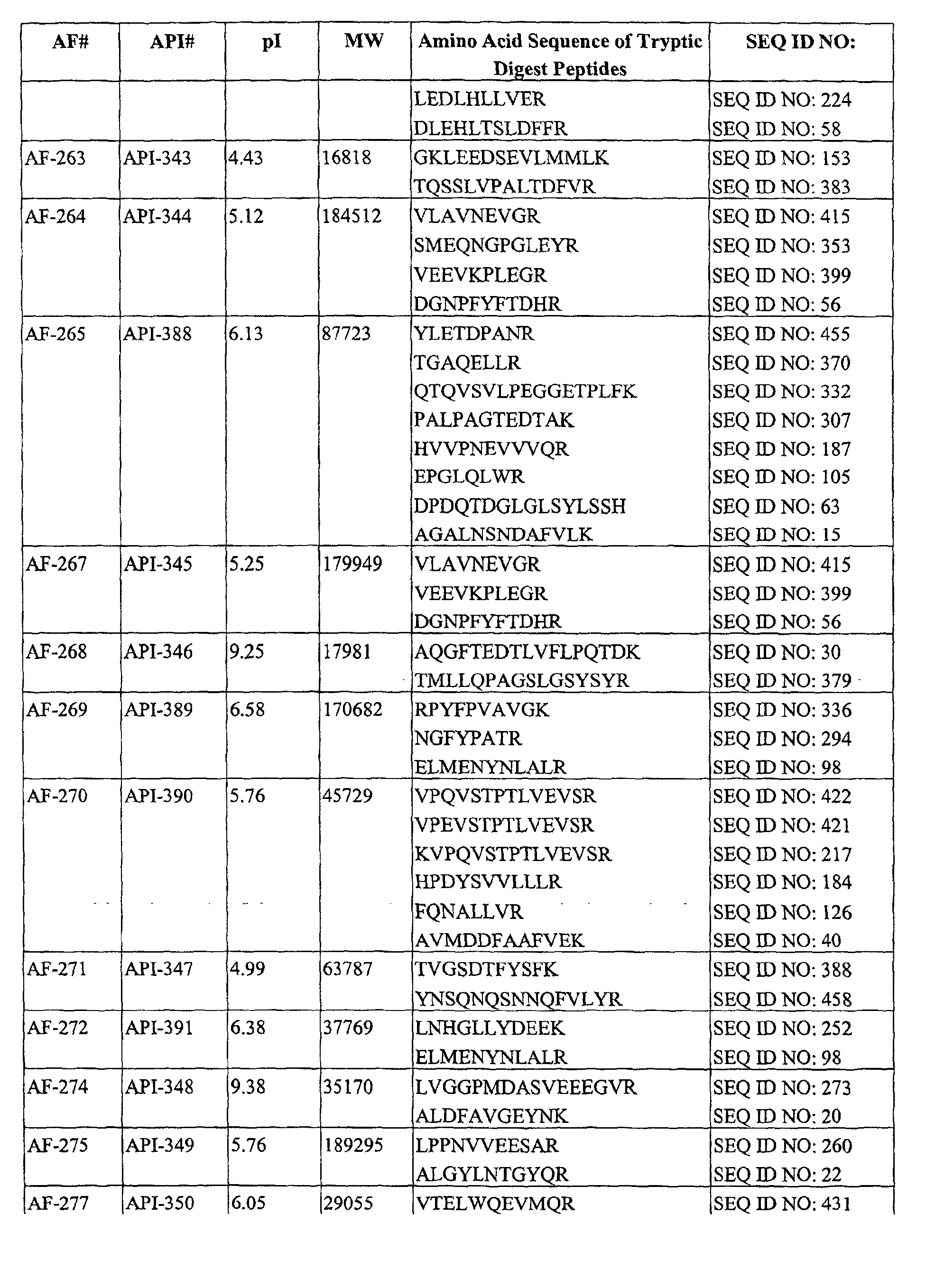

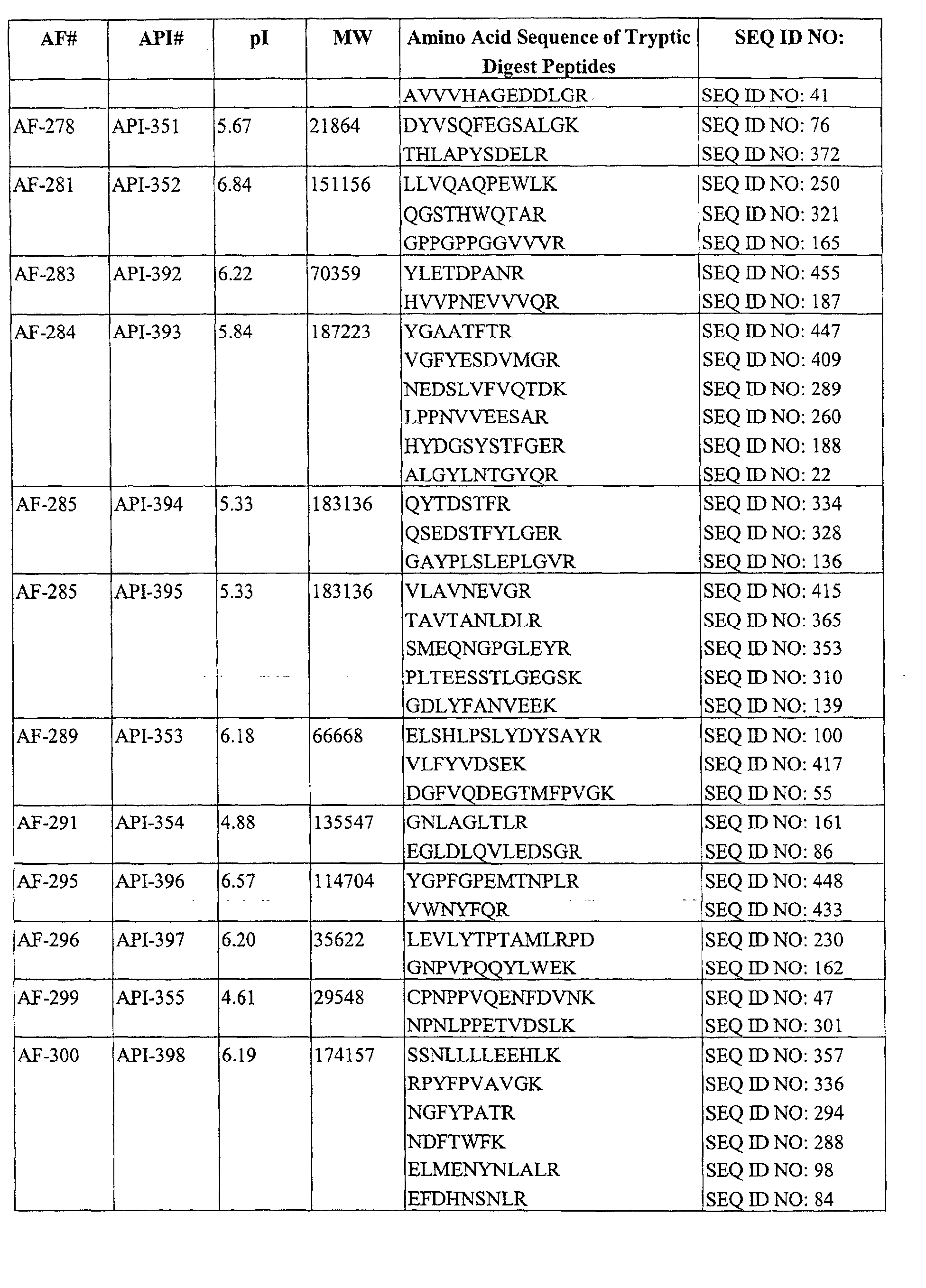

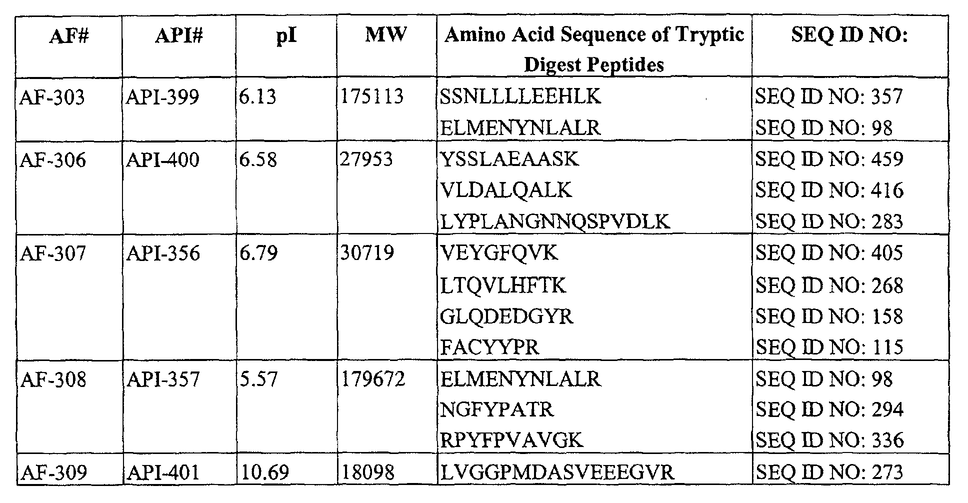

### (b) APIs which can be used in combination with the APIs in Table IV (a) and Table V (a)

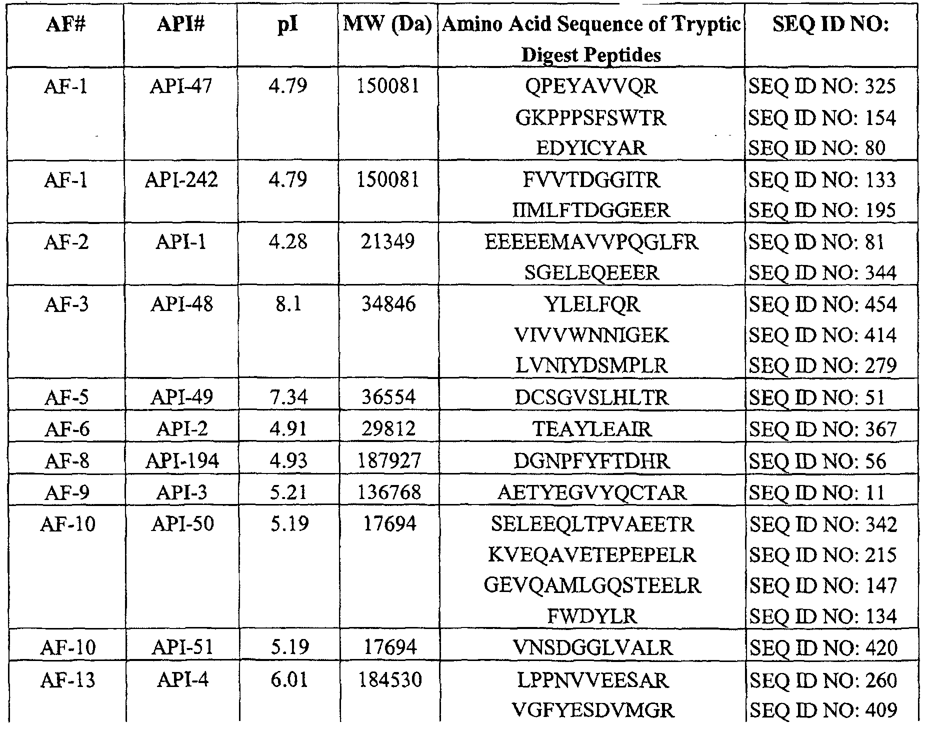

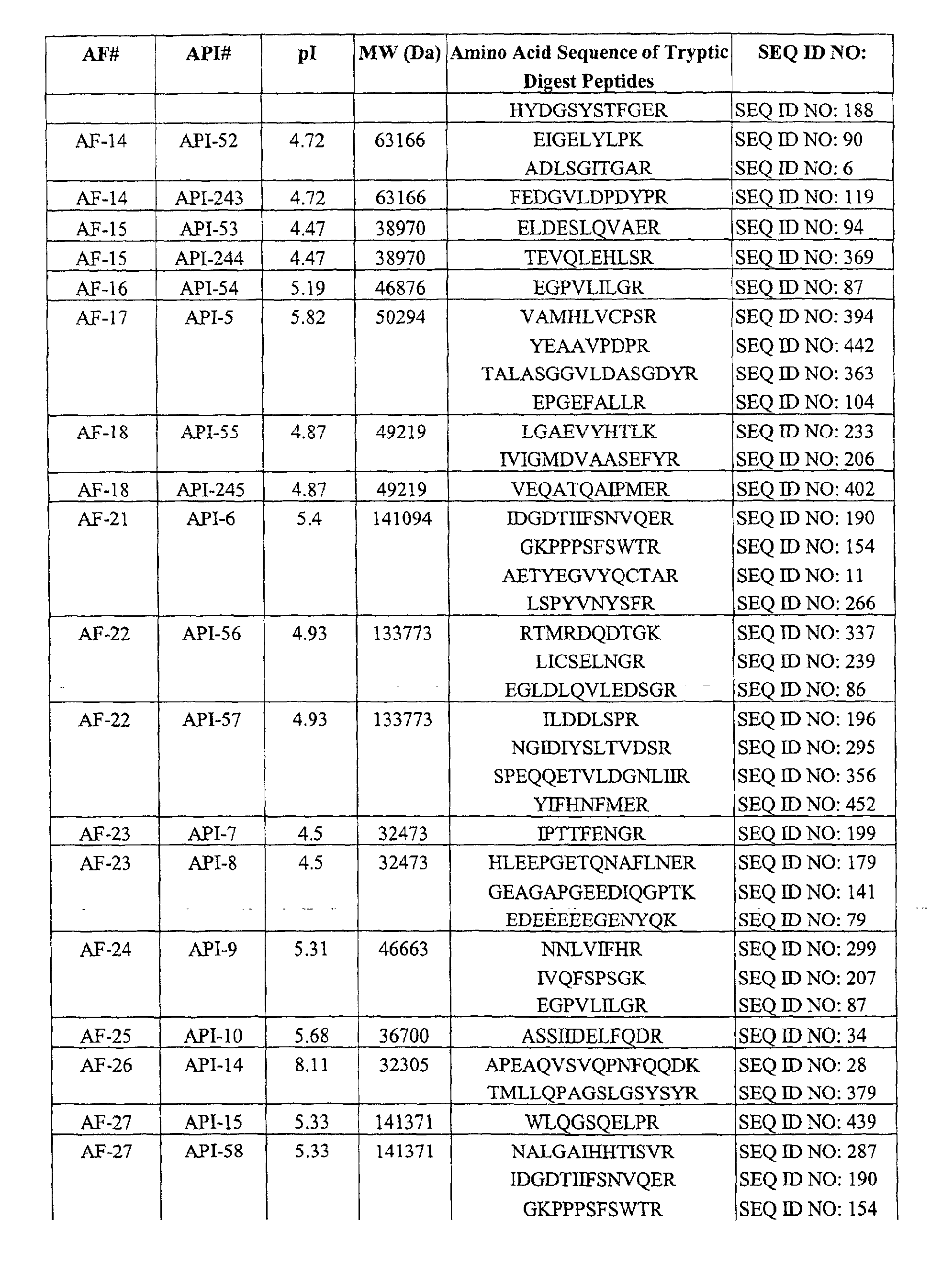

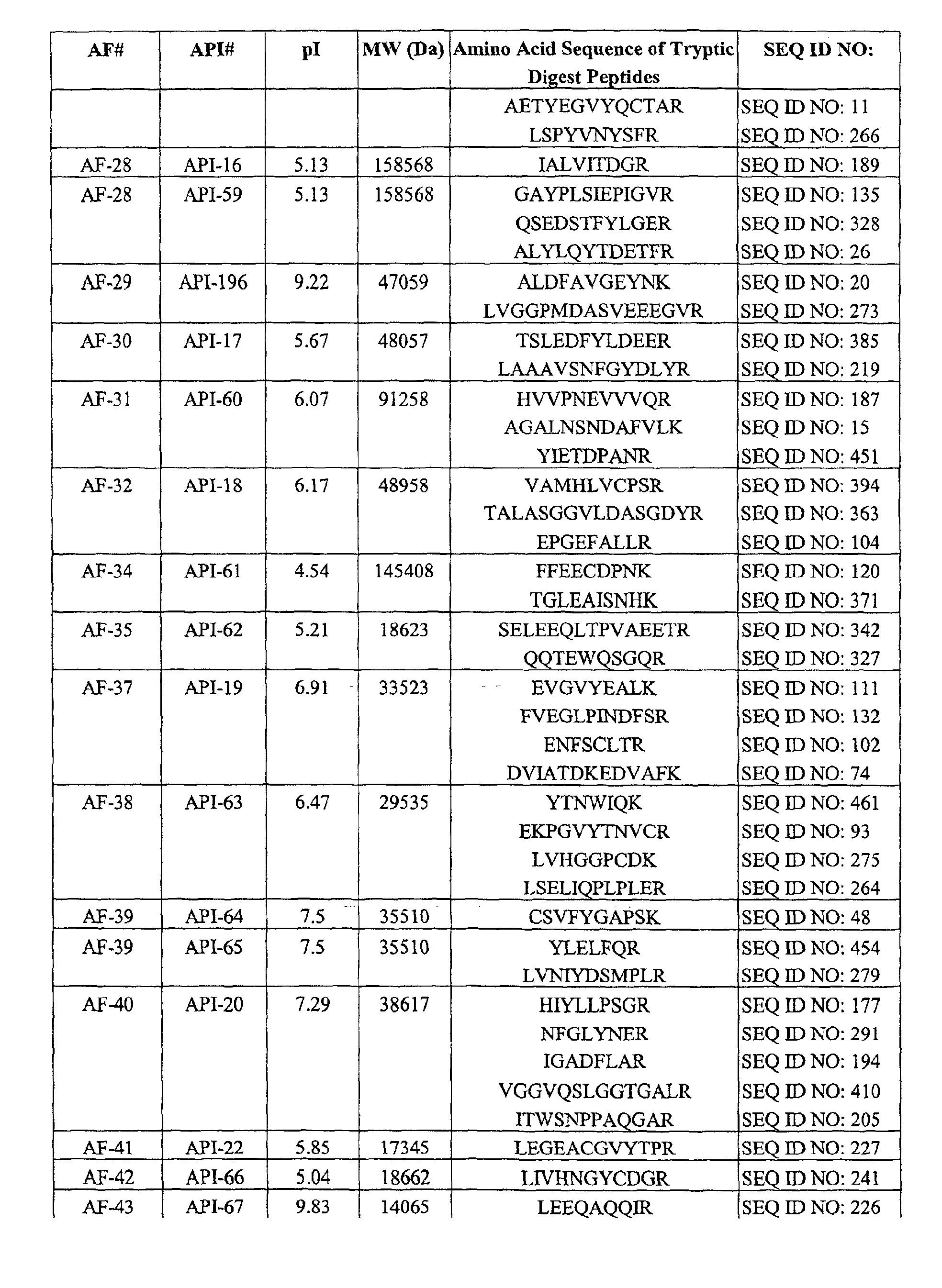

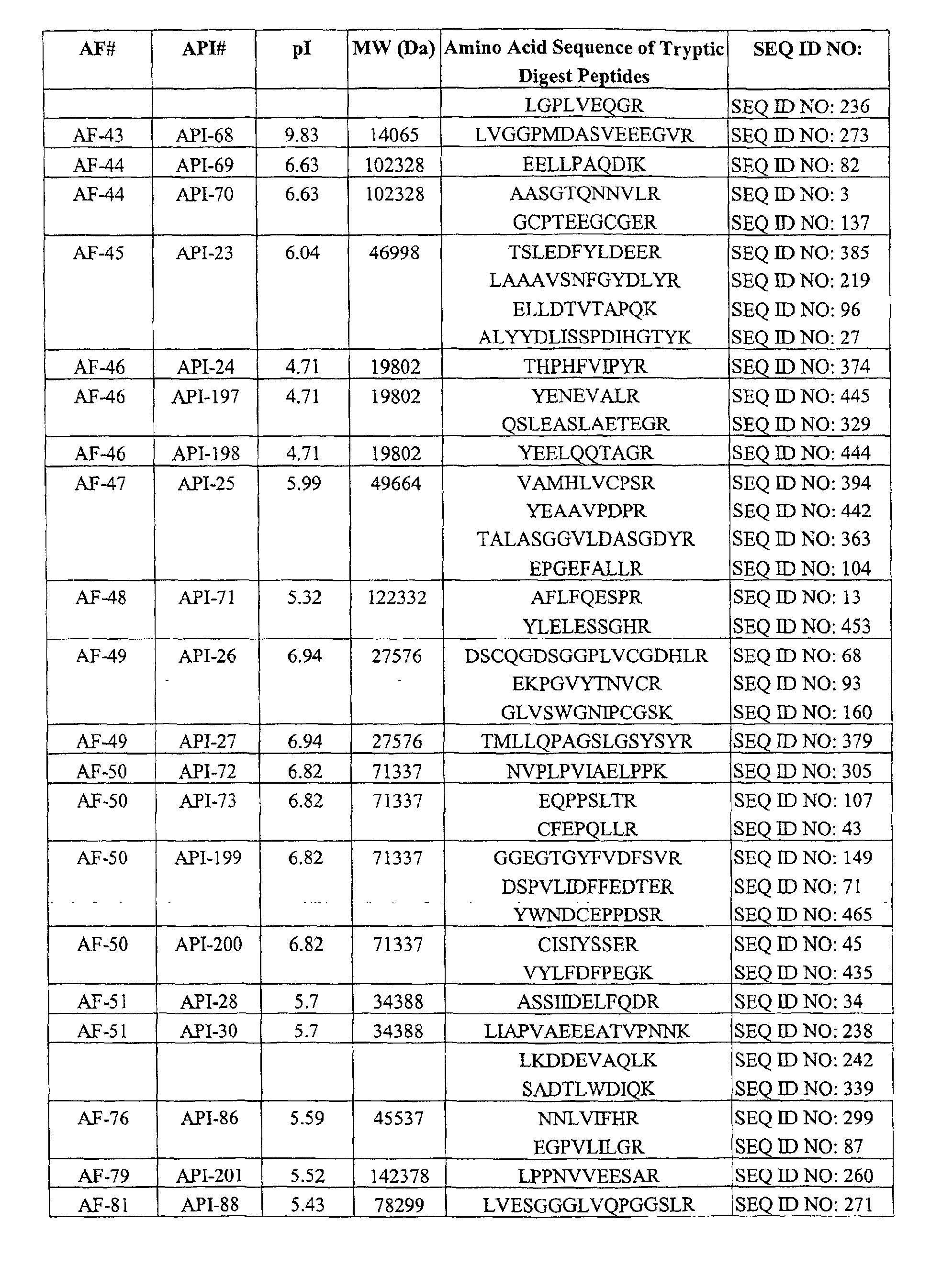

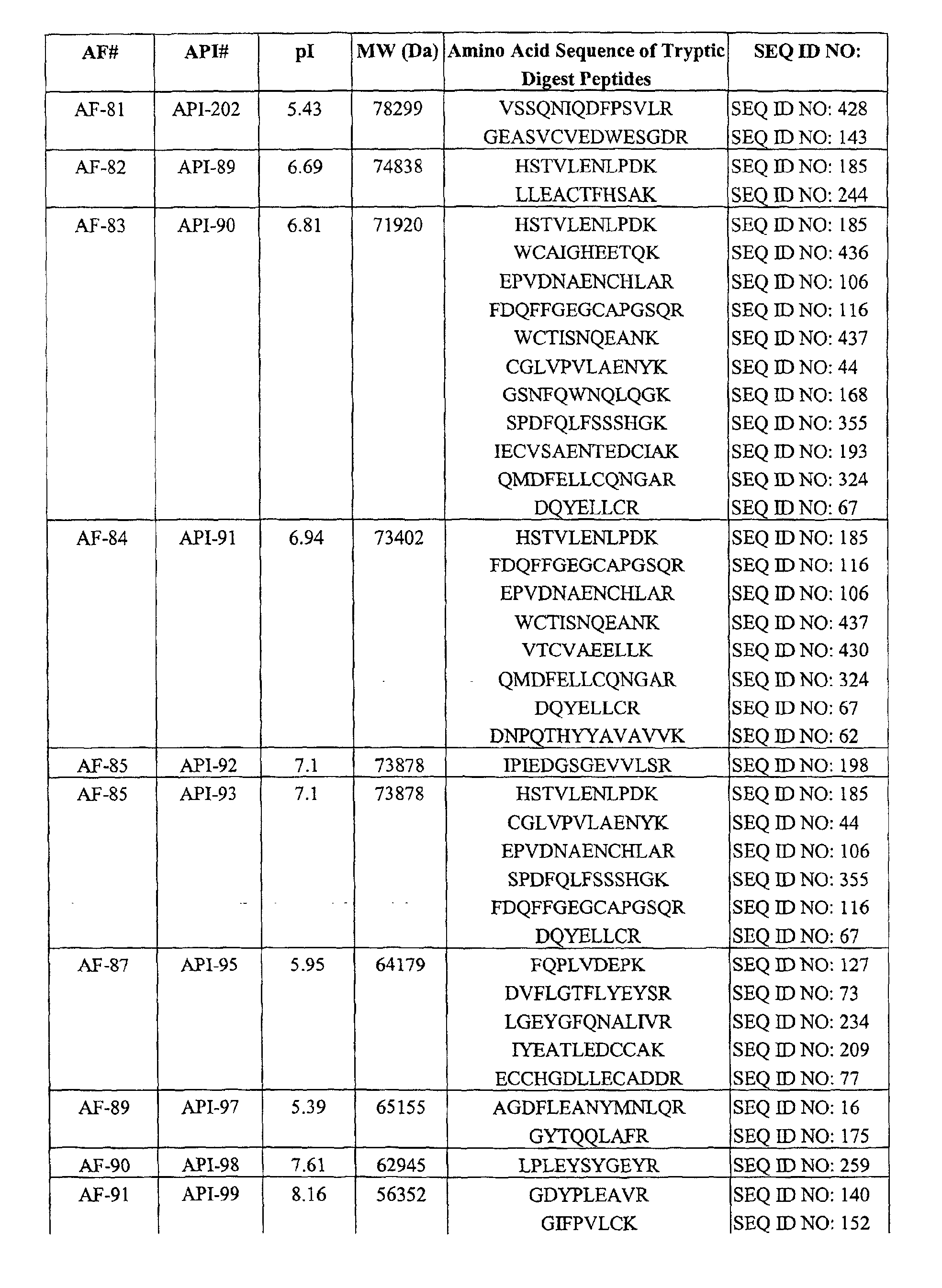

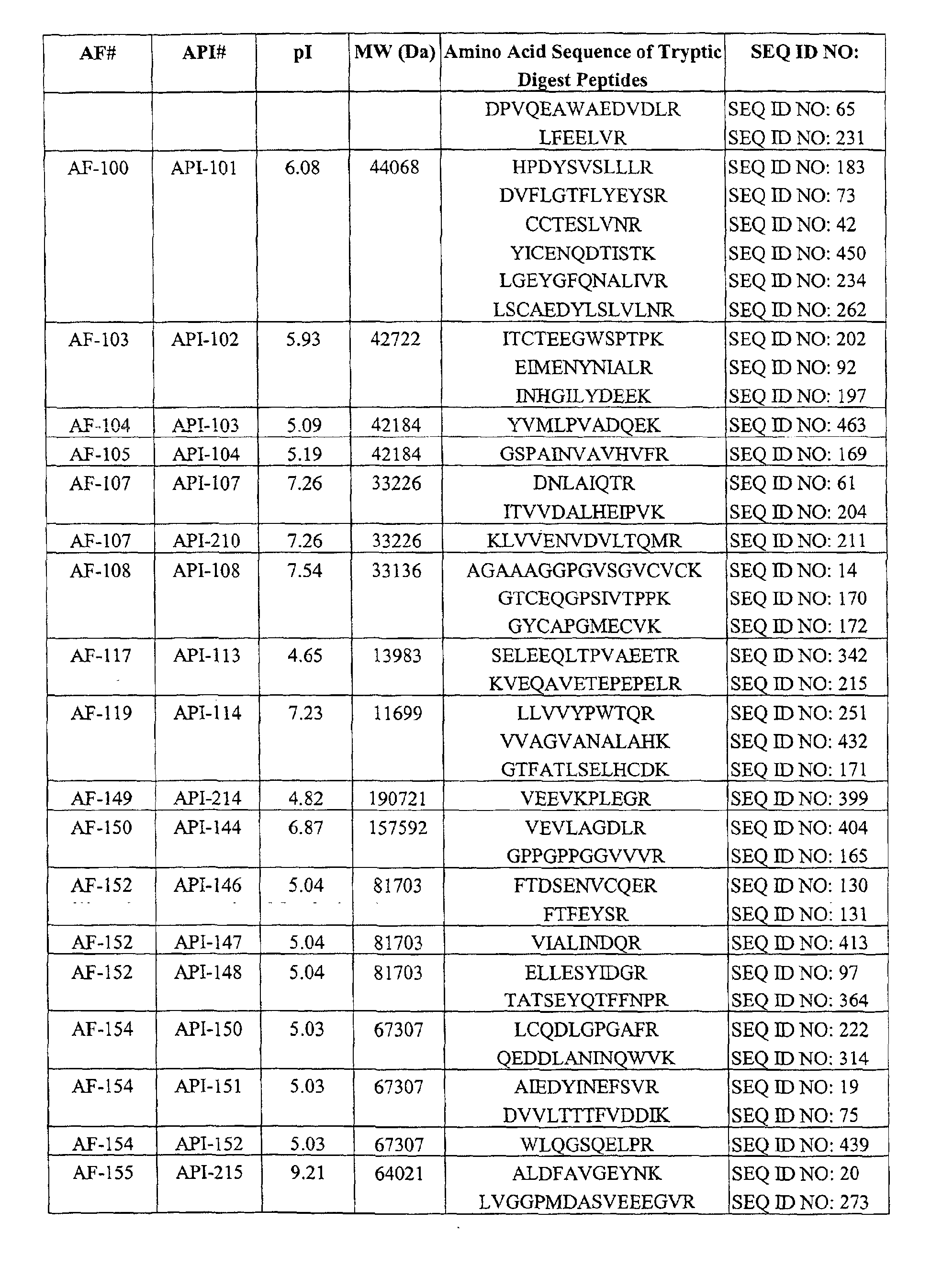

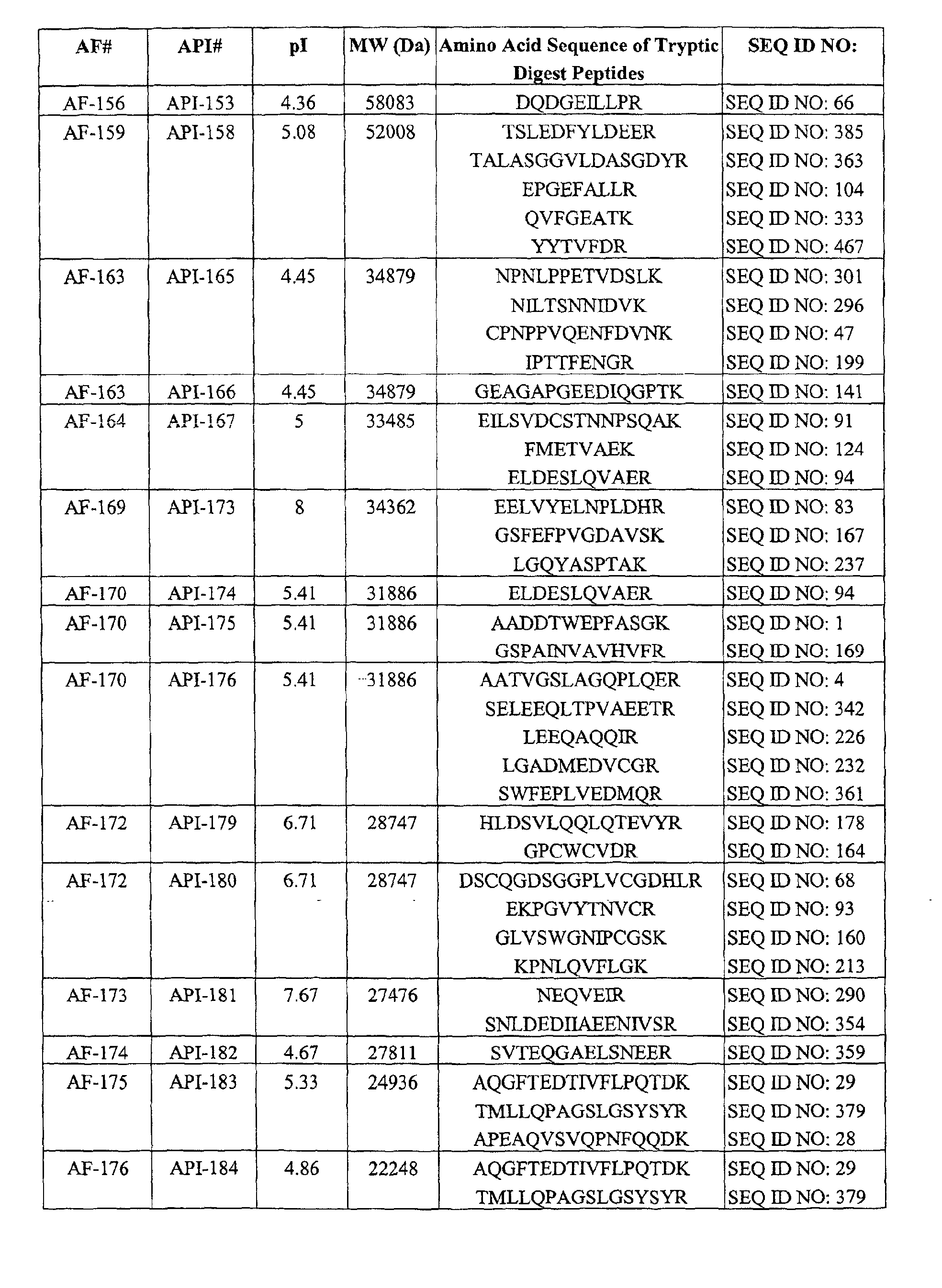

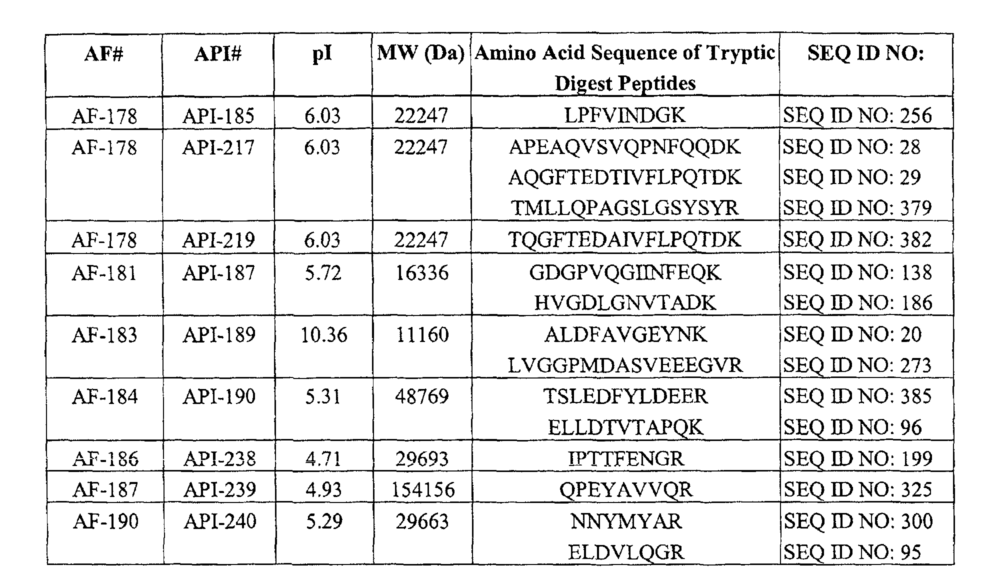

The second group comprises APIs that are increased in the CSF of subjects having AD as compared with the CSF of subjects free from AD. The amino acid sequences of peptides produced from these APIs by proteolysis using trypsin and identified by tandem mass spectrometry and database searching using the SEQUEST program are listed in Table V, in addition to their corresponding pIs and MWs.

### Table V. APIs Increased in CSF of Subjects Having AD

### (a) APIs Increased in CSF of Subjects Having AD

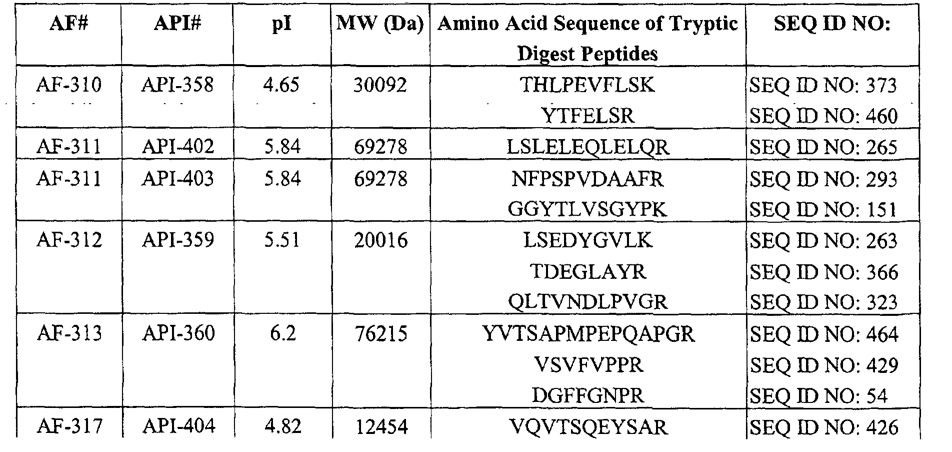

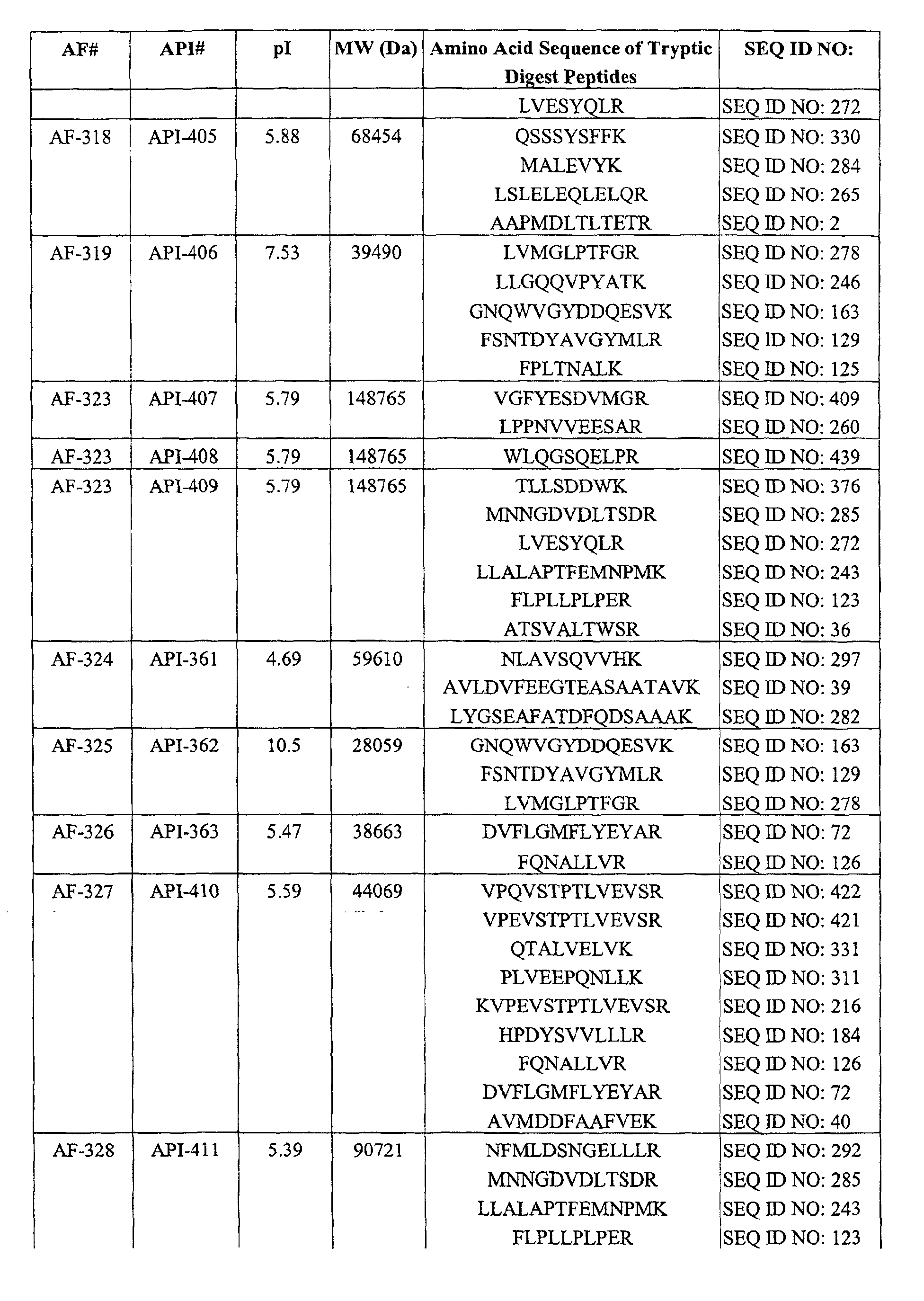

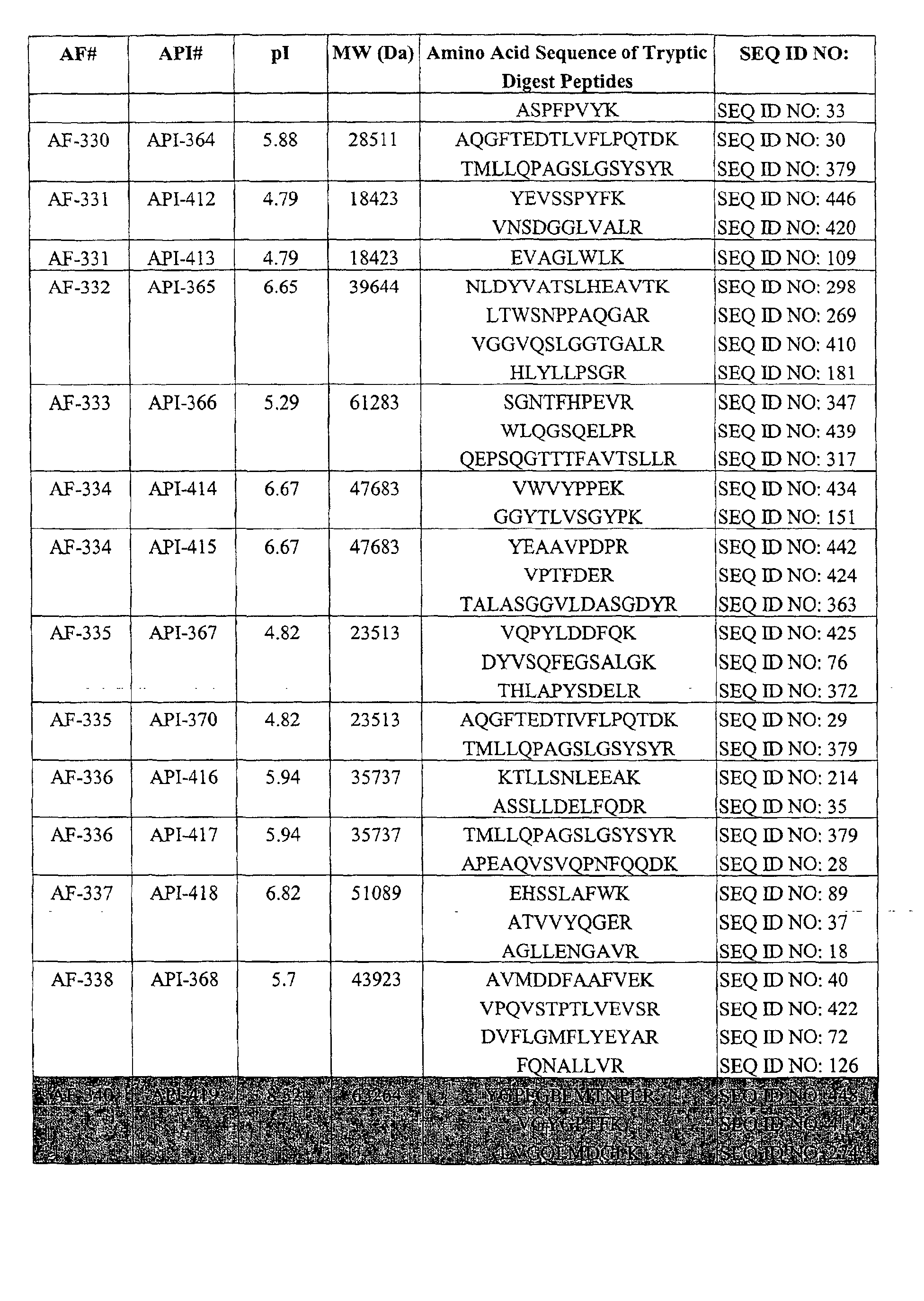

### (b) APIs which can be used in combination with the APIs in Table IV (a) and Table V (a)

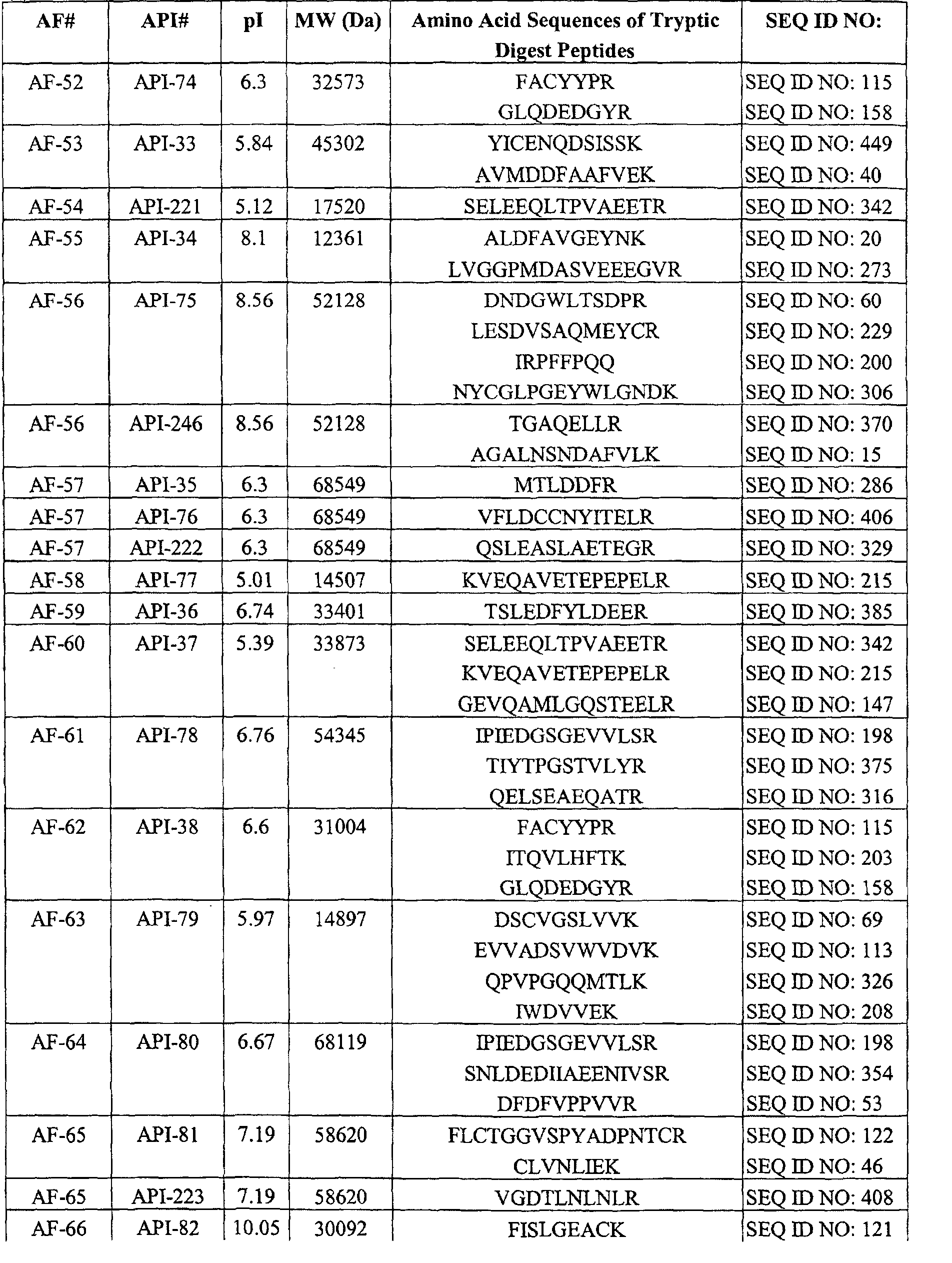

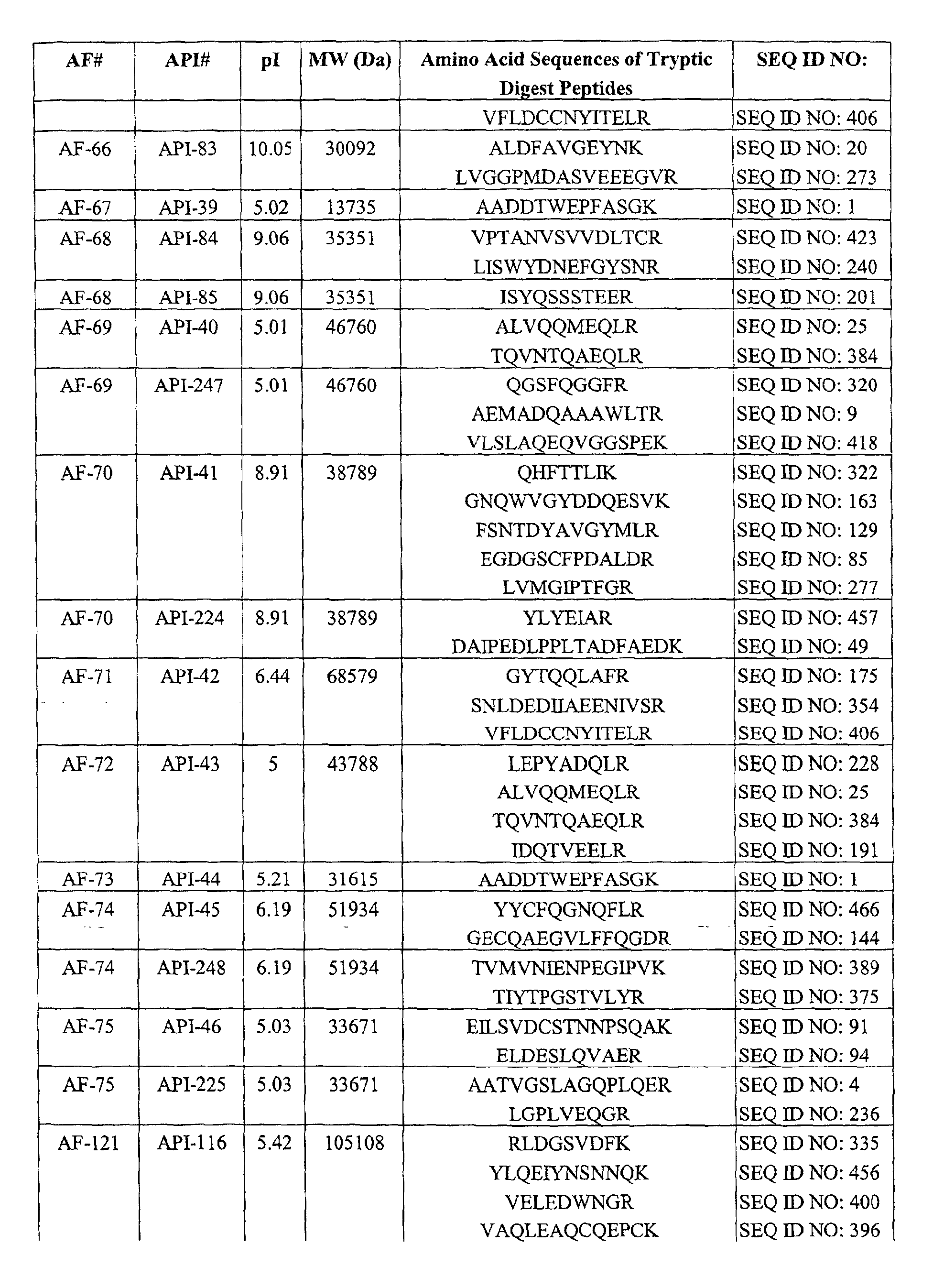

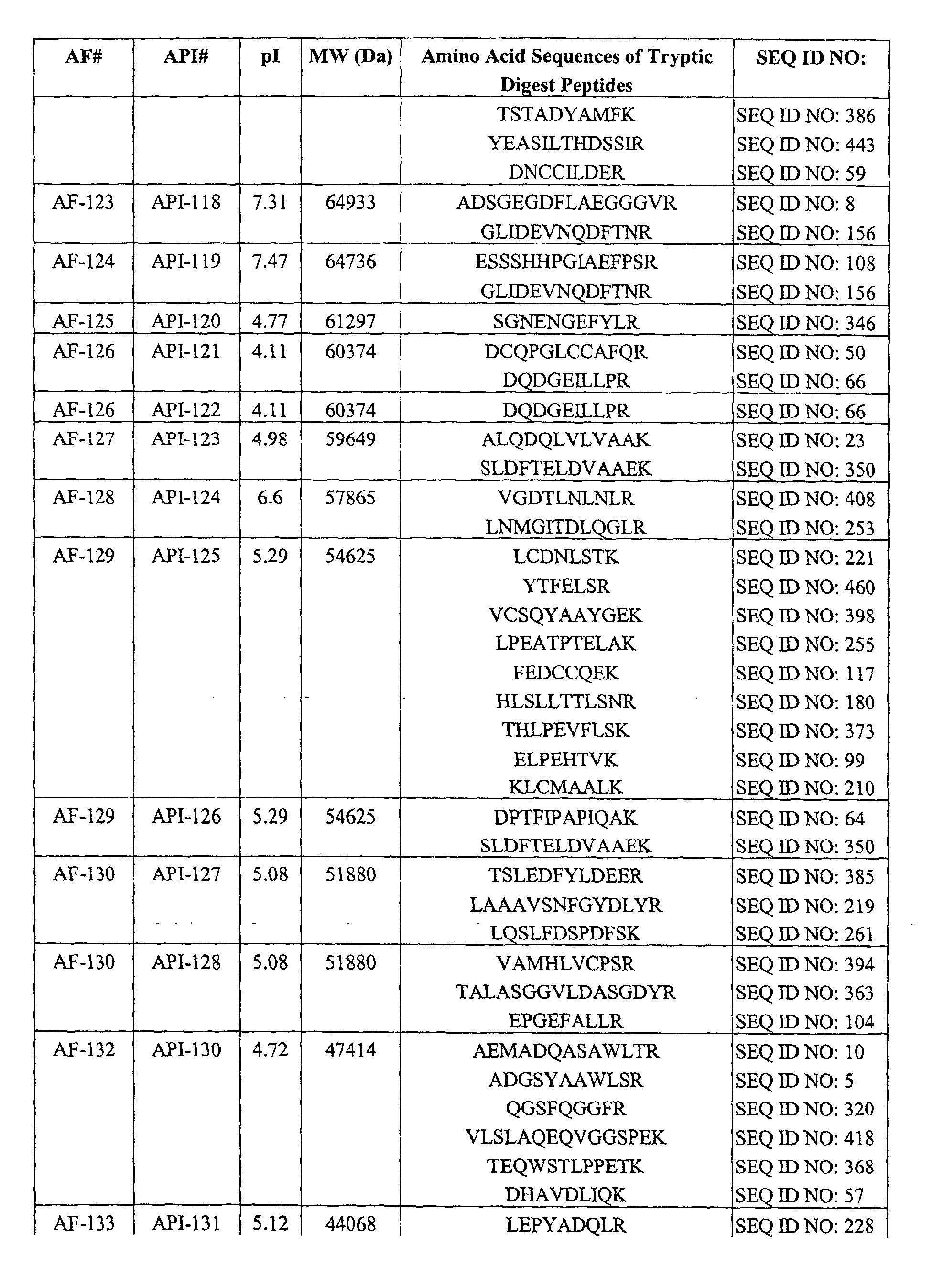

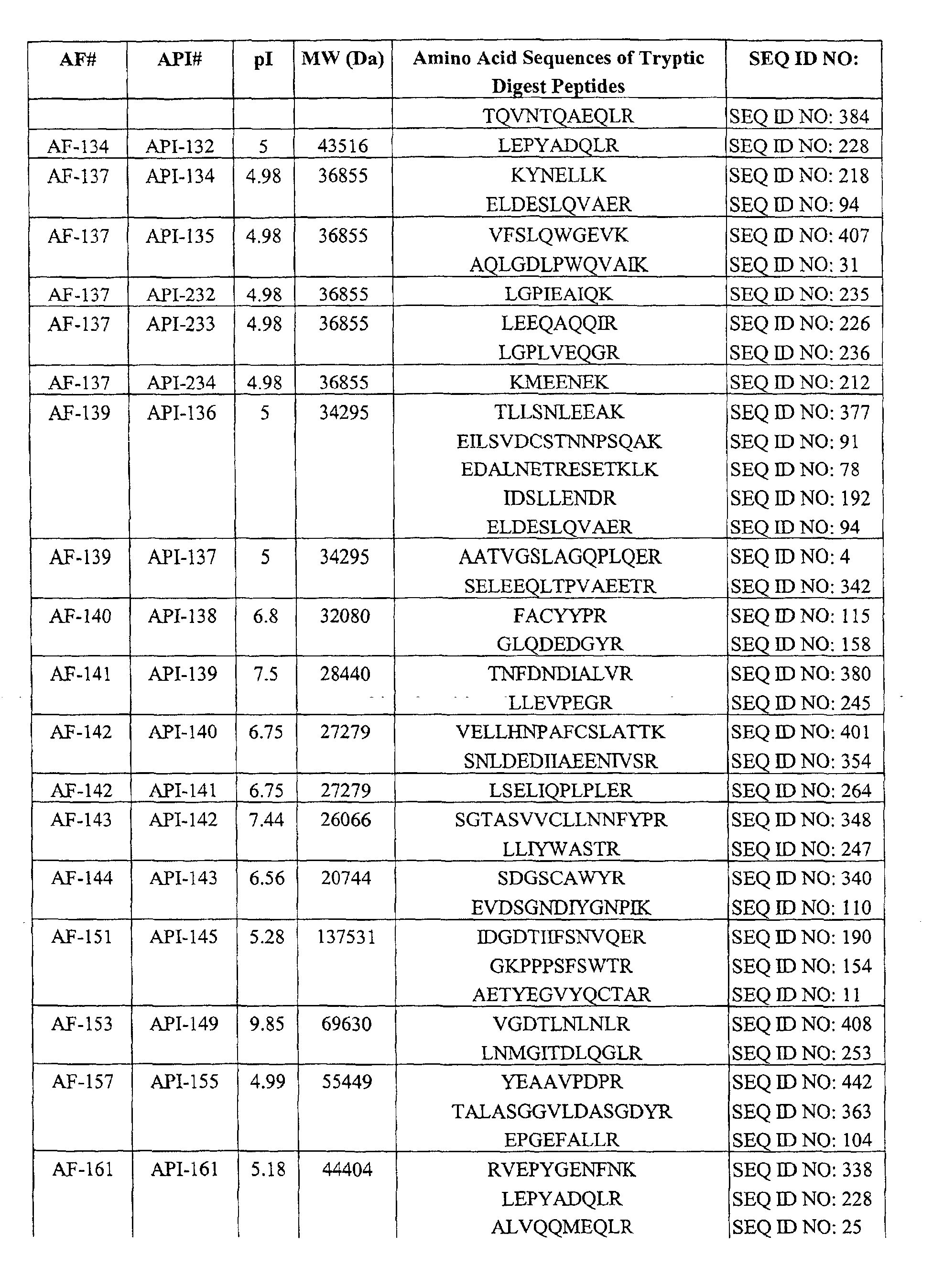

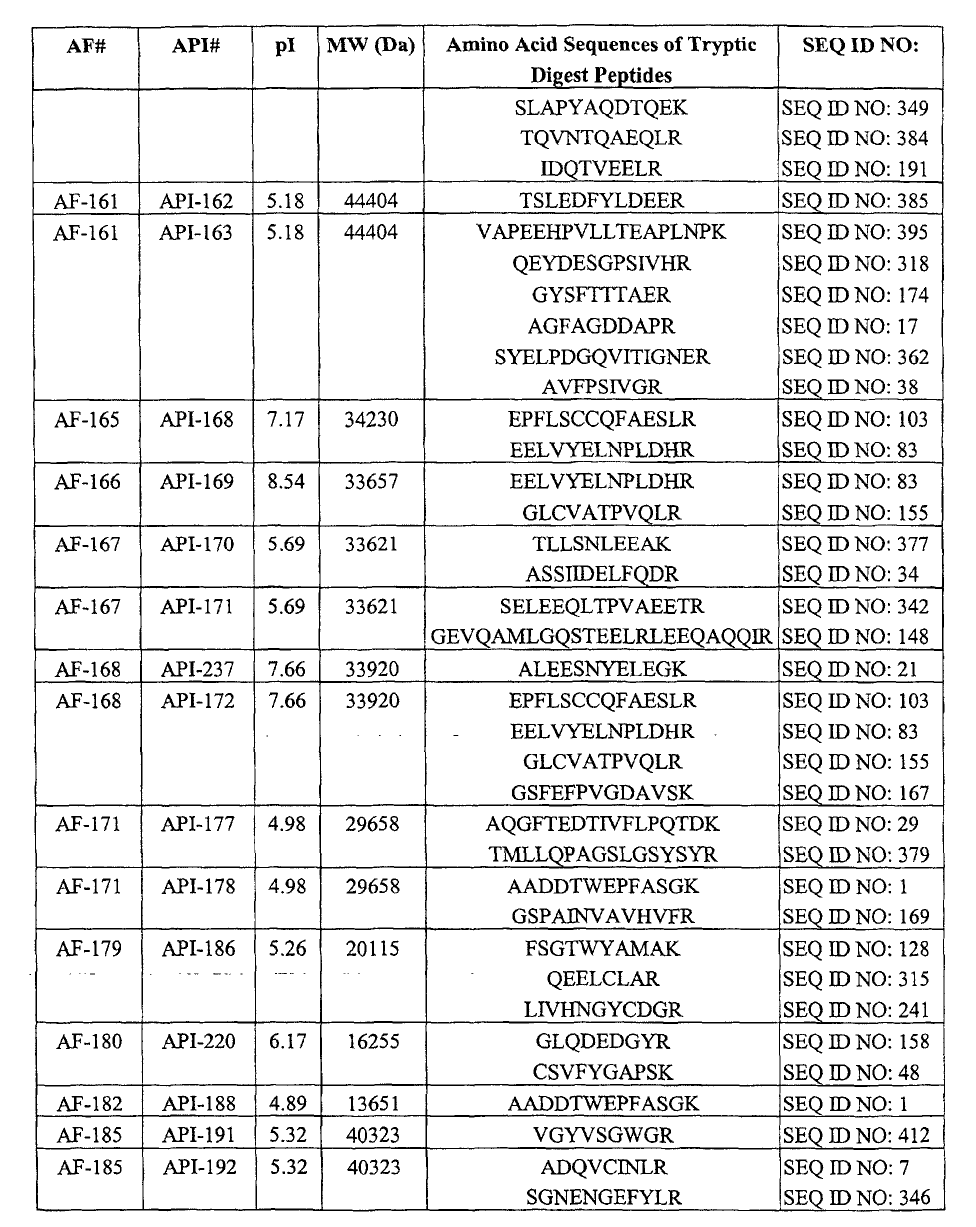

Those skilled in the art will understand, based upon the present description, that a given API can be described according to the data provided for that API in Table IV or V. The API is a protein comprising a peptide sequence described for that API (preferably comprising a plurality of, more preferably all of, the peptide sequences described for that API) and has a pI of about the value stated for that API (preferably within about 10%, more preferably within about 5% still more preferably within about 1% of the stated value) and has a MW of about the value stated for that API (preferably within about 10%, more preferably within about 5%, still more preferably within about 1% of the stated value).

In one embodiment, CSF from a subject is analyzed for quantitative detection of one or more of the APIs as defined in Table N(a), optionally combined with one or more of the APIs as defined in Table IV (b): wherein a decreased abundance of the API or APIs (or any suitable combination of them) in the CSF from the subject relative to CSF from a subject or subjects free from AD (*e.g.*, a control sample or a previously determined reference range) indicates the presence of AD. In another embodiment of the invention, CSF from a subject is analyzed for quantitative detection of one or more of the APIs as defined in Table V(a), optionally combined with the APIs as defined in Table V(b), wherein an increased abundance of the API or APIs (or any suitable combination of them) in CSF from the subject relative to CSF from a subject or subjects free from AD (*e.g.*, a control sample or a previously determined reference range) indicates the presence of AD.

In a further embodiment, CSF from a subject is analyzed for quantitative detection of (a) one or more APIs, or any suitable combination of them, whose decreased abundance indicates the presence of AD, *i.e.*, preferably the APIs set forth in Table IV (a), optionally combined with the APIs set forth in Table IV (b); and (b) one or more APIs, or any suitable combination of them, whose increased abundance indicates the presence of AD, *i.e.*, preferably the APIs set forth in Table V (a), optionally combined with the APIs as set forth in Table V (b).

In yet a further embodiment, CSF from a subject is analyzed for quantitative detection of one or more APIs and one or more previously known biomarkers of AD (*e.g.*, tau, NTP, Aβ). In accordance with this embodiment, the abundance of each API and known biomarker relative to a control or reference range indicates whether a subject has AD.

Preferably, the abundance of an API is normalized to an Expression Reference Protein Isoform (ERPI). ERPIs can be identified by partial amino acid sequence characterization of ERFs, which are described above, and which may be accomplished using e.g. the methods and apparatus of the Preferred Technology. The partial amino acid sequences of an ERPI are presented in Table VI.

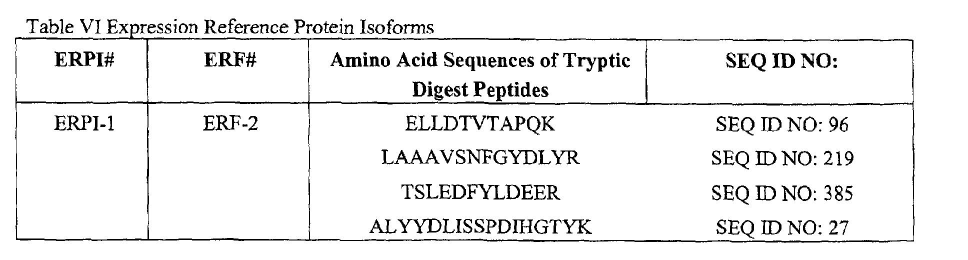

As shown above, the APIs described herein include previously unknown proteins, as well as isoforms of known proteins where the isoforms were not previously known to be associated with AD. For each API, the present invention additionally provides: (a) a preparation comprising the isolated API; (b) a preparation comprising one or more fragments of an API; and (c) antibodies that bind to said API, to said fragments, or both to said API and to said fragments. As used herein, an API is "isolated" when it is present in a preparation that is substantially free of other proteins, *i.e.*, a preparation in which less than 10% (particularly less than 5%, more particularly less than 1%) of the total protein present is contaminating protein(s). Another protein is a protein or protein isoform having a significantly different pI or MW from those of the isolated API, as determined by 2D electrophoresis. As used herein, a "significantly different" pI or MW is one that permits the other protein to be resolved from the API on 2D electrophoresis, performed according to the Reference Protocol.

In one embodiment, an isolated protein is provided, that comprises a peptide with the amino acid sequence identified in Table IV or V for an API, said protein having a pI and MW within 10% (particularly within 5%, more particularly within 1%) of the values identified in Table IV or V for that API.

The APIs of the invention can be qualitatively or quantitatively detected by any method known to those skilled in the art, including but not limited to the Preferred Technology described herein, kinase assays, enzyme assays, binding assays and other functional assays, immunoassays, and western blotting. In one embodiment, the APIs are separated on a 2-D gel by virtue of their MWs and pIs and are visualized by staining the gel. In one embodiment, the APIs are stained with a fluorescent dye and imaged with a fluorescence scanner. Sypro Red (Molecular Probes, Inc., Eugene, Oregon) is a suitable dye for this purpose. A preferred fluorescent dye is Pyridinium, 4-[2-[4-(dipentylamino)-2-trifluoromethylphenyl] ethenyl]-1-(sulfobutyl)-, inner salt. See U.S. 6,335,446.

Alternatively, APIs can be detected in an immunoassay. In one embodiment, an immunoassay is performed by contacting a sample with an anti-API capture reagent, e.g. an antibody under conditions such that immunospecific binding can occur if the API is present, and detecting or measuring the amount of any immunospecific binding by the capture reagent, e.g. an antibody. Anti-API antibodies can be produced by the methods and techniques described herein; examples of such antibodies known in the art are set forth in Table VII. These antibodies shown in Table VII are already known to bind to the protein of which the API is itself a family member. Particularly, the anti-API antibody preferentially binds to the API rather than to other isoforms of the same protein. In a particular embodiment, the anti-API antibody binds to the API with at least 2-fold greater affinity, more particularly at least 5-fold greater affinity, still more particularly at least 10-fold greater affinity, than to said other isoforms of the same protein. When the antibodies shown in Table VII do not display the required preferential selectivity for the target API, one skilled in the art can generate additional antibodies by using the API itself for the generation of such antibodies

APIs can be transferred from a gel to a suitable membrane (*e.g.* a PVDF membrane) and subsequently probed in suitable assays that include, without limitation, competitive and non-competitive assay systems using techniques such as western blots and "sandwich" immunoassays using anti-API antibodies as described herein, *e.g.*, the antibodies identified in Table VII, or others raised against the APIs of interest as those skilled in the art will appreciate based on the present description. The immunoblots can be used to identify those anti-API antibodies displaying the selectivity required to immuno-specifically differentiate an API from other isoforms encoded by the same gene.

### Table VII. Known Antibodies That Recognize APIs or API-Related Polypeptides

**Table VII**

| **API #** | **Antibody** | **Manufacturer** | **Cat. No.** |
|---|---|---|---|
| API-1 | Chromogranin A | BIODESIGN INTERNATIONAL | M54219M |
| API-3 | ANTI-Human CD56 ANTIGEN (NEURAL CELL ADHESION MOLECULE) | RDI RESEARCH DIAGNOSTICS, INC | RDI-CBL159 |
| API-4 | Gel | DAKO-1998 CATALOGUE | A0033 |
| API-6 | ANTI-Human CD56 ANTIGEN (NEURAL CELL ADHESION MOLECULE) | RDI RESEARCH DIAGNOSTICS, INC | RDI-CBL159 |
| API-7 | Apolipoprotein D, Clone: 36C6, Mab anti-Human, paraffin, IH/WB | ACCURATE CHEMICAL & SCIENTIFIC CORPORATION | MED- CLA457 |
| API-10 | Goat anti-Clusterin (human) | RDI RESEARCH DIAGNOSTICS, INC | RDI-CLUSTRCabG |
| API-15 | Monoclonal mouse anti-human IgAl | RDI RESEARCH DIAGNOSTICS, INC | RDI-TRK1A2-2B5 |
| API-16 | Monoclonal anti Human Collagen Type VI | BIODESIGN INTERNATIONAL | M22090M |
| API-22 | RABBIT anti-human INSULIN GROWTH FACTOR BINDING PROTEIN 2 | RDI RESEARCH DIAGNOSTICS, INC | RDI-IGFBP2abr |
| API-28 | Goat anti-Clusterin (human) | RDI RESEARCH DIAGNOSTICS, INC | RDI-CLUSTRCabG |
| API-30 | Lactic Dehydrogenase (LDH) (H-subunit), Clone: HH-17, Mab anti-Human | ACCURATE CHEMICAL & SCIENTIFIC CORPORATION | BYA- 6019-1 |
| API-33 | Albumin, Human, Chicken anti- | ACCURATE CHEMICAL & SCIENTIFIC CORPORATION | IMS-01-026-02 |
| API-34 | Cystatin C, Rabbit anti-Human | ACCURATE CHEMICAL & SCIENTIFIC CORPORATION | AXL- 574 |
| API-37 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-38 | C4 Complement, Chicken anti-Human | ACCURATE CHEMICAL & SCIENTIFIC CORPORATION | IMS-01-032-02 |
| API-39 | Transthyretin, Prealbumin, 55kD, Rabbit anti-Human | ACCURATE CHEMICAL & SCIENTIFIC CORPORATION | MED- CLA 193 |
| API-40 | Apolipoprotein A1 (HDL), Plasminogen absorbed, Sheep anti-Human | ACCURATE CHEMICAL & SCIENTIFIC CORPORATION | ACL- 20076A |
| API-42 | C3 Complement, Chicken anti-Human | ACCURATE CHEMICAL & SCIENTIFIC CORPORATION | IMS-01-001-02 |
| API-43 | Apolipoprotein A1 (HDL), Plasminogen absorbed, Sheep anti-Human | ACCURATE CHEMICAL & SCIENTIFIC CORPORATION | ACL- 20076A |
| API-44 | Transthyretin, Prealbumin, 55kD, Rabbit anti-Human | ACCURATE CHEMICAL & SCIENTIFIC CORPORATION | MED- CLA 193 |
| API-45 | Hemopexin, Beta-1, Rabbit anti-Human, precipitating | ACCURATE CHEMICAL & SCIENTIFIC CORPORATION | YN- RHHPX |
| API-46 | Goat anti-Clusterin (human) | RDI RESEARCH DIAGNOSTICS, INC | RDI-CLUSTRCabG |
| API-47 | ANTI-Human CD56 ANTIGEN (NEURAL CELL ADHESION MOLECULE) | RDI RESEARCH DIAGNOSTICS, INC | RDI-CBL159 |
| API-50 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-52 | Alpha-1-Antichymotrypsin, Rabbit anti-Human | ACCURATE CHEMICAL & SCIENTIFIC CORPORATION | AXL- 145/2 |
| API-53 | Goat anti-Clusterin (human) | RDI RESEARCH DIAGNOSTICS, INC | RDI-CLUSTRCabG |
| API-55 | Monoclonal anti-Neuron Specific Enolase | BIODESIGN INTERNATIONAL | M37403M |
| API-58 | ANTI-Human CD56 ANTIGEN (NEURAL CELL ADHESION MOLECULE) | RDI RESEARCH DIAGNOSTICS, INC | RDI-CBL159 |
| API-60 | Gelsolin, plasma + cytoplasmic, Sheep anti- | ACCURATE CHEMICAL & SCIENTIFIC CORPORATION | YBG- 4628-6210 |
| API-62 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-64 | C4 Complement, Chicken anti-Human | ACCURATE CHEMICAL & SCIENTIFIC CORPORATION | IMS-01-032-02 |
| API-66 | Retinol Binding Protein, Rabbit anti-Human | ACCURATE CHEMICAL & SCIENTIFIC CORPORATION | AXL- 163/2 |
| API-67 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-69 | Complement Factor B, C3 proactivator, Rabbit anti-Human | ACCURATE CHEMICAL & SCIENTIFIC CORPORATION | AXL- 466/2 |
| API-72 | Gel | DAKO-1998 CATALOGUE | A0475 |
| API-74 | C4 Complement, Chicken anti-Human | ACCURATE CHEMICAL & SCIENTIFIC CORPORATION | IMS-01-032-02 |
| API-75 | Fibrinogen, Fibrin I, B-beta chain (Bβ 1-42), Clone: 18C6, Mab anti-Human | ACCURATE CHEMICAL & SCIENTIFIC CORPORATION | NYB- 18C6 |
| API-76 | C3 Complement, Chicken anti-Human | ACCURATE CHEMICAL & SCIENTIFIC CORPORATION | IMS-01-001-02 |
| API-77 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-78 | C3 Complement, Chicken anti-Human | ACCURATE CHEMICAL & SCIENTIFIC CORPORATION | IMS-01-001-02 |
| API-79 | C3 Complement, Chicken anti-Human | ACCURATE CHEMICAL & SCIENTIFIC CORPORATION | IMS-01-001-02 |
| API-80 | C3 Complement, Chicken anti-Human | ACCURATE CHEMICAL & SCIENTIFIC CORPORATION | IMS-01-001-02 |
| API-81 | Complement Factor B, C3 proactivator, Rabbit anti-Human | ACCURATE CHEMICAL & SCIENTIFIC CORPORATION | AXL- 466/2 |
| API-82 | C3 Complement, Chicken anti-Human | ACCURATE CHEMICAL & SCIENTIFIC CORPORATION | IMS-01-001-02 |
| API-84 | Glyceraldehyde-3-Phosphate Dehydrogenase | BIODESIGN INTERNATIONAL | H86504M |
| API-90 | Monoclonal mouse anti-lactoferrin | RDI RESEARCH DIAGNOSTICS, INC | RDI-TRK4L2-LF2B8 |
| API-92 | C3 Complement, Chicken anti-Human | ACCURATE CHEMICAL & SCIENTIFIC CORPORATION | IMS- 01-001-02 |
| API-93 | Monoclonal mouse anti-lactoferrin | RDI RESEARCH DIAGNOSTICS, INC | RDI-TRK4L2-LF2B8 |
| API-95 | Albumin, Human, Chicken anti- | ACCURATE CHEMICAL & SCIENTIFIC CORPORATION | IMS- 01-026-02 |
| API-97 | C3 Complement, Chicken anti-Human | ACCURATE CHEMICAL & SCIENTIFIC CORPORATION | IMS-01-001-02 |
| API-98 | C8 Complement, Goat anti-Human | ACCURATE CHEMICAL & SCIENTIFIC CORPORATION | BMD- G35 |
| API-101 | Albumin, Human, Chicken anti- | ACCURATE CHEMICAL & SCIENTIFIC CORPORATION | IMS-01-026-02 |
| API-102 | Factor H (Complement), Chicken anti-Human | ACCURATE CHEMICAL & SCIENTIFIC CORPORATION | IMS-01-066-02 |
| API-103 | Goat anti-Haptoglobin | BIODESIGN INTERNATIONAL | L15320G |
| API-104 | Transthyretin, Prealbumin, 55kD, Rabbit anti-Human | ACCURATE CHEMICAL & SCIENTIFIC CORPORATION | MED- CLA 193 |
| API-113 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-118 | Monoclonal anti-human Fibrinogen | BIODESIGN INTERNATIONAL | N77190M |
| API-119 | Monoclonal anti-human Fibrinogen | BIODESIGN INTERNATIONAL | N77190M |
| API-123 | AT1 (306) | SANTA CRUZ BIOTECHNOLOGY, INC -RESEARCH ANTIBODIES 98/99 | sc-579 |
| API-124 | C4 Complement, Chicken anti-Human | ACCURATE CHEMICAL & SCIENTIFIC CORPORATION | IMS-01-032-02 |
| API-126 | AT1 (306) | SANTA CRUZ BIOTECHNOLOGY, INC -RESEARCH ANTIBODIES 98/99 | sc-579 |
| API-130 | C4 Complement, Chicken anti-Human | ACCURATE CHEMICAL & SCIENTIFIC CORPORATION | IMS-01-032-02 |
| API-131 | Apolipoprotein A1 (HDL), Plasminogen absorbed, Sheep anti-Human | ACCURATE CHEMICAL & SCIENTIFIC CORPORATION | ACL- 20076A |
| API-132 | Apolipoprotein A1 (HDL), Plasminogen absorbed, Sheep anti-Human | ACCURATE CHEMICAL & SCIENTIFIC CORPORATION | ACL- 20076A |
| API-134 | Goat anti-Clusterin (human) | RDI RESEARCH DIAGNOSTICS, INC | RDI-CLUSTRCabG |
| API-136 | Goat anti-Clusterin (human) | RDI RESEARCH DIAGNOSTICS, INC | RDI-CLUSTRCabG |
| API-137 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-138 | C4 Complement, Chicken anti-Human | ACCURATE CHEMICAL & SCIENTIFIC CORPORATION | IMS-01-032-02 |
| API-140 | C3 Complement, Chicken anti-Human | ACCURATE CHEMICAL & SCIENTIFIC CORPORATION | IMS-01-001-02 |
| API-142 | Kappa Chain, Mab anti-Human | ACCURATE CHEMICAL & SCIENTIFIC CORPORATION | BMD- 021D |
| API-143 | Tissue Inhibitor of Matrix Metalloproteinase 2 (TIMP2) (NO X w/TIMP1), Clone: 3A4, Mab anti-Human, paraffin, IH | ACCURATE CHEMICAL & SCIENTIFIC CORPORATION | MED- CLA498 |
| API-145 | ANTI-Human CD56 ANTIGEN (NEURAL CELL ADHESION MOLECULE) | RDI RESEARCH DIAGNOSTICS, INC | RDI-CBL159 |
| API-149 | C4 Complement, Chicken anti-Human | ACCURATE CHEMICAL & SCIENTIFIC CORPORATION | IMS-01-032-02 |
| API-150 | Sheep anti-Alpha 2 Antiplasmin | BIODESIGN INTERNATIONAL | K90038C |
| API-161 | Apolipoprotein A1 (HDL), Plasminogen absorbed, Sheep anti-Human | ACCURATE CHEMICAL & SCIENTIFIC CORPORATION | ACL- 20076A |
| API-165 | Apolipoprotein D, Clone: 36C6, Mab anti-Human, paraffin, IH/WB | ACCURATE CHEMICAL & SCIENTIFIC CORPORATION | MED- CLA457 |
| API-167 | Goat anti-Clusterin (human) | RDI RESEARCH DIAGNOSTICS, INC | RDI-CLUSTRCabG |
| API-168 | C4 Complement, Chicken anti-Human | ACCURATE CHEMICAL & SCIENTIFIC CORPORATION | IMS-01-032-02 |
| API-169 | C4 Complement, Chicken anti-Human | ACCURATE CHEMICAL & SCIENTIFIC CORPORATION | IMS-01-032-02 |
| API-170 | Goat anti-Clusterin (human) | RDI RESEARCH DIAGNOSTICS, INC | RDI-CLUSTRCabG |
| API-171 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-172 | C4 Complement, Chicken anti-Human | ACCURATE CHEMICAL & SCIENTIFIC CORPORATION | IMS-01-032-02 |
| API-173 | C4 Complement, Chicken anti-Human | ACCURATE CHEMICAL & SCIENTIFIC CORPORATION | IMS-01-032-02 |
| API-174 | Goat anti-Clusterin (human) | RDI RESEARCH DIAGNOSTICS, INC | RDI-CLUSTRCabG |
| API-175 | Transthyretin, Prealbumin, 55kD, Rabbit anti-Human | ACCURATE CHEMICAL & SCIENTIFIC CORPORATION | MED- CLA 193 |
| API-176 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-178 | Transthyretin, Prealbumin, 55kD, Rabbit anti-Human | ACCURATE CHEMICAL & SCIENTIFIC CORPORATION | MED- CLA 193 |
| API-179 | IGFBP6 (M-20) | SANTA CRUZ BIOTECHNOLOGY, INC -RESEARCH ANTIBODIES 98/99 | sc-6008 |
| API-181 | C3 Complement, Chicken anti-Human | ACCURATE CHEMICAL & SCIENTIFIC CORPORATION | IMS-01-001-02 |
| API-182 | Rabbit anti-14-3-38 (Broadly Reactive) | RDI RESEARCH DIAGNOSTICS, INC | RDI-1433BNabr |
| API-186 | Retinol Binding Protein, Rabbit anti-Human | ACCURATE CHEMICAL & SCIENTIFIC CORPORATION | AXL- 163/2 |
| API-187 | Anti-Superoxide Dismutase (Cu/Zn-SOD) IgG fraction (POLYCLONAL) | RDI RESEARCH DIAGNOSTICS, INC | RDI-SODabg |
| API-188 | Transthyretin, Prealbumin, 55kD, Rabbit anti-Human | ACCURATE CHEMICAL & SCIENTIFIC CORPORATION | MED- CLA 193 |
| API-189 | Cystatin C, Rabbit anti-Human | ACCURATE CHEMICAL & SCIENTIFIC CORPORATION | AXL-574 |
| API-191 | Goat anti-Haptoglobin | BIODESIGN INTERNATIONAL | L15320G |
| API-194 | ANTI-Human CD56 ANTIGEN (NEURAL CELL ADHESION MOLECULE) | RDI RESEARCH DIAGNOSTICS, INC | RDI-CBL159 |
| API-196 | Cystatin C, Rabbit anti-Human | ACCURATE CHEMICAL & SCIENTIFIC CORPORATION | AXL-574 |
| API-201 | Gel | DAKO - 1998 CATALOGUE | A0033 |
| API-215 | Cystatin C, Rabbit anti-Human | ACCURATE CHEMICAL & SCIENTIFIC CORPORATION | AXL- 574 |
| API-220 | C4 Complement, Chicken anti-Human | ACCURATE CHEMICAL & SCIENTIFIC CORPORATION | IMS-01-032-02 |
| API-221 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-223 | C4 Complement, Chicken anti-Human | ACCURATE CHEMICAL & SCIENTIFIC CORPORATION | IMS-01-032-02 |
| API-225 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-233 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-238 | Apolipoprotein D, Clone: 36C6, Mab anti-Human, paraffin, IH/WB | ACCURATE CHEMICAL & SCIENTIFIC CORPORATION | MED- CLA457 |
| API-239 | ANTI-Human CD56 ANTIGEN (NEURAL CELL ADHESION MOLECULE) | RDI RESEARCH DIAGNOSTICS, INC | RDI-CBL159 |
| API-300 | C4 Complement, Chicken anti-Human | ACCURATE CHEMICAL & SCIENTIFIC CORPORATION | IMS-01-032-02 |
| API-301 | rabbit anti-human alpha-2-macroglobulin | Cambio Ltd. | CA-0427 |
| API-303 | Anti-prostaglandin D synthase/Prostaglandin H2-D isomerase | Oxford Biomedical Research | PD-01 |
| API-305 | C4 Complement, Chicken anti-Human | ACCURATE CHEMICAL & SCIENTIFIC CORPORATION | IMS-01-032-02 |
| API-308 | Gelsolin, plasma + cytoplasmic, Sheep anti- | ACCURATE CHEMICAL & SCIENTIFIC CORPORATION | YBG- 4628-6210 |
| API-309 | rabbit anti-human alpha-2-macroglobulin | Cambio Ltd. | CA-0427 |
| API-310 | rabbit anti-human alpha-2-macroglobulin | Cambio Ltd. | CA-0427 |
| API-311 | C4 Complement, Chicken anti-Human | ACCURATE CHEMICAL & SCIENTIFIC CORPORATION | IMS-01-032-02 |
| API-312 | Gelsolin, plasma + cytoplasmic, Sheep anti- | ACCURATE CHEMICAL & SCIENTIFIC CORPORATION | YBG- 4628-6210 |
| API-313 | Mouse anti-Human pigment epithelium-derived factor (PEDF) (monoclonal) Clone 10F12.2 | Chemicon International | MAB1059 |
| API-314 | C3 Complement, Chicken anti-Human | ACCURATE CHEMICAL & SCIENTIFIC CORPORATION | IMS-01-001-02 |
| API-315 | C4 Complement, Chicken anti-Human | ACCURATE CHEMICAL & SCIENTIFIC CORPORATION | IMS-01-032-02 |
| API-316 | Anti-prostaglandin D synthase/Prostaglandin H2-D isomerase | Oxford Biomedical Research | PD-01 |
| API-318 | rabbit anti-human alpha-2-macroglobulin | Cambio Ltd. | CA-0427 |
| API-320 | Chromogranin A | BIODESIGN INTERNATIONAL | M54219M |
| API-322 | Retinol Binding Protein, Rabbit anti-Human | ACCURATE CHEMICAL & SCIENTIFIC CORPORATION | AXL-163/2 |
| API-323 | Gelsolin, plasma + cytoplasmic, Sheep anti- | ACCURATE CHEMICAL & SCIENTIFIC CORPORATION | YBG- 4628-6210 |
| API-325 | rabbit anti-human alpha-2-macroglobulin | Cambio Ltd. | CA-0427 |
| API-326 | Apolipoprotein A1 (HDL), Sheep anti-Human | ACCURATE CHEMICAL & SCIENTIFIC CORPORATION | ACL- 20075AP |
| API-327 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-330 | C3 Complement, Chicken anti-Human | ACCURATE CHEMICAL & SCIENTIFIC CORPORATION | IMS-01-001-02 |
| API-332 | Chromogranin A | BIODESIGN INTERNATIONAL | M54219M |
| API-335 | Apolipoprotein A1 (HDL), Sheep anti-Human | ACCURATE CHEMICAL & SCIENTIFIC CORPORATION | ACL- 20075AP |
| API-336 | Anti-prostaglandin D synthase/Prostaglandin H2-D isomerase | Oxford Biomedical Research | PD-01 |
| API-337 | rabbit anti-human alpha-2-macroglobulin | Cambio Ltd. | CA-0427 |
| API-339 | Anti-prostaglandin D synthase/Prostaglandin H2-D isomerase | Oxford Biomedical Research | PD-01 |
| API-340 | Apolipoprotein E, LDL, VLDL, Clone: 3D12, Mab anti-Human, frozen/paraffin | ACCURATE CHEMICAL & SCIENTIFIC CORPORATION | YM- 5029 |
| API-341 | Anti-prostaglandin D synthase/Prostaglandin H2-D isomerase | Oxford Biomedical Research | PD-01 |
| API-346 | Anti-prostaglandin D synthase/Prostaglandin H2-D isomerase | Oxford Biomedical Research | PD-01 |
| API-348 | Cystatin C, Rabbit anti-Human | ACCURATE CHEMICAL & SCIENTIFIC CORPORATION | AXL- 574 |
| API-349 | rabbit anti-human alpha-2-macroglobulin | Cambio Ltd. | CA-0427 |
| API-351 | Apolipoprotein A1 (HDL), Sheep anti-Human | ACCURATE CHEMICAL & SCIENTIFIC CORPORATION | ACL- 20075AP |
| API-353 | C7 Complement, Goat anti-Human | ACCURATE CHEMICAL & SCIENTIFIC CORPORATION | BMD- G34 |
| API-355 | Apolipoprotein D, Clone: 36C6, Mab anti-Human, paraffin, IH/WB | ACCURATE CHEMICAL & SCIENTIFIC CORPORATION | MED- CLA457 |
| API-356 | C4 Complement, Chicken anti-Human | ACCURATE CHEMICAL & SCIENTIFIC CORPORATION | IMS-01-032-02 |
| API-361 | Alpha-1-Antichymotrypsin, Rabbit anti-Human | ACCURATE CHEMICAL & SCIENTIFIC CORPORATION | AXL-145/2 |
| API-363 | Albumin, Human, Chicken anti- | ACCURATE CHEMICAL & SCIENTIFIC CORPORATION | IMS-01-026-02 |
| API-364 | Anti-prostaglandin D synthase/Prostaglandin H2-D isomerase | Oxford Biomedical Research | PD-01 |
| API-366 | Monoclonal mouse anti-human IgA1 | RDI RESEARCH DIAGNOSTICS, INC | RDI-TRK1A2-2B5 |
| API-367 | Apolipoprotein A1 (HDL), Sheep anti-Human | ACCURATE CHEMICAL & SCIENTIFIC CORPORATION | ACL- 20075AP |
| API-368 | Albumin, Human, Chicken anti- | ACCURATE CHEMICAL & SCIENTIFIC CORPORATION | IMS-01-026-02 |
| API-369 | Anti-prostaglandin D synthase/Prostaglandin H2-D isomerase | Oxford Biomedical Research | PD-01 |
| API-370 | Anti-prostaglandin D synthase/Prostaglandin H2-D isomerase | Oxford Biomedical Research | PD-01 |
| API-378 | Monoclonal anti-human Fibrinogen | BIODESIGN INTERNATIONAL | N77190M |
| API-380 | Chromogranin A | BIODESIGN INTERNATIONAL | M54219M |
| API-381 | Monoclonal anti-human Fibrinogen | BIODESIGN INTERNATIONAL | N77190M |
| API-382 | C4 Complement, Chicken anti-Human | ACCURATE CHEMICAL & SCIENTIFIC CORPORATION | IMS-01-032-02 |
| API-383 | C3 Complement, Chicken anti-Human | ACCURATE CHEMICAL & SCIENTIFIC CORPORATION | IMS-01-001-02 |
| API-388 | Gelsolin, plasma + cytoplasmic, Sheep anti- | ACCURATE CHEMICAL & SCIENTIFIC CORPORATION | YBG- 4628-6210 |
| API-390 | Albumin, Human, Chicken anti- | ACCURATE CHEMICAL & SCIENTIFIC CORPORATION | IMS-01-026-02 |
| API-391 | Factor H (Complement), Chicken anti-Human | ACCURATE CHEMICAL & SCIENTIFIC CORPORATION | IMS- 01-066-02 |
| API-392 | Gelsolin, plasma + cytoplasmic, Sheep anti- | ACCURATE CHEMICAL & SCIENTIFIC CORPORATION | YBG- 4628-6210 |
| API-393 | rabbit anti-human alpha-2-macroglobulin | Cambio Ltd. | CA-0427 |
| API-394 | Rabbit Polyclonal Anti-Human Ceruloplasmin | DAKO CORPORATION | A0031 |
| API-400 | Goat anti-human Carbonic Anhydrase I (polyclonal) | Abcam Ltd. | ab6618 |
| API-401 | Cystatin C, Rabbit anti-Human | ACCURATE CHEMICAL & SCIENTIFIC CORPORATION | AXL-574 |
| API-403 | Hemopexin, Beta-1, Rabbit anti-Human, precipitating | ACCURATE CHEMICAL & SCIENTIFIC CORPORATION | YN-RHHPX |
| API-407 | rabbit anti-human alpha-2-macroglobulin | Cambio Ltd. | CA-0427 |
| API-408 | Monoclonal mouse anti-human IgA1 | RDI RESEARCH DIAGNOSTICS, INC | RDI-TRK1A2-2B5 |
| API-409 | Anti-Human Contactin, Mouse monoclonal | BD Biosciences | 610579 |
| API-410 | Albumin, Human, Chicken anti- | ACCURATE CHEMICAL & SCIENTIFIC CORPORATION | IMS-01-026-02 |
| API-411 | Anti-Human Contactin, Mouse monoclonal | BD Biosciences | 610579 |
| API-414 | Hemopexin, Beta-1, Rabbit anti-Human, precipitating | ACCURATE CHEMICAL & SCIENTIFIC CORPORATION | YN-RHHPX |
| API-415 | goat anti-Pyruvate Kinase | SANTA CRUZ | sc-7140 |
| | (polyclonal) | BIOTECHNOLOGY, INC -RESEARCH ANTIBODIES 98/99 | |
| API-416 | Goat anti-Clusterin (human) | RDI RESEARCH DIAGNOSTICS, INC | RDI-CLUSTRCabG |
| API-417 | Anti-prostaglandin D synthase/Prostaglandin H2-D isomerase | Oxford Biomedical Research | PD-01 |
| *Further information about these antibodies can be obtained from their commercial sources at: ACCURATE CHEMICAL & SCIENTIFIC CORPORATION http://www.accuratechemical.com/; BIODESIGN INTERNATIONAL -http://www.biodesign.com/; RDI RESEARCH DIAGNOSTICS, INC - http://www.researchd.com/;SANTA CRUZ BIOTECHNOLOGY, INC - http://www.scbt.com/. | | | |

In one embodiment, binding of antibody in tissue sections can be used to detect API localization or the level of one or more APIs. In a specific embodiment, antibody to an API can be used to assay a tissue sample (*e.g.*, a brain biopsy) from a subject for the level of the API where a substantially changed level of API is indicative of AD. As used herein, a "substantially changed level" means a level that is increased or decreased compared with the level in a subject free from AD or a reference level. If desired, the comparison can be performed with a matched sample from the same subject, taken from a portion of the body not affected by AD.

Any suitable immunoassay can be used to detect an API, including, without limitation, competitive and non-competitive assay systems using techniques such as western blots, radioimmunoassays, ELISAs (enzyme linked immunosorbent assays), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, an API can be detected in a fluid sample (*e.g.*, CSF, blood, urine, or tissue homogenate) by means of a two-step sandwich assay. In the first step, a capture reagent (*e.g.*, an anti-API antibody) is used to capture the API. Examples of such antibodies known in the art are set forth in Table VII. The capture reagent can optionally be immobilized on a solid phase. In the second step, a directly or indirectly labeled detection reagent is used to detect the captured API. In one embodiment, the detection reagent is a lectin. A lectin can be used for this purpose that preferentially binds to the API rather than to other isoforms that have the same core protein as the API or to other proteins that share the antigenic determinant recognized by the antibody. In a preferred embodiment, the chosen lectin binds to the API with at least 2-fold greater affinity, more particularly at least 5-fold greater affinity, still more preferably at least 10-fold greater affinity, than to said other isoforms that have the same core protein as the API or to said other proteins that share the antigenic determinant recognized by the antibody. Based on the present description, a lectin that is suitable for detecting a given API can readily be identified by those skilled in the art using methods well known in the art, for instance upon testing one or more lectins enumerated in Table I on pages 158-159 of Sumar et al., Lectins as Indicators of Disease-Associated Glycoforms, In: Gabius H-J & Gabius S (eds.), 1993, Lectins and Glycobiology, at pp. 158-174. Lectins with the desired oligosaccharide specificity can be identified, for example, by their ability to detect the API in a 2D gel, in a replica of a 2D gel following transfer to a suitable solid substrate such as a nitrocellulose membrane, or in a two-step assay following capture by an antibody. In an alternative embodiment, the detection reagent is an antibody, *e.g.*, an antibody that immunospecifically detects other post-translational modifications, such as an antibody that immunospecifically binds to phosphorylated amino acids. Examples of such antibodies include those that bind to phosphotyrosine (BD Transduction Laboratories, catalog nos.: P 11230-050/P11230-150; P11120; P38820; P39020), those that bind to phosphoserine (Zymed Laboratories Inc., South San Francisco, CA, catalog no. 61-8100) and those that bind to phosphothreonine (Zymed Laboratories Inc., South San Francisco, CA, catalog nos. 71-8200, 13-9200).

If desired, a gene encoding an API, a related gene (*e.g.* a gene having sequence homology), or related nucleic acid sequences or subsequences, including complementary sequences, can also be used in hybridization assays. A nucleotide encoding an API, or subsequences thereof comprising at least 8 nucleotides, preferably at least 12 nucleotides, and most preferably at least 15 nucleotides can be used as a hybridization probe. Hybridization assays can be used for detection, treatment, diagnosis, or monitoring of conditions, disorders, or disease states, associated with aberrant expression of genes encoding APIs, or for differential diagnosis of subjects with signs or symptoms suggestive of AD. In particular, such a hybridization assay can be carried out by a method comprising contacting a subject's sample containing nucleic acid with a nucleic acid probe capable of hybridizing to a DNA or RNA that encodes an API, under conditions such that hybridization can occur, and detecting or measuring any resulting hybridization. Nucleotides can be used for therapy of subjects having AD, as described below.

The invention also provides diagnostic kits, comprising an anti-API capture reagent. In addition, such a kit may optionally comprise one or more of the following: (1) instructions for using the anti-API capture reagent for diagnosis, therapeutic monitoring or any suitable combination of these applications; (2) a labeled binding partner to the capture reagent; (3) a solid phase (such as a reagent strip) upon which the anti-API capture reagent is immobilized; and (4) a label or insert indicating regulatory approval for diagnostic, prognostic or therapeutic use or any suitable combination thereof. If no labeled binding partner to the antibody is provided, the anti-API capture reagent itself can be labeled with a detectable marker, *e.g.*, a chemiluminescent, enzymatic, fluorescent, or radioactive moiety.

The invention also provides a kit comprising a nucleic acid probe capable of hybridizing to RNA encoding an API. In a specific embodiment, a kit comprises in one or more containers a pair of primers (*e.g.*, each in the size range of 6-30 nucleotides, more preferably 10-30 nucleotides and still more preferably 10-20 nucleotides) that under appropriate reaction conditions can prime amplification of at least a portion of a nucleic acid encoding an API, such as by polymerase chain reaction (see, *e.g.*, Innis et al., 1990, PCR Protocols, Academic Press, Inc., San Diego, CA), ligase chain reaction (see EP 320,308) use of Qβ replicase, cyclic probe reaction, or other methods known in the art.

Kits are also provided which allow for the detection of a plurality of APIs or a plurality of nucleic acids each encoding an API. A kit can optionally further comprise a predetermined amount of an isolated API protein or a nucleic acid encoding an API, *e.g.*, for use as a standard or control.

### 5.5 Statistical Techniques for Identifying AFs, APIs and API Clusters

Uni-variate differential analysis tools, such as fold changes, wilcoxon rank sum test and t-test, are useful in identifying individual AFs or APIs that are diagnostically associated with AD or in identifying individual APIs that regulate the disease process. However, those skilled in the art will appreciate that the disease process is associated with a suitable combination of AFs or APIs (and to be regulated by a suitable combination of APIs), rather than individual AFs and APIs in isolation. The strategies for discovering such suitable combinations of AFs and APIs differ from those for discovering individual AFs and APIs. In such cases, each individual AF and API can be regarded as one variable and the disease can be regarded as a joint, multi-variate effect caused by interaction of these variables.

The following steps can be used to identify markers from data produced by the Preferred Technology.

The first step is to identify a collection of AFs or APIs that individually show significant association with AD. The association between the identified individual AFs or individual APIs and A need not be as highly significant when a collection of AFs and APIs is used as a diagnostic as is desirable when an individual AF or API is used as a diagnostic. Any of the tests discussed above (fold changes, Wilcoxon rank sum test, etc.) can be used at this stage. Once a suitable collection of AFs or APIs has been identified, a sophisticated multi-variate analysis capable of identifying clusters can then be used to estimate the significant multivariate associations with AD.

Linear Discriminant Analysis (LDA) is one such procedure, which can be used to detect significant association between a cluster of variables (*i.e.*, AFs or APIs) and AD. In performing LDA, a set of weights is associated with each variable (*i.e.*, AF or API) so that the linear combination of weights and the measured values of the variables can identify the disease state by discriminating between subjects having AD and subjects free from AD. Enhancements to the LDA allow stepwise inclusion (or removal) of variables to optimize the discriminant power of the model. The result of the LDA is therefore a cluster of AFs, or APIs, which can be used for diagnosis, treatment or development of pharmaceutical products. Other enhanced variations of LDA, such as Flexible Discriminant Analysis permit the use of non-linear combinations of variables to discriminate a disease state from a state in which there is no disease. The results of the discriminant analysis can be verified by post-hoc tests and also by repeating the analysis using alternative techniques such as classification trees.

A further category of AFs or APIs can be identified by qualitative measures by comparing the percentage feature presence of an AF or API of one group of samples (*e.g.*, samples from diseased subjects) with the percentage feature presence of an AF or API in another group of samples (*e.g.*, samples from control subjects). The "percentage feature presence" of an AF or API is the percentage of samples in a group of samples in which the AF or API is detectable by the detection method of choice. For example, if an AF is detectable in 95 percent of samples from diseased subjects, the percentage feature presence of that AF in that sample group is 95 percent. If only 5 percent of samples from non-diseased subjects have detectable levels of the same AF, detection of that AF in the sample of a subject would suggest that it is likely that the subject has AD.

Similarly, the use of "nearest neighbor analysis" comprises the visual inspection of a particular group or cluster of AFs or APIs and the consequent association of particular such features or isoforms on the basis of their physical closeness and similarity in appearance, to other features or isoforms that are found to demonstrate a significant presence in samples taken from diseased subjects.

### 5.6. Use in Clinical Studies

The diagnostic methods and compositions of the present invention can assist in monitoring a clinical study, *e.g.* to evaluate therapies for AD. In one embodiment, chemical compounds are tested for their ability to restore AF or API levels in a subject having AD to levels found in subjects free from AD or, in a treated subject (*e.g.* after treatment with a cholinesterase inhibitor), to preserve AF or API levels at or near levels seen in subjects free from AD. The levels of one or more AFs or APIs can be assayed.

In another embodiment, the methods and compositions of the present invention are used to screen individuals for entry into a clinical study to identify individuals having AD; individuals already having AD can then be excluded from the study or can be placed in a separate cohort for treatment or analysis. If desired, the candidates can concurrently be screened to identify individuals with Lewy Body disease and/or senile dementia or a known measure of AD; procedures for these screens are well known in the art (Harding and Halliday, 1998, Neuropathol. Appl. Neurobiol. 24:195-201).

### 5.7. Purification of APIs

In particular aspects, the invention provides isolated mammalian APIs, preferably human APIs, API fragments, API -related polypeptides or API -fusion proteins which comprise an antigenic determinant (*i.e.*, can be recognized by an antibody) or which are otherwise functionally active, as well as nucleic acid sequences encoding the foregoing. "Functionally active" as used herein refers to material displaying one or more functional activities associated with a full-length (wild-type) API, *e.g.*, binding to an API substrate or API binding partner, antigenicity (binding to an anti-API antibody), immunogenicity, enzymatic activity and the like.

In specific embodiments, the invention provides fragments of an API comprising at least 5 amino acids, at least 10 amino acids, at least 50 amino acids, or at least 75 amino acids. Fragments lacking some or all of the regions of an API are also provided, as are proteins (*e.g.*, fusion proteins) comprising such fragments. Nucleic acids encoding the foregoing are provided.

Once a recombinant nucleic acid which encodes the API, a portion of the API, or a precursor of the API is identified, the gene product can be analyzed. This can be achieved by assays based on the physical or functional properties of the given product, including, for example, radioactive labeling of the product followed by analysis by gel electrophoresis, immunoassay, etc.

The APIs identified herein can be isolated and purified by standard methods including chromatography (*e.g.*, ion exchange, affmity, and sizing column chromatography), centrifugation, differential solubility, or by any other standard technique for the purification of proteins.

Alternatively, once a recombinant nucleic acid that encodes the API is identified, the entire amino acid sequence of the API can be deduced from the nucleotide sequence of the gene-coding region contained in the recombinant nucleic acid. As a result, the protein can be synthesized by standard chemical methods known in the art (*e.g.*, see Hunkapiller et al., 1984, Nature 310:105-111).

In another alternative embodiment, native APIs can be purified from natural sources, by standard methods such as those described above (*e.g.*, immunoaffinity purification).

In a preferred embodiment, APIs are isolated by the Preferred Technology described supra. For preparative-scale runs, a narrow-range "zoom gel" having a pH range of 2 pH units or less is preferred for the isoelectric step, according to the method described in Westermeier, 1993, Electrophoresis in Practice (VCH, Weinheim, Germany), pp. 197-209 (which is incorporated herein by reference in its entirety); this modification permits a larger quantity of a target protein to be loaded onto the gel, and thereby increases the quantity of isolated API that can be recovered from the gel. When used in this way for preparative-scale runs, the Preferred Technology typically provides up to 100 ng, and can provide up to 1000 ng, of an isolated API in a single run. Those of skill in the art will appreciate that a zoom gel can be used in any separation strategy which employs gel isoelectric focusing.

The invention thus provides an isolated API, an isolated API fragment, an isolated API-related polypeptide or an isolated API -fusion protein; any of the foregoing can be produced by recombinant DNA techniques or by chemical synthetic methods.

### 5.8 Isolation of DNA Encoding an API

Particular embodiments for the cloning of a gene encoding an API are presented below by way of example and not of limitation.

The nucleotide sequences of the present invention, including DNA and RNA, and comprising a sequence encoding an API, API fragment, API -related polypeptide or API -fusion protein, may be synthesized using methods known in the art, such as using conventional chemical approaches or polymerase chain reaction (PCR) amplification. The nucleotide sequences of the present invention also permit the identification and cloning of the gene encoding an API homolog or API ortholog including, for example, by screening cDNA libraries, genomic libraries or expression libraries.

For example, to clone a gene encoding an API by PCR techniques, anchored degenerate oligonucleotides (or a set of most likely oligonucleotides) can be designed for all API peptide fragments identified as part of the same protein. PCR reactions under a variety of conditions can be performed with relevant cDNA and genomic DNAs (*e.g.*, from brain tissue or from cells of the immune system) from one or more species. Also vectorette reactions can be performed on any available cDNA and genomic DNA using the oligonucleotides (which preferably are nested) as above. Vectorette PCR is a method that enables the amplification of specific DNA fragments in situations where the sequence of only one primer is known. Thus, it extends the application of PCR to stretches of DNA where the sequence information is only available at one end. (Arnold C, 1991, PCR Methods Appl. 1(1): 39-42; Dyer KD, Biotechniques, 1995, 19(4):550-2). Vectorette PCR may be performed with probes that are, for example, anchored degenerate oligonucleotides (or most likely oligonucleotides) coding for API peptide fragments, using as a template a genomic library or cDNA library pools.

Anchored degenerate oligonucleotides (and most likely oligonucleotides) can be designed for all API peptide fragments. These oligonucleotides may be labelled and hybridized to filters containing cDNA and genomic DNA libraries. Oligonucleotides to different peptides from the same protein will often identify the same members of the library. The cDNA and genomic DNA libraries may be obtained from any suitable or desired mammalian species, for example from humans.

Nucleotide sequences comprising a nucleotide sequence encoding an API or API fragment of the present invention are useful, for example, for their ability to hybridize selectively with complementary stretches of genes encoding other proteins. Depending on the application, a variety of hybridization conditions may be employed to obtain nucleotide sequences at least about 30%, 35%, 40%, 45%, 50%, 55%, 60%, 65%, 70%, 75%, 80%, 85%, 90%, 95% or 99% identical, or 100% identical, to the sequence of a nucleotide encoding an API.

For a high degree of selectivity, relatively stringent conditions are used to form the duplexes, such as low salt or high temperature conditions.

Hybridization conditions can also be rendered more stringent by the addition of increasing amounts of formamide, to destabilize the hybrid duplex. Thus, particular hybridization conditions can be readily manipulated, and will generally be chosen depending on the desired results. In general, convenient hybridization temperatures in the presence of 50% formamide are: 42°C for a probe which is 95 to 100% identical to the fragment of a gene encoding an API, 37°C for 90 to 95% identity and 32°C for 70 to 90% identity.

In the preparation of genomic libraries, DNA fragments are generated, some of which will encode parts or the whole of an API. Any suitable method for preparing DNA fragments may be used in the present invention. For example, the DNA may be cleaved at specific sites using various restriction enzymes. Alternatively, one may use DNAse in the presence of manganese to fragment the DNA, or the DNA can be physically sheared, as for example, by sonication. The DNA fragments can then be separated according to size by standard techniques, including but not limited to agarose and polyacrylamide gel electrophoresis, column chromatography and sucrose gradient centrifugation. The DNA fragments can then be inserted into suitable vectors, including but not limited to plasmids, cosmids, bacteriophages lambda or T4, and yeast artificial chromosome (YAC). (See, *e.g.*, Sambrook et al., 1989, Molecular Cloning, A Laboratory Manual, 2d Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II; Ausubel F.M. et al., eds., 1989, Current Protocols in Molecular Biology, Vol. I, Green Publishing Associates, Inc., and John Wiley & sons, Inc., New York). The genomic library may be screened by nucleic acid hybridization to labeled probe (Benton and Davis, 1977, Science 196:180; Grunstein and Hogness, 1975, Proc. Natl. Acad. Sci. U.S.A. 72:3961).

Based on the present description, the genomic libraries may be screened with labeled degenerate oligonucleotide probes corresponding to the amino acid sequence of any peptide of the API using optimal approaches well known in the art. Any probe used is at least 10 nucleotides, at least 15 nucleotides, at least 20 nucleotides, at least 25 nucleotides, at least 30 nucleotides, at least 40 nucleotides, at least 50 nucleotides, at least 60 nucleotides, at least 70 nucleotides, at least 80 nucleotides, or at least 100 nucleotides. Preferably a probe is 10 nucleotides or longer, and more preferably 15 nucleotides or longer.

In Tables IV and V above, some APIs disclosed herein correspond to isoforms of previously identified proteins encoded by genes whose sequences are publicly known. To screen such a gene, any probe may be used that is complementary to the gene or its complement; preferably the probe is 10 nucleotides or longer, more preferably 15 nucleotides or longer. The SWISS-PROT and trEMBL databases (held by the Swiss Institute of Bioinformatics (SIB) and the European Bioinformatics Institute (EBI) which are available at http://www.expasy.ch/) and the GenBank database (held by the National Institute of Health (NIH) which is available at http://www.ncbi.nlm.nih.gov/) provide protein sequences comprising the amino acid sequences listed for the APIs in Tables IV and V under the following accession numbers and each sequence is incorporated herein by reference:

### Table VIII. Nucleotide sequences encoding APIs, API Related Proteins, or ERPIs

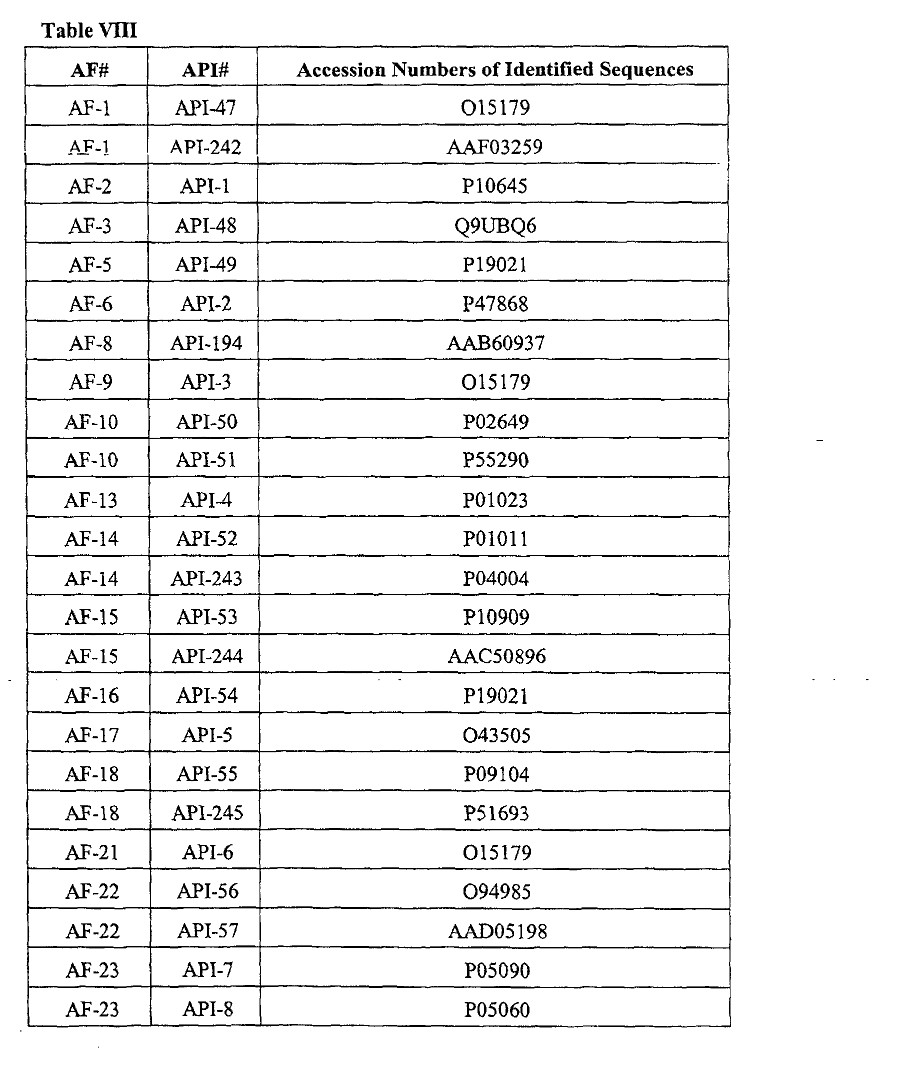

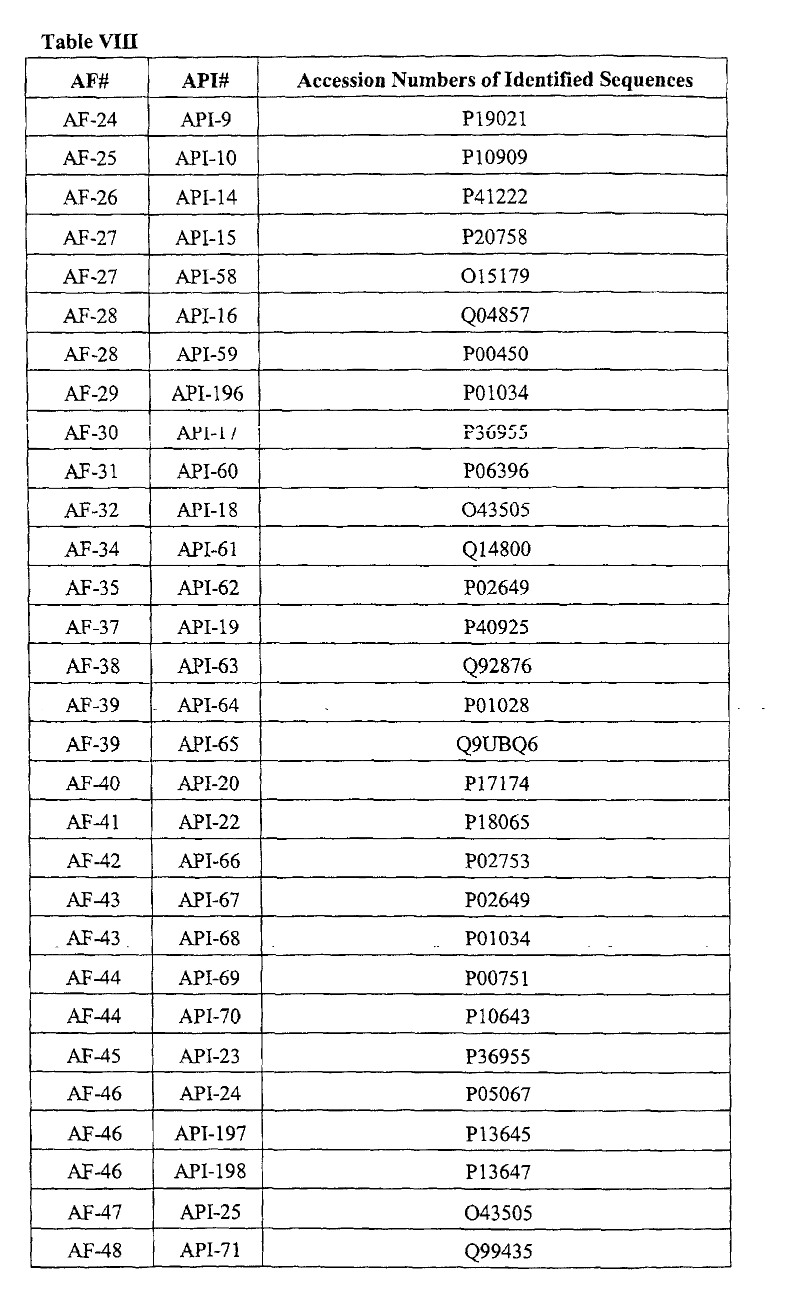

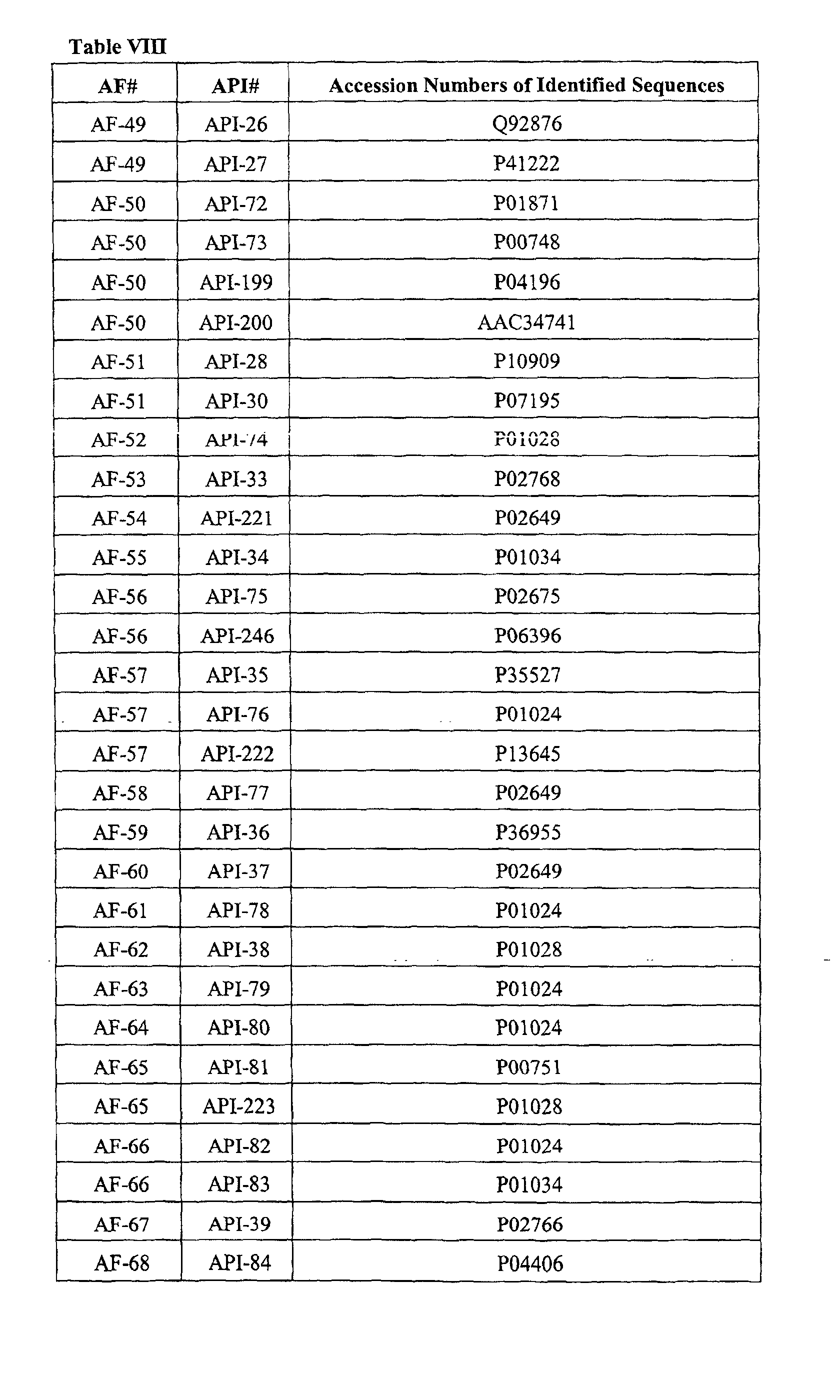

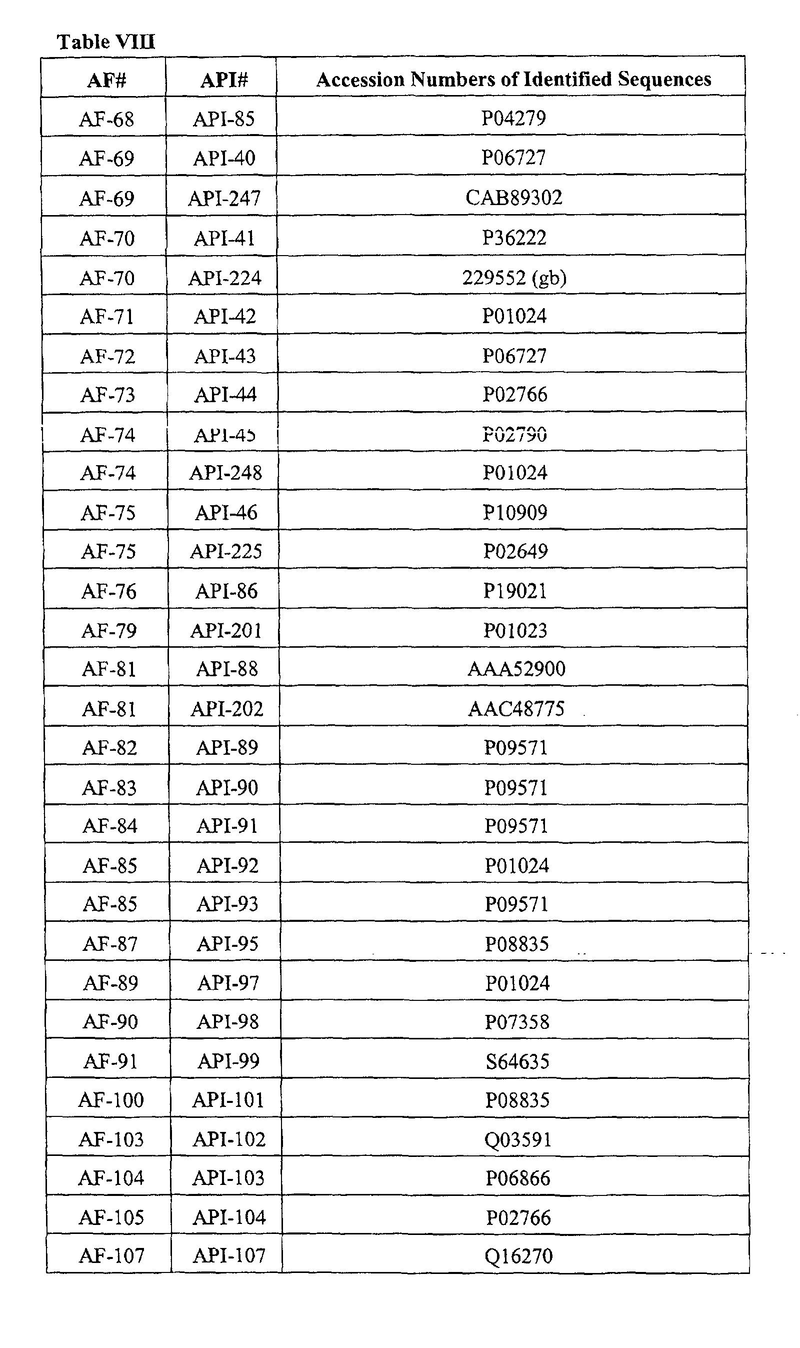

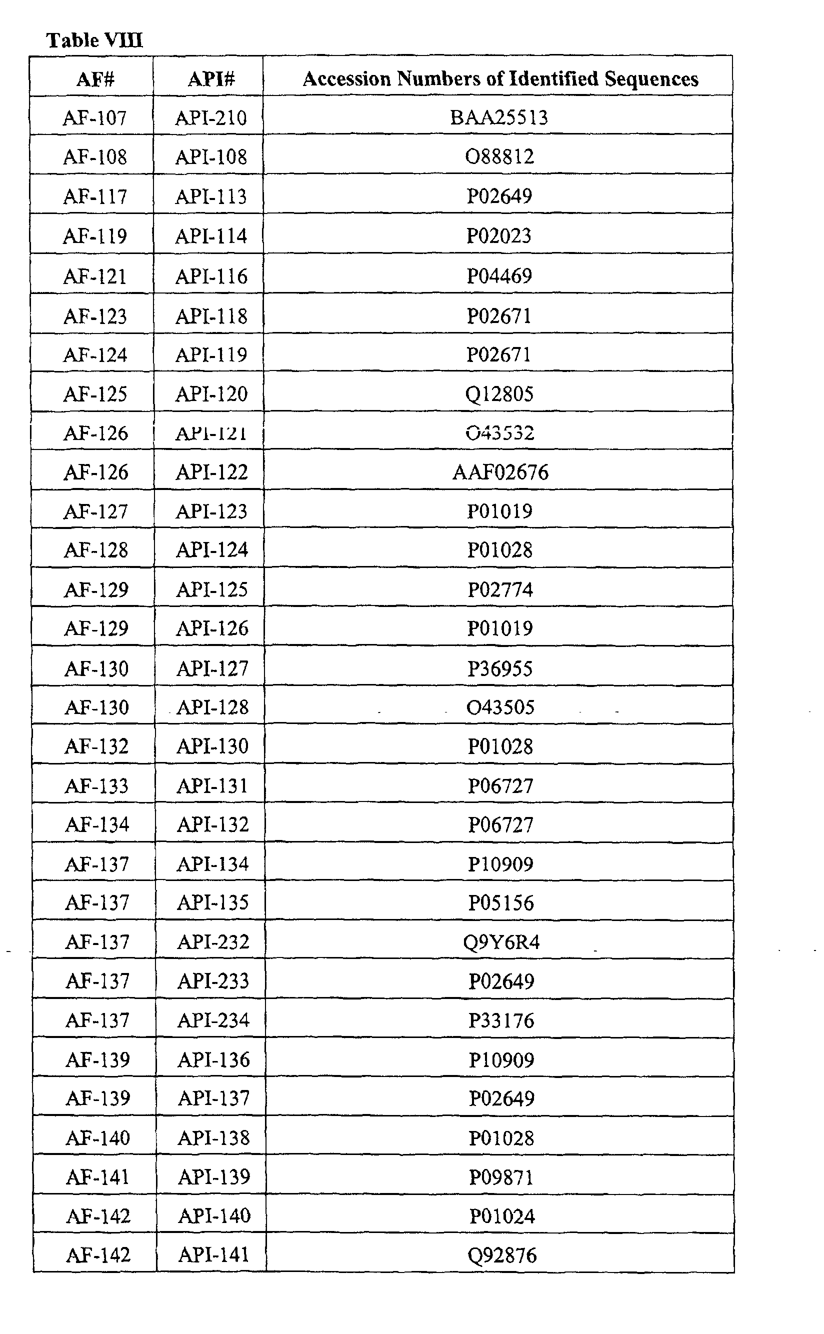

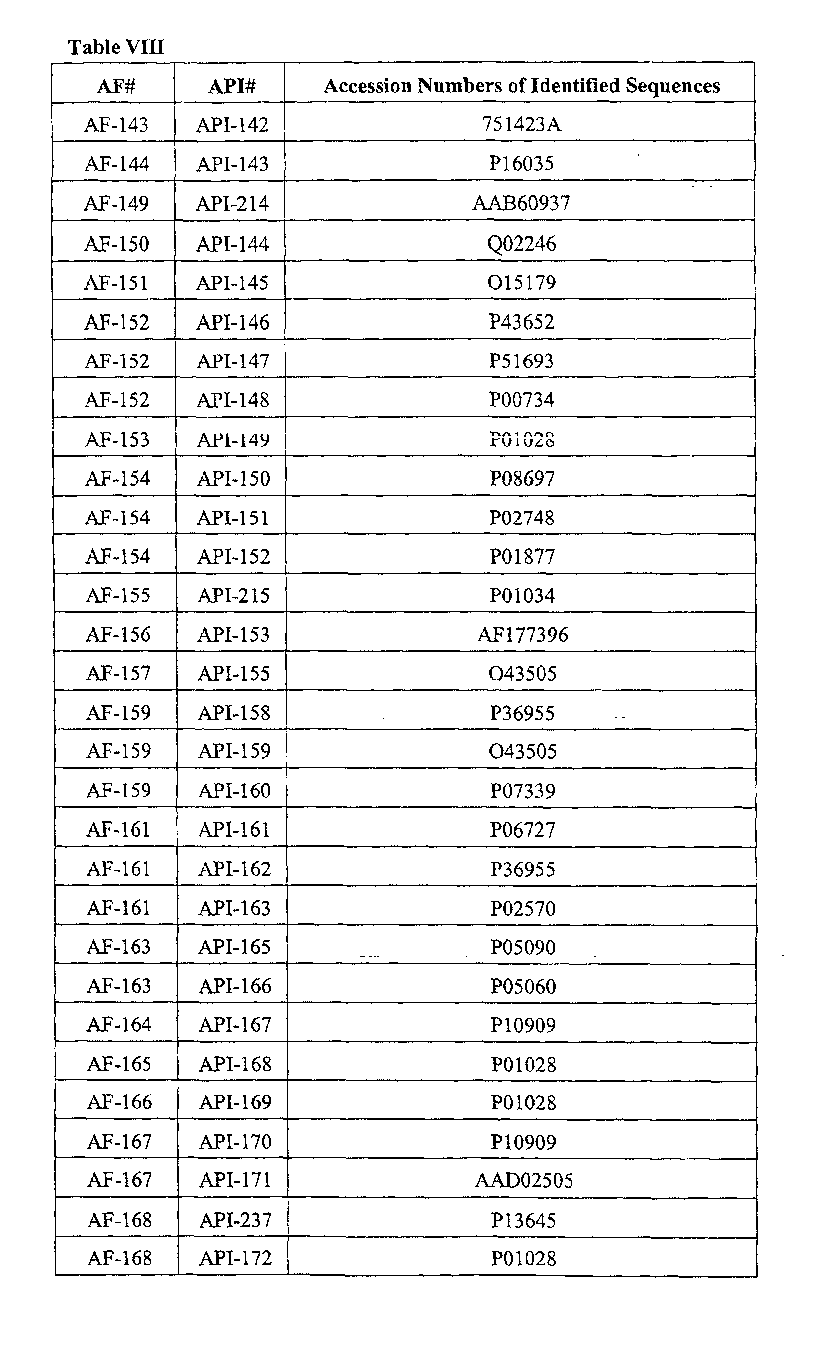

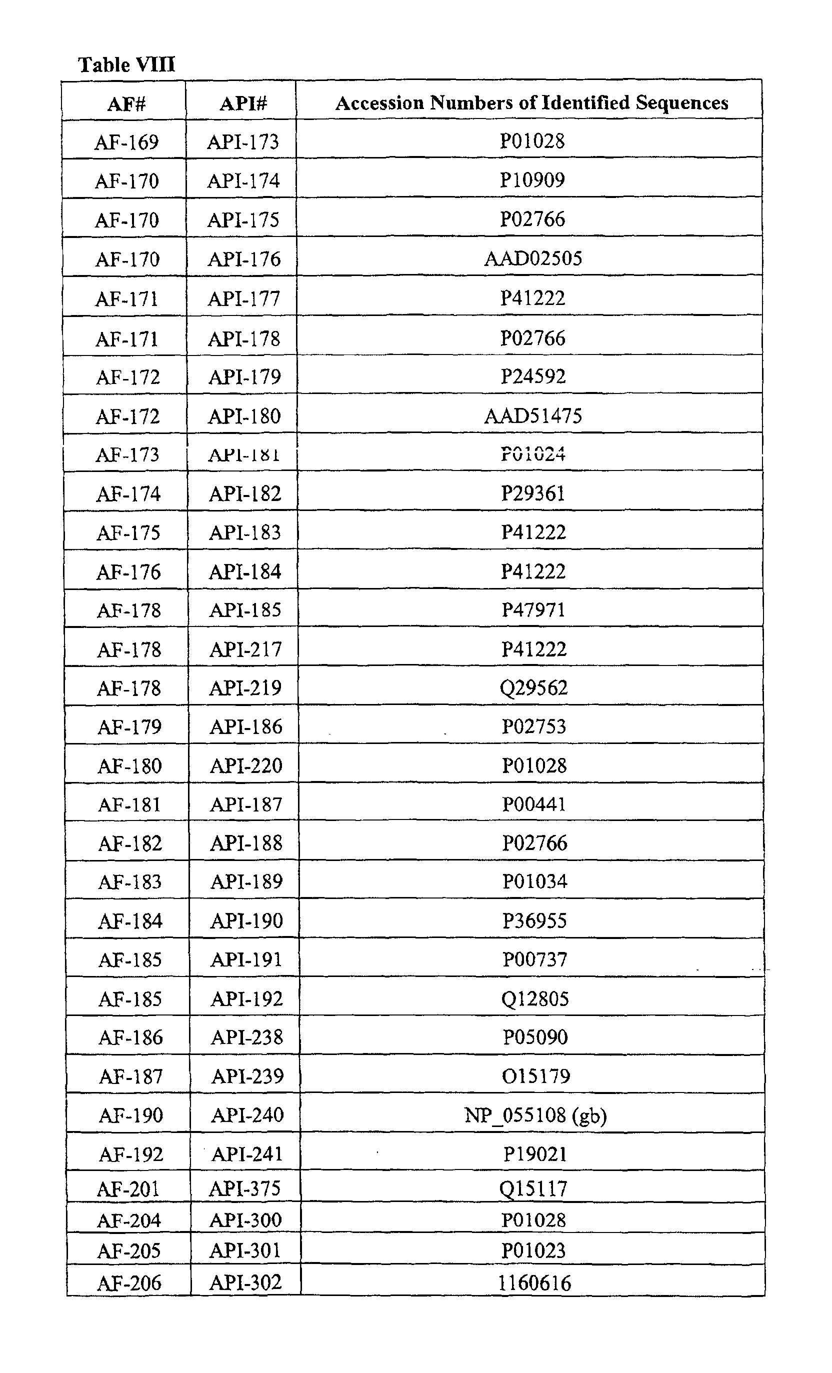

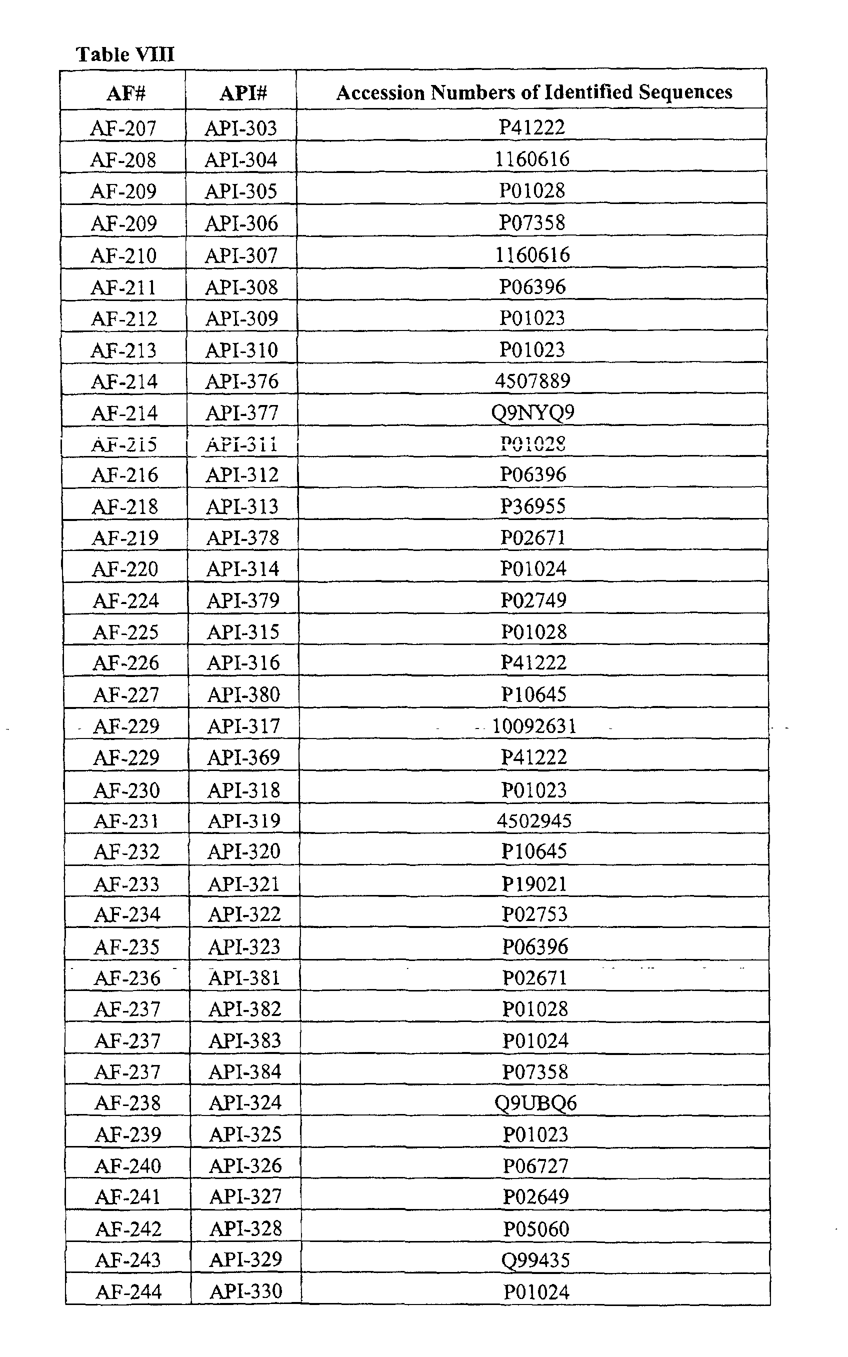

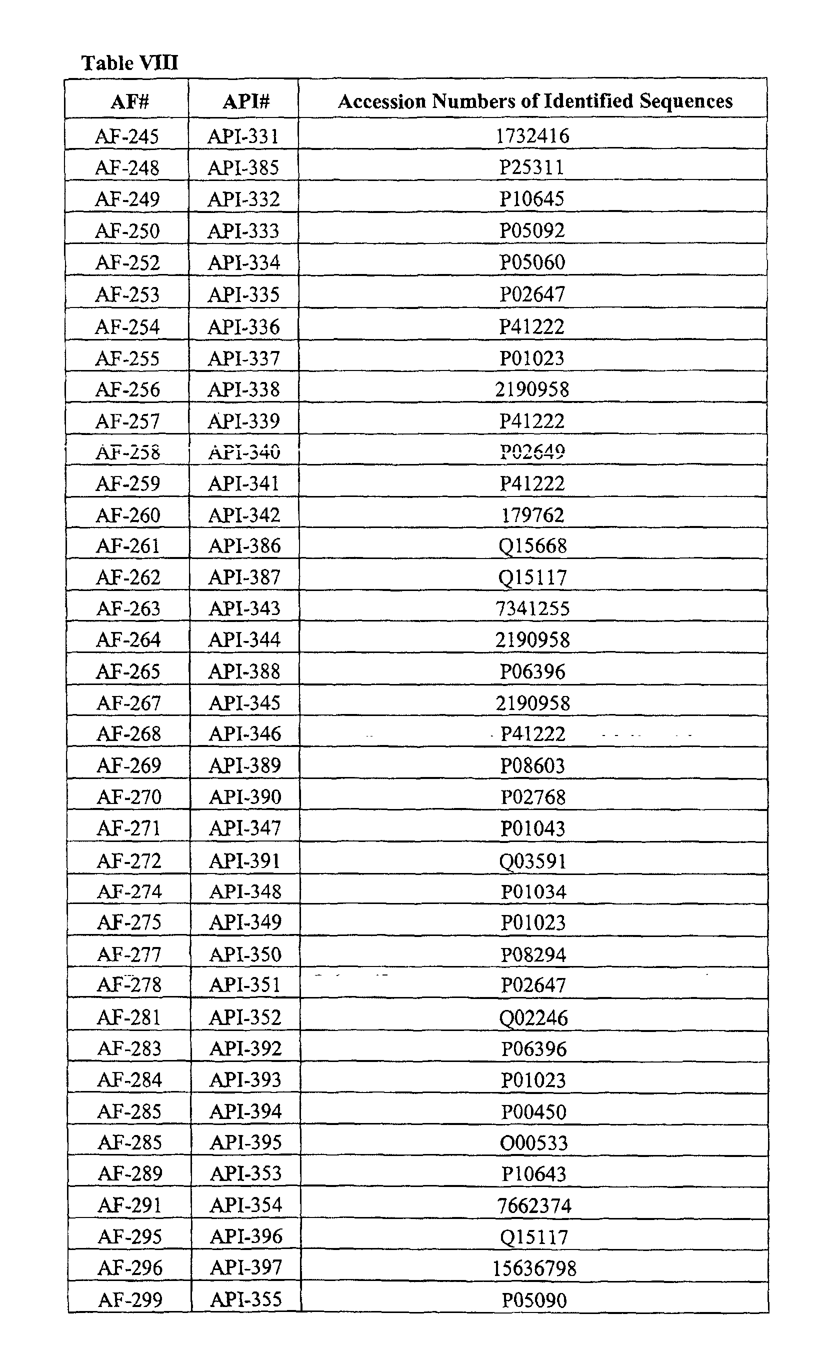

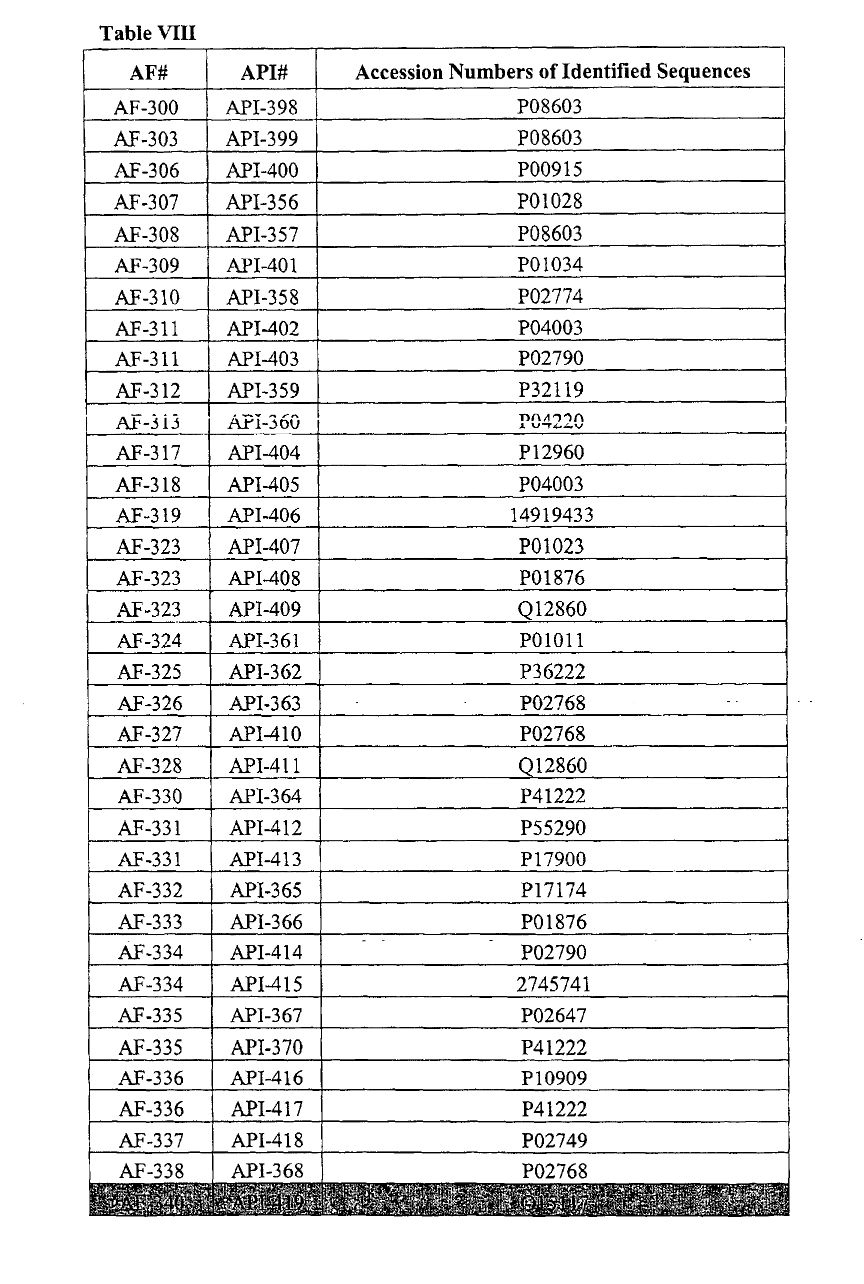

When no nucleotide sequence is known that encodes a protein comprising an amino acid sequence of a given API, degenerate probes can be used for screening. In Table IX, a degenerate set of probes is provided for API-111 and API-112. The partial amino acid sequences listed in Table IX were derived from manual interpretation of the tandem mass spectra of tryptic digest peptides of the API. In the method of tandem mass spectroscopy used for sequencing peptides in the present invention, the following pairs of amino acids could not be distinguished from each other: leucine and isoleucine; and, under certain circumstances, phenylalanine and oxidized methionine. As used herein, an amino acid sequence "as determined by mass spectrometry" refers to the set of amino acid sequences containing at the indicated positions, one or other member of the designated pairs of amino acids. For example, the amino acid sequence P[L/I]A indicates the amino acid sequences PLA and PIA. As will be obvious to one of skill in the art, a sequence containing n designated pairs indicates 2n amino acid sequences. In Table IX, each possible amino acid sequence is listed for each sequence determined by mass spectroscopy, and preferred and fully degenerate sets of probes for each possible amino acid sequence are provided.

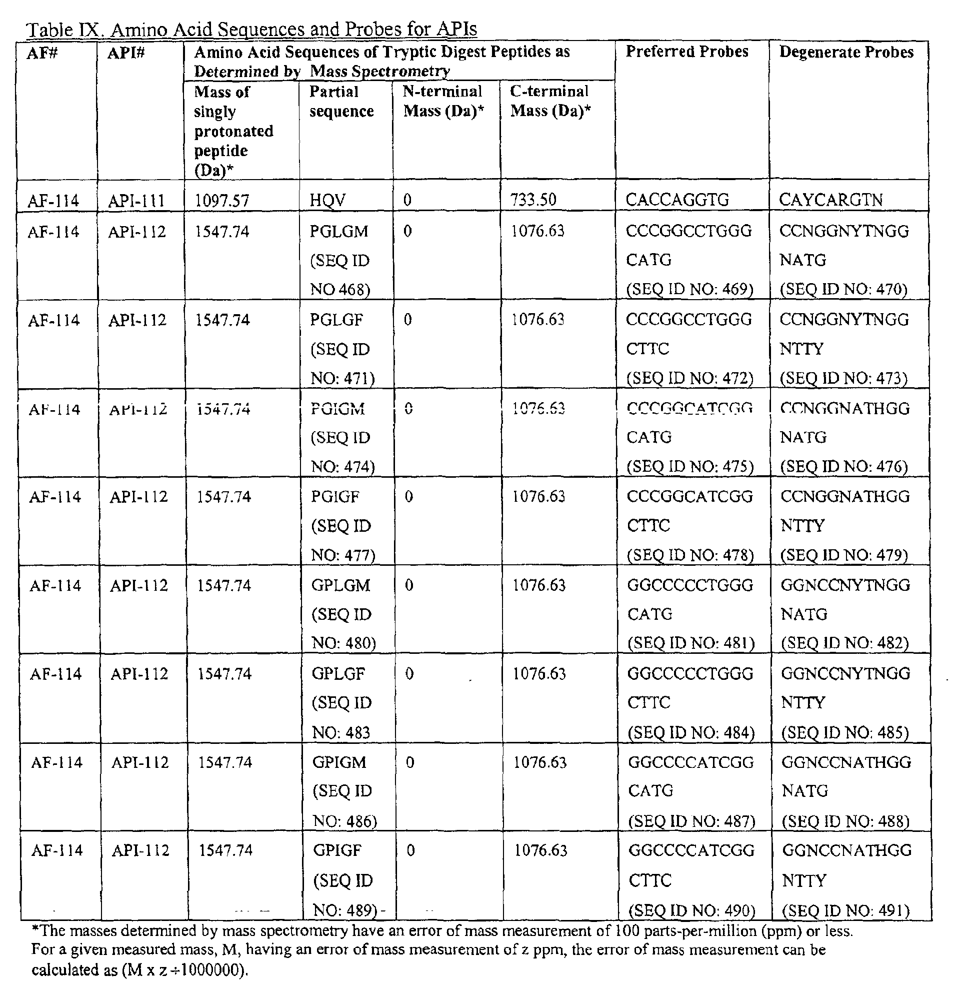

As used herein, the "mass of the singly protonated peptide" is the mass of the singly protonated tryptic digest peptide measured by mass spectrometry (having an error of measurement of approximately 100 parts-per-million or less) and corresponds to the total mass of the constituent amino acid residues of the peptide with the addition of a water molecule (H₂O) and a single proton (H⁺). As used herein, an "amino acid residue" refers to an amino acid residue of the general structure: -NH-CHR-CO- and which have the following symbols, elemental compositions and monoisotopic masses:
Amino acid residue elemental compositions and monoisotopic masses

| Amino Acid | Symbol | Elemental Composition | Monoisotopic mass (Da) |
|---|---|---|---|
| Alanine | A | C₃H₅NO | 71.037114 |
| Arginine | R | C₆H₁₂N₄O | 156.10111 |
| Asparagine | N | C₄H₆N₂O₂ | 114.042927 |
| Aspartic Acid | D | C₄H₅NO₃ | 115.026943 |
| Carboxyamido | C | C₅H₈N₂O₂S | 160.03065 |
| Cysteine¹ | | | |
| Glutamic Acid | E | C₅H₇NO₃ | 129.042593 |
| Glutamine | Q | C₃H₈N₂O₂ | 128.058577 |
| Glycine | G | C₂H₃NO | 57.021464 |
| Histadine | H | C₆H₇N₃O | 137.058912 |
| Isoleucine | I | C₆H₁₁NO | 113.084064 |
| Leucine | L | C₆H₁₁NO | 113.084064 |
| Lysine | K | C₆H₁₂N₂O | 128.094963 |
| Methionine | M | C₅H₉NOS | 131.040485 |
| Oxidised Methionine | M* | C₅H₉NO₂S | 147.035340 |
| Phenylalanine | F | C₉H₉NO | 147.068414 |
| Proline | P | C₅H₇NO | 97.052764 |
| Serine | S | C₃H₅NO₂ | 87.032028 |
| Threonine | T | C₄H₇NO₂ | 101.047678 |
| Tryptophan | W | C₁₁H₁₀N₂O | 186.079313 |
| Tyrosine | Y | C₉H₉NO₂ | 163.063328 |
| Valine | V | C₅H₉NO | 99.068414 |

| | | | |
|---|---|---|---|
| ^{*1*}*All Cysteines are modified to the carboxyamino derivative during our production process.* | | | |

As used herein "tryptic digest peptides" are peptides produced through treatment of the protein with the enzyme trypsin. Trypsin cleaves specifically at the carboxyl side of lysine (Lys) and arginine (Arg) residues, so that the tryptic digest peptides generated should have a Lys or Arg as the C-terminal amino acid, unless the peptide fragment was obtained from the C-terminal of the protein. Similarly, the amino acid directly preceding the N-terminal amino acid of the tryptic digest peptides should also be a Lys or Arg, unless the peptide was obtained from the N-terminal of the protein. The mass of a tryptic digest peptide corresponds to the total mass of the constituent amino acid residues of the peptide with the addition of a water molecule (H₂O). As used herein, the "partial sequence" is an amino acid sequence within the tryptic digest peptide determined from the interpretation of the tandem mass spectrum of the peptide. As used herein, the "N-terminal mass" is the mass measured by mass spectrometry (having an error of measurement of approximately 100 parts-per-million or less) of the portion of the tryptic digest peptide extending from the start of the partial sequence to the N-terminus of the peptide. This is a neutral mass corresponding to the total mass of the constituent amino acid residues extending from the partial sequence to the N-terminus of the peptide. As used herein, the "C-terminal mass" is the mass measured by mass spectrometry (having an error of measurement of approximately 100 parts-per-million or less) of the portion of the tryptic digest peptide extending from the end of the partial sequence to the C-terminus of the peptide. This mass corresponds to the total mass of the constituent amino acid residues extending from the end of the partial sequence to the C-terminus of the peptide with the addition of a water molecular (H₂O), and a single proton (H⁺). In Table IX, *supra*, the preferred and degenerate sets of probes are described using GCG Nucleotide Ambiguity Codes as employed in GCG SeqWeb™ sequence analysis software (SeqWeb™ version 1.1, part of Wisconsin Package Version 10, Genetics Computer Group, Inc.). These Nucleotide Ambiguity Codes have the following meaning:

| *GCG Code* | *Meaning* |
|---|---|
| A | A |
| C | C |
| G | G |
| T | T |
| U | T |
| M | A or C |
| R | A or G |
| W | A or T |
| S | C or G |
| Y | C or T |
| K | G or T |
| V | A or C or G |
| H | A or C or T |
| D | A or G or T |
| B | C or G or T |
| X | G or A or T or C |
| N | G or A or T or C |

GCG uses the letter codes for amino acid codes and nucleotide ambiguity proposed by IUPAC-IUB. These codes are compatible with the codes used by the EMBL, GenBank, and PIR databases. See IUPAC, Commission on Nomenclature of Organic Chemistry. A Guide to IUPAC Nomenclature of Organic Compounds (Recommendations 1993), Blackwell Scientific publications, 1993.

When a library is screened, clones with insert DNA encoding the API of interest or a fragment thereof will hybridize to one or more members of the corresponding set of degenerate oligonucleotide probes (or their complement). Hybridization of such oligonucleotide probes to genomic libraries is carried out using methods known in the art. For example, hybridization with one of the above-mentioned degenerate sets of oligonucleotide probes, or their complement (or with any member of such a set, or its complement) can be performed under highly stringent or moderately stringent conditions as defined above, or can be carried out in 2X SSC, 1.0% SDS at 50°C and washed using the washing conditions described supra for highly stringent or moderately stringent hybridization.

In yet another aspect of the invention, clones containing nucleotide sequences encoding the entire API, a fragment of an API, an API-related polypeptide, or a fragment of an API-related polypeptide or any of the foregoing may also be obtained by screening expression libraries. For example, DNA from the relevant source is isolated and random fragments are prepared and ligated into an expression vector (*e.g.*, a bacteriophage, plasmid, phagemid or cosmid) such that the inserted sequence in the vector is capable of being expressed by the host cell into which the vector is then introduced. Various screening assays can then be used to select for the expressed API or API-related polypeptides. In one embodiment, the various anti-API antibodies of the invention can be used to identify the desired clones using methods known in the art. See, for example, Harlow and Lane, 1988, Antibodies: A Laboratory Manual, Cold Spring Harbor Laboratory Press, Cold Spring Harbor, NY, Appendix IV. Colonies or plaques from the library are brought into contact with the antibodies to identify those clones that bind antibody.

In an embodiment, colonies or plaques containing DNA that encodes an API, API fragment, API -related polypeptide or API -fusion protein can be detected using DYNA Beads according to Olsvick et al., 29th ICAAC, Houston, Tex. 1989, incorporated herein by reference. Anti-API antibodies are crosslinked to tosylated DYNA Beads M280, and these antibody-containing beads are then contacted with colonies or plaques expressing recombinant polypeptides. Colonies or plaques expressing an API, API fragment, API -related polypeptide or API -fusion protein are identified as any of those that bind the beads.

Alternatively, the anti-API antibodies can be nonspecifically immobilized to a suitable support, such as silica or Celite® resin. This material is then used to adsorb to bacterial colonies expressing the API, API fragment, API -related polypeptide or API -fusion protein ptide as described herein.

In another aspect, PCR amplification may be used to isolate from genomic DNA a substantially pure DNA (*i.e.*, a DNA substantially free of contaminating nucleic acids) encoding the entire API or a part thereof. Preferably such a DNA is at least 95% pure, more preferably at least 99% pure. Oligonucleotide sequences, degenerate or otherwise, that correspond to peptide sequences of APIs disclosed herein can be used as primers.

PCR can be carried out, *e.g.*, by use of a Perkin-Elmer Cetus thermal cycler and Taq polymerase (Gene Amp® or AmpliTaq DNA polymerase). One can choose to synthesize several different degenerate primers, for use in the PCR reactions. It is also possible to vary the stringency of hybridization conditions used in priming the PCR reactions, to allow for greater or lesser degrees of nucleotide sequence similarity between the degenerate primers and the corresponding sequences in the DNA. After successful amplification of a segment of the sequence encoding an API, that segment may be molecularly cloned and sequenced, and utilized as a probe to isolate a complete genomic clone. This, in turn, will permit the determination of the gene's complete nucleotide sequence, the analysis of its expression, and the production of its protein product for functional analysis, as described *infra.*

The gene encoding an API can also be identified by mRNA selection by nucleic acid hybridization followed by *in vitro* translation. In this procedure, fragments are used to isolate complementary mRNAs by hybridization. Such DNA fragments may represent available, purified DNA encoding an API of another species (*e.g.*, mouse, human). Immunoprecipitation analysis or functional assays (*e.g.*, aggregation ability *in vitro*; binding to receptor) of the *in vitro* translation products of the isolated products of the isolated mRNAs identifies the mRNA and, therefore, the complementary DNA fragments that contain the desired sequences. In addition, specific mRNAs may be selected by adsorption ofpolysomes isolated from cells to immobilized antibodies that specifically recognize an API. A radiolabelled cDNA encoding an API can be synthesized using the selected mRNA (from the adsorbed polysomes) as a template. The radiolabelled mRNA or cDNA may then be used as a probe to identify the DNA fragments encoding an API from among other genomic DNA fragments.

Alternatives to isolating genomic DNA encoding an API include, but are not limited to, chemically synthesizing the gene sequence itself from a known sequence or making cDNA to the mRNA which encodes the API. For example, RNA for cDNA cloning of the gene encoding an API can be isolated from cells which express the API. Those skilled in the art will understand from the present description that other methods may be used and are within the scope of the invention.

Any suitable eukaryotic cell can serve as the nucleic acid source for the molecular cloning of the gene encoding an API. The nucleic acid sequences encoding the API can be isolated from vertebrate, mammalian, primate, human, porcine, bovine, feline, avian, equine, canine or murine sources. The DNA may be obtained by standard procedures known in the art from cloned DNA (*e.g.*, a DNA "library"), by chemical synthesis, by cDNA cloning, or by the cloning of genomic DNA, or fragments thereof, purified from the desired cell. (See, *e.g.*, Sambrook et al., 1989, Molecular Cloning, A Laboratory Manual, 2d Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II.). Clones derived from genomic DNA may contain regulatory and intron DNA regions in addition to coding regions; clones derived from cDNA will contain only exon sequences.

The identified and isolated gene or cDNA can then be inserted into any suitable cloning vector. A large number of vector-host systems known in the art may be used. As those skilled in the art will appreciate, the vector system chosen should be compatible with the host cell used. Such vectors include, but are not limited to, bacteriophages such as lambda derivatives, plasmids such as PBR322 or pUC plasmid derivatives or the Bluescript vector (Stratagene) or modified viruses such as adenoviruses, adeno-associated viruses or retroviruses. The insertion into a cloning vector can be accomplished, for example, by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. In an alternative method, the cleaved vector and the gene encoding an API may be modified by homopolymeric tailing. Recombinant molecules can be introduced into host cells via transformation, transfection, infection, electroporation, etc., so that many copies of the gene sequence are generated.

In specific embodiments, transformation of host cells with recombinant DNA molecules that incorporate the isolated gene encoding the API, cDNA, or synthesized DNA sequence enables generation of multiple copies of the gene. Thus, the gene may be obtained in large quantities by growing transformants, isolating the recombinant DNA molecules from the transformants and, when necessary, retrieving the inserted gene from the isolated recombinant DNA.

The nucleotide sequences of the present invention include nucleotide sequences encoding amino acid sequences with substantially the same amino acid sequences as native APIs, nucleotide sequences encoding amino acid sequences with functionally equivalent amino acids, nucleotide sequences encoding APIs, API fragments, API -related polypeptides or fragments of API-related polypeptides.

In a specific embodiment, an isolated nucleic acid molecule encoding an API-related polypeptide can be created by introducing one or more nucleotide substitutions, additions or deletions into the nucleotide sequence of an API, such that one or more amino acid substitutions, additions or deletions are introduced into the encoded protein. Standard techniques known to those of skill in the art can be used to introduce mutations, including, for example, site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a side chain with a similar charge. Families of amino acid residues having side chains with similar charges have been defined in the art. These families include amino acids with basic side chains (*e.g.*, lysine, arginine, histidine), acidic side chains (*e.g.*, aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains (*e.g.*, tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity. Following mutagenesis, the encoded protein can be expressed and the activity of the protein can be determined.

### 5.9 Expression of DNA Encoding APIs

The nucleotide sequence coding for an API, API fragment, API -related polypeptide or API-fusion protein, can be inserted into an appropriate expression vector, *i.e.*, a vector which contains the necessary elements for the transcription and translation of the inserted protein-coding sequence. The necessary transcriptional and translational signals can also be supplied by the native gene encoding the API, or its flanking regions, or the native gene encoding the API-related polypeptide or its flanking regions. A variety of host-vector systems may be utilized in the present invention to express the protein-coding sequence. These include but are not limited to mammalian cell systems infected with virus (*e.g.*, vaccinia virus, adenovirus, etc.); insect cell systems infected with virus (*e.g.*, baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used. In specific embodiments, a nucleotide sequence encoding a human gene (or a nucleotide sequence encoding a functionally active portion of a human API) is expressed. In yet another embodiment, a fragment of an API comprising a domain of the API is expressed.

Any of the methods previously described for the insertion of DNA fragments into a vector may be used to construct expression vectors containing a chimeric gene consisting of appropriate transcriptional and translational control signals and the protein coding sequences. These methods may include *in vitro* recombinant DNA and synthetic techniques and *in vivo* recombinants (genetic recombination). Expression of nucleic acid sequence encoding an API, API fragment, API -related polypeptide or API -fusion protein may be regulated by a second nucleic acid sequence so that the API, API fragment, API -related polypeptide or API -fusion protein is expressed in a host transformed with the recombinant DNA molecule. For example, expression of an API may be controlled by any promoter or enhancer element known in the art. Promoters which may be used to control the expression of the gene encoding an API, API fragment, API -related polypeptide or API -fusion protein include, but are not limited to, the SV40 early promoter region (Bemoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42), the tetracycline (Tet) promoter (Gossen et al., 1995, Proc. Nat. Acad. Sci. USA 89:5547-5551); prokaryotic expression vectors such as the β-lactamase promoter (Villa-Kamaroff, et al., 1978, Proc. Natl. Acad. Sci. U.S.A. 75:3727-3731), or the tac promoter (DeBoer, et al., 1983, Proc. Natl. Acad. Sci. U.S.A. 80:21-25; see also "Useful proteins from recombinant bacteria" in Scientific American, 1980, 242:74-94); plant expression vectors comprising the nopaline synthetase promoter region (Herrera-Estrella et al., Nature 303:209-213) or the cauliflower mosaic virus 35S RNA promoter (Gardner, et al., 1981, Nucl. Acids Res. 9:2871), and the promoter of the photosynthetic enzyme ribulose biphosphate carboxylase (Herrera-Estrella et al., 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Omitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), un gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286); neuronal-specific enolase (NSE) which is active in neuronal cells (Morelli et al., 1999, Gen. Virol. 80:571-83); brain-derived neurotrophic factor (BDNF) gene control region which is active in neuronal cells (Tabuchi et al., 1998, Biochem. Biophysic. Res. Com. 253:818-823); glial fibrillary acidic protein (GFAP) promoter which is active in astrocytes (Gomes et al., 1999, Braz J Med Biol Res 32(5):619-631; Morelli et al., 1999, Gen. Virol. 80:571-83) and gonadotropic releasing hormone gene control region which is active in the hypothalamus (Mason et al., 1986, Science 234:1372-1378).

In a specific embodiment, a vector is used that comprises a promoter operably linked to an API-encoding nucleic acid, one or more origins of replication, and, optionally, one or more selectable markers (*e.g.*, an antibiotic resistance gene).

In a specific embodiment, an expression construct is made by subcloning an API or an API-related polypeptide coding sequence into the EcoRI restriction site of each of the three pGEX vectors (Glutathione S-Transferase expression vectors; Smith and Johnson, 1988, Gene 7:31-40). This allows for the expression of the API product or API-related polypeptide from the subclone in the correct reading frame.

In mammalian host cells, a number of viral-based expression systems may be utilized. In cases where an adenovirus is used as an expression vector, the API coding sequence or API-related polypeptide coding sequence may be ligated to an adenovirus transcription/translation control complex, *e.g.*, the late promoter and tripartite leader sequence. This chimeric gene may then be inserted in the adenovirus genome by *in vitro* or *in vivo* recombination. Insertion in a non-essential region of the viral genome (*e.g.*, region E1 or E3) will result in a recombinant virus that is viable and capable of expressing the antibody molecule in infected hosts (*e.g.*, see Logan & Shenk, 1984, Proc. Natl. Acad. Sci. USA 81:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., 1987, Methods in Enzymol. 153:51-544).

Expression vectors containing inserts of a gene encoding an API or an API-related polypeptide can be identified, for example, by three general approaches: (a) nucleic acid hybridization, (b) presence or absence of "marker" gene functions, and (c) expression of inserted sequences. In the first approach, the presence of a gene encoding an API inserted in an expression vector can be detected by nucleic acid hybridization using probes comprising sequences that are homologous to an inserted gene encoding an API. In the second approach, the recombinant vector/host system can be identified and selected based upon the presence or absence of certain "marker" gene functions (*e.g.*, thymidine kinase activity, resistance to antibiotics, transformation phenotype, occlusion body formation in baculovirus, etc.) caused by the insertion of a gene encoding an API in the vector. For example, if the gene encoding the API is inserted within the marker gene sequence of the vector, recombinants containing the gene encoding the API insert can be identified by the absence of the marker gene function. In the third approach, recombinant expression vectors can be identified by assaying the gene product (*i.e.*, API) expressed by the recombinant. Such assays can be based, for example, on the physical or functional properties of the API in *in vitro* assay systems, *e.g.*, binding with anti-API antibody.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered API or API-related polypeptide may be controlled. Furthermore, different host cells have characteristic and specific mechanisms for the translational and post-translational processing and modification (*e.g.*, glycosylation, phosphorylation of proteins). Appropriate cell lines or host systems can be chosen to ensure the desired modification and processing of the foreign protein expressed. For example, expression in a bacterial system will produce an unglycosylated product and expression in yeast will produce a glycosylated product. Eukaryotic host cells which possess the cellular machinery for proper processing of the primary transcript, glycosylation, and phosphorylation of the gene product may be used. Such mammalian host cells include but are not limited to CHO, VERO, BHK, Hela, COS, MDCK, HEK 293, 3T3, WI38, and in particular, neuronal cell lines such as, for example, SK-N-AS, SK-N-FI, SK-N-DZ human neuroblastomas (Sugimoto et al., 1984, J. Natl. Cancer Inst. 73: 51-57), SK-N-SH human neuroblastoma (Biochim. Biophys. Acta, 1982, 704: 450 460), Daoy human cerebellar medulloblastoma (He et al., 1992, Cancer Res, 52: 1144-1148) DBTRG-05MG glioblastoma cells (Kruse et al., 1992, *In vitro* Cell. Dev. Biol. 28A: 609-614), IMR-32 human neuroblastoma (Cancer Res., 1970, 30: 2110-2118), 1321N1 human astrocytoma (Proc. Natl Acad. Sci. USA ,1977, 74: 4816), MOG-G-CCM human astrocytoma (Br. J, Cancer, 1984,49: 269), U87MG human glioblastoma-astrocytoma (Acta Pathol. Microbiol. Scand., 1968, 74: 465-486), A172 human glioblastoma (Olopade et al., 1992, Cancer Res. 52: 2523-2529), C6 rat glioma cells (Benda et al., 1968, Science 161: 370-371), Neuro-2a mouse neuroblastoma (Proc. Natl. Acad. Sci. USA, 1970, 65: 129-136), NB41A3 mouse neuroblastoma (Proc. Natl. Acad. Sci. USA, 1962, 48: 1184-1190), SCP sheep choroid plexus (Bolin et al., 1994, J. Virol. Methods 48: 211-221), G355-5, PG-4 Cat normal astrocyte (Haapala et al., 1985, J. Virol. 53: 827-833), Mpf ferret brain (Trowbridge et al., 1982, *In vitro* 18: 952-960), and normal cell lines such as, for example, CTX TNA2 rat normal cortex brain (Radany et al., 1992, Proc. Natl. Acad. Sci. USA 89: 6467-6471) such as, for example, CRL7030 and Hs578Bst. Furthermore, different vector/host expression systems may effect processing reactions to different extents.

For long-term, high-yield production of recombi-nant proteins, stable expression is preferred. For example, cell lines which stably express the differentially expressed or pathway gene protein may be engineered. Rather than using expression vectors which contain viral origins of replication, host cells can be transformed with DNA controlled by appropriate expression control elements (*e.g.*, promoter, enhancer, sequences, transcription terminators, polyadenylation sites, etc.), and a selectable marker. Following the introduction of the foreign DNA, engineered cells may be allowed to grow for 1-2 days in an enriched medium, and then are switched to a selective medium. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the differentially expressed or pathway gene protein. Such engineered cell lines may be particularly useful in screening and evaluation of compounds that affect the endogenous activity of the differentially expressed or pathway gene protein.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk-, hgprt- or aprt- cells, respectively. Also, antimetabolite resistance can be used as the basis of selection for dhfr, which confers resistance to methotrexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147) genes.

In other embodiments, the API, API fragment or API -related polypeptide may be expressed as a fusion, or chimeric protein product (comprising the protein, fragment, analog, or derivative joined via a peptide bond to a heterologous protein sequence). For example, the polypeptides of the present invention may be fused with the constant domain of immunoglobulins (IgA, IgE, IgG, IgM), or portions thereof (CH1, CH2, CH3, or any combination thereof and portions thereof) resulting in chimeric polypeptides. Such fusion proteins may facilitate purification, increase half-life *in vivo*, and enhance the delivery of an antigen across an epithelial barrier to the immune system. An increase in the half-life *in vivo* and facilitated purification has been shown for chimeric proteins consisting of the first two domains of the human CD4-polypeptide and various domains of the constant regions of the heavy or light chains of mammalian immunoglobulins. See, *e.g.*, EP 394,827; Traunecker et al., Nature, 331:84-86 (1988). Enhanced delivery of an antigen across the epithelial barrier to the immune system has been demonstrated for antigens (*e.g.*, insulin) conjugated to an FcRn binding partner such as IgG or Fc fragments (see, *e.g.*, PCT publications WO 96/22024 and WO 99/04813).

Nucleic acids encoding an API, a fragment of an API, an API-related polypeptide, or a fragment of an API-related polypeptide can be fused to an epitope tag (*e.g.*, the hemagglutinin ("HA") tag or flag tag) to aid in detection and purification of the expressed polypeptide. For example, a system described by Janknecht et al. allows for the ready purification of non-denatured fusion proteins expressed in human cell lines (Janknecht et al., 1991, Proc. Natl. Acad. Sci. USA 88:8972-897).

An API fusion protein can be made by ligating the appropriate nucleic acid sequences encoding the desired amino acid sequences to each other by methods known in the art, in the proper coding frame, and expressing the chimeric product by methods commonly known in the art. Alternatively, an API fusion protein may be made by protein synthetic techniques, *e.g.*, by use of a peptide synthesizer.

Both cDNA and genomic sequences can be cloned and expressed.

### 5.10 Domain Structure of APIs

Domains of some of the APIs provided by the present invention are known in the art and have been described in the scientific literature. Moreover, domains of an API can be identified using techniques known to those of skill in the art. For example, one or more domains of an API can be identified by using one or more of the following programs: ProDom, TMpred, and SAPS. ProDom compares the amino acid sequence of a polypeptide to a database of compiled domains (see, *e.g.*, http://www.toulouse.inra.fr/prodom.html; Corpet F., Gouzy J. & Kahn D., 1999, Nucleic Acids Res., 27:263-267). TMpred predicts membrane-spanning regions of a polypeptide and their orientation. This program uses an algorithm that is based on the statistical analysis of TMbase, a database of naturally occuring transmembrane proteins (see, *e.g.*, http://www.ch.embnet.org/sonware/TMPRED_form.html; Hofmann & Stoffel. (1993) "TMbase - A database of membrane spanning proteins segments." Biol. Chem. Hoppe-Seyler 347,166). The SAPS program analyzes polypeptides for statistically significant features like charge-clusters, repeats, hydrophobic regions, compositional domains (see, *e.g.*, Brendel et al., 1992, Proc. Natl. Acad. Sci. USA 89: 2002-2006). Thus, based on the present description, those skilled in the art can identify domains of an API having enzymatic or binding activity, and further can identify nucleotide sequences encoding such domains. These nucleotide sequences can then be used for recombinant expression of an API fragment that retains the enzymatic or binding activity of the API.

Based on the present description, those skilled in the art can identify domains of an API having enzymatic or binding activity, and further can identify nucleotide sequences encoding such domains. These nucleotide sequences can then be used for recombinant expression of API fragments that retain the enzymatic or binding activity of the API.

In one embodiment, an API has an amino acid sequence sufficiently similar to an identified domain of a known polypeptide. As used herein, the term "functionally similar" refers to a first amino acid or nucleotide sequence which contains a sufficient number of identical or equivalent (*e.g.*, with a similar side chain) amino acid residues or nucleotides to a second amino acid or nucleotide sequence such that the first and second amino acid or nucleotide sequences have or encode a common structural domain or common functional activity or both.

An API domain can be assessed for its function using techniques well known to those of skill in the art. For example, a domain can be assessed for its kinase activity or for its ability to bind to DNA using techniques known to the skilled artisan. Kinase activity can be assessed, for example, by measuring the ability of a polypeptide to phosphorylate a substrate. DNA binding activity can be assessed, for example, by measuring the ability of a polypeptide to bind to a DNA binding element in a electromobility shift assay. In a preferred embodiment, the function of a domain of an API is determined using an assay described in one or more of the references identified in Table IX, *infra*.

### 5.11 Production of Antibodies to APIs

According to the invention API, API fragment, API -related polypeptide or API -fusion protein may be used as an immunogen to generate antibodies which immunospecifically bind such an immunogen. Such immunogens can be isolated by any convenient means, including the methods described above. Antibodies of the invention include, but are not limited to polyclonal, monoclonal, bispecific, humanized or chimeric antibodies, single chain antibodies, Fab fragments and F(ab') fragments, fragments produced by a Fab expression library, anti-idiotypic (anti-Id) antibodies, and epitope-binding fragments of any of the above. The term "antibody" as used herein refers to immunoglobulin molecules and immunologically active portions of immunoglobulin molecules, *i.e.*, molecules that contain an antigen-binding site that specifically binds an antigen. The immunoglobulin molecules of the invention can be of any class (*e.g.*, IgG, IgE, IgM, IgD and IgA ) or subclass of immunoglobulin molecule.

In one embodiment, antibodies that recognize gene products of genes encoding APIs may be prepared. For example, antibodies that recognize these APIs and/or their isoforms include antibodies recognizing API-1, API-3, API-4, API-6, API-7, API-10, API-15, API-16, API-22, API-28, API-30, API-33, API-34, API-37, API-38, API-39, API-40, API-42, API-43, API-44, API-45, API-46, API-47, API-50, API-52, API-53, API-55, APT-58, API-60, API-62, API-64, API-66, API-67, API-69, API-72, API-74, API-75, API-76, API-77, API-78, API-79, API-80, API-81, API-82, API-84, API-90, API-92, API-93, API-95, API-97, API-98, API-101, API-102, API-103, API-104, API-113, API-118, API-119, API-123, API-124, API-126, API-130, API-131, API-132, API-134, API-136, API-137, API-138, API-140, API-142, API-143, API-145, API-149, API-150, API-161, API-165, API-167, API-168, API-169, API-170, API-171, API-172, API-173, API-174, API-175, API-176, API-178, API-179, API-181, API-182, API-186, API-188, API-189, API-191, API-194, API-196, API-201, API-215, API-220, API-221, API-223, API-225, API-233, API-234, API-237, API-238, API-239, API-240, API-241, API-242, API-243, API-244, API-245, API-246, API-247, API-248, API-300, API-301, API-302, API-303, API-304, API-305, API-306, API-307, API-308, API-309, API-310, API-311, API-312, API-313, API-314, API-315, API-316, API-317, APT-318, API-319, API-320, API-321, API-322, API-323, API-324, API-325, API-326, API-327, API-328, API-329, API-330, API-331, API-332, API-333, API-334, API-335, API-336, API-337, API-338, API-339, API-340, API-341, API-342, API-343, API-344, API-345, API-346, API-347, API-348, API-349, API-350, API-351, API-352, API-353, API-354, API-355, API-356, API-357, API-358, API-359, API-360, API-361, API-362, API-363, API-364, API-365, API-366, API-367 or API-368. Certain antibodies are already known and can be purchased from commercial sources as shown in Table VII above. In another embodiment, methods known to those skilled in the art are used to produce antibodies that recognize an API, an API analog, an API-related polypeptide, or a derivative or fragment of any of the foregoing.

In one embodiment of the invention, antibodies to a specific domain of an API are produced. In a specific embodiment, hydrophilic fragments of an API are used as immunogens for antibody production.

In the production of antibodies, screening for the desired antibody can be accomplished by techniques known in the art, *e.g.* ELISA (enzyme-linked immunosorbent assay). For example, to select antibodies which recognize a specific domain of an API, one may assay generated hybridomas for a product which binds to an API fragment containing such domain. For selection of an antibody that specifically binds a first API homolog but which does not specifically bind to (or binds less avidly to) a second API homolog, one can select on the basis of positive binding to the first API homolog and a lack of binding to (or reduced binding to) the second API homolog. Similarly, for selection of an antibody that specifically binds an API but which does not specifically bind to (or binds less avidly to) a different isoform of the same protein (such as a different glycoform having the same core peptide as the API), one can select on the basis of positive binding to the API and a lack of binding to (or reduced binding to) the different isoform (*e.g.*, a different glycoform). Thus, the present invention provides an antibody (particularly a monoclonal antibody) that binds with greater affinity (particularly at least 2-fold, more particularly at least 5-fold still more particularly at least 10-fold greater affinity) to an API than to a different isoform or isoforms (*e.g.*, glycoforms) of the API.

Polyclonal antibodies, which may be used in the methods of the invention, are heterogeneous populations of antibody molecules derived from the sera of immunized animals. Unfractionated immune serum can also be used. Various procedures known in the art may be used for the production of polyclonal antibodies to an API, API fragment, API -related polypeptide or API -fusion protein. In a particular embodiment, rabbit polyclonal antibodies to an epitope of an API, API fragment, API -related polypeptide or API -fusion protein can be obtained. For example, for the production of polyclonal or monoclonal antibodies, various host animals can be immunized by injection with the native or a synthetic (*e.g.*, recombinant) version of an API, API fragment, API -related polypeptide or API -fusion protein, including but not limited to rabbits, mice, rats, etc. Isolated APIs suitable for such immunization may be obtained by the use of discovery techniques, such as the preferred technology described herein. If the API is purified by gel electrophoresis, the API can be used for immunization with or without prior extraction from the polyacrylamide gel. Various adjuvants may be used to enhance the immunological response, depending on the host species, including, but not limited to, complete or incomplete Freund's adjuvant, a mineral gel such as aluminum hydroxide, surface active substance such as lysolecithin, pluronic polyol, a polyanion, a peptide, an oil emulsion, keyhole limpet hemocyanin, dinitrophenol, and an adjuvant such as BCG (bacille Calmette-Guerin) or corynebacterium parvum. Additional adjuvants are also well known in the art.

For preparation of monoclonal antibodies (mAbs) directed toward an API, API fragment, API - related polypeptide or API -fusion protein, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs of the invention may be cultivated *in vitro* or *in vivo*. In an additional embodiment of the invention, monoclonal antibodies can be produced in germ-free animals utilizing known technology (PCT/US90/02545, incorporated herein by reference).

The monoclonal antibodies include but are not limited to human monoclonal antibodies and chimeric monoclonal antibodies (*e.g.*, human-mouse chimeras). Humanized antibodies are antibody molecules from non-human species having one or more complementarily determining regions (CDRs) from the non-human species and a framework region from a human immunoglobulin molecule. (See, *e.g* Queen, U.S. Patent No. 5,585,089, which is incorporated herein by reference in its entirety.)

Chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al., 1988, Science 240:1041-1043; Liu et al., 1987, Proc. Natl. Acad. Sci. USA., 84:3439-3443; Liu et al., 1987, J. Immunol. 139:3521-3526; Sun et al., 1987, Proc. Natl. Acad. Sci. USA., 84:214-218; Nishimura et al., 1987, Canc. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, Bio/Techniques 4:214; U.S. Patent 5,225,539; Jones et al., 1986, Nature 321:552-525; Verhoeyan et al. (1988) Science 239:1534; and Beidler et al., 1988, J. Immunol. 141;4053-4060.

Completely human antibodies (antibodies derived solely from human antigenic material) are particularly desirable for therapeutic treatment of human subjects. Such antibodies can be produced using transgenic mice which are incapable of expressing endogenous immunoglobulin heavy and light chains genes, but which can express human heavy and light chain genes. The transgenic mice are immunized in the normal fashion with selected antigens, *e.g.*, all or a portion of an API of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, see, *e.g.*, U.S. Patent 5,625,126, U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognize a selected epitope can be generated using a technique referred to as "guided selection." In this approach a selected non-human monoclonal antibody, *e.g.*, a mouse antibody, is used to guide the selection of a completely human antibody recognizing the same epitope. (Jespers et al. (1994) Biotechnology 12:899-903).

The antibodies of the present invention can also be generated using various phage display methods known in the art. In phage display methods, functional antibody domains are displayed on the surface of phage particles which carry the polynucleotide sequences encoding them. In a particular, such phage can be utilized to display antigen-binding domains expressed from a repertoire or combinatorial antibody library (*e.g.*, human or murine). Phage expressing an antigen binding domain that binds the antigen of interest can be selected or identified with antigen, *e.g.*, using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Phage display methods that can be used to make the antibodies of the present invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT Application No. PCT/GB91/01134; PCT Publications WO 90/02809; WO 91/10737; WO 92/01047; WO 92/18619; WO 93/11236; WO 95115982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108; each of which is incorporated herein by reference in its entirety,

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g.*, as described in detail below. For example, techniques to recombinantly produce Fab, Fab' and F(ab')2 fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988) (said references incorporated by reference in their entireties).

Examples of suitable techniques which can be used to produce single-chain Fvs and antibodies against APIs of the present invention include those described in U.S. Patents 4,946,778 and 5,258,498; Huston et al., Methods in Enzymology 203:46-88 (1991); Shu et al., PNAS 90:7995-7999 (1993); and Skerra et al., Science 240:1038-1040 (1988).

The invention further provides for the use of bispecific antibodies, which can be made by methods known in the art. Traditional production of full-length bispecific antibodies is based on the coexpression of two immunoglobulin heavy chain-light chain pairs, where the two chains have different specificities (Milstein et al., 1983, Nature 305:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, published 13 May 1993, and in Traunecker et al., 1991, EMBO J. 10:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690 published March 3,1994. For further details for generating bispecific antibodies see, for example, Suresh et al., Methods in Enzymology, 1986, 121:210.

The invention provides functionally active fragments, derivatives or analogs of the anti-API immunoglobulin molecules. Functionally active means that the fragment, derivative or analog is able to elicit anti-anti-idiotype antibodies (i.e., tertiary antibodies) that recognize the same antigen that is recognized by the antibody from which the fragment, derivative or analog is derived. Specifically, in a preferred embodiment the antigenicity of the idiotype of the immunoglobulin molecule may be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any suitable binding assay known in the art.

The present invention provides antibody fragments such as, but not limited to, F(ab')2 fragments and Fab fragments. Antibody fragments which recognize specific epitopes may be generated by known techniques. F(ab')2 fragments consist of the variable region, the light chain constant region and the CH1 domain of the heavy chain and are generated by pepsin digestion of the antibody molecule. Fab fragments are generated by reducing the disulfide bridges of the F(ab')2 fragments. The invention also provides heavy chain and light chain dimers of the antibodies of the invention, or any minimal fragment thereof such as Fvs or single chain antibodies (SCAs) (*e.g.*, as described in U.S. Patent 4,946,778; Bird, 1988, Science 242:423-42; Huston et al., 1988, Proc. Natl. Acad. Sci. USA 85:5879-5883; and Ward et al., 1989, Nature 334:544-54), or any other molecule with the same specificity as the antibody of the invention. Single chain antibodies are formed by linking the heavy and light chain fragments of the Fv region via an amino acid bridge, resulting in a single chain polypeptide. Techniques for the assembly of functional Fv fragments in E. coli may be used (Skerra et al., 1988, Science 242:1038-1041).

In other embodiments, the invention provides fusion proteins of the immunoglobulins of the invention (or functionally active fragments thereof), for example in which the immunoglobulin is fused via a covalent bond (*e.g.*, a peptide bond), at either the N-terminus or the C-terminus to an amino acid sequence of another protein (or portion thereof, preferably at least 10, 20 or 50 amino acid portion of the protein) that is not the immunoglobulin. Preferably the immunoglobulin, or fragment thereof, is covalently linked to the other protein at the N-terminus of the constant domain. As stated above, such fusion proteins may facilitate purification, increase half-life *in vivo*, and enhance the delivery of an antigen across an epithelial barrier to the immune system.

The immunoglobulins of the invention include analogs and derivatives that are either modified, i.e, by the covalent attachment of any type of molecule as long as such covalent attachment that does not impair immunospecific binding. For example, but not by way of limitation, the derivatives and analogs of the immunoglobulins include those that have been further modified, *e.g.*, by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analog or derivative may contain one or more non-classical or unnatural amino acids.

The foregoing antibodies can be used in methods known in the art relating to the localization and activity of the APIs of the invention, *e.g.*, for imaging these proteins, measuring levels thereof in appropriate physiological samples, in diagnostic methods, etc.

### 5.12 Expression Of Antibodies

The antibodies of the invention can be produced by any suitable method known in the art for the synthesis of antibodies, in particular, by chemical synthesis or by recombinant expression, and are preferably produced by recombinant expression techniques.

Recombinant expression of antibodies, or fragments, derivatives or analogs thereof, requires construction of a nucleic acid that encodes the antibody. If the nucleotide sequence of the antibody is known, a nucleic acid encoding the antibody may be assembled from chemically synthesized oligonucleotides (*e.g.*, as described in Kutmeier et al., 1994, BioTechniques 17:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (*e.g.*, an antibody cDNA library, or cDNA library generated from any tissue or cells expressing the antibody) by PCR amplification using synthetic primers hybridizable to the 3' and 5' ends of the sequence or by cloning using an oligonucleotide probe specific for the particular gene sequence.

If an antibody molecule that specifically recognizes a particular antigen is not available (or a source for a cDNA library for cloning a nucleic acid encoding such an antibody), antibodies specific for a particular antigen may be generated by any method known in the art, for example, by immunizing an animal, such as a rabbit, to generate polyclonal antibodies or, more preferably, by generating monoclonal antibodies. Alternatively, a clone encoding at least the Fab portion of the antibody may be obtained by screening Fab expression libraries (*e.g.*, as described in Huse et al., 1989, Science 246:1275-1281) for clones of Fab fragments that bind the specific antigen or by screening antibody libraries (See, *e.g.*, Clackson et al., 1991, Nature 352:624; Hane et al., 1997 Proc. Natl. Acad. Sci. USA 94:4937).

Once a nucleic acid encoding at least the variable domain of the antibody molecule is obtained, it may be introduced into a vector containing the nucleotide sequence encoding the constant region of the antibody molecule (see, *e.g.*, WO 86/05807; WO 89/01036; and U.S. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulfide bond with an amino acid residue that does not contain a sulfhydyl group. Such modifications can be carried out by any method known in the art for the introduction of specific mutations or deletions in a nucleotide sequence, for example, but not limited to, chemical mutagenesis, *in vitro* site directed mutagenesis (Hutchinson et al., 1978, J. Biol. Chem. 253:6551), PCR based methods, etc.

In addition, techniques developed for the production of "chimeric antibodies" (Morrison et al., 1984, Proc. Natl. Acad. Sci. 81:851-855; Neuberger *et al.,* 1984, Nature 312:604-608; Takeda *et al.,* 1985, Nature 314:452-454) by splicing genes from a mouse antibody molecule of appropriate antigen specificity together with genes from a human antibody molecule of appropriate biological activity can be used. As described *supra,* a chimeric antibody is a molecule in which different portions are derived from different animal species, such as those having a variable region derived from a murine mAb and a human antibody constant region, *e.g.*, humanized antibodies.

Once a nucleic acid encoding an antibody molecule of the invention has been obtained, the vector for the production of the antibody molecule may be produced by recombinant DNA technology using techniques well known in the art. Thus, methods for preparing the protein of the invention by expressing nucleic acid containing the antibody molecule sequences are described herein. Methods which are well known to those skilled in the art, can be used to construct expression vectors containing an antibody molecule coding sequences and appropriate transcriptional and translational control signals. These methods include, for example, *in vitro* recombinant DNA techniques, synthetic techniques, and *in vivo* genetic recombination. See, for example, the techniques described in Sambrook et al. (1990, Molecular Cloning, A Laboratory Manual, 2d Ed., Cold Spring Harbor Laboratory, Cold Spring Harbor, NY) and Ausubel et al. (eds., 1998, Current Protocols in Molecular Biology, John Wiley & Sons, NY).

The expression vector is transferred to a host cell by conventional techniques and the transfected cells are then cultured by conventional techniques to produce an antibody of the invention.

The host cells used to express a recombinant antibody of the invention may be either bacterial cells such as *Escherichia coli*, or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus is an effective expression system for antibodies (Foecking *et al.,* 1986, Gene 45:101; Cockett et al., 1990, Bio/Technology 8:2).

A variety of host-expression vector systems may be utilized to express an antibody molecule of the invention. Such host-expression systems represent vehicles by which the coding sequences of interest may be produced and subsequently purified, but also represent cells which may, when transformed or transfected with the appropriate nucleotide coding sequences, express the antibody molecule of the invention in situ. These include but are not limited to microorganisms such as bacteria (*e.g., E. coli, B. subtilis*) transformed with recombinant bacteriophage DNA, plasmid DNA or cosmid DNA expression vectors containing antibody coding sequences; yeast (*e.g., Saccharomyces, Pichia*) transformed with recombinant yeast expression vectors containing antibody coding sequences; insect cell systems infected with recombinant virus expression vectors (*e.g.*, baculovirus) containing the antibody coding sequences; plant cell systems infected with recombinant virus expression vectors (*e.g.*, cauliflower mosaic virus, CaMV; tobacco mosaic virus, TMV) or transformed with recombinant plasmid expression vectors (*e.g.*, Ti plasmid) containing antibody coding sequences; or mammalian cell systems (*e.g.*, COS, CHO, BHK, HEK 293, 3T3 cells) harboring recombinant expression constructs containing promoters derived from the genome of mammalian cells (*e.g.*, metallothionein promoter) or from mammalian viruses (*e.g.*, the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for the generation of pharmaceutical compositions comprising an antibody molecule, vectors which direct the expression of high levels of fusion protein products that are readily purified may be desirable. Such vectors include, but are not limited, to the *E. coli* expression vector pUR278 (Ruther *et al.,* 1983, EMBO J. 2:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter). In mammalian host cells, a number of viral-based expression systems (*e.g.*, an adenovirus expression system) may be utilized.

As discussed above, a host cell strain may be chosen based on the present description which modulates the expression of the inserted sequences, or modifies and processes the gene product in the specific fashion desired. Such modifications (*e.g.*, glycosylation) and processing (*e.g.*, cleavage) of protein products may be important for the function of the protein.

For long-term, high-yield production of recombinant antibodies, stable expression is preferred. For example, cells lines that stably express an antibody of interest can be produced by transfecting the cells with an expression vector comprising the nucleotide sequence of the antibody and the nucleotide sequence of a selectable (*e.g.*, neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, Nature 322:52; Kohler, 1980, Proc. Natl. Acad. Sci. USA 77:2197). The coding sequences for the heavy and light chains may comprise cDNA or genomic DNA.

Once the antibody molecule of the invention has been recombinantly expressed, it may be purified by any method known in the art for purification of an antibody molecule, for example, by chromatography (*e.g.*, ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht *et al.,* 1991, Proc. Natl. Acad. Sci. USA 88:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni2+ nitriloacetic acid-agarose columns and histidine-tagged proteins are selectively eluted with imidazole-containing buffers.

### 5.13 Conjugated Antibodies

In a preferred embodiment, anti-API antibodies or fragments thereof are conjugated to a diagnostic or a therapeutic molecule. The antibodies can be used, for example, for diagnosis or to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present invention. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc.

Anti-API antibodies or fragments thereof can be conjugated to a therapeutic agent or pharmaceutical product to modify a given biological response. The therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator, a thrombotic agent or an anti-angiogenic agent, *e.g.*, angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

Techniques for conjugating such therapeutic moiety to antibodies are well known, see, *e.g.*, Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62:119-58 (1982). These references are incorporated herein in their entirety.

Alternatively, an antibody can be conjugated to a second antibody to form an antibody heteroconjugate as described in U.S. 4,676,980.

An antibody with or without a therapeutic moiety conjugated to it can be used as a therapeutic that is administered alone or in combination with cytotoxic factor(s) and/or cytokine(s).

### 5.14 Diagnosis of AD

In accordance with the present invention, suitable test samples, *e.g.*, of cerebrospinal fluid (CSF), serum, plasma or urine obtained from a subject suspected of having or known to have AD can be used for diagnosis. In one embodiment, a decreased abundance of one or more AFs or APIs (or any combination of them) in a test sample relative to a control sample (from a subject or subjects free from AD) or a previously determined reference range indicates the presence of AD; AFs and APIs suitable for this purpose are identified in Tables I and IV, respectively, as described in detail above. In another embodiment of the invention, an increased abundance of one or more AFs or APIs (or any combination of them) in a test sample compared to a control sample or a previously determined reference range indicates the presence of AD; AFs and APIs suitable for this purpose are identified in Tables II and V, respectively, as described in detail above. In another embodiment, the relative abundance of one or more AFs or APIs (or any combination of them) in a test sample compared to a control sample or a previously determined reference range indicates a subtype of AD (*e.g.*, familial or sporadic AD). In yet another embodiment, the relative abundance of one or more AFs or APIs (or any combination of them) in a test sample relative to a control sample or a previously determined reference range indicates the degree or severity of AD. In any of the aforesaid methods, detection of one or more APIs described herein may optionally be combined with detection of one or more additional biomarkers for AD including, but not limited to apoplipoprotein E (ApoE), amyloid β-peptides (Aβ), tau and neural thread protein (NTP). Any suitable method in the art can be employed to measure the level of AFs and APIs, including but not limited to the Preferred Technology described herein, kinase assays, immunoassays to detect and/or visualize the APIs (*e.g.*, Western blot, immunoprecipitation followed by sodium dodecyl sulfate polyacrylamide gel electrophoresis, immunocytochemistry, etc.). In cases where an API has a known function, an assay for that function may be used to measure API expression. In a further embodiment, a decreased abundance of mRNA encoding one or more APIs identified in Table IV (or any combination of them) in a test sample relative to a control sample or a previously determined reference range indicates the presence of AD. In yet a further embodiment, an increased abundance of mRNA encoding one or more APIs identified in Table V (or any combination of them) in a test sample relative to a control sample or previously determined reference range indicates the presence of AD. Any suitable hybridization assay can be used to detect API expression by detecting and/or visualizing mRNA encoding the API (*e.g.*, Northern assays, dot blots, in situ hybridization, etc.).

In another embodiment of the invention, labeled antibodies, derivatives and analogs thereof, which specifically bind to an API can be used for diagnostic purposes, *e.g.*, to detect, diagnose, or monitor AD. Preferably, AD is detected in an animal, more preferably in a mammal and most preferably in a human.

### 5.15 Screening Assays

The invention provides methods for identifying active agents (*e.g.*, chemical compounds, proteins, or peptides) that bind to an API or have a stimulatory or inhibitory effect on the expression or activity of an API. The term "active agent" includes APIs, API fragments, API-related polypeptides, anti-API antibodies, fragments of anti-API antibodes and agents which modulate the expression of APIs for example, but without limitation, agonists or antagonists of APIs. The invention also provides methods of identifying candidate agents that bind to an API-related polypeptide or an API fusion protein or have a stimulatory or inhibitory effect on the expression or activity of an API-related polypeptide or an API fusion protein. Examples of active agents or candidate agents include, but are not limited to, nucleic acids (*e.g.*, DNA and RNA), carbohydrates, lipids, proteins, peptides, peptidomimetics, small molecules and other drugs. Candidate agents can be obtained using any of the numerous suitable approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, 1997, Anticancer Drug Des. 12:145; U.S. Patent No. 5,738,996; and U.S. Patent No.5,807,683, each of which is incorporated herein in its entirety by reference).

Examples of methods for the synthesis of molecular libraries can be found in the art, for example in: DeWitt et al., 1993, Proc. Natl. Acad. Sci. USA 90:6909; Erb et al., 1994, Proc. Natl. Acad. Sci. USA 91:11422; Zuckermann et al., 1994, J. Med. Chem. 37:2678; Cho et al., 1993, Science 261:1303; Carrell et al., 1994, Angew. Chem. Int. Ed. Engl. 33:2059; Carell et al., 1994, Angew. Chem. Int. Ed. Engl. 33:2061; and Gallop et al., 1994, J. Med. Chem. 37:1233, each of which is incorporated herein in its entirety by reference.

Libraries of compounds may be presented, *e.g.*, presented in solution (*e.g.*, Houghten, 1992, Bio/Techniques 13:412-421), or on beads (Lam, 1991, Nature 354:82-84), chips (Fodor, 1993, Nature 364:555-556), bacteria (U.S. Patent No. 5,223,409), spores (Patent Nos. 5,571,698; 5,403,484; and 5,223,409), plasmids (Cull et al., 1992, Proc. Natl. Acad. Sci. USA 89:1865-1869) or phage (Scott and Smith, 1990, Science 249:386-390; Devlin, 1990, Science 249:404-406; Cwirla et al., 1990, Proc. Natl. Acad. Sci. USA 87:6378-6382; and Felici, 1991, J. Mol. Biol. 222:301-310).

In one embodiment, candidate agents that interact with APIs, API fragments, API -related polypeptides or API -fusion proteins are identified in a cell-based assay system. In accordance with this embodiment, cells expressing an API, API fragment, API-related polypeptide, or API-fusion protein are contacted with a candidate agent or a control agent and the ability of the candidate agent to interact with the API is determined. If desired, this assay may be used to screen a plurality (*e.g*. a library) of candidate agents. The cell, for example, can be of prokaryotic origin (*e.g.*, E. coli) or eukaryotic origin (*e.g.*, yeast or mammalian). Further, the cells can express the an API, API fragment, API-related polypeptide, or API-fusion protein endogenously or be genetically engineered to express the API, API fragment, API-related polypeptide, or API-fusion protein. In some embodiments, the API, fragment of the API, API-related polypeptide, a fragment of the API-related polypeptide, or an API fusion protein or the candidate agent is labeled, for example with a radioactive label (such as ³²P, ³⁵S or ¹²⁵I) or a fluorescent label (such as fluorescein isothiocyanate, rhodamine, phycoerythrin, phycocyanin, allophycocyanin, o-phthaldehyde or fluorescamine) to enable detection of an interaction between an API and a candidate agent. The ability of the candidate agent to interact directly or indirectly with an API, API fragment, API-related polypeptide, or API-fusion protein can be determined by methods known to those of skill in the art. For example, the interaction between a candidate agent and an an API, API fragment, API-related polypeptide, or API-fusion protein can be determined by flow cytometry, a scintillation assay, immunoprecipitation or western blot analysis.

In another embodiment, agents that interact with (*i.e.*, bind to) an API, API fragment, API-related polypeptide, or API-fusion protein are identified in a cell-free assay system. In accordance with this embodiment, a native or recombinant API or fragment thereof, or a native or recombinant API-related polypeptide or fragment thereof, or an API-fusion protein or fragment thereof, is contacted with a candidate agent or a control compound and the ability of the candidate compound to interact with the API, API fragment, API-related polypeptide, or API-fusion protein is determined. If desired, this assay may be used to screen a plurality (*e.g*. a library) of candidate compounds. Preferably, the API, API fragment, API-related polypeptide, or API-fusion protein is first immobilized, by, for example, contacting the API, API fragment, API-related polypeptide, or API-fusion protein with an immobilized antibody which specifically recognizes and binds it, or by contacting a purified preparation of the API, API fragment, API-related polypeptide, or API-fusion protein with a surface designed to bind proteins. The API, API fragment, API-related polypeptide, or API-fusion protein may be partially or completely purified (*e.g.*, partially or completely free of other polypeptides) or part of a cell lysate. Further, the API, API fragment, API-related polypeptide, a fragment of an API-related polypeptide may be a fusion protein comprising the API or a biologically active portion thereof, or API-related polypeptide and a domain such as glutathionine-S-transferase. Alternatively, the API, API fragment, API-related polypeptide, fragment of an API-related polypeptide or API fusion protein can be biotinylated using techniques well known to those of skill in the art (*e.g.*, biotinylation kit, Pierce Chemicals; Rockford, IL). The ability of the candidate compound to interact with an API, API fragment, API-related polypeptide, or API-fusion protein can be can be determined by methods known to those of skill in the art.

In another embodiment, a cell-based assay system is used to identify agents that bind to or modulate the activity of a protein, such as an enzyme, or a biologically active portion thereof, which is responsible for the production or degradation of an API or is responsible for the post- translational modification of an API. In a primary screen, a plurality (*e.g.*, a library) of compounds are contacted with cells that naturally or recombinantly express: (i) an API, API fragment, API-related polypeptide, or API-fusion protein; and (ii) a protein that is responsible for processing of the API, API fragment, API-related polypeptide, or API-fusion protein in order to identify compounds that modulate the production, degradation, or post-translational modification of the API, API fragment, API-related polypeptide, or API-fusion protein. If desired, compounds identified in the primary screen can then be assayed in a secondary screen against cells naturally or recombinantly expressing the specific API of interest. The ability of the candidate compound to modulate the production, degradation or post-translational modification of an API, API fragment, API-related polypeptide, or API-fusion protein can be determined by methods known to those of skill in the art, including without limitation, flow cytometry, a scintillation assay, immunoprecipitation and western blot analysis.

In another embodiment, agents that competitively interact with an API, API fragment, API-related polypeptide, or API-fusion protein are identified in a competitive binding assay. In accordance with this embodiment, cells expressing an API, API fragment, API-related polypeptide, or API-fusion protein are contacted with a candidate compound and a compound known to interact with the API, API fragment, API-related polypeptide, or API-fusion protein; the ability of the candidate compound to competitively interact with the API, API fragment, API-related polypeptide, or API-fusion protein is then determined. Alternatively, agents that competitively interact with an API, API fragment, API-related polypeptide, or API-fusion protein are identified in a cell-free assay system by contacting an API, API fragment, API-related polypeptide, or API-fusion protein with a candidate compound and a compound known to interact with the API, API fragment, API-related polypeptide, or API-fusion protein. As stated above, the ability of the candidate compound to interact with an API, API fragment, API-related polypeptide, or API-fusion protein can be determined by methods known to those of skill in the art. These assays, whether cell-based or cell-free, can be used to screen a plurality (*e.g.*, a library) of candidate compounds.

In another embodiment, agents that modulate the expression of an API, API fragment, API-related polypeptide, or API-fusion protein are identified by contacting cells (*e.g.*, cells of prokaryotic origin or eukaryotic origin) expressing the API, API fragment, API-related polypeptide, or API-fusion protein with a candidate agent or a control agent (*e.g.*, phosphate buffered saline (PBS)) and determining the expression of the API, API fragment, API-related polypeptide, or API-fusion protein, or the expression of mRNA encoding the API, API fragment, API-related polypeptide, or API-fusion protein. The level of expression of a selected API, API fragment, API-related polypeptide, or API-fusion protein or mRNA encoding the API, API fragment, API-related polypeptide, or API-fusion protein in the presence of the candidate compound is compared to the level of expression of the API, API fragment, API-related polypeptide, or API-fusion protein or mRNA encoding the API, API fragment, API-related polypeptide, or API-fusion protein in the absence of the candidate agent (*e.g.*, in the presence of a control agent). The candidate agent can then be identified as a modulator of the expression of the API, API fragment, API-related polypeptide, or API-fusion protein or mRNA encoding the API, API fragment, API-related polypeptide, or API-fusion protein based on this comparison. For example, when expression of the API or mRNA is significantly greater in the presence of the candidate agent than in its absence, the candidate agent is identified as a stimulator of expression of the API or its mRNA. Alternatively, when expression of the API or mRNA is significantly less in the presence of the candidate agent than in its absence, the candidate agent is identified as an inhibitor of the expression of the API or its mRNA. The level of expression of an API or the mRNA that encodes it can be determined by methods known to those of skill in the art based on the present description. For example, mRNA expression can be assessed by Northern blot analysis or RT-PCR, and protein levels can be assessed by western blot analysis.

In another embodiment, agents that modulate the activity of an API, API fragment, API-related polypeptide, or API-fusion protein are identified by contacting a preparation containing the API, API fragment, API-related polypeptide, or API-fusion protein, or cells (*e.g.*, prokaryotic or eukaryotic cells) expressing the API, API fragment, API-related polypeptide, or API-fusion protein with a candidate agent or a control agent and determining the ability of the candidate agent to modulate the activity of the API, API fragment, API-related polypeptide, or API-fusion protein. The activity of an API, API fragment, API-related polypeptide, or API-fusion protein can be assessed by detecting induction of a downstream effector of the API, API fragment, API-related polypeptide, or API-fusion protein (*e.g.*, intracellular Ca²⁺, diacylglycerol, IP3, etc.), detecting catalytic or enzymatic activity of the target on a suitable substrate, detecting the induction of a reporter gene ( *e.g.*, a regulatory element that is responsive to an API, API fragment, API-related polypeptide, or API-fusion protein and is operably linked to a nucleic acid encoding a detectable marker, *e.g.*, luciferase), or detecting a cellular response, for example, cellular differentiation, or cell proliferation as the case may be, based on the present description, techniques known to those of skill in the art can be used for measuring these activities (see, *e.g.*, U.S. 5,401,639). The candidate agent can then be identified as a modulator of the activity of an API, API fragment, API-related polypeptide, or API-fusion protein by comparing the effects of the candidate agent to the control agent. Suitable control agents include phosphate buffered saline (PBS) and normal saline (NS).

In another embodiment, agents that modulate the expression, activity or both the expression and activity of an API, API fragment, API-related polypeptide, or API-fusion protein are identified in an animal model. Examples of suitable animals include, but are not limited to, mice, rats, rabbits, monkeys, guinea pigs, dogs and cats. Preferably, the animal used represent a model of AD (*e.g.*, animals that express human familial AD (FAD) β-amyloid precursor (APP), animals that overexpress human wild-type APP, animals that overexpress β-amyloid 1-42 *(βA),* animals that express FAD presenillin-1 (PS-1). See, *e.g.*, Higgins, LS, 1999, Molecular Medicine Today 5:274-276. In accordance with this embodiment, the candidate agent or a control agent is administered (*e.g.*, orally, rectally or parenterally such as intraperitoneally or intravenously) to a suitable animal and the effect on the expression, activity or both expression and activity of the API, API fragment, API-related polypeptide, or API-fusion protein is determined. Changes in the expression of an API, API fragment, API-related polypeptide, or API-fusion protein can be assessed by any suitable method described above, based on the present description.

In yet another embodiment, an API, API fragment, API-related polypeptide, or API-fusion protein is used as a "bait protein" in a two-hybrid assay or three hybrid assay to identify other proteins that bind to or interact with an API, API fragment, API-related polypeptide, or API-fusion protein (see, *e.g.*, U.S. Patent No. 5,283,317; Zervos et al. (1993) Cell 72:223-232; Madura et al. (1993) J. Biol. Chem. 268:12046-12054; Bartel et al. (1993) Bio/Techniques 14:920-924; Iwabuchi et al. (1993) Oncogene 8:1693-1696; and PCT Publication No. WO 94/10300). As those skilled in the art will appreciate, such binding proteins are also likely to be involved in the propagation of signals by the APIs of the invention as, for example, upstream or downstream elements of a signaling pathway involving the APIs of the invention.

As those skilled in the art will appreciate, Table X enumerates scientific publications describing suitable assays for detecting or quantifying enzymatic or binding activity of an API, API fragment, API-related polypeptide, or API-fusion protein. Each such reference is hereby incorporated in its entirety. In a preferred embodiment, an assay referenced in Table X is used in the screens and assays described herein, for example, to screen for or to identify an agent that modulates the activity of (or that modulates both the expression and activity of) an API, API fragment, API-related polypeptide, or API-fusion protein.

### Table X.

**Table X**

| **API** | **References** |
|---|---|
| API-39, API-44, API-178, API-188 | Structural Biology 7, 312-321, 2000 J. Am. Chem. Soc. 122, 2178-2192, 2000 |
| API-123 API-126 | Neuroendocrinology 1992 Mar 55:3 308-16 |
| API-182 | Biochem J 1997 Mar 1 322 ( Pt 2): 455-60; Biochem Soc Trans 1997 Nov 25:4 S591; Biochim Biophys Acta 1986 Oct 10 888:3 325-31 http://www.promega.com |
| API-322 API-186 | "Assay of human transthyretin-bound holo-retinol-binding protein with reversed-phase high-performance liquid chromatography." J Chromatogr 1991 Jul 5 567:2 369-80 "Liquid-chromatographic assay for free and transthyretin-bound retinol-binding protein in serum from normal humans." Clin Chem 1989 Apr 35:4 582-6 |
| API-300 API-305 API-315 API-311 API-356 API-38 API-74 API-105 API-124 API-130 API-138 API-169 API-172 | "Development of a rapid kinetic assay for the function of the classical pathway of the complement system and for C2 and C4." J Clin Lab Immunol 1986 Dec 21:4 201-7 |
| API-361 API-52 | "High-performance liquid chromatographic assay of chymotrypsin and chymotrypsin-like enzyme activity." J Chromatogr 1987 411:498-501 "Assay for chymotrypsin and trypsin. II. On a few considerations for the assay of the commercial anti-inflammatry enzyme preparations." Eisei Shikenjo Hokoku 1972 90:89-92 "Effect of the reactant mixing sequence on the chymotrypsin inhibition assay." Analyst 1990 Aug 115:8 1143-4 |
| API-330 API-314 API-76 API-78 API-79 API-80 API-82 API-140 | "Time-resolved immunofluorometric assay of complement C3: application to cerebrospinal fluid." Clin Chem 1993 Feb 39:2 309-12 "A fluorimetric assay for native C3. The hemolytically active form of the third component of human complement." J Immunol Methods 1987, 102(1):7-14 |

This invention further provides novel agents identified by the above-described screening assays and uses thereof for treatments as described herein.

### 5.16 Therapeutic Uses Of APIs

The invention provides for treatment or prevention of various diseases and disorders by administration of a therapeutic agent. Such agents include but are not limited to: APIs, API analogs, API-related polypeptides and derivatives (including fragments) thereof; antibodies to the foregoing; nucleic acids encoding API, API fragment, API-related polypeptide, or API-fusion protein; antisense nucleic acids to a gene encoding an API, API fragment, API-related polypeptide, or API-fusion protein; and modulator (*e.g.*, agonists and antagonists) of a gene encoding an API or API-related polypeptide. An important feature of the present invention is the identification of genes encoding APIs involved in AD. AD can be treated (*e.g.* to ameliorate symptoms or to retard onset or progression) or prevented by administration of a therapeutic compound that promotes function or expression of one or more APIs that are decreased in the CSF of subjects having AD, or by administration of a therapeutic compound that reduces function or expression of one or more APIs that are increased in the CSF of subjects having AD.

In one embodiment, one or more antibodies each specifically binding to an API are administered alone or in combination with one or more additional therapeutic compounds or treatments. Examples of such therapeutic compounds or treatments include, but are not limited to, tacrine, donepezil, α-tocopherol, selegeline, NSAIDs, estrogen replacement therapy, physostigmine, rivastigmine, hepastigmine, metrifonate, ENA-713, ginkgo biloba extract, physostigmine, amridin, talsaclidine, zifrosilone, eptastigmine, methanesulfonyl chloride, nefiracetam, ALCAR, talsachidine, xanomeline, galanthamine, and propentofylline.

Preferably, a biological product such as an antibody is allogeneic to the subject to which it is administered. In a preferred embodiment, a human API or a human API-related polypeptide, a nucleotide sequence encoding a human API or a human API-related polypeptide, or an antibody to a human API or a human API-related polypeptide, is administered to a human subject for therapy (*e.g.* to ameliorate symptoms or to retard onset or progression) or prophylaxis.

### 5.16.1 Treatment And Prevention Of AD

AD can be treated or prevented by administration to a subject suspected of having or known to have AD or to be at risk of developing AD following administration of an agent that modulates (*i.e.,* increases or decreases) the level or activity (*i.e.*, function) of one or more APIs or the level of one or more AFs that are differentially present in the CSF of subjects having AD compared with CSF of subjects free from AD. In one embodiment, AD is treated by administering to a subject suspected of having or known to have AD or to be at risk of developing AD an agent that upregulates (*i.e.*, increases) the level or activity (*i.e.*, function) of one or more APIs or the level of one or more AFs that are decreased in the CSF of subjects having AD. In another embodiment, an agent is administered that downregulates the level or activity *(i.e.,* function) of one or more APIs - or the level of one or more AFs - that are increased in the CSF of subjects having AD. Examples of such a compound include but are not limited to: APIs, API fragments and API-related polypeptides; nucleic acids encoding an API, an API fragment and an API-related polypeptide (*e.g*., for use in gene therapy); and, for those APIs or API-related polypeptides with enzymatic activity, compounds or molecules known to modulate that enzymatic activity. Other compounds that can be used, *e.g.*, API agonists, can be identified using in vitro assays, as defined or described above or earlier.

AD is also treated or prevented by administration to a subject suspected of having or known to have AD or to be at risk of developing AD of a compound that downregulates the level or activity of one or more APIs - or the level of one or more AFs - that are increased in the CSF of subjects having AD. In another embodiment, a compound is administered that upregulates the level or activity of one or more APIs - or the level of one or more AFs - that are decreased in the CSF of subjects having AD. Examples of such a compound include, but are not limited to, API antisense oligonucleotides, ribozymes, antibodies directed against APIs, and compounds that inhibit the enzymatic activity of an API. Other useful compounds *e.g.*, API antagonists and small molecule API antagonists, can be identified using *in vitro* assays.

In a preferred embodiment, therapy or prophylaxis is tailored to the needs of an individual subject. Thus, in specific embodiments, compounds that promote the level or function of one or more APIs, or the level of one or more AFs, are therapeutically or prophylactically administered to a subject suspected of having or known to have AD, in whom the levels or functions of said one or more APIs, or levels of said one or more AFs, are absent or are decreased relative to a control or normal reference range. In further embodiments, compounds that promote the level or function of one or more APIs, or the level of one or more AFs, are therapeutically or prophylactically administered to a subject suspected of having or known to have AD in whom the levels or functions of said one or more APIs, or levels of said one or more AFs, are increased relative to a control or to a reference range. In further embodiments, compounds that decrease the level or function of one or more APIs, or the level of one or more AFs, are therapeutically or prophylactically administered to a subject suspected of having or known to have AD in whom the levels or functions of said one or more APIs, or levels of said one or more AFs, are increased relative to a control or to a reference range. In further embodiments, compounds that decrease the level or function of one or more APIs, or the level of one or more AFs, are therapeutically or prophylactically administered to a subject suspected of having or known to have AD in whom the levels or functions of said one or more APIs, or levels of said one or more AFs, are decreased relative to a control or to a reference range. The change in API function or level, or AF level, due to the administration of such compounds can be readily detected, *e.g.*, by obtaining a sample (*e.g.*, a sample of CSF, blood or urine or a tissue sample such as biopsy tissue) and assaying *in vitro* the levels of said AFs or the levels or activities of said APIs, or the levels of mRNAs encoding said APIs. or any combination of the foregoing. Such assays can be performed before and after the administration of the compound as described herein.

The compounds of the invention include but are not limited to any compound, *e.g.*, a small organic molecule, protein, peptide, antibody, nucleic acid, etc. that restores the AD API or AF profile towards normal with the proviso that such compound is not an acetylcholinesterase (AChE) inhibitor (*e.g.*, tacrine, donepezil, rivastigmine, hepastigmine, Metrigonate, physostigmine, Amridin, Talsaclidine, KA-672, Huperzine, P-11012, P- 11149, Zifrosilone, Eptastigmine, Methanesulfonyl chloride, and S-9977), an acetylcholine receptor agonist (*e.g.*, Nefiracetam, LU-25109, and NS2330), a muscarinic receptor agonist (*e.g.*, SB-20206, Talsachidine, AF-1025B, and SR-46559A), a nicotonic cholinergic receptor agonist (*e.g.*, ABT-418), an acetylcholine modulator (*e.g.*, FKS-508 and Galantamine) or propentofylline.

### 5.16.2 Gene Therapy

In another embodiment, nucleic acids comprising a sequence encoding an API, an API fragment, API-related polypeptide or fragment of an API-related polypeptide, are administered to promote API function by way of gene therapy. Gene therapy refers to the administration of an expressed or expressible nucleic acid to a subject. In this embodiment, the nucleic acid produces its encoded polypeptide and the polypeptide mediates a therapeutic effect by promoting API function.

Any suitable methods for gene therapy available in the art can be used according to the present invention.

For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62:191-217; May, 1993, TIBTECH 11(5):155-215. Methods commonly known in the art of recombinant DNA technology which can be used in the present invention are described in Ausubel et. al. (eds.), 1993, Current Protocols in Molecular Biology, John Wiley & Sons, NY; and Kriegler, 1990, Gene Transfer and Expression, A Laboratory Manual, Stockton Press, NY.

In a particular aspect, the compound comprises a nucleic acid encoding an API or fragment or chimeric protein thereof, said nucleic acid being part of an expression vector that expresses an API or fragment or chimeric protein thereof in a suitable host. In particular, such a nucleic acid has a promoter operably linked to the API coding region, said promoter being inducible or constitutive (and, optionally, tissue-specific). In another particular embodiment, a nucleic acid molecule is used in which the API coding sequences and any other desired sequences are flanked by regions that promote homologous recombination at a desired site in the genome, thus providing for intrachromosomal expression of the API nucleic acid (Koller and Smithies, 1989, Proc. Natl. Acad. Sci. USA 86:8932-8935; Zijlstra et al., 1989, Nature 342:435-438).

Delivery of the nucleic acid into a subject may be direct, in which case the subject is directly exposed to the nucleic acid or nucleic acid-carrying vector; this approach is known as *in vivo* gene therapy. Alternatively, delivery of the nucleic acid into the subject may be indirect, in which case cells are first transformed with the nucleic acid *in vitro* and then transplanted into the subject, known as "*ex vivo* gene therapy".

In another embodiment, the nucleic acid is directly administered *in vivo*, where it is expressed to produce the encoded product. This can be accomplished by any of numerous methods known in the art, *e.g.*, by constructing it as part of an appropriate nucleic acid expression vector and administering it so that it becomes intracellular, *e.g.*, by infection using a defective or attenuated retroviral or other viral vector (see U.S. Patent No. 4,980,286); by direct injection of naked DNA; by use of microparticle bombardment (*e.g.*, a gene gun; Biolistic, Dupont); by coating with lipids, cell-surface receptors or transfecting agents; by encapsulation in liposomes, microparticles or microcapsules; by administering it in linkage to a peptide which is known to enter the nucleus; or by administering it in linkage to a ligand subject to receptor-mediated endocytosis (see, *e.g.*, Wu and Wu, 1987, J. Biol. Chem. 262:4429-4432), which can be used to target cell types specifically expressing the receptors. In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (see, *e.g.*, PCT Publications WO 92/06180 dated April 16, 1992 (Wu et al.); WO 92/22635 dated December 23, 1992 (Wilson et al.); WO92/2031 dated November 26, 1992 (Findeis et al.); WO93/14188 dated July 22, 1993 (Clarke et al.), WO 93/20221 dated October 14, 1993 (Young)). Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

In a further embodiment, a viral vector that contains a nucleic acid encoding an API is used. For example, a retroviral vector can be used (see Miller et al., 1993, Meth. Enzymol. 217:581-599). These retroviral vectors have been modified to delete retroviral sequences that are not necessary for packaging of the viral genome and integration into host cell DNA. The nucleic acid encoding the API to be used in gene therapy is cloned into the vector, which facilitates delivery of the gene into a subject. More detail about retroviral vectors can be found in Boesen et al., 1994, Biotherapy 6:291-302, which describes the use of a retroviral vector to deliver the mdr1 gene to hematopoietic stem cells in order to make the stem cells more resistant to chemotherapy. Other references illustrating the use of retroviral vectors in gene therapy are: Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, 1993, Current Opinion in Genetics and Development 3:499-503 present a review of adenovirus-based gene therapy. Bout et al., 1994, Human Gene Therapy 5:3-10 demonstrated the use of adenovirus vectors to transfer genes to the respiratory epithelia of rhesus monkeys. Other instances of the use of adenoviruses in gene therapy can be found in Rosenfeld et al., 1991, Science 252:431-434; Rosenfeld et al., 1992, Cell 68:143-155; Mastrangeli et al., 1993, J. Clin. Invest. 91:225-234; PCT Publication WO94/12649; and Wang, et al., 1995, Gene Therapy 2:775-783.

Adeno-associated virus (AAV) has also been proposed for use in gene therapy (Walsh et al., 1993, Proc. Soc. Exp. Biol. Med. 204:289-300; U.S. Patent No. 5,436,146).

Another suitable approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a subject.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, *e.g.*, Loeffler and Behr, 1993, Meth. Enzymol. 217:599-618; Cohen et al., 1993, Meth. Enzymol. 217:618-644; Cline, 1985, Pharmac. Ther. 29:69-92) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a subject by various methods known in the art. In a preferred embodiment, epithelial cells are injected, i, subcutaneously. In another embodiment, recombinant skin cells may be applied as a skin graft onto the subject. Recombinant blood cells (*e.g.*, hematopoietic stem or progenitor cells) are preferably administered intravenously. The amount of cells envisioned for use depends on the desired effect, the condition of the subject, etc., and can be determined by one skilled in the art.

Cells into which a nucleic acid can be introduced for purposes of gene therapy encompass any desired, available cell type, and include but are not limited to neuronal cells, glial cells (*e.g.*, oligodendrocytes or astrocytes), epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, *e.g.*, as obtained from bone marrow, umbilical cord blood, peripheral blood or fetal liver.

In a preferred embodiment, the cell used for gene therapy is autologous to the subject that is treated.

In an embodiment in which recombinant cells are used in gene therapy, a nucleic acid encoding an API is introduced into the cells such that it is expressible by the cells or their progeny, and the recombinant cells are then administered in vivo for therapeutic effect In a specific embodiment, stem or progenitor cells are used. Any stem or progenitor cells which can be isolated and maintained *in vitro* can be used in accordance with this embodiment of the present invention (see *e.g.* PCT Publication WO 94/08598, dated April 28, 1994; Stemple and Anderson, 1992, Cell 71:973-985; Rheinwald, 1980, Meth. Cell Bio. 21A:229; and Pittelkow and Scott, 1986, Mayo Clinic Proc. 61:771).

In another embodiment, the nucleic acid to be introduced for purposes of gene therapy may comprise an inducible promoter operably linked to the coding region, such that expression of the nucleic acid is controllable by controlling the presence or absence of the appropriate inducer of transcription.

Direct injection of a DNA coding for an API may also be performed according to, for example, the techniques described in United States Patent No. 5,589,466. These techniques involve the injection of "naked DNA", *i.e.*, isolated DNA molecules in the absence of liposomes, cells, or any other material besides a suitable carrier. The injection of DNA encoding a protein and operably linked to a suitable promoter results in the production of the protein in cells near the site of injection and the elicitation of an immune response in the subject to the protein encoded by the injected DNA. In a preferred embodiment, naked DNA comprising (a) DNA encoding an API and (b) a promoter are injected into a subject to elicit an immune response to the API.

### 5.16.3 Inhibition Of APIs To Treat AD

In one embodiment of the invention, AD is treated or prevented by administration of a compound that antagonizes (inhibits) the level(s) and/or function(s) of one or more APIs which are elevated in the CSF of subjects having AD as compared with CSF of subjects free from AD. Compounds useful for this purpose include but are not limited to anti-API antibodies (and fragments and derivatives containing the binding region thereof), API antisense or ribozyme nucleic acids, and nucleic acids encoding dysfunctional APIs that are used to "knockout" endogenous API function by homologous recombination (see, *e.g.*, Capecchi, 1989, Science 244:1288-1292). Other compounds that inhibit API function can be identified by use of known *in vitro* assays, *e.g.*, assays for the ability of a test compound to inhibit binding of an API to another protein or a binding partner, or to inhibit a known API function. Preferably such inhibition is assayed *in vitro* or in cell culture, but genetic assays may also be employed. The Preferred Technology can also be used to detect levels of the API before and after the administration of the compound. Preferably, suitable *in vitro* or *in vivo* assays are utilized to determine the effect of a specific compound and whether its administration is indicated for treatment of the affected tissue, as described in more detail below.

In a particular embodiment, a compound that inhibits an API function is administered therapeutically or prophylactically to a subject in whom an increased CSF level or functional activity of the API (*e.g.*, greater than the normal level or desired level) is detected as compared with CSF of subjects free from AD or a predetermined reference range. Methods standard in the art can be employed to measure the increase in an API level or function, as outlined above. Preferred API inhibitor compositions include small molecules, *i.e.*, molecules of 1000 daltons or less. Such small molecules can be identified by the screening methods described herein.

### 5.16.4 Antisense Regulation of APIs

In a further embodiment, API expression is inhibited by use of API antisense nucleic acids. The present invention provides the therapeutic or prophylactic use of nucleic acids comprising at least six nucleotides that are antisense to a gene or cDNA encoding an API or a portion thereof. As used herein, an API "antisense" nucleic acid refers to a nucleic acid capable of hybridizing by virtue of some sequence complementarity to a portion of an RNA (preferably mRNA) encoding an API. The antisense nucleic acid may be complementary to a coding and/or noncoding region of an mRNA encoding an API. Such antisense nucleic acids have utility as compounds that inhibit API expression, and can be used in the treatment or prevention of AD.

The antisense nucleic acids of the invention are double-stranded or single-stranded oligonucleotides, RNA or DNA or a modification or derivative thereof, and can be directly administered to a cell or produced intracellularly by transcription of exogenous, introduced sequences.

The invention further provides pharmaceutical compositions comprising a therapeutically effective amount of an API antisense nucleic acid, and a pharmaceutically-acceptable carrier, vehicle or diluent.

In another embodiment, the invention provides methods for inhibiting the expression of an API nucleic acid sequence in a prokaryotic or eukaryotic cell comprising providing the cell with an effective amount of a composition comprising an API antisense nucleic acid of the invention.

API antisense nucleic acids and their uses are described in detail below.

### 5.16.5 API Antisense Nucleic Acids

The API antisense nucleic acids are of at least six nucleotides and are preferably oligonucleotides ranging from 6 to about 50 oligonucleotides. In specific aspects, the oligonucleotide is at least 10 nucleotides, at least 15 nucleotides, at least 100 nucleotides, or at least 200 nucleotides. The oligonucleotides can be DNA or RNA or chimeric mixtures or derivatives or modified versions thereof and can be single-stranded or double-stranded. The oligonucleotide can be modified at the base moiety, sugar moiety, or phosphate backbone. The oligonucleotide may include other appended groups such as peptides; agents that facilitate transport across the cell membrane (see, *e.g.*, Letsinger et al., 1989, Proc. Natl. Acad. Sci. USA 86:6553-6556; Lemaitre et al., 1987, Proc. Natl. Acad. Sci. 84:648-652; PCT Publication No. WO 88/09810, published December 15, 1988) or blood-brain barrier (see, *e.g.*, PCT Publication No. WO 89/10134, published April 25, 1988); hybridization-triggered cleavage agents (see, *e.g.*, Krol et al., 1988, BioTechniques 6:958-976) or intercalating agents (see, *e.g.*, Zon, 1988, Pharm. Res. 5:539-549).

In a particular aspect of the invention, an API antisense oligonucleotide is provided, preferably of single-stranded DNA. The oligonucleotide may be modified at any position on its structure with substituents generally known in the art.

The API antisense oligonucleotide may comprise any suitable of the following modified base moieties, *e.g.* 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5□-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, 2,6-diaminopurine, and other base analogs.

In another embodiment, the oligonucleotide comprises at least one modified sugar moiety, *e.g.*, one of the following sugar moieties: arabinose, 2-fluoroarabinose, xylulose, and hexose.

In yet another embodiment, the oligonucleotide comprises at least one of the following modified phosphate backbones: a phosphorothioate, a phosphorodithioate, a phosphoramidothioate, a phosphoramidate, a phosphordiamidate, a methylphosphonate, an alkyl phosphotriester, a formacetal, or an analog of formacetal.

In yet another embodiment, the oligonucleotide is an α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641).

The oligonucleotide may be conjugated to another molecule, *e.g.*, a peptide, hybridization triggered cross-linking agent, transport agent, or hybridization-triggered cleavage agent.

Oligonucleotides of the invention may be synthesized by standard methods known in the art, *e.g.*, by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16:3209), and methylphosphonate oligonucleotides can be prepared by use of controlled pore glass polymer supports (Sarin et al., 1988, Proc. Natl. Acad. Sci. USA 85:7448-7451).

In another embodiment, the API antisense nucleic acid of the invention is produced intracellularly by transcription from an exogenous sequence. For example, a vector can be introduced *in vivo* such that it is taken up by a cell, within which cell the vector or a portion thereof is transcribed, producing an antisense nucleic acid (RNA) of the invention. Such a vector would contain a sequence encoding the API antisense nucleic acid. Such a vector can remain episomal or become chromosomally integrated, as long as it can be transcribed to produce the desired antisense RNA. Such vectors can be constructed by recombinant DNA technology standard in the art. Vectors can be plasmid, viral, or others known in the art, used for replication and expression in mammalian cells. Expression of the sequence encoding the API antisense RNA can be by any promoter known in the art to act in mammalian, preferably human, cells. Such promoters can be inducible or constitutive. Examples of such promoters are outlined above.

The antisense nucleic acids of the invention comprise a sequence complementary to at least a portion of an RNA transcript of a gene encoding an API, preferably a human gene encoding an API. However, absolute complementarity, although preferred, is not required. A sequence "complementary to at least a portion of an RNA," as referred to herein, means a sequence having sufficient complementarity to be able to hybridize under stringent conditions (*e.g.*, highly stringent conditions comprising hybridization in 7% sodium dodecyl sulfate (SDS), 1 mM EDTA at 65 □C and washing in 0.1xSSC/0.1% SDS at 68□C, or moderately stringent conditions comprising washing in 0.2xSSC/0.1% SDS at 42□C ) with the RNA, forming a stable duplex; in the case of double-stranded API antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA encoding an API it may contain and still form a stable duplex (or triplex, as the case may be). One skilled in the art can ascertain a tolerable degree of mismatch by use of standard procedures to determine the melting point of the hybridized complex.

### 5.16.6 Therapeutic Use Of API Antisense Nucleic Acids

The API antisense nucleic acids can be used to treat or prevent AD when the target API is overexpressed in the CSF of subjects suspected of having or suffering from AD. In a preferred embodiment, a single-stranded DNA antisense API oligonucleotide is used.

Cell types which express or overexpress RNA encoding an API can be identified by various methods known in the art. Such cell types include but are not limited to leukocytes (*e.g.*, neutrophils, macrophages, monocytes) and resident cells (*e.g.*, astrocytes, glial cells, neuronal cells, and ependymal cells). Such methods include, but are not limited to, hybridization with an API-specific nucleic acid (*e.g.*, by Northern hybridization, dot blot hybridization, in situ hybridization), observing the ability of RNA from the cell type to be translated in vitro into an API, immunoassay, etc. In a preferred aspect, primary tissue from a subject can be assayed for API expression prior to treatment, *e.g.*, by immunocytochemistry or in situ hybridization.

Pharmaceutical compositions of the invention, comprising an effective amount of an API antisense nucleic acid in a pharmaceutically acceptable carrier, vehicle or diluent can be administered to a subject having AD.

The amount of API antisense nucleic acid which will be effective in the treatment of AD can be determined by standard clinical techniques.

In a specific embodiment, pharmaceutical compositions comprising one or more API antisense nucleic acids are administered via liposomes, microparticles, or microcapsules. In various embodiments of the invention, such compositions may be used to achieve sustained release of the API antisense nucleic acids.

### 5.16.7 Inhibitory Ribozyme And Triple Helix Approaches

In another embodiment, symptoms of AD may be ameliorated by decreasing the level of an API or API activity by using gene sequences encoding the API in conjunction with well-known gene "knock-out," ribozyme or triple helix methods to decrease gene expression of an API. In this approach ribozyme or triple helix molecules are used to modulate the activity, expression or synthesis of the gene encoding the API, and thus to ameliorate the symptoms of AD. Such molecules may be designed to reduce or inhibit expression of a mutant or non-mutant target gene. Techniques for the production and use of such molecules are well known to those of skill in the art.

Ribozyme molecules designed to catalytically cleave gene mRNA transcripts encoding an API can be used to prevent translation of target gene mRNA and, therefore, expression of the gene product. (See, *e.g.*, PCT International Publication WO90/11364, published October 4, 1990; Sarver et al., 1990, Science 247:1222-1225).

Ribozymes are enzymatic RNA molecules capable of catalyzing the specific cleavage of RNA. (For a review, see Rossi, 1994, Current Biology 4, 469-471). The mechanism of ribozyme action involves sequence specific hybridization of the ribozyme molecule to complementary target RNA, followed by an endonucleolytic cleavage event. The composition of ribozyme molecules must include one or more sequences complementary to the target gene mRNA, and must include the well known catalytic sequence responsible for mRNA cleavage. For this sequence, see, *e.g*., U.S. Patent No. 5,093,246, which is incorporated herein by reference in its entirety.

While ribozymes that cleave mRNA at site specific recognition sequences can be used to destroy mRNAs encoding an API, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Myers, 1995, Molecular Biology and Biotechnology: A Comprehensive Desk Reference, VCH Publishers, New York, (see especially Figure 4, page 833) and in Haseloff and Gerlach, 1988, Nature, 334, 585-591, each of which is incorporated herein by reference in its entirety.

Preferably the ribozyme is engineered so that the cleavage recognition site is located near the 5' end of the mRNA encoding the API, *i.e.*, to increase efficiency and minimize the intracellular accumulation of non-functional mRNA transcripts.

The ribozymes of the present invention also include RNA endoribonucleases (hereinafter "Cech-type ribozymes") such as the one that occurs naturally in Tetrahymena thermophila (known as the IVS, or L-19 IVS RNA) and that has been extensively described by Thomas Cech and collaborators (Zaug, et al., 1984, Science, 224, 574-578; Zaug and Cech, 1986, Science, 231, 470-475; Zaug, et al., 1986, Nature, 324, 429-433; published International patent application No. WO 88/04300 by University Patents Inc.; Been and Cech, 1986, Cell, 47, 207-216). The Cech-type ribozymes have an eight base pair active site which hybridizes to a target RNA sequence whereafter cleavage of the target RNA takes place. The invention encompasses those Cech-type ribozymes which target eight base-pair active site sequences that are present in the gene encoding the API.

As in the antisense approach, the ribozymes can be composed of modified oligonucleotides (*e.g.*, for improved stability, targeting, etc.) and should be delivered to cells that express the API *in vivo*. A preferred method of delivery involves using a DNA construct "encoding" the ribozyme under the control of a strong constitutive pol III or pol II promoter, so that transfected cells will produce sufficient quantities of the ribozyme to destroy endogenous mRNA encoding the API and inhibit translation. Because ribozymes, unlike antisense molecules, are catalytic, a lower intracellular concentration is required for efficacy.

Endogenous API expression can also be reduced by inactivating or "knocking out" the gene encoding the API, or the promoter of such a gene, using targeted homologous recombination (*e.g.*, see Smithies, et al., 1985, Nature 317:230-234; Thomas and Capecchi, 1987, Cell 51:503-512; Thompson et al., 1989, Cell 5:313-321; and Zijlstra et al., 1989, Nature 342:435-438, each of which is incorporated by reference herein in its entirety). For example, a mutant gene encoding a non-functional API (or a completely unrelated DNA sequence) flanked by DNA homologous to the endogenous gene (either the coding regions or regulatory regions of the gene encoding the API) can be used, with or without a selectable marker and/or a negative selectable marker, to transfect cells that express the target gene *in vivo*. Insertion of the DNA construct, via targeted homologous recombination, results in inactivation of the target gene. Such approaches are particularly suited in the agricultural field where modifications to ES (embryonic stem) cells can be used to generate animal offspring with an inactive target gene (*e.g*., see Thomas and Capecchi, 1987 and Thompson, 1989, *supra*). However, this approach can be adapted for use in humans provided the recombinant DNA constructs are directly administered or targeted to the required site *in vivo* using appropriate viral vectors.

Alternatively, the endogenous expression of a gene encoding an API can be reduced by targeting deoxyribonucleotide sequences complementary to the regulatory region of the gene (*i.e.*, the gene promoter and/or enhancers) to form triple helical structures that prevent transcription of the gene encoding the API in target cells in the body. (See generally, Helene, 1991, Anticancer Drug Des., 6(6), 569-584; Helene, et al., 1992, Ann. N.Y. Acad. Sci., 660, 27-36; and Maher, 1992, Bioassays 14(12), 807-815).

Nucleic acid molecules to be used in triplex helix formation for the inhibition of transcription in the present invention should be single stranded and composed of deoxynucleotides. The base composition of these oligonucleotides must be designed to promote triple helix formation via Hoogsteen base pairing rules, which generally require sizeable stretches of either purines or pyrimidines to be present on one strand of a duplex. Nucleotide sequences may be pyrimidine-based, which will result in TAT and CGC+ triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, contain a stretch of G residues. These molecules will form a triple helix with a DNA duplex that is rich in GC pairs, in which the majority of the purine residues are located on a single strand of the targeted duplex, resulting in GGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

In one embodiment, wherein the antisense, ribozyme, or triple helix molecules described herein are utilized to inhibit mutant gene expression, it is possible that the technique may so efficiently reduce or inhibit the transcription (triple helix) or translation (antisense, ribozyme) of mRNA produced by normal gene alleles of an API that the situation may arise wherein the concentration of API present may be lower than is necessary for a normal phenotype. In such cases, to ensure that substantially normal levels of activity of a gene encoding an API are maintained, gene therapy may be used to introduce into cells nucleic acid molecules that encode and express the API that exhibit normal gene activity and that do not contain sequences susceptible to whatever antisense, ribozyme, or triple helix treatments are being utilized. Alternatively, in instances whereby the gene encodes an extracellular protein, normal API can be co-administered in order to maintain the requisite level of API activity.

Antisense RNA and DNA, ribozyme, and triple helix molecules of the invention may be prepared by any method known in the art for the synthesis of DNA and RNA molecules, as discussed above. These include techniques for chemically synthesizing oligodeoxyri-bonucleotides and - oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors that incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

### 5.17 Assays For Therapeutic Or Prophylactic Compounds

The present invention also provides assays for use in discovery of pharmaceutical products in order to identify or verify the efficacy of compounds for treatment or prevention of AD. Agents can be assayed for their ability to restore AF or API levels in a subject having AD towards levels found in subjects free from AD or to produce similar changes in experimental animal models of AD. Compounds able to restore AF or API levels in a subject having AD towards levels found in subjects free from AD or to produce similar changes in experimental animal models of Alzheimer's disease can be used as lead compounds for further drug discovery, or used therapeutically. AF and API expression can be assayed by the Preferred Technology, immunoassays, gel electrophoresis followed by visualization, detection of API activity, or any other method taught herein or known to those skilled in the art. Such assays can be used to screen candidate drugs, in clinical monitoring or in drug development, where abundance of an AF or API can serve as a surrogate marker for clinical disease.

In various embodiments, *in vitro* assays can be carried out with cells representative of cell types involved in a subject's disorder, to determine if a compound has a desired effect upon such cell types.

Compounds for use in therapy can be tested in suitable animal model systems prior to testing in humans, including but not limited to rats, mice, chicken, cows, monkeys, rabbits, etc. For *in vivo* testing, prior to administration to humans, any animal model system known in the art may be used. Examples of animal models of AD include, but are not limited to, animals that express human familial AD (FAD) β-amyloid precursor (APP), animals that overexpress human wild-type APP, animals that overexpress β-amyloid 1-42 (βA), animals that express FAD presenillin-1 (PS-1) (see, *e.g.*, Higgins, LS, 1999, Molecular Medicine Today 5:274-276). Further, animal models for Downs syndrome (*e.g.*, TgSOD1, TgPFKL, TgS100β, TgAPP, TgEts2, TgHMG14, TgMNB, Ts65Dn, and Ts1Cje (see, *e.g.*, Kola et al., 1999, Molecular Medicine Today 5:276-277) can be utilized to test compounds that modulate AF or API levels since the neuropathology exhibited by individuals with Downs syndrome is similar to that of AD. It is also apparent to the skilled artisan that, based upon the present disclosure, transgenic animals can be produced with "knock-out" mutations of the gene or genes encoding one or more APIs. A "knock-out" mutation of a gene is a mutation that causes the mutated gene to not be expressed, or expressed in an aberrant form or at a low level, such that the activity associated with the gene product is nearly or entirely absent. Preferably, the transgenic animal is a mammal, more preferably, the transgenic animal is a mouse.

In one embodiment, test compounds that modulate the expression of an API are identified in non-human animals (*e.g.*, mice, rats, monkeys, rabbits, and guinea pigs), preferably non-human animal models for AD or Downs syndrome, expressing the API. In accordance with this embodiment, a test compound or a control compound is administered to the animals, and the effect of the test compound on expression of one or more APIs is determined. A test compound that alters the expression of an API (or a plurality of APIs) can be identified by comparing the level of the selected API or APIs (or mRNA(s) encoding the same) in an animal or group of animals treated with a test compound with the level of the API(s) or mRNA(s) in an animal or group of animals treated with a control compound. Techniques known to those of skill in the art can be used to determine the mRNA and protein levels, for example, in situ hybridization. The animals may or may not be sacrificed to assay the effects of a test compound.

In another embodiment, test compounds that modulate the activity of an API or a biologically active portion thereof are identified in non-human animals (*e.g.*, mice, rats, monkeys, rabbits, and guinea pigs), preferably non-human animal models for AD or Downs syndrome, expressing the API. In accordance with this embodiment, a test compound or a control compound is administered to the animals, and the effect of a test compound on the activity of an API is determined. A test compound that alters the activity of an API (or a plurality of APIs) can be identified by assaying animals treated with a control compound and animals treated with the test compound. The activity of the API can be assessed by detecting induction of a downstream effector of the API (*e.g.*, intracellular Ca2+, diacylglycerol, IP3, etc.), detecting catalytic or enzymatic activity of the API or binding partner thereof, detecting the induction of a reporter gene (*e.g.*, a regulatory element that is responsive to an API of the invention operably linked to a nucleic acid encoding a detectable marker, such as luciferase or green fluorescent protein), or detecting a cellular response (*e.g.*, cellular differentiation or cell proliferation). Techniques known to those of skill in the art can be utilized to detect changes in the activity of an API (see, *e.g.*, U.S. Patent No. 5,401,639, which is incorporated herein in its entirety by reference).

In yet another embodiment, test compounds that modulate the level or expression of an API (or plurality of APIs) are identified in human subjects having AD or Downs syndrome, preferably those having mild to severe AD and most preferably these having mild AD. In accordance with this embodiment, a test compound or a control compound is administered to the human subject, and the effect of a test compound on API expression is determined by analyzing the expression of the API or the mRNA encoding the same in a biological sample (*e.g.*, CSF, serum, plasma, or urine). A test compound that alters the expression of an API can be identified by comparing the level of the API or mRNA encoding the same in a subject or group of subjects treated with a control compound to that in a subject or group of subjects treated with a test compound. Alternatively, alterations in the expression of an API can be identified by comparing the level of the API or mRNA encoding the same in a subject or group of subjects before and after the administration of a test compound. Any suitable techniques known to those of skill in the art can be used to obtain the biological sample and analyze the mRNA or protein expression. For example, the Preferred Technology described herein can be used to assess changes in the level of an API.

In another embodiment, test compounds that modulate the activity of an API (or plurality of APIs) are identified in human subjects having AD or Downs syndrome, preferably those having mild to severe AD and most preferably those with mild AD. In this embodiment, a test compound or a control compound is administered to the human subject, and the effect of a test compound on the activity of an API is determined. A test compound that alters the activity of an API can be identified by comparing biological samples from subjects treated with a control compound to samples from subjects treated with the test compound. Alternatively, alterations in the activity of an API can be identified by comparing the activity of an API in a subject or group of subjects before and after the administration of a test compound. The activity of the API can be assessed by detecting in a biological sample (*e.g.*, CSF, serum, plasma, or urine) induction of a downstream effector of the API (*e.g.*, intracellular Ca2+, diacylglycerol, IP3, etc.), catalytic or enzymatic activity of the API or a binding partner thereof, or a cellular response, for example, cellular differentiation, or cell proliferation. Techniques known to those of skill in the art can be used to detect changes in the induction of a second messenger of an API or changes in a cellular response. For example, RT-PCR can be used to detect changes in the induction of a cellular second messenger.

In a particular embodiment, an agent that changes the level or expression of an API towards levels detected in control subjects (*e.g.*, humans free from AD) is selected for further testing or therapeutic use. In another preferred embodiment, a test compound that changes the activity of an API towards the activity found in control subjects (*e.g.*, humans free from AD) is selected for further testing or therapeutic use.

In another embodiment, test compounds that reduce the severity of one or more symptoms associated with AD are identified in human subjects having AD or Downs syndrome, preferably subjects having mild to severe AD and most preferably subjects with mild AD. In accordance with this embodiment, a test compound or a control compound is administered to the subjects, and the effect of a test compound on one or more symptoms of AD is determined. A test compound that reduces one or more symptoms can be identified by comparing the subjects treated with a control compound to the subjects treated with the test compound. Techniques known to physicians familiar with AD can be used to determine whether a test compound reduces one or more symptoms associated with AD. For example, a test compound that enhances memory or reduces confusion in a subject having AD will be beneficial for treating subjects having AD.

In a preferred embodiment, an agent that reduces the severity of one or more symptoms associated with AD in a human having AD is selected for further testing or therapeutic use.

### 5.18 Therapeutic and Prophylactic Compositions and Their Use

The invention provides methods of treatment comprising administering to a subject an effective amount of an agent of the invention. In a preferred aspect, the compound is substantially purified (*e.g.*, substantially free from substances that limit its effect or produce undesired side-effects). The subject is preferably an animal, including but not limited to animals such as cows, pigs, horses, chickens, cats, dogs, etc., and is preferably a mammal, and most preferably human. In a specific embodiment, a non-human mammal is the subject.

Formulations and methods of administration that can be employed when the compound comprises a nucleic acid are described above; additional appropriate formulations and routes of administration are described below.

Various delivery systems are known and can be used to administer a compound of the invention, *e.g.*, encapsulation in liposomes, microparticles, microcapsules, recombinant cells capable of expressing the compound, receptor-mediated endocytosis (see, *e.g.*, Wu and Wu, 1987, J. Biol. Chem. 262:4429-4432), construction of a nucleic acid as part of retroviral or other vector, etc. Methods of introduction can be enteral or parenteral and include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (*e.g.*, oral mucosa, rectal and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g.*, by use of an inhaler or nebulizer, and formulation with an aerosolizing agent.

In a specific embodiment, it may be desirable to administer the pharmaceutical compositions of the invention locally to the area in need of treatment; this may be achieved, for example, and not by way of limitation, by local infusion during surgery, topical application, *e.g.*, by injection, by means of a catheter, or by means of an implant, said implant being of a porous, non-porous, or gelatinous material, including membranes, such as sialastic membranes, or fibers. In one embodiment, administration can be by direct injection into CSF or at the site (or former site) of neurodegeneration or to CNS tissue.

In another embodiment, the compound can be delivered in a vesicle, in particular a liposome (see Langer, 1990, Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.)

In yet another embodiment, the compound can be delivered in a controlled release system. In one embodiment, a pump may be used (see Langer, *supra*; Sefton, 1987, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., 1983, Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105 ). In yet another embodiment, a controlled release system can be placed in proximity of the therapeutic target, *i.e.*, the brain, thus requiring only a fraction of the systemic dose (see, *e.g.*, Goodson, in Medical Applications of Controlled Release, *supra*, vol. 2, pp. 115-138 (1984)).

Other suitable controlled release systems are discussed in the review by Langer (1990, Science 249:1527-1533).

In another embodiment where the compound of the invention is a nucleic acid encoding a protein, the nucleic acid can be administered *in vivo* to promote expression of its encoded protein, by constructing it as part of an appropriate nucleic acid expression vector and administering it so that it becomes intracellular, *e.g.*, by use of a retroviral vector (see U.S. Patent No. 4,980,286), or by direct injection, or by use of microparticle bombardment (*e.g*., a gene gun; Biolistic, Dupont), or coating with lipids or cell-surface receptors or transfecting agents, or by administering it in linkage to a homeobox-like peptide which is known to enter the nucleus (see *e.g.*, Joliot et al., 1991, Proc. Natl. Acad. Sci. USA 88:1864-1868), etc. Alternatively, a nucleic acid can be introduced intracellularly and incorporated within host cell DNA for expression, by homologous recombination.

The present invention also provides pharmaceutical compositions. Such compositions comprise a therapeutically effective amount of an agent, and a pharmaceutically acceptable carrier. In a particular embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" Ed. E.W. Martin, ISBN: 0-912734-04-3, Mack Publishing Co. Such compositions will contain a therapeutically effective amount of the compound, preferably in purified form, together with a suitable amount of carrier so as to provide the form for proper administration to the subject. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

The amount of the compound of the invention which will be effective in the treatment of AD can be determined by standard clinical techniques based on the present description. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each subject's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

### 6. EXAMPLE: IDENTIFICATION OF PROTEINS DIFFERENTIALLY EXPRESSED IN THE CSF IN AD

Using the following exemplary and non-limiting procedure, proteins in CSF samples from (a) 148 subjects having AD, (b) 60 family members of these AD subjects, and (c) 32 unrelated controls were separated by isoelectric focusing followed by SDS-PAGE and analyzed. From some subjects, serial samples were taken over time. Parts 6.1.1 to 6.1.9 (inclusive) of the procedure set forth below are hereby designated as the "Reference Protocol".

### 6.1 MATERIALS AND METHODS

### 6.1.1 Sample Preparation

A protein assay (Pierce BCA Cat # 23225) was performed on each CSF sample as received. Prior to protein separation, each sample was processed for selective depletion of certain proteins, in order to enhance and simplify protein separation and facilitate analysis by removing proteins that may interfere with or limit analysis of proteins of interest. See International Patent Application No. PCT/GB99/01742, filed June 1, 1999, which is incorporated by reference in its entirety, with particular reference to pages 3 and 6.

Removal of albumin, haptoglobin, transferrin and immunoglobin G (IgG) from CSF ("CSF depletion") was achieved by an affinity chromatography purification step in which the sample was passed through a series of 'Hi-Trap' columns containing immobilized antibodies for selective removal of albumin, haptoglobin and transferrin, and protein G for selective removal of immunoglobin G. Two affinity columns in a tandem assembly were prepared by coupling antibodies to protein G-sepharose contained in Hi-Trap columns (Protein G-Sepharose Hi-Trap columns (1 ml) Pharmacia Cat. No. 17-0404-01). This was done by circulating the following solutions sequentially through the columns: (1) Dulbecco's Phosphate Buffered Saline (Gibco BRL Cat. No. 14190-094); (2) concentrated antibody solution; (3) 200 mM sodium carbonate buffer, pH 8.35; (4) cross-linking solution (200 mM sodium carbonate buffer, pH 8.35, 20 mM dimethylpimelimidate); and (5) 500 mM ethanolamine, 500 mM NaCl. A third (un-derivatised) protein G Hi-Trap column was then attached to the lower end of the tandem column assembly.

The chromatographic procedure was automated using an Akta Fast Protein Liquid Chromatography (FPLC) System such that a series of up to seven runs could be performed sequentially. The samples were passed through the series of 3 Hi-Trap columns in which the affinity chromatography media selectively bind the above proteins thereby removing them from the sample. Fractions (typically 3 ml per tube) were collected of unbound material ("Flowthrough fractions") that eluted through the column during column loading and washing stages and of bound proteins ("Bound/Eluted fractions") that were eluted by step elution with Immunopure Gentle Ag/Ab Elution Buffer (Pierce Cat. No. 21013). The eluate containing unbound material was collected in fractions which were pooled, desalted/concentrated by centrifugal ultrafiltration and stored to await further analysis by 2D PAGE.

A volume of depleted CSF containing approximately 300 µg of total protein was aliquoted and an equal volume of 10% (w/v) SDS (Fluka 71729), 2.3% (w/v) dithiothreitol (BDH 443852A) was added. The sample was heated at 95°C for 5 mins, and then allowed to cool to 20°C . 125µl of the following buffer was then added to the sample:
8M urea (BDH 452043w )
4% CHAPS (Sigma C3023)
65mM dithiotheitol (DTT)
2% (v/v) Resolytes 3.5-10 (BDH 44338 2x)

This mixture was vortexed, and centrifuged at 13000 rpm for 5 mins at 15°C, and the supernatant was analyzed by isoelectric focusing.

### 6.1.2 Isoelectric Focusing

Isoelectric focusing (IEF), was performed using the Immobiline® DryStrip Kit (Pharmacia BioTech), following the procedure described in the manufacturer's instructions, see Instructions for Immobiline® DryStrip Kit, Pharmacia, # 18-1038-63, Edition AB (incorporated herein by reference in its entirety). Immobilized pH Gradient (IPG) strips (18cm, pH 3-10 non-linear strips; Pharmacia Cat. # 17-1235-01) were rehydrated overnight at 20°C in a solution of 8M urea, 2% (w/v) CHAPS, 10mM DTT, 2% (v/v) Resolytes 3.5-10, as described in the Immobiline DryStrip Users Manual. For IEF, 50µl of supernatant (prepared as above) was loaded onto a strip, with the cup-loading units being placed at the basic end of the strip. The loaded gels were then covered with mineral oil (Pharmacia 17-3335-01) and a voltage was immediately applied to the strips according to the following profile, using a Pharmacia EPS3500XL power supply (Cat 19-3500-01):
Initial voltage = 300V for 2 hrs
Linear Ramp from 300V to 3500V over 3hrs
Hold at 3500V for 19hrs

For all stages of the process, the current limit was set to 10mA for 12 gels, and the wattage limit to 5W. The temperature was held at 20°C throughout the run.

### 6.1.3 Gel Equilibration and SDS-PAGE

After the final 19hr step, the strips were immediately removed and immersed for 10 mins at 20°C in a first solution of the following composition: 6M urea; 2% (w/v) DTT; 2% (w/v) SDS; 30% (v/v) glycerol (Fluka 49767); 0.05M Tris/HCl, pH 6.8 (Sigma Cat T-1503). The strips were removed from the first solution and immersed for 10 mins at 20°C in a second solution of the following composition: 6M urea; 2% (w/v) iodoacetamide (Sigma 1-6125); 2% (w/v) SDS; 30% (v/v) glycerol; 0.05M Tris/HCl, pH 6.8. After removal from the second solution, the strips were loaded onto supported gels for SDS-PAGE according to Hochstrasser et al., 1988, Analytical Biochemistry 173: 412-423 (incorporated herein by reference in its entirety), with modifications as specified below.

### 6.1.4 Preparation of supported gels

The gels were cast between two glass plates of the following dimensions: 23cm wide x 24cm long (back plate); 23cm wide x 24cm long with a 2cm deep notch in the central 19cm (front plate). To promote covalent attachment of SDS-PAGE gels, the back plate was treated with a 0.4% solution of γ-methacryl-oxypropyltrimethoxysilane in ethanol (BindSilane™; Pharmacia Cat. # 17-1330-01). The front plate was treated with a 2% solution of dimethyldichlorosilane dissolved in octamethyl cyclo-octasilane (RepelSilane™ Pharmacia Cat. # 17-1332-01) to reduce adhesion of the gel. Excess reagent was removed by washing with water, and the plates were allowed to dry. At this stage, both as identification for the gel, and as a marker to identify the coated face of the plate, an adhesive bar-code was attached to the back plate in a position such that it would not come into contact with the gel matrix.

The dried plates were assembled into a casting box with a capacity of 13 gel sandwiches. The top and bottom plates of each sandwich were spaced by means of 1mm thick spacers, 2.5 cm wide. The sandwiches were interleaved with acetate sheets to facilitate separation of the sandwiches after gel polymerization. Casting was then carried out according to Hochstrasser et al., *op. cit.*

A 9-16% linear polyacrylamide gradient was cast, extending up to a point 2cm below the level of the notch in the front plate, using the Angelique gradient casting system (Large Scale Biology). Stock solutions were as follows. Acrylamide (40% in water) was from Serva (Cat. # 10677). The cross-linking agent was PDA (BioRad 161-0202), at a concentration of 2.6% (w/w) of the total starting monomer content. The gel buffer was 0.375M Tris/HCl, pH 8.8. The polymerization catalyst was 0.05% (v/v) TEMED (BioRad 161-0801), and the initiator was 0.1% (w/v) APS (BioRad 161-0700). No SDS was included in the gel and no stacking gel was used. The cast gels were allowed to polymerize at 20°C overnight, and then stored at 4°C in sealed polyethylene bags with 6ml of gel buffer, and were used within 4 weeks.

### 6.1.5 SDS-PAGE

A solution of 0.5% (w/v) agarose (Fluka Cat 05075) was prepared in running buffer (0.025M Tris, 0.198M glycine (Fluka 50050), 1% (w/v) SDS, supplemented by a trace of bromophenol blue). The agarose suspension was heated to 70°C with stirring, until the agarose had dissolved. The top of the supported 2nd D gel was filled with the agarose solution, and the equilibrated strip was placed into the agarose, and tapped gently with a palette knife until the gel was intimately in contact with the 2nd D gel. The gels were placed in the 2nd D running tank, as described by Amess et al., 1995, Electrophoresis 16: 1255-1267 (incorporated herein by reference in its entirety). The tank was filled with running buffer (as above) until the level of the buffer was just higher than the top of the region of the 2nd D gels which contained polyacrylamide, so as to achieve efficient cooling of the active gel area. Running buffer was added to the top buffer compartments formed by the gels, and then voltage was applied immediately to the gels using a Consort E-833 power supply. For 1 hour, the gels were run at 20mA/gel. The wattage limit was set to 150W for a tank containing 6 gels, and the voltage limit was set to 600V. After 1 hour, the gels were then run at 40mA/gel, with the same voltage and wattage limits as before, until the bromophenol blue line was 0.5cm from the bottom of the gel. The temperature of the buffer was held at 16°C throughout the run. Gels were not run in duplicate.

### 6.1.6 Staining

Upon completion of the electrophoresis run, the gels were immediately removed from the tank for fixation. The top plate of the gel cassette was carefully removed, leaving the gel bonded to the bottom plate. The bottom plate with its attached gel was then placed into a staining apparatus, which can accommodate 12 gels. The gels were completely immersed in fixative solution of 40% (v/v) ethanol (BDH 28719), 10% (v/v) acetic acid (BDH 100016X), 50% (v/v) water (MilliQ-Millipore), which was continuously circulated over the gels. After an overnight incubation, the fixative was drained from the tank, and the gels were primed by immersion in 7.5% (v/v) acetic acid, 0.05% (w/v) SDS, 92.5% (v/v) water for 30 mins. The priming solution was then drained, and the gels were stained by complete immersion for 4 hours in a staining solution of Pyridinium, 4-[2-[4-(dipentylamino)-2-trifluoromethylphenyl] ethenyl]-1-(sulfobutyl)-, inner salt, prepared by diluting a stock solution of this dye (2mg/ml in DMSO) in 7.5% (v/v) aqueous acetic acid to give a final concentration of 1.2 mg/l; the staining solution was vacuum filtered through a 0.4µm filter (Duropore) before use.

### 6.1.7 Imaging of the gel

A computer-readable output was produced by imaging the fluorescently stained gels with the Apollo 2 scanner (Oxford Glycosciences, Oxford, UK) described in section 5.1, *supra*. This scanner has a gel carrier with four integral fluorescent markers (Designated M1, M2, M3, M4) that are used to correct the image geometry and are a quality control feature to confirm that the scanning has been performed correctly.

For scanning, the gels were removed from the stain, rinsed with water and allowed to air dry briefly, and imaged on the Apollo 2. After imaging, the gels were sealed in polyethylene bags containing a small volume of staining solution, and then stored at 4°C.

### 6.1.8 Digital Analysis of the Data

The data were processed as described in U.S. Application Serial No. 08/980,574, (published as WO 98/23950) at Sections 5.4 and 5.5 (incorporated herein by reference), as set forth more particularly below.

The output from the scanner was first processed using the MELANIE® II 2D PAGE analysis program (Release 2.2, 1997, BioRad Laboratories, Hercules, California, Cat. # 170-7566) to autodetect the registration points, M1, M2, M3 and M4; to autocrop the images (i.e., to eliminate signals originating from areas of the scanned image lying outside the boundaries of the gel, *e.g.* the reference frame); to filter out artifacts due to dust; to detect and quantify features; and to create image files in GIF format. Features were detected using the following parameters:
Smooths =2
Laplacian threshold 50
Partials threshold 1
Saturation = 100
Peakedness = 0
Minimum Perimeter = 10

### 6.1.9 Assignment of pI and MW Values

Landmark identification was used to determine the pI and MW of features detected in the images. Twelve landmark features, designated CSF1 to CSF12, were identified in a standard CSF image obtained from a pooled sample. These landmark features are identified in Figure 1 and were assigned the pI and/or MW values identified in Table XI.

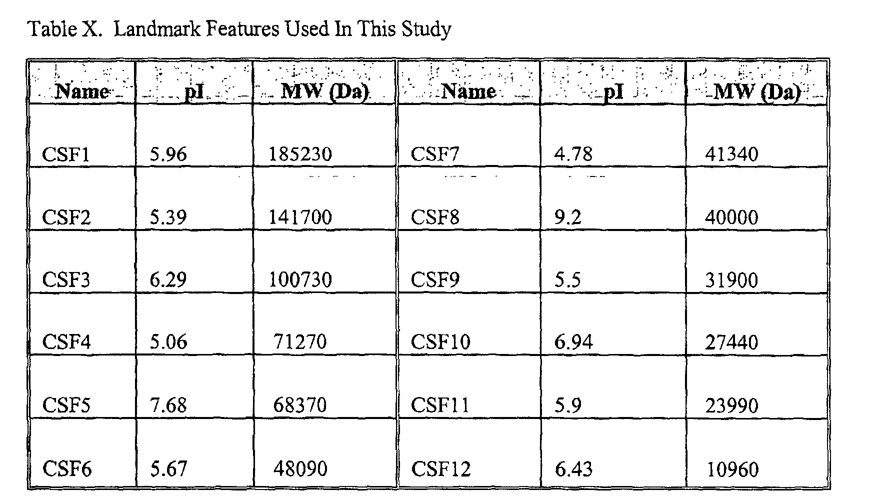

As many of these landmarks as possible were identified in each gel image of the dataset. Each feature in the study gels was then assigned a pI value by linear interpolation or extrapolation (using the MELANIE®-II software) to the two nearest landmarks, and was assigned a MW value by linear interpolation or extrapolation (using the MELANLE®-II software) to the two nearest landmarks.

### 6.1.10 Matching With Primary Master Image

Images were edited to remove gross artifacts such as dust, to reject images which had gross abnormalities such as smearing of protein features, or were of too low a loading or overall image intensity to allow identification of more than the most intense features, or were of too poor a resolution to allow accurate detection of features. Images were then compared by pairing with one common image from the whole sample set. This common image, the "primary master image", was selected on the basis of protein load (maximum load consistent with maximum feature detection), a well resolved myoglobin region, (myoglobin was used as an internal standard), and general image quality. Additionally, the primary master image was chosen to be an image which appeared to be generally representative of all those to be included in the analysis. (This process by which a primary master gel was judged to be representative of the study gels was rechecked by the method described below and in the event that the primary master gel was seen to be unrepresentative, it was rejected and the process repeated until a representative primary master gel was found.)

Each of the remaining study gel images was individually matched to the primary master image such that common protein features were paired between the primary master image and each individual study gel image as described below.

### 6.1.11 Cross-matching Between Samples

To facilitate statistical analysis of large numbers of samples for purposes of identifying features that are differentially expressed, the geometry of each study gel was adjusted for maximum alignment between its pattern of protein features, and that of the primary master, as follows. Each of the study gel images was individually transformed into the geometry of the primary master image using a multi-resolution warping procedure. This procedure corrects the image geometry for the distortions brought about by small changes in the physical parameters of the electrophoresis separation process from one sample to another. The observed changes are such that the distortions found are not simple geometric distortions, but rather a smooth flow, with variations at both local and global scale.

The fundamental principle in multi-resolution modeling is that smooth signals may be modeled as an evolution through 'scale space', in which details at successively finer scales are added to a low resolution approximation to obtain the high resolution signal. This type of model is applied to the flow field of vectors (defined at each pixel position on the reference image) and allows flows of arbitrary smoothness to be modeled with relatively few degrees of freedom. Each image is first reduced to a stack, or pyramid, of images derived from the initial image, but smoothed and reduced in resolution by a factor of 2 in each direction at every level (Gaussian pyramid) and a corresponding difference image is also computed at each level, representing the difference between the smoothed image and its progenitor (Laplacian pyramid). Thus the Laplacian images represent the details in the image at different scales.

To estimate the distortion between any 2 given images, a calculation was performed at level 7 in the pyramid (*i.e.* after 7 successive reductions in resolution). The Laplacian images were segmented into a grid of 16x16 pixels, with 50% overlap between adjacent grid positions in both directions, and the cross correlation between corresponding grid squares on the reference and the test images was computed. The distortion displacement was then given by the location of the maximum in the correlation matrix. After all displacements had been calculated at a particular level, they were interpolated to the next level in the pyramid, applied to the test image, and then further corrections to the displacements were calculated at the next scale.

The warping process brought about good alignment between the common features in the primary master image, and the images for the other samples. The MELANIE® II 2D PAGE analysis program was used to calculate and record approximately 500-700 matched feature pairs between the primary master and each of the other images. The accuracy of this program was significantly enhanced by the alignment of the images in the manner described above. To improve accuracy still further, all pairings were finally examined by eye in the MelView interactive editing program and residual recognizably incorrect pairings were removed. Where the number of such recognizably incorrect pairings exceeded the overall reproducibility of the Preferred Technology (as measured by repeat analysis of the same biological sample) the gel selected to be the primary master gel was judged to be insufficiently representative of the study gels to serve as a primary master gel. In that case, the gel chosen as the primary master gel was rejected, and different gel was selected as the primary master gel, and the process was repeated.

All the images were then added together to create a composite master image, and the positions and shapes of all the gel features of all the component images were super-imposed onto this composite master as described below.

Once all the initial pairs had been computed, corrected and saved, a second pass was performed whereby the original (unwarped) images were transformed a second time to the geometry of the primary master, this time using a flow field computed by smooth interpolation of the multiple tie-points defined by the centroids of the paired gel features. A composite master image was thus generated by initialising the primary master image with its feature descriptors. As each image was transformed into the primary master geometry, it was digitally summed pixel by pixel into the composite master image, and the features that had not been paired by the procedure outlined above were likewise added to the composite master image description, with their centroids adjusted to the master geometry using the flow field correction.

The final stage of processing was applied to the composite master image and its feature descriptors, which now represent all the features from all the images in the study transformed to a common geometry. The features were grouped together into linked sets or "clusters", according to the degree of overlap between them. Each cluster was then given a unique identifying index, the molecular cluster index (MCI).

An MCI identifies a set of matched features on different images. Thus an MCI represents a protein or proteins eluting at equivalent positions in the 2D separation in different samples.

### 6.1.12. Construction of Profiles

After matching all component gels in the study to the final composite master image, the intensity of each feature was measured and stored. The end result of this analysis was the generation of a digital profile which contained, for each identified feature: 1) a unique identification code relative to corresponding feature within the composite master image (MCI), 2) the x, y coordinates of the features within the gel, 3) the isoelectric point (pI) of the AFs, 4) the apparent molecular weight (MW) of the AFs, 5) the signal value, 6) the standard deviation for each of the preceding measurements, and 7) a method of linking the MCI of each feature to the master gel to which this feature was matched. By virtue of a Laboratory Information Management System (LIMS), this MCI profile was traceable to the actual stored gel from which it was generated, so that proteins identified by computer analysis of gel profile databases could be retrieved. The LIMS also permitted the profile to be traced back to an original sample or patient.

### 6.1.13. Differental Analysis of the Profiles

For the pooled gel data within each sample set (Alzheimer's CSF and normal CSF), the profiles were analyzed to identify and select those features differentially present in the profiles. These selected features were then assembled into an Alzheimer's pooled gel feature set. Matching features of each feature set were then compared to identify those features showing at least a 2-fold difference in mean intensity between Alzheimer's CSF and normal CSF. Differentially present features were identified as Alzheimer's Disease Associated Features (AFs).

### 6.1.14. Statistical Analysis of the Profiles

The MCI data was represented in statistical models in two forms: 1) percent of total protein volume for a given gel (PCTVOL) and 2) absolute volume, scaled by the total volume loaded on the gel (VOL). A value of 0 was entered for PCTVOL and VOL if an MCI did not appear on a particular gel. For most analyzes, in order for an MCI to be considered in the statistical model, it had to have non-zero values for PCTVOL and VOL in at least 75% of gels in at least one of the diagnosis groups in the analysis (described below).

The complementary statistical strategies specified below were used to identify AFs from the MCIs within the mastergroup.

### (I) Group Analysis

The purpose of these analyses was to characterize differences among gels from individuals with different clinical diagnoses. The diagnosis groups were 1) autopsy-confirmed (AD) vs. normal controls (NCO) at their first sample, 2) Dementia Alzheimer's type (DAT) with an initial sample within 3 years of disease onset vs. NCO, and 3) last sample of first-degree relatives of individuals diagnosed with dementia of Alzheimer's type without a clinical diagnosis of dementia (NCF) vs. NCO.
The following statistical techniques were used in the group analyses:

### (1) Linear model

A linear model controlling for age and gender that compared a DAT group vs the NCO group with regard to the rank of the volume.

### (2) Classification trees

Classification trees were used with the MCI volumes as predictors, and clinical diagnosis as the response. The algorithm looks for 'split points' in the predictors that partition the data into homogeneous sets according to the response variable. After evaluating all possible splits for a given node of the tree, the split is chosen that maximizes the change in deviance according to a multinomial likelihood model. Tree models were fit to both the original data and data from bootstrap samples of the original data (sampling with replacement). The statistical test involved whether a given MCI proved to be an important'split point' to determine diagnosis, either in the original data tree or a bootstrap sample tree.

### (3) Logistic regression model

A logistic regression model was used to model the probability of being AD. The volumes of the various MCI's were used as the explanatory variables. A stepwise procedure was used to select 5 MCI's.
Criteria for inclusion based on the group analyses:
Information from all of the above described analyses were used to select MCI's that:
   1. Were among the 5-6 MCI's with the smallest p-value for a given analysis
   2. Appeared in the smallest 100 p-values for 2 or more analysis
   3. Appeared as an important split-point in a classification tree
   4. Had desired distributional properties

### (4) Nearest Neighbor Analysis

In addition to the techniques described above, the gels were examined with nearest neighbor analysis. In this analysis, the gels were visually examined and MCIs located proximate to other MCIs that demonstrated a volume or other characteristics that are indicative of disease, and that themselves demonstrated a similar or like appearance and intensity, were noted and categorized as of like significance. These MCIs were thus included in the group for further examination and analysis in relation to their role in the disease.

### (II) Longitudinal Analysis

The purpose of the longitudinal analyses was to identify AF's associated with changes in disease state as measured by the MMSEM, a combination of the MMSE, CDR, and GDS assessment measures. DAT subjects with two or more samples were used in these analyses.

There were two models employed in the longitudinal analyses. In the first, the goal was to identify AF's for which changes in volume were significantly correlated with changes in the MMSEM. For each AF, MMSEM was regressed on the rank of the volume after controlling for age and subject. AF's with p-values less than 0.05, in the top 100 of any of the group analyses, and consistent with the group analyses in terms of up or down regulation were included.

The goal of the second model was to identify AF's for which volume in a subject's first sample was a significant predictor of disease progression rate during the period following the time of the first sample. First, a simple linear regression model was used to estimate a progression rate based on the MMSEM for each subject. Only subjects with an initial MMSEM greater than or equal to 12 and with greater than four months between the first and last samples were used. In addition, only samples within the first three years of the first sample were used. Regression modeling and split-sample validation were then used to identify significant AF's. More specifically, subjects were first randomly divided into two groups. For each group, stepwise weighted least-squares (WLS) regression using the rank of volume from each subject's first sample was used to select the five best AF's for predicting progression rate. If an AF was in the top five in one group and yielded a slope estimate with the same sign when included in the other group, it was included. In addition, the top five AF's from a stepwise WLS on both groups combined were included.

### 6.1.15 Recovery and analysis of selected proteins

Proteins in AFs were robotically excised and processed to generate tryptic digest peptides. Tryptic peptides were analyzed by mass spectrometry using a PerSeptive Biosystems Voyager- DE™ STR Matrix-Assisted Laser Desorption Ionization Time-of-Flight (MALDI-TOF) mass spectrometer, and selected tryptic peptides were analyzed by tandem mass spectrometry (MS/MS) using a Micromass Quadrupole Time-of-Flight (Q-TOF) mass spectrometer (Micromass, Altrincham, U.K.) equipped with a nanoflow™ electrospray Z-spray source. For partial amino acid sequencing and identification of APIs uninterpreted tandem mass spectra of tryptic peptides were searched using the SEQUEST search program (Eng et al., 1994, J. Am. Soc. Mass Spectrom. 5:976-989), version v.C.1. Criteria for database identification included: the cleavage specificity of trypsin; the detection of a suite of a, b and y ions in peptides returned from the database, and a mass increment for all Cys residues to account for carbamidomethylation. The database searched was database constructed of protein entries in the non-redundant database held by the National Centre for Biotechnology Information (NCBI) which is accessible at http://www.ncbi.nlm.nih.gov/. Following identification of proteins through spectral-spectral correlation using the SEQUEST program, masses detected in MALDI-TOF mass spectra were assigned to tryptic digest peptides within the proteins identified. In cases where no proteins could be identified through searching with uninterpreted MS/MS spectra of tryptic digest peptides using the SEQUEST program, tandem mass spectra of the peptides were interpreted manually, using methods known in the art. (In the case of interpretation of low-energy fragmentation mass spectra of peptide ions see Gaskell et al., 1992, Rapid Commun. Mass Spectrom. 6:658-662). The method described in PCT Application No. PCT/GB01/04034, which is incorporated herein by reference in its entirety, was also used to interpret mass spectra.

### 6.2 RESULTS

These initial experiments identified 111 features that were decreased and 30 features that were increased in AD CSF as compared with normal CSF. Details of these AFs are provided in Tables I (a) and II (a). Each AF was differentially present in AD CSF as compared with normal CSF. For some preferred AFs (AF-200, AF-201, AF-203, AF-204, AF-205, AF-206, AF-207, AF-208, AF-209, AF-210, AF-211, AF-212, AF-213, AF-214, AF-215, AF-216, AF-217, AF-218, AF-219, AF-220, AF-221, AF-222, AF-223, AF-224, AF-225, AF-226, AF-227, AF-227, AF-229, AF-230, AF-231, AF-232, AF-233, AF-234, AF-235, AF-236, AF-237, AF-238, AF-239, AF-240, AF-241, AF-242, AF-243, AF-244, AF-245, AF-248, AF-249, AF-251, AF-252, AF-253, AF-258, AF-262, AF-263, AF-278, AF-287, AF-303, AF 310, AF-311, AF-313, AF-314, AF-316, AF-317, AF-318, AF-325) the difference was highly significant (p < 0.01), and for certain highly preferred AFs (AF-200, AF-201, AF-203, AF-204, AF-205, AF-206, AF-207, AF-208, AF-209, AF-210, AF-211, AF-212, AF-213, AF-214, AF-216, AF-217, AF-218, AF-219, AF-220, AF-221, AF-222, AF-223, AF-224, AF-225, AF-226, AF-227, AF-227, AF-229, AF-231, AF-232, AF-233, AF-234, AF-235, AF-236, AF-237, AF-238, AF-240, AF-241, AF-242, AF-243, AF-245, AF-253, AF-258, AF-262, AF-287, AF-303, AF-311, AF-316), the difference was still more significant (p < 0.001).

Partial amino acid sequences were determined for the differentially present APIs in these AFs. Details of these APIs are provided in Tables IV and V . Computer searches of public databases identified at least one API for which neither the partial amino acid sequence, nor any oligonucleotide encoding such a peptide sequence, was described in any public database examined.

### 7. EXAMPLE: DIAGNOSIS AND TREATMENT OF AD

The following example illustrate the use of an API of the invention for screening, treatment or diagnosis of AD. The following example also illustrates the use of modulators (*e.g*., agonist or antagonists) of an API of the invention to treat or prevent AD.

Pigment epithelium-derived factor (PEDF) is a neurotrophic protein synthesized and secreted by retinal pigment epithelial cells in early embryogenesis and has been shown to be present in the extracellular matrix between the RPE cells and the neural retina. It induces neuronal differentiation and promotes survival of neurons of the central nervous system from degeneration caused by serum withdrawal or glutamate cytotoxicity. PEDF has been shown to protect immature but not mature cerebellar cells from apoptotic death, acting as a survival factor for such cells, as well as protecting them against glutamate and hydrogen peroxide toxicity. PEDF binds to glycosaminoglycans and to an 80 kDa receptor present on the surface of retinoblastoma and cerebellar granule cells. PEDF binding to the 80 kDa receptor, as well as PEDF activity, may be blocked by antibodies recognizing PEDF, and by a 44 amino acid fragment (amino acids 78-121) of PEDF.

The expression of an isoform of PEDF with a molecular weight of 33,401 kDa and pI of 6.74 has been shown herein to be significantly increased in the cerebrospinal fluid (CSF) of subjects having AD as compared with the CSF of subjects free from AD (AF-218/API-313, see Tables I(a) and IV(a)). Thus, quantitative detection of PEDF in CSF can be used to diagnose AD, determine the progression of AD or monitor the effectiveness of therapy for AD.

In one embodiment of the invention, compounds that modulate (*i.e.*, upregulate or downregulate) the expression, activity or both the expression and activity of PEDF are administered to a subject in need of treatment or for prophylaxis of AD. Antibodies that modulate the expression, activity or both the expression and activity of PEDF are suitable for this purpose. In addition, nucleic acids coding for all or a portion of PEDF, or nucleic acids complementary to all or a portion of PEDF, may be administered. PEDF, or fragments of the PEDF polypeptide may also be administered.

The invention also provides screening assays to identify additional compounds that modulate the expression of PEDF or activity of PEDF. Compounds that modulate the expression of PEDF *in vitro* can be identified by comparing the expression of PEDF in cells treated with a test compound to the expression of PEDF in cells treated with a control compound (*e.g.*, saline). Methods for detecting expression of PEDF are known in the art and include measuring the level of PEDF RNA (*e.g*., by northern blot analysis or RT-PCR) and measuring PEDF protein (*e.g*., by immunoassay or western blot analysis). Compounds that modulate the activity of PEDF can be identified by comparing the ability of a test compound to agonize or antagonize a function of PEDF, such as its neurotrophic activity or its binding to the 80 kDa receptor, to the ability of a control compound (*e.g.*, saline) to inhibit the same function of PEDF. Compounds capable of modulating PEDF binding to its receptor or PEDF activity are identified as compounds suitable for further development as a compound useful for the treatment of AD.

Binding between PEDF and its receptor can be determined by, for example, contacting PEDF with cells known to express the PEDF receptor and assaying the extent of binding between PEDF and the cell surface receptor, or by contacting PEDF with its receptor in a cell-free assay, *i.e.,* an assay where the PEDF and PEDF receptor are isolated, and, preferably, recombinantly produced, and assaying the extent of binding between PEDF and its receptor. Through the use of such assays, candidate compounds may be tested for their ability to agonize or antagonize the binding of PEDF to its receptor.

Compounds identified *in vitro* that affect the expression or activity of PEDF can be tested *in vivo* in animal models of AD or Downs syndrome, or in subjects having AD, to determine their therapeutic efficacy.

The present invention is not to be limited in terms of the particular embodiments described in this application, which are intended as single illustrations of individual aspects of the invention. Functionally equivalent methods and apparatus within the scope of the invention, in addition to those enumerated herein, will be apparent to those skilled in the art from the foregoing description and accompanying drawings. Such modifications and variations are intended to fall within the scope of the appended claims. The contents of each reference, patent and patent application cited in this application is hereby incorporated by reference in its entirety.

SEQUENCE LISTING

<110> Pfizer Products Inc
Oxford GlycoSciences (UK) Ltd

<120> NUCLEIC ACID MOLECULES, POLYPEPTIDES AND USES THEREFOR, INCLUDING
DIAGNOSIS AND TREATMENT OF ALZHEIMER'S DISEASE

<130> P0113-WO01

<140> EP 02256893.5
<141> 2002-10-03

<150> US 60/326708
<151> 2001-10-03

<160> 491

<170> PatentIn version 3.1

<210> 1
<211> 13
<212> PRT
<213> Homo sapiens

<400> 1

Ala Ala Asp Asp Thr Trp Glu Pro Phe Ala Ser Gly Lys
1               5                   10


<210> 2
<211> 12
<212> PRT
<213> Homo sapiens

<400> 2

Ala Ala Pro Met Asp Leu Thr Leu Thr Glu Thr Arg
1               5                   10


<210> 3
<211> 11
<212> PRT
<213> Homo sapiens

<400> 3

Ala Ala Ser Gly Thr Gln Asn Asn Val Leu Arg
1               5                   10


<210> 4
<211> 15
<212> PRT
<213> Homo sapiens

<400>  4

Ala Ala Thr Val Gly Ser Leu Ala Gly Gln Pro Leu Gln Glu Arg
1               5                   10                  15


<210>  5
<211>  11
<212>  PRT
<213>  Homo sapiens

<400>  5

Ala Asp Gly Ser Tyr Ala Ala Trp Leu Ser Arg
1               5                   10


<210>  6
<211>  10
<212>  PRT
<213>  Homo sapiens

<400>  6

Ala Asp Leu Ser Gly Ile Thr Gly Ala Arg
1               5                   10


<210>  7
<211>  9
<212>  PRT
<213>  Homo sapiens

<400>  7

Ala Asp Gln Val Cys Ile Asn Leu Arg
1               5


<210>  8
<211>  16
<212>  PRT
<213>  Homo sapiens

<400>  8

Ala Asp Ser Gly Glu Gly Asp Phe Leu Ala Glu Gly Gly Gly Val Arg
1               5                   10                  15


<210>  9
<211>  13
<212>  PRT
<213>  Homo sapiens

<400>  9

Ala Glu Met Ala Asp Gln Ala Ala Ala Trp Leu Thr Arg
1               5                   10

<210>  10
<211>  13
<212>  PRT
<213>  Homo sapiens

<400>  10

Ala Glu Met Ala Asp Gln Ala Ser Ala Trp Leu Thr Arg
1               5                   10

<210>  11
<211>  13
<212>  PRT
<213>  Homo sapiens

<400>  11

Ala Glu Thr Tyr Glu Gly Val Tyr Gln Cys Thr Ala Arg
1               5                   10

<210>  12
<211>  9
<212>  PRT
<213>  Homo sapiens

<400>  12

Ala Phe Leu Phe Gln Asp Thr Pro Arg
1               5

<210>  13
<211>  9
<212>  PRT
<213>  Homo sapiens

<400>  13

Ala Phe Leu Phe Gln Glu Ser Pro Arg
1               5

<210>  14
<211>  17
<212>  PRT
<213>  Homo sapiens

<400>  14

Ala Gly Ala Ala Ala Gly Gly Pro Gly Val Ser Gly Val Cys Val Cys

1               5                   10                  15

Lys

<210>  15
<211>  13
<212>  PRT
<213>  Homo sapiens

<400>  15

Ala Gly Ala Leu Asn Ser Asn Asp Ala Phe Val Leu Lys
1               5                   10

<210>  16
<211>  14
<212>  PRT
<213>  Homo sapiens

<400>  16

Ala Gly Asp Phe Leu Glu Ala Asn Tyr Met Asn Leu Gln Arg
1               5                   10

<210>  17
<211>  10
<212>  PRT
<213>  Homo sapiens

<400>  17

Ala Gly Phe Ala Gly Asp Asp Ala Pro Arg
1               5                   10

<210>  18
<211>  10
<212>  PRT
<213>  Homo sapiens

<400>  18

Ala Gly Leu Leu Glu Asn Gly Ala Val Arg
1               5                   10

<210>  19
<211>  12
<212>  PRT
<213>  Homo sapiens

<400>  19

Ala Ile Glu Asp Tyr Ile Asn Glu Phe Ser Val Arg
1               5                   10


<210>  20
<211>  11
<212>  PRT
<213>  Homo sapiens

<400>  20

Ala Leu Asp Phe Ala Val Gly Glu Tyr Asn Lys
1               5                   10


<210>  21
<211>  12
<212>  PRT
<213>  Homo sapiens

<400>  21

Ala Leu Glu Glu Ser Asn Tyr Glu Leu Glu Gly Lys
1               5                   10


<210>  22
<211>  11
<212>  PRT
<213>  Homo sapiens

<400>  22

Ala Leu Gly Tyr Leu Asn Thr Gly Tyr Gln Arg
1               5                   10


<210>  23
<211>  12
<212>  PRT
<213>  Homo sapiens

<400>  23

Ala Leu Gln Asp Gln Leu Val Leu Val Ala Ala Lys
1               5                   10


<210>  24
<211>  9
<212>  PRT
<213>  Homo sapiens

<400>  24

Ala Leu Thr Asp Met Pro Gln Met Arg

1               5


<210>  25
<211>  10
<212>  PRT
<213>  Homo sapiens

<400>  25

Ala Leu Val Gln Gln Met Glu Gln Leu Arg
1               5                   10


<210>  26
<211>  12
<212>  PRT
<213>  Homo sapiens

<400>  26

Ala Leu Tyr Leu Gln Tyr Thr Asp Glu Thr Phe Arg
1               5                   10


<210>  27
<211>  17
<212>  PRT
<213>  Homo sapiens

<400>  27

Ala Leu Tyr Tyr Asp Leu Ile Ser Ser Pro Asp Ile His Gly Thr Tyr
1               5                   10                  15


Lys


<210>  28
<211>  16
<212>  PRT
<213>  Homo sapiens

<400>  28

Ala Pro Glu Ala Gln Val Ser Val Gln Pro Asn Phe Gln Gln Asp Lys
1               5                   10                  15


<210>  29
<211>  17
<212>  PRT
<213>  Homo sapiens

<400>  29

Ala Gln Gly Phe Thr Glu Asp Thr Ile Val Phe Leu Pro Gln Thr Asp
1               5                   10                  15

Lys

<210>  30
<211>  17
<212>  PRT
<213>  Homo sapiens

<400>  30

Ala Gln Gly Phe Thr Glu Asp Thr Leu Val Phe Leu Pro Gln Thr Asp
1               5                   10                  15

Lys

<210>  31
<211>  13
<212>  PRT
<213>  Homo sapiens

<400>  31

Ala Gln Leu Gly Asp Leu Pro Trp Gln Val Ala Ile Lys
1               5                   10

<210>  32
<211>  13
<212>  PRT
<213>  Homo sapiens

<400>  32

Ala Ser Glu Glu Glu Pro Glu Tyr Gly Glu Glu Leu Lys
1               5                   10

<210>  33
<211>  8
<212>  PRT
<213>  Homo sapiens

<400>  33

Ala Ser Pro Phe Pro Val Tyr Lys
1               5

<210>  34
<211>  12
<212>  PRT
<213>  Homo sapiens

<400>  34

Ala Ser Ser Ile Ile Asp Glu Leu Phe Gln Asp Arg
1               5                   10


<210>  35
<211>  12
<212>  PRT
<213>  Homo sapiens

<400>  35

Ala Ser Ser Leu Leu Asp Glu Leu Phe Gln Asp Arg
1               5                   10


<210>  36
<211>  10
<212>  PRT
<213>  Homo sapiens

<400>  36

Ala Thr Ser Val Ala Leu Thr Trp Ser Arg
1               5                   10


<210>  37
<211>  9
<212>  PRT
<213>  Homo sapiens

<400>  37

Ala Thr Val Val Tyr Gln Gly Glu Arg
1               5


<210>  38
<211>  9
<212>  PRT
<213>  Homo sapiens

<400>  38

Ala Val Phe Pro Ser Ile Val Gly Arg
1               5


<210>  39
<211>  19

<212>  PRT
<213>  Homo sapiens

<400>  39

Ala Val Leu Asp Val Phe Glu Glu Gly Thr Glu Ala Ser Ala Ala Thr
1               5                   10                  15

Ala Val Lys

<210>  40
<211>  12
<212>  PRT
<213>  Homo sapiens

<400>  40

Ala Val Met Asp Asp Phe Ala Ala Phe Val Glu Lys
1               5                   10

<210>  41
<211>  13
<212>  PRT
<213>  Homo sapiens

<400>  41

Ala Val Val Val His Ala Gly Glu Asp Asp Leu Gly Arg
1               5                   10

<210>  42
<211>  9
<212>  PRT
<213>  Homo sapiens

<400>  42

Cys Cys Thr Glu Ser Leu Val Asn Arg
1               5

<210>  43
<211>  8
<212>  PRT
<213>  Homo sapiens

<400>  43

Cys Phe Glu Pro Gln Leu Leu Arg
1               5

<210>  44
<211>  12
<212>  PRT
<213>  Homo sapiens

<400>  44

Cys Gly Leu Val Pro Val Leu Ala Glu Asn Tyr Lys
1               5                   10


<210>  45
<211>  9
<212>  PRT
<213>  Homo sapiens

<400>  45

Cys Ile Ser Ile Tyr Ser Ser Glu Arg
1               5


<210>  46
<211>  8
<212>  PRT
<213>  Homo sapiens

<400>  46

Cys Leu Val Asn Leu Ile Glu Lys
1               5


<210>  47
<211>  14
<212>  PRT
<213>  Homo sapiens

<400>  47

Cys Pro Asn Pro Pro Val Gln Glu Asn Phe Asp Val Asn Lys
1               5                   10


<210>  48
<211>  10
<212>  PRT
<213>  Homo sapiens

<400>  48

Cys Ser Val Phe Tyr Gly Ala Pro Ser Lys
1               5                   10


<210>  49
<211>  18

<212>  PRT
<213>  Homo sapiens

<400>  49

Asp Ala Ile Pro Glu Asp Leu Pro Pro Leu Thr Ala Asp Phe Ala Glu
1               5                   10                  15

Asp Lys

<210>  50
<211>  12
<212>  PRT
<213>  Homo sapiens

<400>  50

Asp Cys Gln Pro Gly Leu Cys Cys Ala Phe Gln Arg
1               5                   10

<210>  51
<211>  11
<212>  PRT
<213>  Homo sapiens

<400>  51

Asp Cys Ser Gly Val Ser Leu His Leu Thr Arg
1               5                   10

<210>  52
<211>  10
<212>  PRT
<213>  Homo sapiens

<400>  52

Asp Glu Pro Pro Gln Ser Pro Trp Asp Arg
1               5                   10

<210>  53
<211>  10
<212>  PRT
<213>  Homo sapiens

<400>  53

Asp Phe Asp Phe Val Pro Pro Val Val Arg
1               5                   10

<210>  54
<211>  8
<212>  PRT
<213>  Homo sapiens

<400>  54

Asp Gly Phe Phe Gly Asn Pro Arg
1               5


<210>  55
<211>  15
<212>  PRT
<213>  Homo sapiens

<400>  55

Asp Gly Phe Val Gln Asp Glu Gly Thr Met Phe Pro Val Gly Lys
1               5                   10                  15


<210>  56
<211>  11
<212>  PRT
<213>  Homo sapiens

<400>  56

Asp Gly Asn Pro Phe Tyr Phe Thr Asp His Arg
1               5                   10


<210>  57
<211>  9
<212>  PRT
<213>  Homo sapiens

<400>  57

Asp His Ala Val Asp Leu Ile Gln Lys
1               5


<210>  58
<211>  12
<212>  PRT
<213>  Homo sapiens

<400>  58

Asp Leu Glu His Leu Thr Ser Leu Asp Phe Phe Arg
1               5                   10


<210>  59
<211>  9

<212>  PRT
<213>  Homo sapiens

<400>  59

Asp Asn Cys Cys Ile Leu Asp Glu Arg
1               5


<210>  60
<211>  11
<212>  PRT
<213>  Homo sapiens

<400>  60

Asp Asn Asp Gly Trp Leu Thr Ser Asp Pro Arg
1               5                   10


<210>  61
<211>  8
<212>  PRT
<213>  Homo sapiens

<400>  61

Asp Asn Leu Ala Ile Gln Thr Arg
1               5


<210>  62
<211>  14
<212>  PRT
<213>  Homo sapiens

<400>  62

Asp Asn Pro Gln Thr His Tyr Tyr Ala Val Ala Val Val Lys
1               5                   10


<210>  63
<211>  16
<212>  PRT
<213>  Homo sapiens

<400>  63

Asp Pro Asp Gln Thr Asp Gly Leu Gly Leu Ser Tyr Leu Ser Ser His
1               5                   10                  15


<210>  64
<211>  12
<212>  PRT
<213>  Homo sapiens

<400>  64

Asp Pro Thr Phe Ile Pro Ala Pro Ile Gln Ala Lys
1               5                   10


<210>  65
<211>  14
<212>  PRT
<213>  Homo sapiens

<400>  65

Asp Pro Val Gln Glu Ala Trp Ala Glu Asp Val Asp Leu Arg
1               5                   10


<210>  66
<211>  10
<212>  PRT
<213>  Homo sapiens

<400>  66

Asp Gln Asp Gly Glu Ile Leu Leu Pro Arg
1               5                   10


<210>  67
<211>  8
<212>  PRT
<213>  Homo sapiens

<400>  67

Asp Gln Tyr Glu Leu Leu Cys Arg
1               5


<210>  68
<211>  18
<212>  PRT
<213>  Homo sapiens

<400>  68

Asp Ser Cys Gln Gly Asp Ser Gly Gly Pro Leu Val Cys Gly Asp His
1               5                   10                  15


Leu Arg


<210>  69
<211>  10

<212>  PRT
<213>  Homo sapiens

<400>  69

Asp Ser Cys Val Gly Ser Leu Val Val Lys
1               5                   10


<210>  70
<211>  14
<212>  PRT
<213>  Homo sapiens

<400>  70

Asp Ser His Ser Leu Thr Thr Asn Leu Met Glu Leu Leu Arg
1               5                   10


<210>  71
<211>  14
<212>  PRT
<213>  Homo sapiens

<400>  71

Asp Ser Pro Val Leu Ile Asp Phe Phe Glu Asp Thr Glu Arg
1               5                   10


<210>  72
<211>  13
<212>  PRT
<213>  Homo sapiens

<400>  72

Asp Val Phe Leu Gly Met Phe Leu Tyr Glu Tyr Ala Arg
1               5                   10


<210>  73
<211>  13
<212>  PRT
<213>  Homo sapiens

<400>  73

Asp Val Phe Leu Gly Thr Phe Leu Tyr Glu Tyr Ser Arg
1               5                   10


<210>  74
<211>  13
<212>  PRT
<213>  Homo sapiens

<400>  74

Asp Val Ile Ala Thr Asp Lys Glu Asp Val Ala Phe Lys
1               5                   10


<210>  75
<211>  13
<212>  PRT
<213>  Homo sapiens

<400>  75

Asp Val Val Leu Thr Thr Thr Phe Val Asp Asp Ile Lys
1               5                   10


<210>  76
<211>  13
<212>  PRT
<213>  Homo sapiens

<400>  76

Asp Tyr Val Ser Gln Phe Glu Gly Ser Ala Leu Gly Lys
1               5                   10


<210>  77
<211>  14
<212>  PRT
<213>  Homo sapiens

<400>  77

Glu Cys Cys His Gly Asp Leu Leu Glu Cys Ala Asp Asp Arg
1               5                   10


<210>  78
<211>  15
<212>  PRT
<213>  Homo sapiens

<400>  78

Glu Asp Ala Leu Asn Glu Thr Arg Glu Ser Glu Thr Lys Leu Lys
1               5                   10                  15


<210>  79
<211>  13
<212>  PRT
<213>  Homo sapiens

<400>  79

Glu Asp Glu Glu Glu Glu Glu Gly Glu Asn Tyr Gln Lys
1               5                   10

<210>  80
<211>  8
<212>  PRT
<213>  Homo sapiens

<400>  80

Glu Asp Tyr Ile Cys Tyr Ala Arg
1               5

<210>  81
<211>  15
<212>  PRT
<213>  Homo sapiens

<400>  81

Glu Glu Glu Glu Glu Met Ala Val Val Pro Gln Gly Leu Phe Arg
1               5                   10                  15

<210>  82
<211>  10
<212>  PRT
<213>  Homo sapiens

<400>  82

Glu Glu Leu Leu Pro Ala Gln Asp Ile Lys
1               5                   10

<210>  83
<211>  13
<212>  PRT
<213>  Homo sapiens

<400>  83

Glu Glu Leu Val Tyr Glu Leu Asn Pro Leu Asp His Arg
1               5                   10

<210>  84
<211>  9
<212>  PRT
<213>  Homo sapiens

<400>  84

Glu Phe Asp His Asn Ser Asn Leu Arg

1               5


<210>  85
<211>  13
<212>  PRT
<213>  Homo sapiens

<400>  85

Glu Gly Asp Gly Ser Cys Phe Pro Asp Ala Leu Asp Arg
1               5                   10


<210>  86
<211>  13
<212>  PRT
<213>  Homo sapiens

<400>  86

Glu Gly Leu Asp Leu Gln Val Leu Glu Asp Ser Gly Arg
1               5                   10


<210>  87
<211>  9
<212>  PRT
<213>  Homo sapiens

<400>  87

Glu Gly Pro Val Leu Ile Leu Gly Arg
1               5


<210>  88
<211>  9
<212>  PRT
<213>  Homo sapiens

<400>  88

Glu Gly Pro Val Leu Leu Leu Gly Arg
1               5


<210>  89
<211>  9
<212>  PRT
<213>  Homo sapiens

<400>  89

Glu His Ser Ser Leu Ala Phe Trp Lys
1               5

<210> 90
<211> 9
<212> PRT
<213> Homo sapiens

<400> 90

Glu Ile Gly Glu Leu Tyr Leu Pro Lys
1               5


<210> 91
<211> 16
<212> PRT
<213> Homo sapiens

<400> 91

Glu Ile Leu Ser Val Asp Cys Ser Thr Asn Asn Pro Ser Gln Ala Lys
1               5                   10                  15


<210> 92
<211> 11
<212> PRT
<213> Homo sapiens

<400> 92

Glu Ile Met Glu Asn Tyr Asn Ile Ala Leu Arg
1               5                   10


<210> 93
<211> 11
<212> PRT
<213> Homo sapiens

<400> 93

Glu Lys Pro Gly Val Tyr Thr Asn Val Cys Arg
1               5                   10


<210> 94
<211> 11
<212> PRT
<213> Homo sapiens

<400> 94

Glu Leu Asp Glu Ser Leu Gln Val Ala Glu Arg
1               5                   10


<210> 95

<211>  8
<212>  PRT
<213>  Homo sapiens

<400>  95

Glu Leu Asp Val Leu Gln Gly Arg
1               5


<210>  96
<211>  11
<212>  PRT
<213>  Homo sapiens

<400>  96

Glu Leu Leu Asp Thr Val Thr Ala Pro Gln Lys
1               5                   10


<210>  97
<211>  10
<212>  PRT
<213>  Homo sapiens

<400>  97

Glu Leu Leu Glu Ser Tyr Ile Asp Gly Arg
1               5                   10


<210>  98
<211>  11
<212>  PRT
<213>  Homo sapiens

<400>  98

Glu Leu Met Glu Asn Tyr Asn Leu Ala Leu Arg
1               5                   10


<210>  99
<211>  8
<212>  PRT
<213>  Homo sapiens

<400>  99

Glu Leu Pro Glu His Thr Val Lys
1               5


<210>  100
<211>  15
<212>  PRT

<213>  Homo sapiens

<400>  100

Glu Leu Ser His Leu Pro Ser Leu Tyr Asp Tyr Ser Ala Tyr Arg
1               5                   10                  15


<210>  101
<211>  13
<212>  PRT
<213>  Homo sapiens

<400>  101

Glu Met Ser Gly Ser Pro Ala Ser Gly Leu Pro Val Lys
1               5                   10


<210>  102
<211>  8
<212>  PRT
<213>  Homo sapiens

<400>  102

Glu Asn Phe Ser Cys Leu Thr Arg
1               5


<210>  103
<211>  14
<212>  PRT
<213>  Homo sapiens

<400>  103

Glu Pro Phe Leu Ser Cys Cys Gln Phe Ala Glu Ser Leu Arg
1               5                   10


<210>  104
<211>  9
<212>  PRT
<213>  Homo sapiens

<400>  104

Glu Pro Gly Glu Phe Ala Leu Leu Arg
1               5


<210>  105
<211>  8
<212>  PRT
<213>  Homo sapiens

<400>  105

Glu Pro Gly Leu Gln Leu Trp Arg
1               5


<210>  106
<211>  13
<212>  PRT
<213>  Homo sapiens

<400>  106

Glu Pro Val Asp Asn Ala Glu Asn Cys His Leu Ala Arg
1               5                   10


<210>  107
<211>  8
<212>  PRT
<213>  Homo sapiens

<400>  107

Glu Gln Pro Pro Ser Leu Thr Arg
1               5


<210>  108
<211>  15
<212>  PRT
<213>  Homo sapiens

<400>  108

Glu Ser Ser Ser His His Pro Gly Ile Ala Glu Phe Pro Ser Arg
1               5                   10                  15


<210>  109
<211>  8
<212>  PRT
<213>  Homo sapiens

<400>  109

Glu Val Ala Gly Leu Trp Leu Lys
1               5


<210>  110
<211>  14
<212>  PRT
<213>  Homo sapiens

<400>  110

Glu Val Asp Ser Gly Asn Asp Ile Tyr Gly Asn Pro Ile Lys
1               5                   10

<210>  111
<211>  9
<212>  PRT
<213>  Homo sapiens

<400>  111

Glu Val Gly Val Tyr Glu Ala Leu Lys
1               5

<210>  112
<211>  15
<212>  PRT
<213>  Homo sapiens

<400>  112

Glu Val Gln Gly Phe Glu Ser Ala Thr Phe Leu Gly Tyr Phe Lys
1               5                   10                  15

<210>  113
<211>  12
<212>  PRT
<213>  Homo sapiens

<400>  113

Glu Val Val Ala Asp Ser Val Trp Val Asp Val Lys
1               5                   10

<210>  114
<211>  10
<212>  PRT
<213>  Homo sapiens

<400>  114

Glu Tyr Glu Ser Tyr Ser Asp Phe Glu Arg
1               5                   10

<210>  115
<211>  7
<212>  PRT
<213>  Homo sapiens

<400>  115

Phe Ala Cys Tyr Tyr Pro Arg
1               5

<210>  116
<211>  15
<212>  PRT
<213>  Homo sapiens

<400>  116

Phe Asp Gln Phe Phe Gly Glu Gly Cys Ala Pro Gly Ser Gln Arg
1               5                   10                  15


<210>  117
<211>  8
<212>  PRT
<213>  Homo sapiens

<400>  117

Phe Glu Asp Cys Cys Gln Glu Lys
1               5


<210>  118
<211>  9
<212>  PRT
<213>  Homo sapiens

<400>  118

Phe Glu Asp Glu Asn Phe Leu Leu Lys
1               5


<210>  119
<211>  12
<212>  PRT
<213>  Homo sapiens

<400>  119

Phe Glu Asp Gly Val Leu Asp Pro Asp Tyr Pro Arg
1               5                   10


<210>  120
<211>  9
<212>  PRT
<213>  Homo sapiens

<400>  120

Phe Phe Glu Glu Cys Asp Pro Asn Lys
1               5

<210>  121
<211>  9
<212>  PRT
<213>  Homo sapiens

<400>  121

Phe Ile Ser Leu Gly Glu Ala Cys Lys
1               5


<210>  122
<211>  17
<212>  PRT
<213>  Homo sapiens

<400>  122

Phe Leu Cys Thr Gly Gly Val Ser Pro Tyr Ala Asp Pro Asn Thr Cys
1               5                   10                  15


Arg



<210>  123
<211>  10
<212>  PRT
<213>  Homo sapiens

<400>  123

Phe Leu Pro Leu Leu Pro Leu Pro Glu Arg
1               5                   10


<210>  124
<211>  8
<212>  PRT
<213>  Homo sapiens

<400>  124

Phe Met Glu Thr Val Ala Glu Lys
1               5


<210>  125
<211>  8
<212>  PRT
<213>  Homo sapiens

<400>  125

Phe Pro Leu Thr Asn Ala Leu Lys
1               5

<210>  126
<211>  8
<212>  PRT
<213>  Homo sapiens

<400>  126

Phe Gln Asn Ala Leu Leu Val Arg
1               5


<210>  127
<211>  9
<212>  PRT
<213>  Homo sapiens

<400>  127

Phe Gln Pro Leu Val Asp Glu Pro Lys
1               5


<210>  128
<211>  10
<212>  PRT
<213>  Homo sapiens

<400>  128

Phe Ser Gly Thr Trp Tyr Ala Met Ala Lys
1               5                   10


<210>  129
<211>  13
<212>  PRT
<213>  Homo sapiens

<400>  129

Phe Ser Asn Thr Asp Tyr Ala Val Gly Tyr Met Leu Arg
1               5                   10


<210>  130
<211>  11
<212>  PRT
<213>  Homo sapiens

<400>  130

Phe Thr Asp Ser Glu Asn Val Cys Gln Glu Arg
1               5                   10

<210>  131
<211>  7
<212>  PRT
<213>  Homo sapiens

<400>  131

Phe Thr Phe Glu Tyr Ser Arg
1               5


<210>  132
<211>  12
<212>  PRT
<213>  Homo sapiens

<400>  132

Phe Val Glu Gly Leu Pro Ile Asn Asp Phe Ser Arg
1               5                   10


<210>  133
<211>  10
<212>  PRT
<213>  Homo sapiens

<400>  133

Phe Val Val Thr Asp Gly Gly Ile Thr Arg
1               5                   10


<210>  134
<211>  6
<212>  PRT
<213>  Homo sapiens

<400>  134

Phe Trp Asp Tyr Leu Arg
1               5


<210>  135
<211>  13
<212>  PRT
<213>  Homo sapiens

<400>  135

Gly Ala Tyr Pro Leu Ser Ile Glu Pro Ile Gly Val Arg
1               5                   10


<210>  136
<211>  13

<212>  PRT
<213>  Homo sapiens

<400>  136

Gly Ala Tyr Pro Leu Ser Leu Glu Pro Leu Gly Val Arg
1               5                   10


<210>  137
<211>  11
<212>  PRT
<213>  Homo sapiens

<400>  137

Gly Cys Pro Thr Glu Glu Gly Cys Gly Glu Arg
1               5                   10


<210>  138
<211>  14
<212>  PRT
<213>  Homo sapiens

<400>  138

Gly Asp Gly Pro Val Gln Gly Ile Ile Asn Phe Glu Gln Lys
1               5                   10


<210>  139
<211>  11
<212>  PRT
<213>  Homo sapiens

<400>  139

Gly Asp Leu Tyr Phe Ala Asn Val Glu Glu Lys
1               5                   10


<210>  140
<211>  9
<212>  PRT
<213>  Homo sapiens

<400>  140

Gly Asp Tyr Pro Leu Glu Ala Val Arg
1               5


<210>  141
<211>  16
<212>  PRT
<213>  Homo sapiens

<400> 141

Gly Glu Ala Gly Ala Pro Gly Glu Glu Asp Ile Gln Gly Pro Thr Lys
1               5                   10                  15


<210> 142
<211> 16
<212> PRT
<213> Homo sapiens

<400> 142

Gly Glu Ala Gly Ala Pro Gly Glu Glu Asp Leu Gln Gly Pro Thr Lys
1               5                   10                  15


<210> 143
<211> 15
<212> PRT
<213> Homo sapiens

<400> 143

Gly Glu Ala Ser Val Cys Val Glu Asp Trp Glu Ser Gly Asp Arg
1               5                   10                  15


<210> 144
<211> 15
<212> PRT
<213> Homo sapiens

<400> 144

Gly Glu Cys Gln Ala Glu Gly Val Leu Phe Phe Gln Gly Asp Arg
1               5                   10                  15


<210> 145
<211> 15
<212> PRT
<213> Homo sapiens

<400> 145

Gly Glu Ser Gly Pro Ser Gly Pro Ala Gly Pro Thr Gly Ala Arg
1               5                   10                  15


<210> 146
<211> 18
<212> PRT
<213> Homo sapiens

<400> 146

Gly Glu Thr Gly Pro Ala Gly Pro Ala Gly Pro Val Gly Pro Val Gly
1               5                   10                  15

Ala Arg

<210>  147
<211>  15
<212>  PRT
<213>  Homo sapiens

<400>  147

Gly Glu Val Gln Ala Met Leu Gly Gln Ser Thr Glu Glu Leu Arg
1               5                   10                  15

<210>  148
<211>  24
<212>  PRT
<213>  Homo sapiens

<400>  148

Gly Glu Val Gln Ala Met Leu Gly Gln Ser Thr Glu Glu Leu Arg Leu
1               5                   10                  15

Glu Glu Gln Ala Gln Gln Ile Arg
            20

<210>  149
<211>  14
<212>  PRT
<213>  Homo sapiens

<400>  149

Gly Gly Glu Gly Thr Gly Tyr Phe Val Asp Phe Ser Val Arg
1               5                   10

<210>  150
<211>  16
<212>  PRT
<213>  Homo sapiens

<400>  150

Gly Gly Ser Thr Ser Tyr Gly Thr Gly Ser Glu Thr Glu Ser Pro Arg
1               5                   10                  15

<210>  151
<211>  11
<212>  PRT
<213>  Homo sapiens

<400>  151

Gly Gly Tyr Thr Leu Val Ser Gly Tyr Pro Lys
1               5                   10


<210>  152
<211>  8
<212>  PRT
<213>  Homo sapiens

<400>  152

Gly Ile Phe Pro Val Leu Cys Lys
1               5


<210>  153
<211>  14
<212>  PRT
<213>  Homo sapiens

<400>  153

Gly Lys Leu Glu Glu Asp Ser Glu Val Leu Met Met Leu Lys
1               5                   10


<210>  154
<211>  11
<212>  PRT
<213>  Homo sapiens

<400>  154

Gly Lys Pro Pro Pro Ser Phe Ser Trp Thr Arg
1               5                   10


<210>  155
<211>  11
<212>  PRT
<213>  Homo sapiens

<400>  155

Gly Leu Cys Val Ala Thr Pro Val Gln Leu Arg
1               5                   10


<210>  156
<211>  13

<212>  PRT
<213>  Homo sapiens

<400>  156

Gly Leu Ile Asp Glu Val Asn Gln Asp Phe Thr Asn Arg
1               5                   10


<210>  157
<211>  13
<212>  PRT
<213>  Homo sapiens

<400>  157

Gly Leu Leu Asp Glu Val Asn Gln Asp Phe Thr Asn Arg
1               5                   10


<210>  158
<211>  9
<212>  PRT
<213>  Homo sapiens

<400>  158

Gly Leu Gln Asp Glu Asp Gly Tyr Arg
1               5


<210>  159
<211>  11
<212>  PRT
<213>  Homo sapiens

<400>  159

Gly Leu Ser Ala Glu Pro Gly Trp Gln Ala Lys
1               5                   10


<210>  160
<211>  13
<212>  PRT
<213>  Homo sapiens

<400>  160

Gly Leu Val Ser Trp Gly Asn Ile Pro Cys Gly Ser Lys
1               5                   10


<210>  161
<211>  9
<212>  PRT
<213>  Homo sapiens

<400> 161

Gly Asn Leu Ala Gly Leu Thr Leu Arg
1               5


<210> 162
<211> 12
<212> PRT
<213> Homo sapiens

<400> 162

Gly Asn Pro Val Pro Gln Gln Tyr Leu Trp Glu Lys
1               5                   10


<210> 163
<211> 14
<212> PRT
<213> Homo sapiens

<400> 163

Gly Asn Gln Trp Val Gly Tyr Asp Asp Gln Glu Ser Val Lys
1               5                   10


<210> 164
<211> 8
<212> PRT
<213> Homo sapiens

<400> 164

Gly Pro Cys Trp Cys Val Asp Arg
1               5


<210> 165
<211> 12
<212> PRT
<213> Homo sapiens

<400> 165

Gly Pro Pro Gly Pro Pro Gly Gly Val Val Val Arg
1               5                   10


<210> 166
<211> 8
<212> PRT
<213> Homo sapiens

<400> 166

Gly Gln Leu Val Phe Met Asn Arg
1               5

<210>  167
<211>  13
<212>  PRT
<213>  Homo sapiens

<400>  167

Gly Ser Phe Glu Phe Pro Val Gly Asp Ala Val Ser Lys
1               5                   10

<210>  168
<211>  12
<212>  PRT
<213>  Homo sapiens

<400>  168

Gly Ser Asn Phe Gln Trp Asn Gln Leu Gln Gly Lys
1               5                   10

<210>  169
<211>  13
<212>  PRT
<213>  Homo sapiens

<400>  169

Gly Ser Pro Ala Ile Asn Val Ala Val His Val Phe Arg
1               5                   10

<210>  170
<211>  14
<212>  PRT
<213>  Homo sapiens

<400>  170

Gly Thr Cys Glu Gln Gly Pro Ser Ile Val Thr Pro Pro Lys
1               5                   10

<210>  171
<211>  13
<212>  PRT
<213>  Homo sapiens

<400>  171

Gly Thr Phe Ala Thr Leu Ser Glu Leu His Cys Asp Lys

1                   5                        10


<210>  172
<211>  11
<212>  PRT
<213>  Homo sapiens

<400>  172

Gly Tyr Cys Ala Pro Gly Met Glu Cys Val Lys
1                   5                        10


<210>  173
<211>  15
<212>  PRT
<213>  Homo sapiens

<400>  173

Gly Tyr Pro Gly Val Gln Ala Pro Glu Asp Leu Glu Trp Glu Arg
1                   5                        10                       15


<210>  174
<211>  10
<212>  PRT
<213>  Homo sapiens

<400>  174

Gly Tyr Ser Phe Thr Thr Thr Ala Glu Arg
1                   5                        10


<210>  175
<211>  9
<212>  PRT
<213>  Homo sapiens

<400>  175

Gly Tyr Thr Gln Gln Leu Ala Phe Arg
1                   5


<210>  176
<211>  13
<212>  PRT
<213>  Homo sapiens

<400>  176

Gly Tyr Tyr Tyr Glu Leu Pro Ser Leu Gly Ala Leu Arg
1                   5                        10

<210> 177
<211> 9
<212> PRT
<213> Homo sapiens

<400> 177

His Ile Tyr Leu Leu Pro Ser Gly Arg
1               5


<210> 178
<211> 15
<212> PRT
<213> Homo sapiens

<400> 178

His Leu Asp Ser Val Leu Gln Gln Leu Gln Thr Glu Val Tyr Arg
1               5                   10                  15


<210> 179
<211> 16
<212> PRT
<213> Homo sapiens

<400> 179

His Leu Glu Glu Pro Gly Glu Thr Gln Asn Ala Phe Leu Asn Glu Arg
1               5                   10                  15


<210> 180
<211> 11
<212> PRT
<213> Homo sapiens

<400> 180

His Leu Ser Leu Leu Thr Thr Leu Ser Asn Arg
1               5                   10


<210> 181
<211> 9
<212> PRT
<213> Homo sapiens

<400> 181

His Leu Tyr Leu Leu Pro Ser Gly Arg
1               5


<210> 182

<211>  15
<212>  PRT
<213>  Homo sapiens

<400>  182

His Pro Asp Glu Ala Ala Phe Phe Asp Thr Ala Ser Thr Gly Lys
1               5                   10                  15


<210>  183
<211>  11
<212>  PRT
<213>  Homo sapiens

<400>  183

His Pro Asp Tyr Ser Val Ser Leu Leu Leu Arg
1               5                   10


<210>  184
<211>  11
<212>  PRT
<213>  Homo sapiens

<400>  184

His Pro Asp Tyr Ser Val Val Leu Leu Leu Arg
1               5                   10


<210>  185
<211>  11
<212>  PRT
<213>  Homo sapiens

<400>  185

His Ser Thr Val Leu Glu Asn Leu Pro Asp Lys
1               5                   10


<210>  186
<211>  12
<212>  PRT
<213>  Homo sapiens

<400>  186

His Val Gly Asp Leu Gly Asn Val Thr Ala Asp Lys
1               5                   10


<210>  187
<211>  11
<212>  PRT

<213>  Homo sapiens

<400>  187

His Val Val Pro Asn Glu Val Val Val Gln Arg
1               5                   10


<210>  188
<211>  12
<212>  PRT
<213>  Homo sapiens

<400>  188

His Tyr Asp Gly Ser Tyr Ser Thr Phe Gly Glu Arg
1               5                   10


<210>  189
<211>  9
<212>  PRT
<213>  Homo sapiens

<400>  189

Ile Ala Leu Val Ile Thr Asp Gly Arg
1               5


<210>  190
<211>  14
<212>  PRT
<213>  Homo sapiens

<400>  190

Ile Asp Gly Asp Thr Ile Ile Phe Ser Asn Val Gln Glu Arg
1               5                   10


<210>  191
<211>  9
<212>  PRT
<213>  Homo sapiens

<400>  191

Ile Asp Gln Thr Val Glu Glu Leu Arg
1               5


<210>  192
<211>  9
<212>  PRT
<213>  Homo sapiens

<400>  192

Ile Asp Ser Leu Leu Glu Asn Asp Arg
1               5


<210>  193
<211>  15
<212>  PRT
<213>  Homo sapiens

<400>  193

Ile Glu Cys Val Ser Ala Glu Asn Thr Glu Asp Cys Ile Ala Lys
1               5                   10                  15


<210>  194
<211>  8
<212>  PRT
<213>  Homo sapiens

<400>  194

Ile Gly Ala Asp Phe Leu Ala Arg
1               5


<210>  195
<211>  12
<212>  PRT
<213>  Homo sapiens

<400>  195

Ile Ile Met Leu Phe Thr Asp Gly Gly Glu Glu Arg
1               5                   10


<210>  196
<211>  8
<212>  PRT
<213>  Homo sapiens

<400>  196

Ile Leu Asp Asp Leu Ser Pro Arg
1               5


<210>  197
<211>  11
<212>  PRT
<213>  Homo sapiens

<400>  197

Ile Asn His Gly Ile Leu Tyr Asp Glu Glu Lys
1               5                   10

<210>  198
<211>  14
<212>  PRT
<213>  Homo sapiens

<400>  198

Ile Pro Ile Glu Asp Gly Ser Gly Glu Val Val Leu Ser Arg
1               5                   10

<210>  199
<211>  9
<212>  PRT
<213>  Homo sapiens

<400>  199

Ile Pro Thr Thr Phe Glu Asn Gly Arg
1               5

<210>  200
<211>  8
<212>  PRT
<213>  Homo sapiens

<400>  200

Ile Arg Pro Phe Phe Pro Gln Gln
1               5

<210>  201
<211>  11
<212>  PRT
<213>  Homo sapiens

<400>  201

Ile Ser Tyr Gln Ser Ser Ser Thr Glu Glu Arg
1               5                   10

<210>  202
<211>  13
<212>  PRT
<213>  Homo sapiens

<400>  202

Ile Thr Cys Thr Glu Glu Gly Trp Ser Pro Thr Pro Lys
1               5                   10

<210> 203
<211> 9
<212> PRT
<213> Homo sapiens

<400> 203

Ile Thr Gln Val Leu His Phe Thr Lys
1               5


<210> 204
<211> 13
<212> PRT
<213> Homo sapiens

<400> 204

Ile Thr Val Val Asp Ala Leu His Glu Ile Pro Val Lys
1               5                   10


<210> 205
<211> 12
<212> PRT
<213> Homo sapiens

<400> 205

Ile Thr Trp Ser Asn Pro Pro Ala Gln Gly Ala Arg
1               5                   10


<210> 206
<211> 14
<212> PRT
<213> Homo sapiens

<400> 206

Ile Val Ile Gly Met Asp Val Ala Ala Ser Glu Phe Tyr Arg
1               5                   10


<210> 207
<211> 9
<212> PRT
<213> Homo sapiens

<400> 207

Ile Val Gln Phe Ser Pro Ser Gly Lys
1               5

<210>  208
<211>  7
<212>  PRT
<213>  Homo sapiens

<400>  208

Ile Trp Asp Val Val Glu Lys
1               5


<210>  209
<211>  12
<212>  PRT
<213>  Homo sapiens

<400>  209

Ile Tyr Glu Ala Thr Leu Glu Asp Cys Cys Ala Lys
1               5                   10


<210>  210
<211>  8
<212>  PRT
<213>  Homo sapiens

<400>  210

Lys Leu Cys Met Ala Ala Leu Lys
1               5


<210>  211
<211>  14
<212>  PRT
<213>  Homo sapiens

<400>  211

Lys Leu Val Val Glu Asn Val Asp Val Leu Thr Gln Met Arg
1               5                   10


<210>  212
<211>  7
<212>  PRT
<213>  Homo sapiens

<400>  212

Lys Met Glu Glu Asn Glu Lys
1               5


<210>  213
<211>  10

<212>  PRT
<213>  Homo sapiens

<400>  213

Lys Pro Asn Leu Gln Val Phe Leu Gly Lys
1               5                   10


<210>  214
<211>  11
<212>  PRT
<213>  Homo sapiens

<400>  214

Lys Thr Leu Leu Ser Asn Leu Glu Glu Ala Lys
1               5                   10


<210>  215
<211>  15
<212>  PRT
<213>  Homo sapiens

<400>  215

Lys Val Glu Gln Ala Val Glu Thr Glu Pro Glu Pro Glu Leu Arg
1               5                   10                  15


<210>  216
<211>  15
<212>  PRT
<213>  Homo sapiens

<400>  216

Lys Val Pro Glu Val Ser Thr Pro Thr Leu Val Glu Val Ser Arg
1               5                   10                  15


<210>  217
<211>  15
<212>  PRT
<213>  Homo sapiens

<400>  217

Lys Val Pro Gln Val Ser Thr Pro Thr Leu Val Glu Val Ser Arg
1               5                   10                  15


<210>  218
<211>  7
<212>  PRT
<213>  Homo sapiens

<400>  218

Lys Tyr Asn Glu Leu Leu Lys
1               5


<210>  219
<211>  14
<212>  PRT
<213>  Homo sapiens

<400>  219

Leu Ala Ala Ala Val Ser Asn Phe Gly Tyr Asp Leu Tyr Arg
1               5                   10


<210>  220
<211>  14
<212>  PRT
<213>  Homo sapiens

<400>  220

Leu Ala Gln Trp Gln Ser Phe Gln Leu Glu Gly Gly Leu Lys
1               5                   10


<210>  221
<211>  8
<212>  PRT
<213>  Homo sapiens

<400>  221

Leu Cys Asp Asn Leu Ser Thr Lys
1               5


<210>  222
<211>  11
<212>  PRT
<213>  Homo sapiens

<400>  222

Leu Cys Gln Asp Leu Gly Pro Gly Ala Phe Arg
1               5                   10


<210>  223
<211>  7
<212>  PRT
<213>  Homo sapiens

<400>  223

Leu Asp Val His Trp Thr Arg
1               5


<210>  224
<211>  10
<212>  PRT
<213>  Homo sapiens

<400>  224

Leu Glu Asp Leu His Leu Leu Val Glu Arg
1               5                   10


<210>  225
<211>  10
<212>  PRT
<213>  Homo sapiens

<400>  225

Leu Glu Glu Glu Leu Gly Asp Glu Ala Arg
1               5                   10


<210>  226
<211>  9
<212>  PRT
<213>  Homo sapiens

<400>  226

Leu Glu Glu Gln Ala Gln Gln Ile Arg
1               5


<210>  227
<211>  12
<212>  PRT
<213>  Homo sapiens

<400>  227

Leu Glu Gly Glu Ala Cys Gly Val Tyr Thr Pro Arg
1               5                   10


<210>  228
<211>  9
<212>  PRT
<213>  Homo sapiens

<400>  228

Leu Glu Pro Tyr Ala Asp Gln Leu Arg

1               5


<210>  229
<211>  13
<212>  PRT
<213>  Homo sapiens

<400>  229

Leu Glu Ser Asp Val Ser Ala Gln Met Glu Tyr Cys Arg
1               5                   10


<210>  230
<211>  14
<212>  PRT
<213>  Homo sapiens

<400>  230

Leu Glu Val Leu Tyr Thr Pro Thr Ala Met Leu Arg Pro Asp
1               5                   10


<210>  231
<211>  7
<212>  PRT
<213>  Homo sapiens

<400>  231

Leu Phe Glu Glu Leu Val Arg
1               5


<210>  232
<211>  11
<212>  PRT
<213>  Homo sapiens

<400>  232

Leu Gly Ala Asp Met Glu Asp Val Cys Gly Arg
1               5                   10


<210>  233
<211>  10
<212>  PRT
<213>  Homo sapiens

<400>  233

Leu Gly Ala Glu Val Tyr His Thr Leu Lys
1               5                   10

<210>  234
<211>  13
<212>  PRT
<213>  Homo sapiens

<400>  234

Leu Gly Glu Tyr Gly Phe Gln Asn Ala Leu Ile Val Arg
1               5                   10


<210>  235
<211>  9
<212>  PRT
<213>  Homo sapiens

<400>  235

Leu Gly Pro Ile Glu Ala Ile Gln Lys
1               5


<210>  236
<211>  9
<212>  PRT
<213>  Homo sapiens

<400>  236

Leu Gly Pro Leu Val Glu Gln Gly Arg
1               5


<210>  237
<211>  10
<212>  PRT
<213>  Homo sapiens

<400>  237

Leu Gly Gln Tyr Ala Ser Pro Thr Ala Lys
1               5                   10


<210>  238
<211>  16
<212>  PRT
<213>  Homo sapiens

<400>  238

Leu Ile Ala Pro Val Ala Glu Glu Glu Ala Thr Val Pro Asn Asn Lys
1               5                   10                  15


<210>  239

<211>  9
<212>  PRT
<213>  Homo sapiens

<400>  239

Leu Ile Cys Ser Glu Leu Asn Gly Arg
1               5


<210>  240
<211>  14
<212>  PRT
<213>  Homo sapiens

<400>  240

Leu Ile Ser Trp Tyr Asp Asn Glu Phe Gly Tyr Ser Asn Arg
1               5                   10


<210>  241
<211>  11
<212>  PRT
<213>  Homo sapiens

<400>  241

Leu Ile Val His Asn Gly Tyr Cys Asp Gly Arg
1               5                   10


<210>  242
<211>  10
<212>  PRT
<213>  Homo sapiens

<400>  242

Leu Lys Asp Asp Glu Val Ala Gln Leu Lys
1               5                   10


<210>  243
<211>  14
<212>  PRT
<213>  Homo sapiens

<400>  243

Leu Leu Ala Leu Ala Pro Thr Phe Glu Met Asn Pro Met Lys
1               5                   10


<210>  244
<211>  11
<212>  PRT

<213>  Homo sapiens

<400>  244

Leu Leu Glu Ala Cys Thr Phe His Ser Ala Lys
1               5                   10


<210>  245
<211>  8
<212>  PRT
<213>  Homo sapiens

<400>  245

Leu Leu Glu Val Pro Glu Gly Arg
1               5


<210>  246
<211>  11
<212>  PRT
<213>  Homo sapiens

<400>  246

Leu Leu Gly Gln Gln Val Pro Tyr Ala Thr Lys
1               5                   10


<210>  247
<211>  9
<212>  PRT
<213>  Homo sapiens

<400>  247

Leu Leu Ile Tyr Trp Ala Ser Thr Arg
1               5


<210>  248
<211>  12
<212>  PRT
<213>  Homo sapiens

<400>  248

Leu Leu Met Leu Phe Thr Asp Gly Gly Glu Glu Arg
1               5                   10


<210>  249
<211>  11
<212>  PRT
<213>  Homo sapiens

<400>  249

Leu Leu Val His Asn Gly Tyr Cys Asp Gly Arg
1               5                   10


<210>  250
<211>  11
<212>  PRT
<213>  Homo sapiens

<400>  250

Leu Leu Val Gln Ala Gln Pro Glu Trp Leu Lys
1               5                   10


<210>  251
<211>  10
<212>  PRT
<213>  Homo sapiens

<400>  251

Leu Leu Val Val Tyr Pro Trp Thr Gln Arg
1               5                   10


<210>  252
<211>  11
<212>  PRT
<213>  Homo sapiens

<400>  252

Leu Asn His Gly Leu Leu Tyr Asp Glu Glu Lys
1               5                   10


<210>  253
<211>  12
<212>  PRT
<213>  Homo sapiens

<400>  253

Leu Asn Met Gly Ile Thr Asp Leu Gln Gly Leu Arg
1               5                   10


<210>  254
<211>  12
<212>  PRT
<213>  Homo sapiens

<400>  254

Leu Asn Met Gly Leu Thr Asp Leu Gln Gly Leu Arg
1               5                   10

<210>  255
<211>  11
<212>  PRT
<213>  Homo sapiens

<400>  255

Leu Pro Glu Ala Thr Pro Thr Glu Leu Ala Lys
1               5                   10

<210>  256
<211>  9
<212>  PRT
<213>  Homo sapiens

<400>  256

Leu Pro Phe Val Ile Asn Asp Gly Lys
1               5

<210>  257
<211>  15
<212>  PRT
<213>  Homo sapiens

<400>  257

Leu Pro Gly Leu Phe Glu Leu Gly Leu Ser Ser Gln Ser Asp Arg
1               5                   10                  15

<210>  258
<211>  14
<212>  PRT
<213>  Homo sapiens

<400>  258

Leu Pro Leu Glu Asp Gly Ser Gly Glu Val Val Leu Ser Arg
1               5                   10

<210>  259
<211>  11
<212>  PRT
<213>  Homo sapiens

<400>  259

Leu Pro Leu Glu Tyr Ser Tyr Gly Glu Tyr Arg
1               5                   10

<210> 260
<211> 11
<212> PRT
<213> Homo sapiens

<400> 260

Leu Pro Pro Asn Val Val Glu Glu Ser Ala Arg
1               5                   10


<210> 261
<211> 12
<212> PRT
<213> Homo sapiens

<400> 261

Leu Gln Ser Leu Phe Asp Ser Pro Asp Phe Ser Lys
1               5                   10


<210> 262
<211> 14
<212> PRT
<213> Homo sapiens

<400> 262

Leu Ser Cys Ala Glu Asp Tyr Leu Ser Leu Val Leu Asn Arg
1               5                   10


<210> 263
<211> 9
<212> PRT
<213> Homo sapiens

<400> 263

Leu Ser Glu Asp Tyr Gly Val Leu Lys
1               5


<210> 264
<211> 12
<212> PRT
<213> Homo sapiens

<400> 264

Leu Ser Glu Leu Ile Gln Pro Leu Pro Leu Glu Arg
1               5                   10

<210>  265
<211>  12
<212>  PRT
<213>  Homo sapiens

<400>  265

Leu Ser Leu Glu Leu Glu Gln Leu Glu Leu Gln Arg
1               5                   10


<210>  266
<211>  10
<212>  PRT
<213>  Homo sapiens

<400>  266

Leu Ser Pro Tyr Val Asn Tyr Ser Phe Arg
1               5                   10


<210>  267
<211>  9
<212>  PRT
<213>  Homo sapiens

<400>  267

Leu Ser Tyr Glu Gly Glu Val Thr Lys
1               5


<210>  268
<211>  9
<212>  PRT
<213>  Homo sapiens

<400>  268

Leu Thr Gln Val Leu His Phe Thr Lys
1               5


<210>  269
<211>  12
<212>  PRT
<213>  Homo sapiens

<400>  269

Leu Thr Trp Ser Asn Pro Pro Ala Gln Gly Ala Arg
1               5                   10


<210>  270
<211>  10

<212>  PRT
<213>  Homo sapiens

<400>  270

Leu Val Glu Ala Phe Gly Gly Ala Thr Lys
1               5                   10


<210>  271
<211>  16
<212>  PRT
<213>  Homo sapiens

<400>  271

Leu Val Glu Ser Gly Gly Gly Leu Val Gln Pro Gly Gly Ser Leu Arg
1               5                   10                  15


<210>  272
<211>  8
<212>  PRT
<213>  Homo sapiens

<400>  272

Leu Val Glu Ser Tyr Gln Leu Arg
1               5


<210>  273
<211>  16
<212>  PRT
<213>  Homo sapiens

<400>  273

Leu Val Gly Gly Pro Met Asp Ala Ser Val Glu Glu Glu Gly Val Arg
1               5                   10                  15


<210>  274
<211>  10
<212>  PRT
<213>  Homo sapiens

<400>  274

Leu Val Gly Gln Leu Met Asp Gly Leu Lys
1               5                   10


<210>  275
<211>  9
<212>  PRT
<213>  Homo sapiens

<400>  275

Leu Val His Gly Gly Pro Cys Asp Lys
1               5


<210>  276
<211>  14
<212>  PRT
<213>  Homo sapiens

<400>  276

Leu Val Leu Gly Met Asp Val Ala Ala Ser Glu Phe Tyr Arg
1               5                   10


<210>  277
<211>  10
<212>  PRT
<213>  Homo sapiens

<400>  277

Leu Val Met Gly Ile Pro Thr Phe Gly Arg
1               5                   10


<210>  278
<211>  10
<212>  PRT
<213>  Homo sapiens

<400>  278

Leu Val Met Gly Leu Pro Thr Phe Gly Arg
1               5                   10


<210>  279
<211>  11
<212>  PRT
<213>  Homo sapiens

<400>  279

Leu Val Asn Ile Tyr Asp Ser Met Pro Leu Arg
1               5                   10


<210>  280
<211>  11
<212>  PRT
<213>  Homo sapiens

<400>  280

Leu Val Asn Leu Tyr Asp Ser Met Pro Leu Arg
1               5                   10


<210>  281
<211>  11
<212>  PRT
<213>  Homo sapiens

<400>  281

Leu Val Val Glu Trp Gln Leu Gln Asp Asp Lys
1               5                   10


<210>  282
<211>  18
<212>  PRT
<213>  Homo sapiens

<400>  282

Leu Tyr Gly Ser Glu Ala Phe Ala Thr Asp Phe Gln Asp Ser Ala Ala
1               5                   10                  15


Ala Lys



<210>  283
<211>  16
<212>  PRT
<213>  Homo sapiens

<400>  283

Leu Tyr Pro Leu Ala Asn Gly Asn Asn Gln Ser Pro Val Asp Leu Lys
1               5                   10                  15


<210>  284
<211>  7
<212>  PRT
<213>  Homo sapiens

<400>  284

Met Ala Leu Glu Val Tyr Lys
1               5


<210>  285
<211>  12
<212>  PRT
<213>  Homo sapiens

<400>  285

Met Asn Asn Gly Asp Val Asp Leu Thr Ser Asp Arg
1               5                   10


<210>  286
<211>  7
<212>  PRT
<213>  Homo sapiens

<400>  286

Met Thr Leu Asp Asp Phe Arg
1               5


<210>  287
<211>  13
<212>  PRT
<213>  Homo sapiens

<400>  287

Asn Ala Leu Gly Ala Ile His His Thr Ile Ser Val Arg
1               5                   10


<210>  288
<211>  7
<212>  PRT
<213>  Homo sapiens

<400>  288

Asn Asp Phe Thr Trp Phe Lys
1               5


<210>  289
<211>  12
<212>  PRT
<213>  Homo sapiens

<400>  289

Asn Glu Asp Ser Leu Val Phe Val Gln Thr Asp Lys
1               5                   10


<210>  290
<211>  7
<212>  PRT
<213>  Homo sapiens

<400>  290

Asn Glu Gln Val Glu Ile Arg
1               5


<210>  291
<211>  8
<212>  PRT
<213>  Homo sapiens

<400>  291

Asn Phe Gly Leu Tyr Asn Glu Arg
1               5


<210>  292
<211>  13
<212>  PRT
<213>  Homo sapiens

<400>  292

Asn Phe Met Leu Asp Ser Asn Gly Glu Leu Leu Leu Arg
1               5                   10


<210>  293
<211>  11
<212>  PRT
<213>  Homo sapiens

<400>  293

Asn Phe Pro Ser Pro Val Asp Ala Ala Phe Arg
1               5                   10


<210>  294
<211>  8
<212>  PRT
<213>  Homo sapiens

<400>  294

Asn Gly Phe Tyr Pro Ala Thr Arg
1               5


<210>  295
<211>  13
<212>  PRT
<213>  Homo sapiens

<400>  295

Asn Gly Ile Asp Ile Tyr Ser Leu Thr Val Asp Ser Arg

1               5                   10


<210>  296
<211>  11
<212>  PRT
<213>  Homo sapiens

<400>  296

Asn Ile Leu Thr Ser Asn Asn Ile Asp Val Lys
1               5                   10


<210>  297
<211>  10
<212>  PRT
<213>  Homo sapiens

<400>  297

Asn Leu Ala Val Ser Gln Val Val His Lys
1               5                   10


<210>  298
<211>  15
<212>  PRT
<213>  Homo sapiens

<400>  298

Asn Leu Asp Tyr Val Ala Thr Ser Leu His Glu Ala Val Thr Lys
1               5                   10                  15


<210>  299
<211>  8
<212>  PRT
<213>  Homo sapiens

<400>  299

Asn Asn Leu Val Ile Phe His Arg
1               5


<210>  300
<211>  7
<212>  PRT
<213>  Homo sapiens

<400>  300

Asn Asn Tyr Met Tyr Ala Arg
1               5

<210> 301
<211> 13
<212> PRT
<213> Homo sapiens

<400> 301

Asn Pro Asn Leu Pro Pro Glu Thr Val Asp Ser Leu Lys
1               5                   10


<210> 302
<211> 17
<212> PRT
<213> Homo sapiens

<400> 302

Asn Ser Glu Pro Gln Asp Glu Gly Glu Leu Phe Gln Gly Val Asp Pro
1               5                   10                  15


Arg



<210> 303
<211> 14
<212> PRT
<213> Homo sapiens

<400> 303

Asn Ser Val Ala Tyr Met Asp Gln Glu Thr Gly Asn Leu Lys
1               5                   10


<210> 304
<211> 12
<212> PRT
<213> Homo sapiens

<400> 304

Asn Val Glu Ser Tyr Thr Pro Gln Thr Gln Gly Lys
1               5                   10


<210> 305
<211> 13
<212> PRT
<213> Homo sapiens

<400> 305

Asn Val Pro Leu Pro Val Ile Ala Glu Leu Pro Pro Lys

1                   5                   10


<210>  306
<211>  15
<212>  PRT
<213>  Homo sapiens

<400>  306

Asn Tyr Cys Gly Leu Pro Gly Glu Tyr Trp Leu Gly Asn Asp Lys
1               5                   10                  15


<210>  307
<211>  12
<212>  PRT
<213>  Homo sapiens

<400>  307

Pro Ala Leu Pro Ala Gly Thr Glu Asp Thr Ala Lys
1               5                   10


<210>  308
<211>  13
<212>  PRT
<213>  Homo sapiens

<400>  308

Pro Asp Asp Leu Pro Phe Ser Thr Val Val Pro Leu Lys
1               5                   10


<210>  309
<211>  14
<212>  PRT
<213>  Homo sapiens

<400>  309

Pro Gly Ser Thr Gly Thr Trp Asn Pro Gly Ser Ser Glu Arg
1               5                   10


<210>  310
<211>  14
<212>  PRT
<213>  Homo sapiens

<400>  310

Pro Leu Thr Glu Glu Ser Ser Thr Leu Gly Glu Gly Ser Lys
1               5                   10

<210> 311
<211> 11
<212> PRT
<213> Homo sapiens

<400> 311

Pro Leu Val Glu Glu Pro Gln Asn Leu Leu Lys
1               5                   10


<210> 312
<211> 8
<212> PRT
<213> Homo sapiens

<400> 312

Gln Ala Leu Glu Glu Phe Gln Lys
1               5


<210> 313
<211> 12
<212> PRT
<213> Homo sapiens

<400> 313

Gln Ala Val Glu Thr Glu Pro Glu Pro Glu Leu Arg
1               5                   10


<210> 314
<211> 13
<212> PRT
<213> Homo sapiens

<400> 314

Gln Glu Asp Asp Leu Ala Asn Ile Asn Gln Trp Val Lys
1               5                   10


<210> 315
<211> 8
<212> PRT
<213> Homo sapiens

<400> 315

Gln Glu Glu Leu Cys Leu Ala Arg
1               5


<210> 316

<211>  11
<212>  PRT
<213>  Homo sapiens

<400>  316

Gln Glu Leu Ser Glu Ala Glu Gln Ala Thr Arg
1               5                   10


<210>  317
<211>  17
<212>  PRT
<213>  Homo sapiens

<400>  317

Gln Glu Pro Ser Gln Gly Thr Thr Thr Phe Ala Val Thr Ser Leu Leu
1               5                   10                  15


Arg



<210>  318
<211>  13
<212>  PRT
<213>  Homo sapiens

<400>  318

Gln Glu Tyr Asp Glu Ser Gly Pro Ser Ile Val His Arg
1               5                   10


<210>  319
<211>  16
<212>  PRT
<213>  Homo sapiens

<400>  319

Gln Phe Ser Phe Pro Leu Ser Ser Glu Pro Phe Gln Gly Ser Tyr Lys
1               5                   10                  15


<210>  320
<211>  9
<212>  PRT
<213>  Homo sapiens

<400>  320

Gln Gly Ser Phe Gln Gly Gly Phe Arg
1               5

<210> 321
<211> 10
<212> PRT
<213> Homo sapiens

<400> 321

Gln Gly Ser Thr His Trp Gln Thr Ala Arg
1               5                   10


<210> 322
<211> 8
<212> PRT
<213> Homo sapiens

<400> 322

Gln His Phe Thr Thr Leu Ile Lys
1               5


<210> 323
<211> 11
<212> PRT
<213> Homo sapiens

<400> 323

Gln Leu Thr Val Asn Asp Leu Pro Val Gly Arg
1               5                   10


<210> 324
<211> 13
<212> PRT
<213> Homo sapiens

<400> 324

Gln Met Asp Phe Glu Leu Leu Cys Gln Asn Gly Ala Arg
1               5                   10


<210> 325
<211> 9
<212> PRT
<213> Homo sapiens

<400> 325

Gln Pro Glu Tyr Ala Val Val Gln Arg
1               5


<210> 326

<211>  11
<212>  PRT
<213>  Homo sapiens

<400>  326

Gln Pro Val Pro Gly Gln Gln Met Thr Leu Lys
1               5                   10


<210>  327
<211>  10
<212>  PRT
<213>  Homo sapiens

<400>  327

Gln Gln Thr Glu Trp Gln Ser Gly Gln Arg
1               5                   10


<210>  328
<211>  12
<212>  PRT
<213>  Homo sapiens

<400>  328

Gln Ser Glu Asp Ser Thr Phe Tyr Leu Gly Glu Arg
1               5                   10


<210>  329
<211>  13
<212>  PRT
<213>  Homo sapiens

<400>  329

Gln Ser Leu Glu Ala Ser Leu Ala Glu Thr Glu Gly Arg
1               5                   10


<210>  330
<211>  9
<212>  PRT
<213>  Homo sapiens

<400>  330

Gln Ser Ser Ser Tyr Ser Phe Phe Lys
1               5


<210>  331
<211>  9
<212>  PRT

<213>  Homo sapiens

<400>  331

Gln Thr Ala Leu Val Glu Leu Val Lys
1               5


<210>  332
<211>  17
<212>  PRT
<213>  Homo sapiens

<400>  332

Gln Thr Gln Val Ser Val Leu Pro Glu Gly Gly Glu Thr Pro Leu Phe
1               5                   10                  15


Lys


<210>  333
<211>  8
<212>  PRT
<213>  Homo sapiens

<400>  333

Gln Val Phe Gly Glu Ala Thr Lys
1               5


<210>  334
<211>  8
<212>  PRT
<213>  Homo sapiens

<400>  334

Gln Tyr Thr Asp Ser Thr Phe Arg
1               5


<210>  335
<211>  9
<212>  PRT
<213>  Homo sapiens

<400>  335

Arg Leu Asp Gly Ser Val Asp Phe Lys
1               5


<210>  336

<211>  10
<212>  PRT
<213>  Homo sapiens

<400>  336

Arg Pro Tyr Phe Pro Val Ala Val Gly Lys
1               5                   10


<210>  337
<211>  10
<212>  PRT
<213>  Homo sapiens

<400>  337

Arg Thr Met Arg Asp Gln Asp Thr Gly Lys
1               5                   10


<210>  338
<211>  11
<212>  PRT
<213>  Homo sapiens

<400>  338

Arg Val Glu Pro Tyr Gly Glu Asn Phe Asn Lys
1               5                   10


<210>  339
<211>  10
<212>  PRT
<213>  Homo sapiens

<400>  339

Ser Ala Asp Thr Leu Trp Asp Ile Gln Lys
1               5                   10


<210>  340
<211>  9
<212>  PRT
<213>  Homo sapiens

<400>  340

Ser Asp Gly Ser Cys Ala Trp Tyr Arg
1               5


<210>  341
<211>  9
<212>  PRT

<213>  Homo sapiens

<400>  341

Ser Asp Leu Glu Val Ala His Tyr Lys
1               5


<210>  342
<211>  15
<212>  PRT
<213>  Homo sapiens

<400>  342

Ser Glu Leu Glu Glu Gln Leu Thr Pro Val Ala Glu Glu Thr Arg
1               5                   10                  15


<210>  343
<211>  10
<212>  PRT
<213>  Homo sapiens

<400>  343

Ser Phe Glu Gly Pro Val Leu Asp His Arg
1               5                   10


<210>  344
<211>  10
<212>  PRT
<213>  Homo sapiens

<400>  344

Ser Gly Glu Leu Glu Gln Glu Glu Glu Arg
1               5                   10


<210>  345
<211>  8
<212>  PRT
<213>  Homo sapiens

<400>  345

Ser Gly Phe Ser Phe Gly Phe Lys
1               5


<210>  346
<211>  11
<212>  PRT
<213>  Homo sapiens

<400>  346

Ser Gly Asn Glu Asn Gly Glu Phe Tyr Leu Arg
1               5                   10


<210>  347
<211>  10
<212>  PRT
<213>  Homo sapiens

<400>  347

Ser Gly Asn Thr Phe His Pro Glu Val Arg
1               5                   10


<210>  348
<211>  16
<212>  PRT
<213>  Homo sapiens

<400>  348

Ser Gly Thr Ala Ser Val Val Cys Leu Leu Asn Asn Phe Tyr Pro Arg
1               5                   10                  15


<210>  349
<211>  12
<212>  PRT
<213>  Homo sapiens

<400>  349

Ser Leu Ala Pro Tyr Ala Gln Asp Thr Gln Glu Lys
1               5                   10


<210>  350
<211>  13
<212>  PRT
<213>  Homo sapiens

<400>  350

Ser Leu Asp Phe Thr Glu Leu Asp Val Ala Ala Glu Lys
1               5                   10


<210>  351
<211>  11
<212>  PRT
<213>  Homo sapiens

<400>  351

Ser Leu Met Leu His Tyr Glu Phe Leu Gln Arg
1               5                   10


<210>  352
<211>  10
<212>  PRT
<213>  Homo sapiens

<400>  352

Ser Leu Ser Lys Gln Leu Glu Asn Leu Arg
1               5                   10


<210>  353
<211>  12
<212>  PRT
<213>  Homo sapiens

<400>  353

Ser Met Glu Gln Asn Gly Pro Gly Leu Glu Tyr Arg
1               5                   10


<210>  354
<211>  16
<212>  PRT
<213>  Homo sapiens

<400>  354

Ser Asn Leu Asp Glu Asp Ile Ile Ala Glu Glu Asn Ile Val Ser Arg
1               5                   10                  15


<210>  355
<211>  13
<212>  PRT
<213>  Homo sapiens

<400>  355

Ser Pro Asp Phe Gln Leu Phe Ser Ser Ser His Gly Lys
1               5                   10


<210>  356
<211>  16
<212>  PRT
<213>  Homo sapiens

<400>  356

Ser Pro Glu Gln Gln Glu Thr Val Leu Asp Gly Asn Leu Ile Ile Arg
1               5                   10                  15

<210> 357
<211> 12
<212> PRT
<213> Homo sapiens

<400> 357

Ser Ser Asn Leu Leu Leu Leu Glu Glu His Leu Lys
1               5                   10

<210> 358
<211> 17
<212> PRT
<213> Homo sapiens

<400> 358

Ser Thr Gly Gly Leu Ser Val Pro Gly Pro Met Gly Pro Ser Gly Pro
1               5                   10                  15

Arg

<210> 359
<211> 14
<212> PRT
<213> Homo sapiens

<400> 359

Ser Val Thr Glu Gln Gly Ala Glu Leu Ser Asn Glu Glu Arg
1               5                   10

<210> 360
<211> 19
<212> PRT
<213> Homo sapiens

<400> 360

Ser Val Val Ala Pro Ala Thr Asp Gly Gly Leu Asn Leu Thr Ser Thr
1               5                   10                  15

Phe Leu Arg

<210> 361
<211> 12
<212> PRT

<213>  Homo sapiens

<400>  361

Ser Trp Phe Glu Pro Leu Val Glu Asp Met Gln Arg
1               5                   10


<210>  362
<211>  16
<212>  PRT
<213>  Homo sapiens

<400>  362

Ser Tyr Glu Leu Pro Asp Gly Gln Val Ile Thr Ile Gly Asn Glu Arg
1               5                   10                  15


<210>  363
<211>  16
<212>  PRT
<213>  Homo sapiens

<400>  363

Thr Ala Leu Ala Ser Gly Gly Val Leu Asp Ala Ser Gly Asp Tyr Arg
1               5                   10                  15


<210>  364
<211>  13
<212>  PRT
<213>  Homo sapiens

<400>  364

Thr Ala Thr Ser Glu Tyr Gln Thr Phe Phe Asn Pro Arg
1               5                   10


<210>  365
<211>  10
<212>  PRT
<213>  Homo sapiens

<400>  365

Thr Ala Val Thr Ala Asn Leu Asp Leu Arg
1               5                   10


<210>  366
<211>  8
<212>  PRT
<213>  Homo sapiens

<400>  366

Thr Asp Glu Gly Leu Ala Tyr Arg
1               5


<210>  367
<211>  9
<212>  PRT
<213>  Homo sapiens

<400>  367

Thr Glu Ala Tyr Leu Glu Ala Ile Arg
1               5


<210>  368
<211>  12
<212>  PRT
<213>  Homo sapiens

<400>  368

Thr Glu Gln Trp Ser Thr Leu Pro Pro Glu Thr Lys
1               5                   10


<210>  369
<211>  10
<212>  PRT
<213>  Homo sapiens

<400>  369

Thr Glu Val Gln Leu Glu His Leu Ser Arg
1               5                   10


<210>  370
<211>  8
<212>  PRT
<213>  Homo sapiens

<400>  370

Thr Gly Ala Gln Glu Leu Leu Arg
1               5


<210>  371
<211>  10
<212>  PRT
<213>  Homo sapiens

<400>  371

Thr Gly Leu Glu Ala Ile Ser Asn His Lys
1               5                   10

<210>  372
<211>  11
<212>  PRT
<213>  Homo sapiens

<400>  372

Thr His Leu Ala Pro Tyr Ser Asp Glu Leu Arg
1               5                   10

<210>  373
<211>  10
<212>  PRT
<213>  Homo sapiens

<400>  373

Thr His Leu Pro Glu Val Phe Leu Ser Lys
1               5                   10

<210>  374
<211>  10
<212>  PRT
<213>  Homo sapiens

<400>  374

Thr His Pro His Phe Val Ile Pro Tyr Arg
1               5                   10

<210>  375
<211>  12
<212>  PRT
<213>  Homo sapiens

<400>  375

Thr Ile Tyr Thr Pro Gly Ser Thr Val Leu Tyr Arg
1               5                   10

<210>  376
<211>  8
<212>  PRT
<213>  Homo sapiens

<400>  376

Thr Leu Leu Ser Asp Asp Trp Lys
1               5

<210>  377
<211>  10
<212>  PRT
<213>  Homo sapiens

<400>  377

Thr Leu Leu Ser Asn Leu Glu Glu Ala Lys
1               5                   10


<210>  378
<211>  12
<212>  PRT
<213>  Homo sapiens

<400>  378

Thr Leu Tyr Thr Pro Gly Ser Thr Val Leu Tyr Arg
1               5                   10


<210>  379
<211>  16
<212>  PRT
<213>  Homo sapiens

<400>  379

Thr Met Leu Leu Gln Pro Ala Gly Ser Leu Gly Ser Tyr Ser Tyr Arg
1               5                   10                  15


<210>  380
<211>  11
<212>  PRT
<213>  Homo sapiens

<400>  380

Thr Asn Phe Asp Asn Asp Ile Ala Leu Val Arg
1               5                   10


<210>  381
<211>  12
<212>  PRT
<213>  Homo sapiens

<400>  381

Thr Asn Ser Phe Glu Gly Pro Val Leu Asp His Arg
1               5                   10

<210> 382
<211> 17
<212> PRT
<213> Homo sapiens

<400> 382

Thr Gln Gly Phe Thr Glu Asp Ala Ile Val Phe Leu Pro Gln Thr Asp
1               5                   10                  15

Lys

<210> 383
<211> 14
<212> PRT
<213> Homo sapiens

<400> 383

Thr Gln Ser Ser Leu Val Pro Ala Leu Thr Asp Phe Val Arg
1               5                   10

<210> 384
<211> 11
<212> PRT
<213> Homo sapiens

<400> 384

Thr Gln Val Asn Thr Gln Ala Glu Gln Leu Arg
1               5                   10

<210> 385
<211> 12
<212> PRT
<213> Homo sapiens

<400> 385

Thr Ser Leu Glu Asp Phe Tyr Leu Asp Glu Glu Arg
1               5                   10

<210> 386
<211> 10
<212> PRT
<213> Homo sapiens

<400> 386

Thr Ser Thr Ala Asp Tyr Ala Met Phe Lys
1               5                   10

<210> 387
<211> 14
<212> PRT
<213> Homo sapiens

<400> 387

Thr Thr Asn Leu Gln Gly Leu Asn Leu Leu Phe Ser Ser Arg
1               5                   10


<210> 388
<211> 11
<212> PRT
<213> Homo sapiens

<400> 388

Thr Val Gly Ser Asp Thr Phe Tyr Ser Phe Lys
1               5                   10


<210> 389
<211> 15
<212> PRT
<213> Homo sapiens

<400> 389

Thr Val Met Val Asn Ile Glu Asn Pro Glu Gly Ile Pro Val Lys
1               5                   10                  15


<210> 390
<211> 15
<212> PRT
<213> Homo sapiens

<400> 390

Thr Val Met Val Asn Leu Glu Asn Pro Glu Gly Leu Pro Val Lys
1               5                   10                  15


<210> 391
<211> 7
<212> PRT
<213> Homo sapiens

<400> 391

Thr Tyr Phe Glu Gly Glu Arg
1               5

<210>  392
<211>  10
<212>  PRT
<213>  Homo sapiens

<400>  392

Thr Tyr Leu His Thr Tyr Glu Ser Glu Leu
1               5                   10


<210>  393
<211>  7
<212>  PRT
<213>  Homo sapiens

<400>  393

Thr Tyr Ser Tyr Leu Asn Lys
1               5


<210>  394
<211>  10
<212>  PRT
<213>  Homo sapiens

<400>  394

Val Ala Met His Leu Val Cys Pro Ser Arg
1               5                   10


<210>  395
<211>  18
<212>  PRT
<213>  Homo sapiens

<400>  395

Val Ala Pro Glu Glu His Pro Val Leu Leu Thr Glu Ala Pro Leu Asn
1               5                   10                  15


Pro Lys



<210>  396
<211>  13
<212>  PRT
<213>  Homo sapiens

<400>  396

Val Ala Gln Leu Glu Ala Gln Cys Gln Glu Pro Cys Lys
1               5                   10

<210>  397
<211>  9
<212>  PRT
<213>  Homo sapiens

<400>  397

Val Ala Gln Leu Pro Leu Ser Leu Lys
1               5


<210>  398
<211>  11
<212>  PRT
<213>  Homo sapiens

<400>  398

Val Cys Ser Gln Tyr Ala Ala Tyr Gly Glu Lys
1               5                   10


<210>  399
<211>  10
<212>  PRT
<213>  Homo sapiens

<400>  399

Val Glu Glu Val Lys Pro Leu Glu Gly Arg
1               5                   10


<210>  400
<211>  9
<212>  PRT
<213>  Homo sapiens

<400>  400

Val Glu Leu Glu Asp Trp Asn Gly Arg
1               5


<210>  401
<211>  16
<212>  PRT
<213>  Homo sapiens

<400>  401

Val Glu Leu Leu His Asn Pro Ala Phe Cys Ser Leu Ala Thr Thr Lys
1               5                   10                  15

<210>  402
<211>  12
<212>  PRT
<213>  Homo sapiens

<400>  402

Val Glu Gln Ala Thr Gln Ala Ile Pro Met Glu Arg
1               5                   10


<210>  403
<211>  14
<212>  PRT
<213>  Homo sapiens

<400>  403

Val Glu Gln Ala Val Glu Thr Glu Pro Glu Pro Glu Leu Arg
1               5                   10


<210>  404
<211>  9
<212>  PRT
<213>  Homo sapiens

<400>  404

Val Glu Val Leu Ala Gly Asp Leu Arg
1               5


<210>  405
<211>  8
<212>  PRT
<213>  Homo sapiens

<400>  405

Val Glu Tyr Gly Phe Gln Val Lys
1               5


<210>  406
<211>  13
<212>  PRT
<213>  Homo sapiens

<400>  406

Val Phe Leu Asp Cys Cys Asn Tyr Ile Thr Glu Leu Arg
1               5                   10


<210>  407
<211>  10

<212>  PRT
<213>  Homo sapiens

<400>  407

Val Phe Ser Leu Gln Trp Gly Glu Val Lys
1               5                   10


<210>  408
<211>  10
<212>  PRT
<213>  Homo sapiens

<400>  408

Val Gly Asp Thr Leu Asn Leu Asn Leu Arg
1               5                   10


<210>  409
<211>  11
<212>  PRT
<213>  Homo sapiens

<400>  409

Val Gly Phe Tyr Glu Ser Asp Val Met Gly Arg
1               5                   10


<210>  410
<211>  14
<212>  PRT
<213>  Homo sapiens

<400>  410

Val Gly Gly Val Gln Ser Leu Gly Gly Thr Gly Ala Leu Arg
1               5                   10


<210>  411
<211>  8
<212>  PRT
<213>  Homo sapiens

<400>  411

Val Gly Tyr Gly Pro Thr Phe Lys
1               5


<210>  412
<211>  9
<212>  PRT
<213>  Homo sapiens

<400> 412

Val Gly Tyr Val Ser Gly Trp Gly Arg
1               5


<210> 413
<211> 9
<212> PRT
<213> Homo sapiens

<400> 413

Val Ile Ala Leu Ile Asn Asp Gln Arg
1               5


<210> 414
<211> 11
<212> PRT
<213> Homo sapiens

<400> 414

Val Ile Val Val Trp Asn Asn Ile Gly Glu Lys
1               5                   10


<210> 415
<211> 9
<212> PRT
<213> Homo sapiens

<400> 415

Val Leu Ala Val Asn Glu Val Gly Arg
1               5


<210> 416
<211> 9
<212> PRT
<213> Homo sapiens

<400> 416

Val Leu Asp Ala Leu Gln Ala Leu Lys
1               5


<210> 417
<211> 9
<212> PRT
<213> Homo sapiens

<400> 417

Val Leu Phe Tyr Val Asp Ser Glu Lys
1               5

<210>  418
<211>  15
<212>  PRT
<213>  Homo sapiens

<400>  418

Val Leu Ser Leu Ala Gln Glu Gln Val Gly Gly Ser Pro Glu Lys
1               5                   10                  15

<210>  419
<211>  11
<212>  PRT
<213>  Homo sapiens

<400>  419

Val Leu Val Val Trp Asn Asn Leu Gly Glu Lys
1               5                   10

<210>  420
<211>  11
<212>  PRT
<213>  Homo sapiens

<400>  420

Val Asn Ser Asp Gly Gly Leu Val Ala Leu Arg
1               5                   10

<210>  421
<211>  14
<212>  PRT
<213>  Homo sapiens

<400>  421

Val Pro Glu Val Ser Thr Pro Thr Leu Val Glu Val Ser Arg
1               5                   10

<210>  422
<211>  14
<212>  PRT
<213>  Homo sapiens

<400>  422

Val Pro Gln Val Ser Thr Pro Thr Leu Val Glu Val Ser Arg

1               5                   10


<210>  423
<211>  14
<212>  PRT
<213>  Homo sapiens

<400>  423

Val Pro Thr Ala Asn Val Ser Val Val Asp Leu Thr Cys Arg
1               5                   10


<210>  424
<211>  7
<212>  PRT
<213>  Homo sapiens

<400>  424

Val Pro Thr Phe Asp Glu Arg
1               5


<210>  425
<211>  10
<212>  PRT
<213>  Homo sapiens

<400>  425

Val Gln Pro Tyr Leu Asp Asp Phe Gln Lys
1               5                   10


<210>  426
<211>  11
<212>  PRT
<213>  Homo sapiens

<400>  426

Val Gln Val Thr Ser Gln Glu Tyr Ser Ala Arg
1               5                   10


<210>  427
<211>  9
<212>  PRT
<213>  Homo sapiens

<400>  427

Val Ser Phe Glu Leu Phe Ala Asp Lys
1               5

<210> 428
<211> 14
<212> PRT
<213> Homo sapiens

<400> 428

Val Ser Ser Gln Asn Ile Gln Asp Phe Pro Ser Val Leu Arg
1               5                   10


<210> 429
<211> 8
<212> PRT
<213> Homo sapiens

<400> 429

Val Ser Val Phe Val Pro Pro Arg
1               5


<210> 430
<211> 10
<212> PRT
<213> Homo sapiens

<400> 430

Val Thr Cys Val Ala Glu Glu Leu Leu Lys
1               5                   10


<210> 431
<211> 11
<212> PRT
<213> Homo sapiens

<400> 431

Val Thr Glu Leu Trp Gln Glu Val Met Gln Arg
1               5                   10


<210> 432
<211> 12
<212> PRT
<213> Homo sapiens

<400> 432

Val Val Ala Gly Val Ala Asn Ala Leu Ala His Lys
1               5                   10


<210> 433

<211>  7
<212>  PRT
<213>  Homo sapiens

<400>  433

Val Trp Asn Tyr Phe Gln Arg
1               5


<210>  434
<211>  8
<212>  PRT
<213>  Homo sapiens

<400>  434

Val Trp Val Tyr Pro Pro Glu Lys
1               5


<210>  435
<211>  10
<212>  PRT
<213>  Homo sapiens

<400>  435

Val Tyr Leu Phe Asp Phe Pro Glu Gly Lys
1               5                   10


<210>  436
<211>  11
<212>  PRT
<213>  Homo sapiens

<400>  436

Trp Cys Ala Ile Gly His Glu Glu Thr Gln Lys
1               5                   10


<210>  437
<211>  11
<212>  PRT
<213>  Homo sapiens

<400>  437

Trp Cys Thr Ile Ser Asn Gln Glu Ala Asn Lys
1               5                   10


<210>  438
<211>  10
<212>  PRT

<213>  Homo sapiens

<400>  438

Trp Glu Ala Glu Pro Val Tyr Val Gln Arg
1               5                   10


<210>  439
<211>  10
<212>  PRT
<213>  Homo sapiens

<400>  439

Trp Leu Gln Gly Ser Gln Glu Leu Pro Arg
1               5                   10


<210>  440
<211>  9
<212>  PRT
<213>  Homo sapiens

<400>  440

Trp Gln Glu Glu Met Glu Leu Tyr Arg
1               5


<210>  441
<211>  7
<212>  PRT
<213>  Homo sapiens

<400>  441

Trp Val Glu Glu Leu Met Lys
1               5


<210>  442
<211>  9
<212>  PRT
<213>  Homo sapiens

<400>  442

Tyr Glu Ala Ala Val Pro Asp Pro Arg
1               5


<210>  443
<211>  13
<212>  PRT
<213>  Homo sapiens

<400>  443

Tyr Glu Ala Ser Ile Leu Thr His Asp Ser Ser Ile Arg
1               5                   10


<210>  444
<211>  10
<212>  PRT
<213>  Homo sapiens

<400>  444

Tyr Glu Glu Leu Gln Gln Thr Ala Gly Arg
1               5                   10


<210>  445
<211>  8
<212>  PRT
<213>  Homo sapiens

<400>  445

Tyr Glu Asn Glu Val Ala Leu Arg
1               5


<210>  446
<211>  9
<212>  PRT
<213>  Homo sapiens

<400>  446

Tyr Glu Val Ser Ser Pro Tyr Phe Lys
1               5


<210>  447
<211>  8
<212>  PRT
<213>  Homo sapiens

<400>  447

Tyr Gly Ala Ala Thr Phe Thr Arg
1               5


<210>  448
<211>  13
<212>  PRT
<213>  Homo sapiens

<400>  448

Tyr Gly Pro Phe Gly Pro Glu Met Thr Asn Pro Leu Arg
1               5                   10


<210>  449
<211>  12
<212>  PRT
<213>  Homo sapiens

<400>  449

Tyr Ile Cys Glu Asn Gln Asp Ser Ile Ser Ser Lys
1               5                   10


<210>  450
<211>  12
<212>  PRT
<213>  Homo sapiens

<400>  450

Tyr Ile Cys Glu Asn Gln Asp Thr Ile Ser Thr Lys
1               5                   10


<210>  451
<211>  9
<212>  PRT
<213>  Homo sapiens

<400>  451

Tyr Ile Glu Thr Asp Pro Ala Asn Arg
1               5


<210>  452
<211>  9
<212>  PRT
<213>  Homo sapiens

<400>  452

Tyr Ile Phe His Asn Phe Met Glu Arg
1               5


<210>  453
<211>  10
<212>  PRT
<213>  Homo sapiens

<400>  453

Tyr Leu Glu Leu Glu Ser Ser Gly His Arg
1               5                   10

<210> 454
<211> 7
<212> PRT
<213> Homo sapiens

<400> 454

Tyr Leu Glu Leu Phe Gln Arg
1               5


<210> 455
<211> 9
<212> PRT
<213> Homo sapiens

<400> 455

Tyr Leu Glu Thr Asp Pro Ala Asn Arg
1               5


<210> 456
<211> 12
<212> PRT
<213> Homo sapiens

<400> 456

Tyr Leu Gln Glu Ile Tyr Asn Ser Asn Asn Gln Lys
1               5                   10


<210> 457
<211> 7
<212> PRT
<213> Homo sapiens

<400> 457

Tyr Leu Tyr Glu Ile Ala Arg
1               5


<210> 458
<211> 15
<212> PRT
<213> Homo sapiens

<400> 458

Tyr Asn Ser Gln Asn Gln Ser Asn Asn Gln Phe Val Leu Tyr Arg
1               5                   10                  15

<210>  459
<211>  10
<212>  PRT
<213>  Homo sapiens

<400>  459

Tyr Ser Ser Leu Ala Glu Ala Ala Ser Lys
1               5                   10


<210>  460
<211>  7
<212>  PRT
<213>  Homo sapiens

<400>  460

Tyr Thr Phe Glu Leu Ser Arg
1               5


<210>  461
<211>  7
<212>  PRT
<213>  Homo sapiens

<400>  461

Tyr Thr Asn Trp Ile Gln Lys
1               5


<210>  462
<211>  9
<212>  PRT
<213>  Homo sapiens

<400>  462

Tyr Val Leu Pro Asn Phe Glu Val Lys
1               5


<210>  463
<211>  11
<212>  PRT
<213>  Homo sapiens

<400>  463

Tyr Val Met Leu Pro Val Ala Asp Gln Glu Lys
1               5                   10


<210>  464
<211>  15

<212>  PRT
<213>  Homo sapiens

<400>  464

Tyr Val Thr Ser Ala Pro Met Pro Glu Pro Gln Ala Pro Gly Arg
1               5                   10                  15


<210>  465
<211>  11
<212>  PRT
<213>  Homo sapiens

<400>  465

Tyr Trp Asn Asp Cys Glu Pro Pro Asp Ser Arg
1               5                   10


<210>  466
<211>  11
<212>  PRT
<213>  Homo sapiens

<400>  466

Tyr Tyr Cys Phe Gln Gly Asn Gln Phe Leu Arg
1               5                   10


<210>  467
<211>  7
<212>  PRT
<213>  Homo sapiens

<400>  467

Tyr Tyr Thr Val Phe Asp Arg
1               5


<210>  468
<211>  5
<212>  PRT
<213>  Homo sapiens

<400>  468

Pro Gly Leu Gly Met
1               5


<210>  469
<211>  15
<212>  DNA
<213>  Artificial sequence

<220>
<223>  DNA probe

<400>  469
cccggcctgg gcatg                                                        15


<210>  470
<211>  15
<212>  DNA
<213>  Artificial sequence

<220>
<223>  DNA probe

<220>
<221>  misc_feature
<222>  (3)..(3)
<223>  Where "n" represents "a", "t", "g" or "g"


<220>
<221>  misc_feature
<222>  (6)..(6)
<223>  Where "n" represents "a", "t", "g" or "g"


<220>
<221>  misc_feature
<222>  (9)..(9)
<223>  Where "n" represents "a", "t", "g" or "g"


<220>
<221>  misc_feature
<222>  (12)..(12)
<223>  Where "n" represents "a", "t", "g" or "g"


<400>  470
ccnggnytng gnatg                                                        15


<210>  471
<211>  5
<212>  PRT
<213>  Homo sapiens

<400>  471

Pro Gly Leu Gly Phe
1               5


<210>  472
<211>  15

<212>  DNA
<213>  Artificial sequence

<220>
<223>  DNA probe

<400>  472
cccggcctgg gcttc                                                        15


<210>  473
<211>  15
<212>  DNA
<213>  Artificial sequence

<220>
<223>  DNA probe

<220>
<221>  misc_feature
<222>  (3)..(3)
<223>  Where "n" represents "a", "t", "c" or "g"


<220>
<221>  misc_feature
<222>  (6)..(6)
<223>  Where "n" represents "a", "t", "c" or "g"


<220>
<221>  misc_feature
<222>  (9)..(9)
<223>  Where "n" represents "a", "t", "c" or "g"


<220>
<221>  misc_feature
<222>  (12)..(12)
<223>  Where "n" represents "a", "t", "c" or "g"


<400>  473
ccnggnytng gntty                                                        15


<210>  474
<211>  5
<212>  PRT
<213>  Homo sapiens

<400>  474

Pro Gly Ile Gly Met
1               5

<210>  475
<211>  15
<212>  DNA
<213>  Artificial sequence

<220>
<223>  DNA probe

<400>  475
cccggcatcg gcatg                                                        15


<210>  476
<211>  15
<212>  DNA
<213>  Artificial sequence

<220>
<223>  DNA probe

<220>
<221>  misc_feature
<222>  (3)..(3)
<223>  Where "n" represents "a", "t", "c", or "g"


<220>
<221>  misc_feature
<222>  (6)..(6)
<223>  Where "n" represents "a", "t", "c", or "g"


<220>
<221>  misc_feature
<222>  (12)..(12)
<223>  Where "n" represents "a", "t", "c", or "g"


<400>  476
ccnggnathg gnatg                                                        15


<210>  477
<211>  5
<212>  PRT
<213>  Homo sapiens

<400>  477

Pro Gly Ile Gly Phe
1               5


<210>  478
<211>  15
<212>  DNA
<213>  Artificial sequence

<220>
<223>  DNA probe

<400>  478
cccggcatcg gcttc                                                        15


<210>  479
<211>  15
<212>  DNA
<213>  Artificial sequence

<220>
<223>  DNA probe

<220>
<221>  misc_feature
<222>  (3)..(3)
<223>  Where "n" represents "a", "t", "c" or "g"


<220>
<221>  misc_feature
<222>  (6)..(6)
<223>  Where "n" represents "a", "t", "c" or "g"


<220>
<221>  misc_feature
<222>  (12)..(12)
<223>  Where "n" represents "a", "t", "c" or "g"


<400>  479
ccnggnathg gntty                                                        15


<210>  480
<211>  5
<212>  PRT
<213>  Homo sapiens

<400>  480

Gly Pro Leu Gly Met
1               5


<210>  481
<211>  15
<212>  DNA
<213>  Artificial sequence

<220>
<223>  DNA probe

<400> 481
ggccccctgg gcatg                                                        15


<210> 482
<211> 15
<212> DNA
<213> Artificial sequence

<220>
<223> DNA probe

<220>
<221> misc_feature
<222> (3)..(3)
<223> Where "n" represents "a", "t", "c", or "g"


<220>
<221> misc_feature
<222> (6)..(6)
<223> Where "n" represents "a", "t", "c", or "g"


<220>
<221> misc_feature
<222> (9)..(9)
<223> Where "n" represents "a", "t", "c", or "g"


<220>
<221> misc_feature
<222> (12)..(12)
<223> Where "n" represents "a", "t", "c", or "g"


<400> 482
ggnccnytng gnatg                                                        15


<210> 483
<211> 5
<212> PRT
<213> Homo sapiens

<400> 483

Gly Pro Leu Gly Phe
1               5


<210> 484
<211> 15
<212> DNA
<213> Artificial sequence

<220>

<223>  DNA probe

<400>  484
ggccccctgg gcttc                                                        15


<210>  485
<211>  15
<212>  DNA
<213>  Artificial sequence

<220>
<223>  DNA probe

<220>
<221>  misc_feature
<222>  (3)..(3)
<223>  Where "n" represents "a", "t", "c" or "g"


<220>
<221>  misc_feature
<222>  (6)..(6)
<223>  Where "n" represents "a", "t", "c" or "g"


<220>
<221>  misc_feature
<222>  (9)..(9)
<223>  Where "n" represents "a", "t", "c" or "g"


<220>
<221>  misc_feature
<222>  (12)..(12)
<223>  Where "n" represents "a", "t", "c" or "g"


<400>  485
ggnccnytng gntty                                                        15


<210>  486
<211>  5
<212>  PRT
<213>  Homo sapiens

<400>  486

Gly Pro Ile Gly Met
1               5


<210>  487
<211>  15
<212>  DNA
<213>  Artificial sequence

<220>
<223>  DNA probe

<400>  487
ggccccatcg gcatg                                                        15


<210>  488
<211>  15
<212>  DNA
<213>  Artificial sequence

<220>
<223>  DNA probe

<220>
<221>  misc_feature
<222>  (3)..(3)
<223>  Where "n" represents "a", "t", "c" or "t"


<220>
<221>  misc_feature
<222>  (6)..(6)
<223>  Where "n" represents "a", "t", "c" or "t"


<220>
<221>  misc_feature
<222>  (12)..(12)
<223>  Where "n" represents "a", "t", "c" or "t"


<400>  488
ggnccnathg gnatg                                                        15


<210>  489
<211>  5
<212>  PRT
<213>  Homo sapiens

<400>  489

Gly Pro Ile Gly Phe
1               5


<210>  490
<211>  15
<212>  DNA
<213>  Artificial sequence

<220>
<223>  DNA probe

<400> 490
ggccccatcg gcttc 15

<210> 491
<211> 15
<212> DNA
<213> Artificial sequence

<220>
<223> DNA probe

<220>
<221> misc_feature
<222> (3)..(3)
<223> Where "n" represents "a", "t", "c" or "g"

<220>
<221> misc_feature
<222> (6)..(6)
<223> Where "n" represents "a", "t", "c" or "g"

<220>
<221> misc_feature
<222> (12)..(12)
<223> Where "n" represents "a", "t", "c" or "g"

<400> 491
ggnccnathg gntty 15

## Claims

1. A method for screening or diagnosis of Alzheimer's diseasein a subject, for determining the stage or severity of Alzheimer's diseasein a subject, for identifying a subject at risk of developing Alzheimer's disease, or for monitoring the effect of therapy administered to a subject having Alzheimer's disease, said method comprising:
(a) analysing a test sample of body fluid from the subject by two-dimensional electrophoresis to generate a two-dimensional array of features, said array comprising one or more of the following Alzheimer's disease-Associated Features (AFs): AF-200, AF-201, AF-202, AF-203, AF-204, AF-205, AF-206, AF-207, AF-208, AF-209, AF-210, AF-211, AF-212, AF-213, AF-214, AF-215, AF-216, AF-217, AF-218, AF-219, AF-220, AF-221, AF-222, AF-223, AF-224, AF-225, AF-226, AF-227, AF-228, AF-229, AF-230, AF-231, AF-232, AF-233, AF-234, AF-235, AF-236, AF-237, AF-238, AF-239, AF-240, AF-241, AF-242, AF-243, AF-244, AF-245, AF-246, AF-247, AF-248, AF-249, AF-250, AF-251, AF-252, AF-253, AF-254, AF-255, AF-256, AF-257, AF-258, AF-259, AF-260, AF-261, AF-262, AF-263, AF-264, AF-265, AF-266, AF-267, AF-268, AF-269, AF-270, AF-271, AF-272, AF-273, AF-274, AF-275, AF-276, AF-277, AF-278, AF-279, AF-280, AF-281, AF-282, AF-283, AF-284, AF-285, AF-286, AF-287, AF-288, AF-289, AF-290, AF-291, AF-292, AF-293, AF-294, AF-295, AF-296, AF-297, AF-298, AF-299, AF-300, AF-301, AF-302, AF-303, AF-304, AF-305, AF-306, AF-307, AF-308, AF-309, AF-310, AF-311, AF-312, AF-313, AF-314, AF-315, AF-316, AF-317, AF-318, AF-319, AF-320, AF-321, AF-322, AF-323, AF-324, AF-325, AF-326, AF-327, AF-328, AF-329, AF-330, AF-331, AF-332, AF-333, AF-334, AF-335, AF-336, AF-337, AF-338, AF-339, AF-340; optionally in combination with one or more of the following AFs:, AF-1, AF-2, AF-3, AF-4, AF-5, AF-6, AF-7, AF-8, AF-9, AF-10, AF-13, AF-14, AF-15, AF-16, AF-17, AF-18, AF-19, AF-20, AF-21, AF-22, AF-23, AF-24, AF-25, AF-26, AF-27, AF-28, AF-29, AF-30, AF-31, AF-32, AF-33, AF-34, AF-35, AF-36, AF-37, AF-38, AF-39, AF-40, AF-41, AF-42, AF-43, AF-44, AF-45, AF-46, AF-47, AF-48, AF-49, AF-50, AF-51, AF-76, AF-149, AF-150, AF-152, AF-154, AF-155, AF-156, AF-159, AF-160, AF-162, AF-163, AF-164, AF-169, AF-170, AF-172, AF-173, AF-174, AF-175, AF-176, AF-177, AF-178, AF-181, AF-183, AF-184, AF-186, AF-187, AF-188, AF-189, AF-190, AF-191, AF-78, AF-79, AF-80, AF-81, AF-82, AF-83, AF-84, AF-85, AF-86, AF-87, AF-88, AF-89, AF-90, AF-91, AF-92, AF-93, AF-94, AF-95, AF-96, AF-98, AF-99, AF-100, AF-101, AF-102, AF-103, AF-104, AF-105, AF-107, AF-108, AF-110, AF-111, AF-112, AF-114, AF-115, AF-116, AF-117, AF-118, AF-119, AF-52, AF-53, AF-54, AF-55, AF-56, AF-57, AF-58, AF-59, AF-60, AF-61, AF-62, AF-63, AF-64, AF-65, AF-66, AF-67, AF-68, AF-69, AF-70, AF-71, AF-72, AF-73, AF-74, AF-75, AF-77, AF-151, AF-153, AF-157, AF-161, AF-165, AF-166, AF-167, AF-168, AF-171, AF-179, AF-180, AF-182, AF-185, AF-192, AF-121, AF-122, AF-123, AF-124, AF-125, AF-126, AF-127, AF-128, AF-129, AF-130, AF-131, AF-132, AF-133, AF-134, AF-137, AF-139, AF-140, AF-141, AF-142, AF-143, AF-144, AF-145, AF-146, AF-147, AF-148; and
(b) comparing the abundance of the one or more AFs in the test sample with the abundance of the one or more AFs in a body fluid sample from one or more subjects free from Alzheimer's disease, or with a previously determined reference range for that feature in subjects free from Alzheimer's disease, or with the abundance at least one Expression Reference Feature (ERF) in the test sample.

2. A method for screening or diagnosis of Alzheimer's diseasein a subject, for determining the stage or severity of Alzheimer's disease in a subject, for identifying a subject at risk of developing Alzheimer's disease, or for monitoring the effect of therapy administered to a subject having Alzheimer's disease, said method comprising quantitatively detecting, in a test sample of body fluid from the subject, one or more of the following Alzheimer's disease-Associated Protein Isoforms (APIs): API-375, API-300, API-301, API-302, API-303, API-304, API-305, API-306, API-307, API-308, API-309, API-310, API-376, API-377, API-311, API-312, API-313, API-378, API-314, API-379, API-315, API-316, API-380, API-317, API-369, API-318, API-319, API-320, API-321, API-322, API-323, API-381, API-382, API-383, API-384, API-324, API-325, API-326, API-327, API-328, API-329, API-330, API-331, API-385, API-332, API-333, API-334, API-335, API-336, API-337, API-338, API-339, API-340, API-341, API-342, API-386, API-387, API-343, API-344, API-388, API-345, API-346, API-389, API-390, API-347, API-391, API-348, API-349, API-350, API-351, API-352, API 392, API-393, API-394, API-395, API-353, API-354, API-396, API-397, API-355, API-398, API-399, API-400,-API-356, API-357, API-401, API-358, API-402, API-403, API-359, API-360, API-404, API-405, API-406, API-407, API-408, API-409, API-361, API-362, API-363, API-410, API-411, API-364, API-412, API-413, API-365, API-366, API-414, API-415, API-367, API-370, API-416, API-417, API-418, API-368, API-419; optionally combined with one or more of the following APIs: API-47, API-242, API-1, API-48, API-49, API-2, API-194, API-3, API-50, API-51, API-4, API-52, API-243, API-53, API-244, API-54, API-5, API-55, API-245, API-6, API-56, API-57, API-7, API-8, API-9, API-10, API-14, API-15, API-58, API-16, API-59, API-196, API-17, API-60, API-18, API-61, API-62, API-19, API-63, API-64, API-65, API-20, API-22, API-66, API-67, API-68, API-69, API-70, API-23, API-24, API-197, API-198, API-25, API-71, API-26, API-27, API-72, API-73, API-199, API-200, API-28, API-30, API-86, API-201, API-88, API-202, API-89, API-90, API-91, API-92, API-93, API-95, API-97, API-98, API-99, API-101, API-102, API-103, API-104, API-107, API-210, API-108, API- 111 , API-112, API-113, API- 114, API-214, API-144, API-146, API-147, API-148, API-150, API-151, API-152, API-215, API-153, API-158, API-159, API-160, API-165, API-166, API-167, API-173, API-174, API-175, API-176, API-179, API-180, API-181, API-182, API-183, API-184, API-185, API-217, API-219, API-187, API-189, API-190, API-238, API-239, API-240, API-74, API-33, API-221, API-34, API-75, API-246, API-35, API-76, API-222, API-77, API-36, API-37, API-78, API-38, API-79, API-80, API-81, API-223, API-82, API-83, API-39, API-84, API-85, API-40, API-247, API-41, API-224, API-42, API-43, API-44, API-45, API-248, API-46, API-225, API-116, API-118, API-119, API-120, API-121, API-122, API-123, API-124, API-125, API-126, API-127, API-128, API-130, API-131, API-132, API-134, API-135, API-232, API-233, API-234, API-136, API-137, API-138, API-139, API-140, API-141, API-142, API-143, API-145, API-149, API-155, API-161, API-162, API-163, API-168, API-169, API-170, APT-171, API-237, API-172, API-177, API-178, API-186, API-220, API-188, API-191, API-192

3. The method according to claim 1 or 2 where the body fluid is Cerebrospinal Fluid.

4. The method according to claim 2 or 3 where the abundance of the one or more APIs in the test sample is compared with the abundance of the one ore more APIs in a sample from one or more subjects free from Alzheimer's disease, or with a previously determined reference range for that feature in subjects free from Alzheimer's disease, or with the abundance at least one Expression Reference Feature (ERF) in the test sample.

5. The method according to any one of claims 2 to 4, wherein the step of quantitatively detecting comprises testing at least one aliquot of the first sample, said step of testing comprising:
(a) contacting the aliquot with an antibody that is immunospecific for a API;
(b) quantitatively measuring the binding of the antibody and the API; and
(c) comparing the results of step (b) with a predetermined reference range.

6. The method according to claim 5, wherein the step of quantitatively detecting comprises testing a plurality of aliquots with a plurality of antibodies cognate for a plurality of preselected APIs.

7. A pharmaceutical composition comprising an Alzheimer's disease-Associated Protein Isoform (API) as defined in claim 2, or a nucleic acid encoding an API, and a pharmaceutically acceptable carrier.

8. The pharmaceutical composition according to claim 7, wherein the Alzheimer's disease-Associated Protein Isoform (API) is in recombinant form.

9. An antibody capable of immunospecific binding to a Alzheimer's disease-Associated Protein Isoform (API) as defined in claim 2.

10. The method according to claim 5 or 6 or an antibody according to claim 9, wherein the antibody is a monoclonal antibody.

11. The method according to claim 5 or 6 or an antibody according to claim 9 or 10, wherein the antibody is a chimeric antibody.

12. The method according to claim 5 or 6 or an antibody according to claim 9 or 10, wherein the antibody is a bispecific antibody.

13. The method according to claim 5 or 6 or an antibody according to claim 9 or 10, wherein the antibody is a humanised antibody.

14. The method according to claim 5 or 6 or an antibody according to any one of claims 9 to 13, wherein the antibody binds to the API with greater affinity than to another isoform of the API.

15. A kit comprising one or more antibodies as claimed in any one of claims 9 to 14 and/or one or more APIs as defined in claim 2, other reagents and instructions for use.

16. The kit of claim 15 for use in the screening or diagnosis of Alzheimer's disease in a subject, for determining the stage or severity of Alzheimer's disease in a subject, for identifying a subject at risk of developing Alzheimer's disease, or for monitoring the effect of therapy administered to a subject having Alzheimer's disease.

17. The kit according to claim 15 or 16 comprising a plurality of antibodies as claimed in any one of claims 9 to 14 and/or a plurality of APIs as defined in claim 2.

18. A pharmaceutical composition comprising a therapeutically effective amount of an antibody, or a fragment or derivative of an antibody according to any one of claims 9 to 14 and a pharmaceutically acceptable carrier.

19. The use of an antibody as claimed in any one of claims 9 to 14 in the manufacture of a medicamant for treating or preventing Alzheimer's disease.

20. The use of one or more of the Alzheimer's disease-Associated Protein Isoforms (APIs) as defined in claim 2 and/or a nucleic acid encoding said APIs in the manufacture of a medicamant for treating or preventing Alzheimer's disease.

21. The use of a nucleic acid that inhibits the function of one or more of the Alzheimer's disease-Associated Protein Isoforms (APIs) as defined in claim 2 in the manufacture of a medicamant for treating or preventing Alzheimer's disease.

22. The use according to claim 21, wherein the nucleic acid is a API antisense nucleic acid or ribozyme.

23. A method of screening for agents that interact with one or more Alzheimer's disease-Associated Protein Isoforms (APIs) as defined in claim 2, fragments of APIs (API fragment), polypeptides related to APIs (API-related polypeptide), or API-fusion proteins said method comprising:
(a) contacting an API, a fragment of an API, an API-related polypeptide, or an API-fusion protein with a candidate agent; and
(b) determining whether or not the candidate agent interacts with the API, the fragment of an API, the API-related polypeptide, or the API-fusion protein.

24. The method according to clain 23, wherein the determination of interaction between the candidate agent and the API, API fragment, API-related polypeptide or API-fusion protein comprises quantitatively detecting binding of the candidate agent and the API, API fragment, API-related polypeptide or API-fusion protein.

25. A method of screening for or identifying agents that modulate the expression or activity of one or more Alzheimer's disease-Associated Protein Isoforms (APIs) as defined in claim 2, fragments of API (API fragment), polypeptides related to APIs (API-related polypeptide) or API-fusion proteins comprising:
(a) contacting a first population of cells expressing the API, API fragment, API-related polypeptide, or API-fusion protein with a candidate agent;
(b) contacting a second population of cells expressing said API, API fragment, API-related polypeptide, or API-fusion protein with a control agent; and
(c) comparing the level of said API, API fragment, API-related polypeptide, or API-fusion protein or mRNA encoding said API, API fragment, API-related polypeptide, or API-fusion protein in the first and second populations of cells, or comparing the level of induction of a downstream effector in the first and second populations of cells.

26. A method of screening for or identifying agents that modulate the expression or activity of one or more Alzheimer's disease Associated Protein Isoforms (APIs) as defined in claim 2, fragments of APIs (API fragment), polypeptides related to APIs (API-related polypeptide) or API-fusion proteins said method comprising:
(a) administering a candidate agent to a first non-human mammal or group of non-human mammals;
(b) administering a control agent to a second non-human mammal or group of non-human mammals; and
(c) comparing the level of expression of the API, API fragment, API-related polypeptide or API-fusion protein, or mRNA encoding said API, API fragment, API-related polypeptide or API-fusion protein in the first and second groups, or comparing the level of induction of a downstream effector in the first and second groups.

27. The method as claimed in claim 26, wherein the non-human mammals are animal models for Alzheimer's disease.

28. The method as claimed in claim 25, 26 or 27, wherein the levels of said API, API fragment, API-related polypeptide, or API-fusion protein, or mRNA encoding said API, API fragment, API-related polypeptide, or API-fusion protein, or of said downstream effector in the first and second groups are further compared to the level of said API, API fragment, API-related polypeptide or API fusion protein, or mRNA encoding said API, API fragment, API-related polypeptide or API fusion protein in normal control mammals.

29. A method of screening for or identifying agents that modulate the activity of one or more of the Alzheimer's disease-Associated Proteins Isoforms (APIs) as defined in claim 2, fragments of APIs (API fragment), polypeptides related to APIs (API-related polypeptide) or API-fusion proteins said method comprising:
(a) in a first aliquot, contacting a candidate agent with the API, API fragment, API-related polypeptide or API fusion protein, and
(b) determining and comparing the activity of the API, API fragment, API-related polypeptide or API fusion protein in the first aliquot after addition of the candidate agent with the activity of the API, API fragment, API-related polypeptide or API fusion protein in a control aliquot, or with a previously determined reference range.

30. The method according to any one of claims 23 to 29, wherein the API, API fragment, API-related polypeptide, or API fusion protein is a recombinant protein.

31. The method according to claim 23, 24, 30 or 31, wherein the API, API fragment, API-related polypeptide or API fusion protein is immobilized on a solid phase.

32. A method for screening or diagnosis of Alzheimer's disease in a subject or for monitoring the effect of an anti-Alzheimer' disease drug or therapy administered to a subject, comprising:
(a) contacting at least one oligonucleotide probe comprising 10 or more consecutive nucleotides complementary to a nucleotide sequence encoding an API as defined in claim 2 with RNA obtained from a biological sample from the subject or with cDNA copied from the RNA wherein said contacting occurs under conditions that permit hybridization of the probe to the nucleotide sequence if present;
(b) detecting hybridization, if any, between the probe and the nucleotide sequence; and
(c) comparing the hybridization, if any, detected in step (b) with the hybridization detected in a control sample, or with a previously determined reference range.

33. The method as claimed in claim 32, wherein step (a) includes the step of hybridizing the nucleotide sequence to a DNA array, wherein one or more members of the array are the probes complementary to a plurality of nucleotide sequences encoding distinct APIs.

34. The use of an agent identified by the method of any one of claims 23 to 31 in the manufacture of a medicament for modulating the activity of one or more of the Alzheimer's disease-Associated Protein Isoforms as defined in claim 2.

35. The use of an agent that modulates the activity of one or more of the Alzheimer' disease-Associated Protein Isoforms as defined in claim 2 in the manufacture of a medicament for treating or preventing Alzheimer's disease.

36. A method for identifying targets for therapeutic modulation of Alzheimer's disease wherein the activity of one or more of the Alzheimer's disease-Associated Protein Isoforms as defined in claim 2 is utilized as a measure to determine whether a candidate target is effective for modulation of Alzheimer's disease.
